# EUROPÄISCHE PATENTSCHRIFT

(11) **EP 3 004 094 B1**
(45) Veröffentlichungstag und Bekanntmachung des Hinweises auf die Patenterteilung: **01.03.2017**
(21) Anmeldenummer: 14727828.7
(22) Anmeldetag: 04.06.2014
(51) Int. Cl.: C07D 471/04, A61K 31/437, A61P 7/02, A61P 9/04, A61P 9/12, A61P 9/10

(54) **3-ARYL-SUBSTITUIERTE IMIDAZO[1,2-A]PYRIDINE UND IHRE VERWENDUNG**
3-ARYL-SUBSTITUTED IMIDAZO[1,2-A]PYRIDINES AND THE USE THEREOF
IMIDAZO[1,2-A]PYRIDINES À SUBSTITUTION 3-ARYLE ET LEUR UTILISATION

(30) Priorität: 04.06.2013 EP 13170371
(43) Veröffentlichungstag der Anmeldung: 13.04.2016
(73) Patentinhaber: Bayer Pharma Aktiengesellschaft, 13353 Berlin (DE)
(72) Erfinder: VAKALOPOULOS, Alexandros, 40721 Hilden (DE); FOLLMANN, Markus, 50859 Köln (DE); HARTUNG, Ingo, 13158 Berlin (DE); BUCHGRABER, Philipp, 10435 Berlin (DE); GROMOV, Alexey, 40699 Erkrath (DE); LINDNER, Niels, 42115 Wuppertal (DE); WUNDER, Frank, 42117 Wuppertal (DE); STASCH, Johannes-Peter, 81679 München (DE); REDLICH, Gorden, 44799 Bochum (DE); LI, Volkhart Min-Jian, 42553 Velbert (DE)
(74) Vertreter: BIP Patents
(86) Internationale Anmeldenummer: PCT/EP2014/061523
(87) Internationale Veröffentlichungsnummer: WO 2014/195333

(56) Entgegenhaltungen:
- WO-A1-2010/079120
- WO-A1-2012/165399

## Beschreibung

Die vorliegende Anmeldung betrifft neue 3-Aryl-substituierte Imidazo[1,2-a]pyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in EP 0 266 890-A1, WO 89/03833-A1, JP 01258674-A [vgl. Chem. Abstr. 112:178986], WO 96/34866-A1, EP 1 277 754-A1, WO 2001/096335, WO 2006/015737-A1, WO 2006/135667, WO 2008/008539-A2, WO 2008/082490-A2, WO 2008/134553-A1, WO 2010 /030538-A2, WO 2011/113606-A1 und WO 2012/165399-A1 sind verschiedene Imidazo[1,2-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Gegenstand der vorliegenden Erfindung sind Verbindungen der allgemeinen Formel (I) 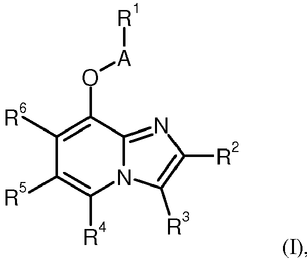 in welcher
- A: für CH₂, CD₂ oder CH(CH₃) steht,
- R¹: für (C₃-C₇)-Cycloalkyl, Phenyl oder Pyridyl steht,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann, wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy und Difluormethoxy substituiert ist,
und
wobei Pyridyl mit 1 oder 2 Substituenten unabhängig voneinander ausgewählt aus der Gruppe Halogen, Cyano und (C₁-C₄)-Alkyl substituiert ist,
- R²: für (C₁-C₄)-Alkyl, Cyclopropyl, Monofluormethyl, Difluormethyl oder Trifluormethyl steht,
- R³: für Phenyl oder 5- bis 10-gliedriges Heteroaryl steht,
wobei Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkyl-carbonyl, (C₁-C₄)-Alkoxycarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy und (C₃-C₇)-Cycloalkyl substituiert sein kann,
worin (C₁-C₆)-Alkyl mit 1 oder 2 Substituenten ausgewählt aus der Gruppe Fluor, Trifluormethoxy, (C₁-C₄)-Alkylcarbonyl, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
      und
   worin (C₃-C₆)-Cycloalkyl mit Amino oder Hydroxy substituiert sein kann,
   und worin

R⁷ und R⁸ jeweils unabhängig voneinander für Wasserstoff, (C₁-C₄)-Alkyl oder (C₃-C₇)-Cycloalkyl stehen, wobei 5- bis 10-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl und (C₃-C₇)-Cycloalkyl substituiert sein kann,
worin (C₁-C₆)-Alkyl mit 1 bis 3 Substituenten ausgewählt aus der Gruppe Halogen, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxycarbonyl,-(C=O)NR⁷R⁸, -O(C=O)NR⁷R⁸, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
   worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
   worin Piperidinyl mit 1 bis 4 Substituenten Fluor substituiert sein kann,
   worin Phenyl mit 1 bis 3 Substituenten ausgewählt aus der Gruppe Halogen, (C₁-C₄)-Alkyl und (C₁-C₄)-Alkoxy substituiert sein kann,
   worin Azetidin mit Hydroxy substituiert sein kann,
      und
   worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
worin (C₃-C₇)-Cycloalkyl mit 1 oder 2 Substituenten unabhängig ausgewählt aus der Gruppe Halogen, (C₁-C₆)-Alkyl, (C₁-C₄)-Alkoxycarbonyl und Hydroxy-carbonyl substituiert sein kann,
worin Amino mit (C₁-C₄)-Alkyl substituiert sein kann,
worin Phenyl, Pyridyl, Pyrimidyl und 1,3-Thiazol-5-yl mit 1 oder 2 Substituenten ausgewählt aus der Gruppe Methyl, Ethyl und Fluor substituiert sein können,
   worin

R⁷ und R⁸ jeweils unabhängig voneinander für Wasserstoff, (C₁-C₄)-Alkyl oder (C₃-C₇)-Cycloalkyl stehen,
und
mit der Maßgabe, dass wenn 5- bis 10-gliedriges Heteroaryl für Pyridyl steht, Pyridyl nicht mit Amino substituiert sein kann,
- R⁴: für Wasserstoff steht,
- R⁵: für Wasserstoff, Halogen, Cyano, (C₁-C₄)-Alkyl, (C₁-C₄)-Alkoxy, (C₃-C₅)-Cycloalkyl, Difluormethoxy, Difluormethyl, Trifluormethyl, 4- bis 7-gliedriges Heterocyclyl oder 5-oder 6-gliedriges Heteroaryl steht,
- R⁶: für Wasserstoff oder Halogen steht,
sowie ihre N-Oxide, Salze, Solvate, Salze der N-Oxide und Solvate der N-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.
Cycloalkyl bzw. Carbocyclus steht in Rahmen der Erfindung für einen monocyclischen, gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl und Cycloheptyl.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 4 Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Alkylsulfonyl steht in Rahmen der Erfindung für einen linearen oder verzweigten Alkylrest mit 1 bis 4 Kohlenstoffatomen, der über eine Sulfonylgruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Ein 4- bis 7-gliedrige Heterocyclus steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.
Heteroaryl steht im Rahmen der Erfindung für einen monocyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, 1H-Pyrazol-4-yl, 1H-Pyrazol-5-yl, Imidazolyl, 1,3-Thiazol-5-yl, 1,3-Thiazol-2-yl, 1,3-Oxazol-5-yl, 1,3-Oxazol-2-yl, Isoxazolyl, Isothiazolyl, Triazolyl, 1,3,4-Oxadiazol-2-yl, 1,2,4-Oxadiazol-3-yl, 1,2,4-Oxadiazol-5-yl, 1,3,4-Thiadiazol-2-yl, 1,2,4-Thiadiazol-3-yl, 1,2,4-Thiadiazol-5-yl, Pyridyl, Pyrimidinyl, Pyridazinyl, Pyrazinyl und Triazinyl.
Halogen schließt im Rahmen der Erfindung Fluor, Chlor, Brom und Iod ein. Bevorzugt sind Chlor oder Fluor.

In der Formel der Gruppe, für die R³ bzw. R¹ stehen kann, steht der Endpunkt der Linie, an dem die Zeichen *, # oder ## stehen, nicht für ein Kohlenstoffatom beziehungsweise eine CH₂-Gruppe, sondern ist Bestandteil der Bindung zu dem jeweils bezeichneten Atom, an das R³ bzw. R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Bevorzugt sind im Rahmen der vorliegenden Erfindung Verbindungen der Formel (I), in welcher
- A: für CH₂ oder CD₂ steht,
- R¹: für Cyclohexyl, Phenyl oder Pyridyl steht,
wobei Phenyl mit 1 bis 4 Substituenten unabhängig voneinander ausgewählt aus der Gruppe Fluor, Brom, Chlor, Cyano und Methyl substituiert ist,
und
wobei Pyridyl mit 1 oder 2 Substituenten unabhängig voneinander ausgewählt aus der Gruppe Fluor, Cyano und Methyl substituiert ist,
- R²: für (C₁-C₄)-Alkyl, Cyclopropyl oder Trifluormethyl steht,
- R³: für Phenyl steht,
wobei Phenyl mit 1 bis 3 Substituenten ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxy-carbonyl, Hydroxycarbonyl, -(C=O)NR⁷R⁸, (C₁-C₄)-Alkylsulfonyl, (C₁-C₄)-Alkylthio, (C₁-C₄)-Alkoxy, Trifluormethoxy, Difluormethoxy, Phenoxy, Hydroxy und (C₃-C₇)-Cycloalkyl substituiert sein kann,
worin (C₁-C₆)-Alkyl mit 1 oder 2 Substituenten ausgewählt aus der Gruppe Trifluormethoxy, (C₁-C₄)-Alkylcarbonyl, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus (C₁-C₆)-Alkyl, (C₁-C₄)-Alkylcarbonyl, (C₁-C₄)-Alkylsulfonyl und Methoxy-(C₁-C₄)-alkyl substituiert sein kann,
worin (C₃-C₆)-Cycloalkyl mit Amino oder Hydroxy substituiert sein kann,
und worin

R⁷ und R⁸ jeweils unabhängig voneinander für Wasserstoff, (C₁-C₄)-Alkyl oder (C₃-C₅)-Cycloalkyl stehen oder
für 5-gliedriges Heteroaryl steht,
wobei 5-gliedriges Heteroaryl mit 1 oder 2 Substituenten unabhängig voneinander ausgewählt aus der Gruppe Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl oder (C₃-C₆)-Cycloalkyl substituiert ist,
worin (C₁-C₆)-Alkyl mit 1 bis 3 Substituenten ausgewählt aus der Gruppe Fluor, Cyano, Trifluormethyl, Difluormethyl, (C₁-C₄)-Alkylcarbonyl, (C₁-C₄)-Alkoxy-carbonyl, Hydroxycarbonyl, -(C=O)NR⁷R⁸, -O(C=O)NR⁷R⁸, (C₁-C₄)-Alkylsulfonyl, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, Hydroxy, Amino, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
   worin 5-gliedriges Heteroaryl mit 1 oder 2 Substituenten Methyl oder Ethyl substituiert sein kann,
   worin Piperidinyl mit 1 bis 4 Substituenten Fluor substituiert sein kann,
   worin Phenyl mit 1 oder 2 Substituenten ausgewählt aus der Gruppe Fluor, Chlor und Methyl substituiert sein kann,
   worin Azetidin mit Hydroxy substituiert sein kann,
      worin

   R⁷ und R⁸ jeweils unabhängig voneinander für Wasserstoff, (C₁-C₄)-Alkyl oder (C₃-C₇)-Cycloalkyl stehen, und
   worin Piperazinyl mit 1 oder 2 Substituenten Methyl oder Ethyl substituiert sein kann,
wobei Phenyl, Pyridyl, Pyrimidyl und 1,3-Thiazol-5-yl mit 1 oder 2 Substituenten ausgewählt aus der Gruppe Methyl, Ethyl und Fluor substituiert sein können,
wobei (C₃-C₆)-Cycloalkyl mit 1 oder 2 Substituenten unabhängig ausgewählt aus der Gruppe Fluor, Methyl, Ethyl, (C₁-C₄)-Alkoxycarbonyl und Hydroxycarbonyl substituiert sein kann,
   und worin

R⁷ und R⁸ jeweils unabhängig voneinander für Wasserstoff, (C₁-C₄)-Alkyl o der (C₃-C₇)-Cycloalkyl stehen,
- R⁴: für Wasserstoff steht,
- R⁵: für Wasserstoff, Halogen, Cyano, (C₁-C₄)-Alkyl, Methoxy, Ethoxy, (C₃-C₅)-Cycloalkyl oder Difluormethyl steht,
- R⁶: für Wasserstoff oder Fluor steht,
sowie ihre N-Oxide, Salze, Solvate, Salze der N-Oxide und Solvate der N-Oxide und Salze.

Bevorzugt sind im Rahmen der vorliegenden Erfindung Verbindungen der Formel (I), in welcher
- A: für CH₂ steht,
- R¹: für Cyclohexyl oder Phenyl steht,
wobei Phenyl mit 1 bis 3 Substituenten Fluor substituiert ist,
oder
für eine Pyridyl-Gruppe der Formel 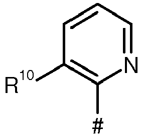 steht, wobei
# für die Anknüpfstelle an A steht,
und
R¹⁰ für Fluor steht,
- R²: für Methyl oder Ethyl steht,
- R³: für Phenyl steht,
wobei Phenyl mit 1 oder 2 Substituenten ausgewählt aus der Gruppe Fluor, Brom, Chlor, Cyano, Trifluormethyl, Difluormethyl, Methyl, Ethyl, -(C=O)NR⁷R⁸, Amino, Hydroxycarbonyl, Methylsulfonyl, Ethylsulfonyl, Methoxy, Ethoxy, Trifluormethoxy, Difluormethoxy und Cyclobutyl substituiert sein kann,
worin Methyl und Ethyl mit 1 oder 2 Substituenten ausgewählt aus der Gruppe Trifluormethoxy, -(C=O)NR⁷R⁸, Methoxy, Ethoxy, Morpholinyl, Piperidinyl, Pyrrolidinyl, Piperazinyl, Hydroxy und Amino substituiert sein können,
   worin Amino mit 1 oder 2 Substituenten unabhängig voneinander ausgewählt aus der Gruppe (C₁-C₄)-Alkyl, Methylcarbonyl, Ethylcarbonyl, Methylsulfonyl, Ethylsulfonyl und Methoxyethyl substituiert sein kann,
worin Cyclobutyl mit Amino oder Hydroxy substituiert sein kann,
worin Amino mit 1 oder 2 Substituenten unabhängig voneinander ausgewählt aus Methyl, Ethyl, Methylcarbonyl, Ethylcarbonyl, Methylsulfonyl oder Ethylsulfonyl substituiert sein kann,
   und worin

R⁷ und R⁸ unabhängig voneinander für Wasserstoff, Methyl, Ethyl oder Cyclopropyl stehen, oder
für eine Gruppe der Formel 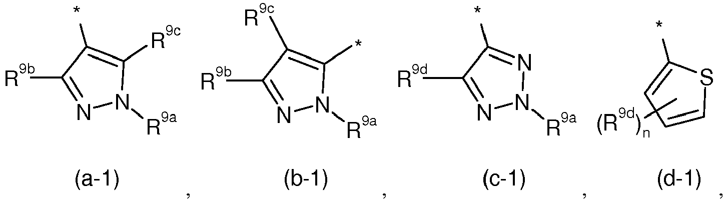 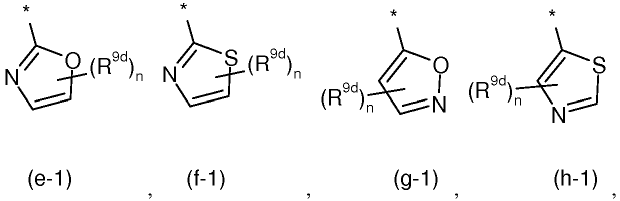 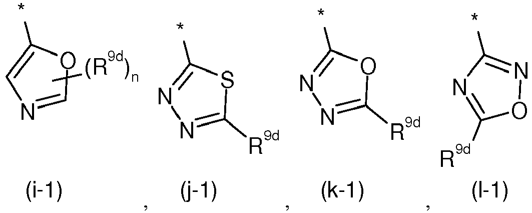 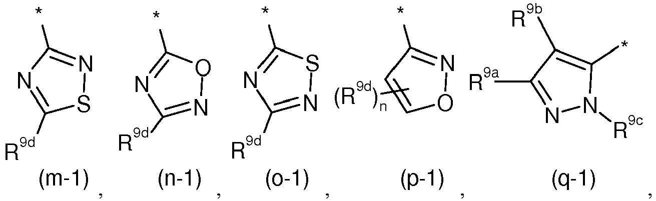
steht, wobei
- *: für die Anknüpfungsstelle an das Imidazopyridin steht,
- n: für eine Zahl 1 oder 2 steht,
- R^{9a}: für (C₁-C₆)-Alkyl, Phenyl, Pyridyl oder Cyclopropyl steht,
wobei (C₁-C₆)-Alkyl mit 1 oder 2 Substituenten ausgewählt aus der Gruppe Fluor, Cyano, Trifluormethyl, Difluormethyl, Methylcarbonyl, Methoxycarbonyl, Ethoxycarbonyl, Hydroxycarbonyl, -(C=O)NR⁷R⁸, -O(C=O)NR⁷R⁸, Methylsulfonyl, Ethylsulfonyl, Methoxy, Phenyl, Pyridyl, 1H-Pyrazolyl, 1H-Tetrazolyl, 1,2-Oxazolyl, Hydroxy, Amino, (C₃-C₅)-Cycloalkyl, Morpholinyl, Piperidinyl, Pyrrolidinyl, Piperazinyl, Thiomorpholinyl-1,1-dioxid und Azetidin substituiert sein kann,
worin 1H-Pyrazolyl, 1H-Tetrazolyl und 1,2-Oxazolyl mit 1 oder 2 Substituenten Methyl oder Ethyl substituiert sein können,
worin Piperidinyl mit 1 bis 2 Substituenten Fluor substituiert sein kann,
worin Azetidin mit Hydroxy substituiert sein kann,
   und
worin Piperazinyl mit Methyl substituiert sein kann,
wobei Cyclopropyl mit 1 oder 2 Substituenten unabhängig ausgewählt aus der Gruppe Methyl, Ethyl, Methoxycarbonyl, Ethoxycarbonyl und Hydroxycarbonyl substituiert sein kann,
wobei Phenyl und Pyridyl mit 1 oder 2 Substituenten Fluor substituiert sein können,
und worin
R⁷ und R⁸ unabhängig voneinander für Wasserstoff, Methyl, Ethyl oder Cyclopropyl stehen,
- R^{9b}: für Wasserstoff oder (C₁-C₄)-Alkyl steht,
- R^{9c}: für Wasserstoff oder (C₁-C₄)-Alkyl steht,
- R^{9d}: für Wasserstoff, (C₁-C₆)-Alkyl, Trifluormethyl, Methoxy, Ethoxy, Amino, Methoxycarbonyl, Ethoxycarbonyl, Hydroxycarbonyl, -(C=O)NR⁷R⁸, Phenyl, Pyridyl, Pyrimidyl, 1,3-Thiazolyl, Tetrahydrothiophenyl-1,1-dioxid oder Cyclopropyl steht,
wobei (C₁-C₆)-Alkyl mit 1 oder 2 Substituenten ausgewählt aus der Gruppe Trifluormethyl, Difluormethyl, (C₁-C₄)-Alkoxy, 2-Oxopyrrolidin-1-yl, Phenyl, Pyridyl, Pyrimidyl, 1H-1,2,4-Triazolyl, Hydroxy und Amino substituiert sein kann,
worin 1H-1,2,4-Triazolyl mit 1 oder 2 Substituenten Methyl oder Ethyl substituiert sein kann,
   und
worin Amino mit (C₁-C₄)-Alkyl substituiert sein kann,
wobei Amino mit (C₁-C₄)-Alkyl substituiert sein kann,
wobei Phenyl, Pyridyl, Pyrimidyl und 1,3-Thiazolyl jeweils mit 1 oder 2 Substituenten Methyl oder Ethyl substituiert sein können, und worin
R⁷ und R⁸ jeweils unabhängig voneinander für Wasserstoff, Methyl, Ethyl oder Cyclopropyl stehen,
- R⁴: für Wasserstoff steht,
- R⁵: für Wasserstoff, Chlor, Fluor, Methyl, Ethyl, Difluormethyl oder Cyclopropyl steht,
- R⁶: für Wasserstoff steht,
sowie ihre N-Oxide, Salze, Solvate, Salze der N-Oxide und Solvate der N-Oxide und Salze.

Besonders bevorzugt sind im Rahmen der vorliegenden Erfindung Verbindungen der Formel (I), in welcher
- A: für CH₂ steht,
- R¹: für Cyclohexyl steht,
oder
für eine Phenyl-Gruppe der Formel 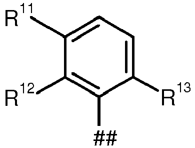 steht, wobei
## für die Anknüpfstelle an A steht,
   und

R¹¹, R¹² und R¹³ unabhängig voneinander für Wasserstoff oder Fluor stehen,
mit der Maßgabe, dass mindestens zwei der Reste R¹¹, R¹², R¹³ von Wasserstoff verschieden sind,
oder
für eine Pyridyl-Gruppe der Formel 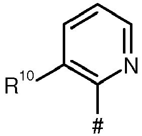 steht, wobei
# für die Anknüpfstelle an A steht,
   und

R¹⁰ für Fluor steht,
R² für Methyl oder Ethyl steht,
R³ für Phenyl steht,
wobei Phenyl mit 1 oder 2 Substituenten ausgewählt aus der Gruppe Fluor, Chlor, Cyano, Amino, Trifluormethyl, Difluormethyl, Methyl, -(C=O)NR⁷R⁸, Methoxy, Piperidinyl und Cyclobutyl substituiert sein kann,
worin Methyl mit 1 oder 2 Substituenten ausgewählt aus der Gruppe-(C=O)NR⁷R⁸, Methoxy, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus Methyl, Ethyl und Methoxyethyl substituiert sein kann,
worin Amino mit 1 oder 2 Substituenten unabhängig voneinander ausgewählt aus Methyl, Ethyl, Methylcarbonyl, Ethylcarbonyl, Methylsulfonyl oder Ethylsulfonyl substituiert sein kann,
worin Cyclobutyl mit Amino substituiert ist,
   und worin

R⁷ und R⁸ jeweils unabhängig voneinander für Wasserstoff, Methyl, Ethyl oder Cyclopropyl stehen, oder
für eine Gruppe der Formel 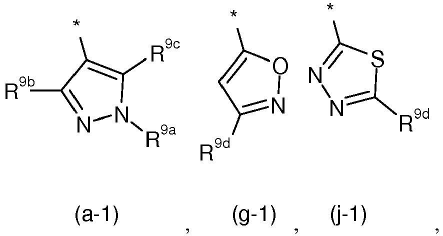 steht, wobei
* für die Anknüpfungsstelle an das Imidazopyridin steht,
R^{9a} für (C₁-C₆)-Alkyl, Phenyl, Pyridyl oder Cyclopropyl steht,
wobei (C₁-C₆)-Alkyl mit Fluor, Cyano, Trifluormethyl, Difluormethyl, Methyl-carbonyl, Methoxycarbonyl, Ethoxycarbonyl, Hydroxycarbonyl, -(C=O)NR⁷R⁸,-O(C=O)NR⁷R⁸, Methylsulfonyl, Ethylsulfonyl, Methoxy, Ethoxy, Trifluormethoxy, Difluormethoxy, Phenyl, Pyridyl, 1H-Pyrazolyl, 1H-Tetrazolyl, 1,2-Oxazolyl, Hydroxy, Amino, Cyclopropyl, Cyclobutyl, Morpholinyl, Piperidinyl, Pyrrolidinyl, Piperazinyl, Thiomorpholinyl-1,1-dioxid oder Azetidin substituiert sein kann,
worin 1H-Pyrazolyl, 1H-Tetrazolyl und 1,2-Oxazolyl mit 1 oder 2 Substituenten Methyl oder Ethyl substituiert sein können,
worin Piperidinyl mit 1 bis 2 Substituenten Fluor substituiert sein kann,
worin Azetidin mit Hydroxy substituiert sein kann,
   und
worin Piperazinyl mit Methyl substituiert sein kann,
wobei Cyclopropyl mit Methoxycarbonyl, Ethoxycarbonyl oder Hydroxycarbonyl substituiert sein kann,
wobei Phenyl und Pyridyl mit 1 oder 2 Substituenten Fluor substituiert sein können,
und worin

R⁷ und R⁸ unabhängig voneinander für Wasserstoff, Methyl, Ethyl oder Cyclopropyl stehen,
R^{9b} für Wasserstoff oder Methyl steht,
R^{9c} für Wasserstoff oder Methyl steht,
R^{9d} für Wasserstoff, (C₁-C₆)-Alkyl, Trifluormethyl, Methoxy, Ethoxy, Amino, Methoxycarbonyl, Ethoxycarbonyl, Hydroxycarbonyl, -(C=O)NR⁷R⁸, Phenyl, Pyridyl, Pyrimidyl, 1,3-Thiazolyl, Tetrahydrothiophenyl-1,1-dioxid oder Cyclopropyl steht,
wobei (C₁-C₆)-Alkyl mit 1 oder 2 Substituenten ausgewählt aus der Gruppe Trifluormethyl, Difluormethyl, (C₁-C₄)-Alkoxy, 2-Oxopyrrolidin-1-yl, Phenyl, Pyridyl, Pyrimidyl, 1H-1,2,4-Triazolyl, Hydroxy und Amino substituiert sein kann,
worin 1H-1,2,4-Triazolyl mit 1 oder 2 Substituenten Methyl oder Ethyl substituiert sein kann,
   und
worin Amino mit (C₁-C₄)-Alkyl substituiert sein kann,
wobei Amino mit (C₁-C₄)-Alkyl substituiert sein kann,
wobei Phenyl, Pyridyl, Pyrimidyl und 1,3-Thiazolyl jeweils mit 1 oder 2 Substituenten Methyl oder Ethyl substituiert sein können,
und worin
R⁷ und R⁸ jeweils unabhängig voneinander für Wasserstoff, Methyl, Ethyl oder Cyclopropyl stehen,
R⁴ für Wasserstoff steht,
R⁵ für Wasserstoff, Chlor, Fluor, Methyl, Ethyl, Difluormethyl oder Cyclopropyl steht,
R⁶ für Wasserstoff steht,
sowie ihre N-Oxide, Salze, Solvate, Salze der N-Oxide und Solvate der N-Oxide und Salze.

Besonders bevorzugt im Rahmen der vorliegenden Erfindung sind auch Verbindungen der Formel (I), in welcher
- A: für CH₂ steht,
- R¹: für Cyclohexyl steht,
oder
für eine Phenyl-Gruppe der Formel 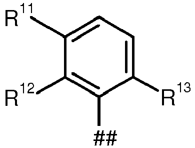 steht, wobei
## für die Anknüpfstelle an A steht, und
R¹¹, R¹² und R¹³ unabhängig voneinander für Wasserstoff oder Fluor stehen, mit der Maßgabe, dass mindestens zwei der Reste R¹¹, R¹², R¹³ von Wasserstoff verschieden sind,
   oder
   für eine Pyridyl-Gruppe der Formel 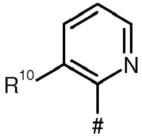
steht, wobei
# für die Anknüpfstelle an A steht,
   und
R¹⁰ für Fluor steht,
- R²: für Methyl steht,
- R³: für Phenyl steht,
wobei Phenyl mit 1 oder 2 Substituenten ausgewählt aus der Gruppe Fluor, Chlor, Cyano, Amino, Methyl, -(C=O)NR⁷R⁸, Methoxy, Piperidinyl und Cyclobutyl substituiert sein kann,
worin Methyl mit 1 oder 2 Substituenten ausgewählt aus der Gruppe-(C=O)NR⁷R⁸, Methoxy, Morpholinyl, Piperidinyl, Pyrrolidinyl, Piperazinyl, Hydroxy und Amino substituiert sein kann,
   worin Amino mit 1 oder 2 Substituenten unabhängig voneinander ausgewählt aus Methyl, Ethyl und Methoxyethyl substituiert sein kann,
worin Amino mit 1 oder 2 Substituenten unabhängig voneinander ausgewählt aus Methyl, Ethyl, Methylcarbonyl, Ethylcarbonyl, Methylsulfonyl oder Ethylsulfonyl substituiert sein kann,
worin Cyclobutyl mit Amino substituiert ist,
   und worin

R⁷ und R⁸ jeweils unabhängig voneinander für Wasserstoff, Methyl, oder Cyclopropyl stehen, oder
für eine Gruppe der Formel 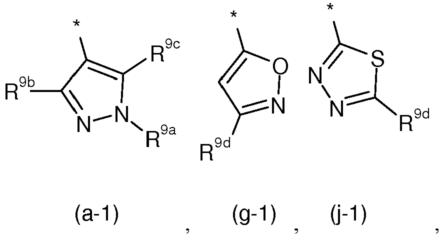 steht, wobei
* für die Anknüpfungsstelle an das Imidazopyridin steht,
R^{9a} für (C₁-C₄)-Alkyl oder Cyclopropyl steht,
wobei (C₁-C₄)-Alkyl mit Fluor, Cyano, Methoxycarbonyl, Hydroxycarbonyl,-(C=O)NR⁷R⁸, -O(C=O)NR⁷R⁸, Methylsulfonyl, Phenyl, 1H-Pyrazolyl, 1H-Tetrazolyl, 1,2-Oxazolyl, Hydroxy, Amino, Cyclopropyl, Morpholinyl, Piperidinyl, Pyrrolidinyl, 2-Oxopyrrolidin-1-yl, Piperazinyl, Thiomorpholinyl-1,1-dioxid oder Azetidin substituiert sein kann,
worin 1H-Pyrazolyl, 1H-Tetrazolyl und 1,2-Oxazolyl mit 1 oder 2 Substituenten Methyl substituiert sein können,
worin Piperidinyl mit 1 oder 2 Substituenten Fluor substituiert sein kann,
worin Phenyl mit 1 oder 2 Substituenten Fluor substituiert sein kann,
worin Azetidin mit Hydroxy substituiert ist,
worin Piperazinyl mit Methyl substituiert ist,
   und worin
R⁷ und R⁸ jeweils unabhängig voneinander für Wasserstoff, Methyl oder Cyclopropyl stehen,
und
wobei Cyclopropyl mit Methoxycarbonyl oder Hydroxycarbonyl substituiert ist,
R^{9b} für Wasserstoff steht,
R^{9c} für Wasserstoff steht,
R^{9d} für (C₁-C₄)-Alkyl steht,
wobei (C₁-C₄)-Alkyl mit Amino oder Hydroxy substituiert ist,
- R⁴: für Wasserstoff steht,
- R⁵: für Wasserstoff, Chlor oder Methyl steht,
- R⁶: für Wasserstoff steht,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in welcher
- R¹: für eine Phenyl-Gruppe der Formel 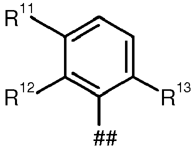 steht, wobei
## für die Anknüpfstelle an A steht,
   und
R¹¹, R¹² und R¹³ unabhängig voneinander für Wasserstoff oder Fluor stehen,
mit der Maßgabe, dass mindestens zwei der Reste R¹¹, R¹², R¹³ von Wasserstoff verschieden sind,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in welcher
- R¹: für eine Pyridyl-Gruppe der Formel 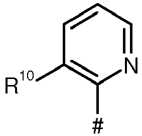 steht, wobei
# für die Anknüpfstelle an A steht,
   und
R¹⁰ für Fluor steht,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R²: für Methyl steht,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R³: für eine Gruppe der Formel 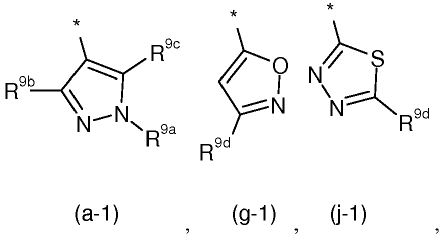 steht, wobei
* für die Anknüpfungsstelle an das Imidazopyridin steht,
R^{9a} für (C₁-C₄)-Alkyl oder Cyclopropyl steht,
wobei (C₁-C₄)-Alkyl mit Fluor, Cyano, Methoxycarbonyl, Hydroxycarbonyl,-(C=O)NR⁷R⁸, -O(C=O)NR⁷R⁸, Methylsulfonyl, Phenyl, 1H-Pyrazolyl, 1H-Tetrazolyl, 1,2-Oxazolyl, Hydroxy, Amino, Cyclopropyl, Morpholinyl, Piperidinyl, Pyrrolidinyl, 2-Oxopyrrolidin-1-yl, Piperazinyl, Thiomorpholinyl-1,1-dioxid oder Azetidin substituiert sein kann,
worin 1H-Pyrazolyl, 1H-Tetrazolyl und 1,2-Oxazolyl mit 1 oder 2 Substituenten Methyl substituiert sein können,
worin Piperidinyl mit 1 oder 2 Substituenten Fluor substituiert sein kann,
worin Phenyl mit 1 oder 2 Substituenten Fluor substituiert sein kann,
worin Azetidin mit Hydroxy substituiert ist,
worin Piperazinyl mit Methyl substituiert ist,
   und

R⁷ und R⁸ jeweils unabhängig voneinander für Wasserstoff, Methyl oder Cyclopropyl stehen, und
wobei Cyclopropyl mit Methoxycarbonyl oder Hydroxycarbonyl substituiert ist,
R^{9b} für Wasserstoff steht,
R^{9c} für Wasserstoff steht,
R^{9d} für Wasserstoff oder (C₁-C₄)-Alkyl steht,
wobei (C₁-C₄)-Alkyl mit Amino oder Hydroxy substituiert ist,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R³: für eine Gruppe der Formel 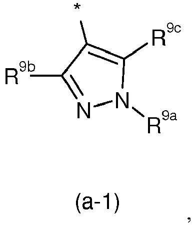 steht, wobei
* für die Anknüpfungsstelle an das Imidazopyridin steht,
R^{9a} für (C₁-C₄)-Alkyl steht,
wobei (C₁-C₄)-Alkyl mit Hydroxy oder Amino substituiert ist,
R^{9b} für Wasserstoff steht,
R^{9c} für Wasserstoff steht,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R³: für eine Gruppe der Formel 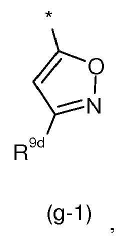 steht, wobei
* für die Anknüpfungsstelle an das Imidazopyridin steht,
R^{9d} für (C₁-C₄)-Alkyl steht,
wobei (C₁-C₄)-Alkyl mit Hydroxy oder Amino substituiert ist,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R³: für eine Gruppe der Formel 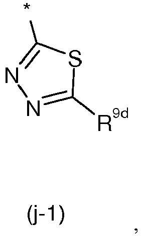 steht, wobei
* für die Anknüpfungsstelle an das Imidazopyridin steht,
R^{9d} für (C₁-C₄)-Alkyl steht,
wobei (C₁-C₄)-Alkyl mit Hydroxy oder Amino substituiert ist,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R⁵: für Wasserstoff, Chlor, Fluor, Methyl, Ethyl, Difluormethyl oder Cyclopropyl steht,
sowie ihre N-Oxide, Salze, Solvate, Salze der N-Oxide und Solvate der N-Oxide und Salze.

Bevorzugt sind im Rahmen der vorliegenden Erfindung auch Verbindungen der Formel (I), in
welcher
- R⁵: für Wasserstoff, Chlor oder Methyl steht,
sowie ihre N-Oxide, Salze, Solvate, Salze der N-Oxide und Solvate der N-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man

### [A] eine Verbindung der Formel (II)

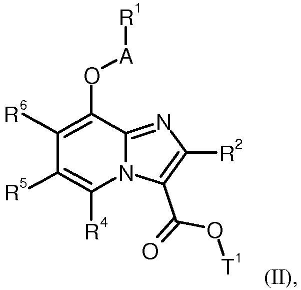

in welcher A, R¹, R², R⁴, R⁵ und R⁶ jeweils die oben angegebenen Bedeutungen haben und
- T¹: für (C₁-C₄)-Alkyl oder Benzyl steht,
in einem inerten Lösungsmittel in Gegenwart einer geeigneten Base oder Säure zu einer Carbonsäure der Formel (III) 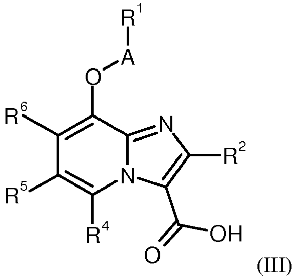 in welcher A, R¹, R², R⁴, R⁵ und R⁶ jeweils die oben angegebenen Bedeutungen haben, umsetzt,
[A] und diese in der Folge in Gegenwart einer geeigneten Säure zu einem Imidazo[1,2-a]-pyridin der Formel (IV) 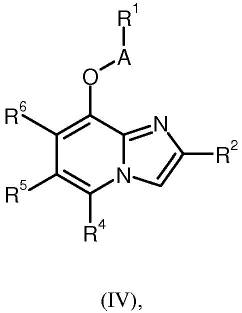 in welcher A, R¹, R², R⁴, R⁵ und R⁶ jeweils die oben angegebenen Bedeutungen haben, umsetzt, und dieses dann mit einem Halogen-Äquivalent in eine Verbindung der Formel (V) 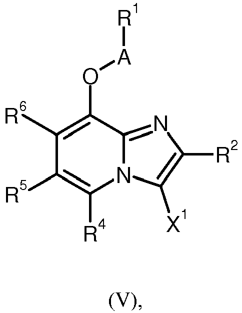 in welcher A, R¹, R², R⁴, R⁵ und R⁶ jeweils die oben angegebenen Bedeutungen haben und
   - X¹: für Chlor, Brom oder Iod steht,
   überführt, und diese im Anschluß in einem inerten Lösungsmittel in Gegenwart eines geeigneten Übergangsmetallkatalysators mit einer Verbindung der Formel (VI), 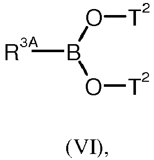 in welcher
   - R^{3A}: die oben für R³ angegebenen Bedeutungen hat und
   - T²: für Wasserstoff oder (C₁-C₄)-Alkyl steht, oder beide Reste T² zusammen eine -C(CH₃)₂-C(CH₃)₂-Brücke bilden,
   zu einer Verbindung der Formel (I-A) 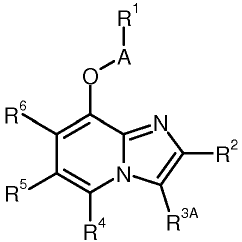 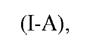 umsetzt und anschließend für den Fall, dass R^{3A} für 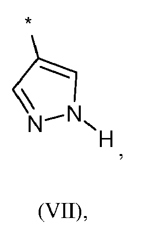 steht, diese Verbindungen in einem inerten Lösungsmittel in Gegenwart einer geeigneten Base mit einer Verbindung der Formel (VIII)

   R¹⁴-X¹ (VIII),

   in welcher
   - X¹: für eine geeignete Abgangsgruppe, insbesondere Chlor, Brom, Iod, Mesylat, Triflat oder Tosylat steht,
   und
   - R¹⁴: für (C₁-C₆)-Alkyl steht,
   wobei (C₁-C₆)-Alkyl mit 1 bis 3 Substituenten ausgewählt aus der Gruppe Fluor, Cyano, Trifluormethyl, Difluormethyl, (C₁-C₄)-Alkylcarbonyl, (C₁-C₄)-Alkoxy-carbonyl, Hydroxycarbonyl, -(C=O)NR⁷R⁸, -O(C=O)NR⁷R⁸, (C₁-C₄)-Alkylsulfonyl, (C₁-C₄)-Alkoxy, Trifluormethoxy, Difluormethoxy, Phenyl, 1H-Pyrazolyl, 1H-1,2,4-Triazolyl, 1H-Tetrazolyl, 1,2-Oxazolyl, Tetrahydrothiophenyl-1,1-dioxid, Hydroxy, Amino, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
      worin 1H-Pyrazolyl, 1H-1,2,4-Triazolyl, 1H-Tetrazolyl und 1,2-Oxazolyl mit 1 oder 2 Substituenten Methyl oder Ethyl substituiert sein können,
      worin Piperidinyl mit 1 oder 2 Substituenten Fluor substituiert sein kann,
      worin Phenyl mit 1 oder 2 Substituenten Fluor substituiert sein kann,
      worin Piperazinyl mit Methyl substituiert sein kann, und worin
         R⁷ und R⁸ jeweils unabhängig voneinander für Wasserstoff, Methyl oder Cyclopropyl stehen,
         oder
         R⁷ und R⁸ zusammen mit dem Kohlenstoffatom, an das sie gebunden sind, einen 3- bis 5-gliedrigen Carbocyclus bilden,
   zu einer Verbindung der Formel (I-B) 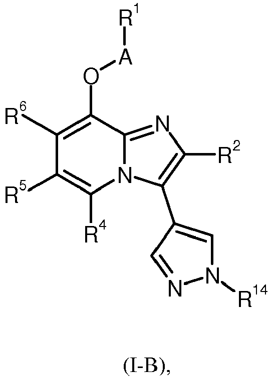 umsetzt,
   in welcher A, R¹, R², R⁴, R⁵, R⁶ und R¹⁴ jeweils die oben angegebenen Bedeutungen haben und
   anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
   oder
[B] eine Verbindung der Formel (II) in Gegenwart von Hydrazinhydrat in eine Verbindung der Formel (IX) 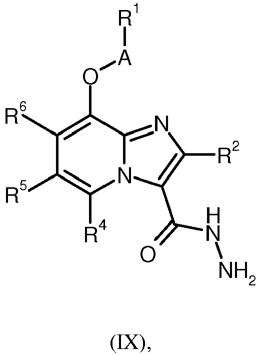 in welcher A, R¹, R², R⁴, R⁵ und R⁶ jeweils die oben angegebenen Bedeutungen haben,
   überführt, und diese in der Folge in einem inerten Lösungsmittel unter Amidkupplungsbedingungen mit einer Carbonsäure der Formel (X) 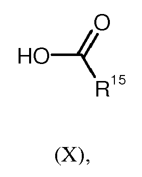 in welchem R¹⁵ für (C₁-C₆)-Alkyl steht,
   wobei (C₁-C₆)-Alkyl mit 1 bis 3 Substituenten ausgewählt aus der Gruppe Trifluormethyl, Difluormethyl, Hydroxy und Amino substituiert sein kann,
   zu einer Verbindung der Formel (XI) umsetzt, 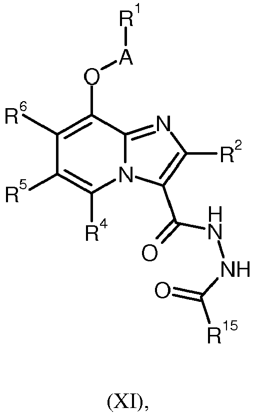 in welcher A, R¹, R², R⁴, R⁵ , R⁶ und R¹⁵ jeweils die oben angegebenen Bedeutungen haben,
   und diese Verbindung anschließend mit 2,4-Bis(4-methoxyphenyl)-1,3,2,4-dithiadiphosphetan-2,4-disulfid [Lawesson-Reagenz] in eine Verbindung der Formel (I-C) 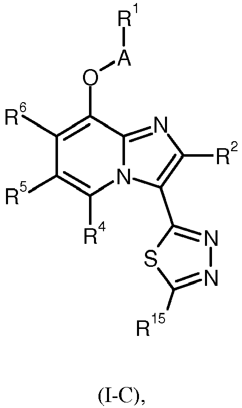 überführt,
   in welcher A, R¹, R², R⁴, R⁵, R⁶ und R¹⁵ jeweils die oben angegebenen Bedeutungen haben,
   anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A), (I-B) und (I-C) bilden eine Teilmenge der erfindungsgemäßen Verbindungen der Formel (I).

Die beschriebenen Herstellverfahren können durch die folgenden Syntheseschemata (Schema 1 und 2) beispielhaft verdeutlicht werden: 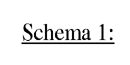 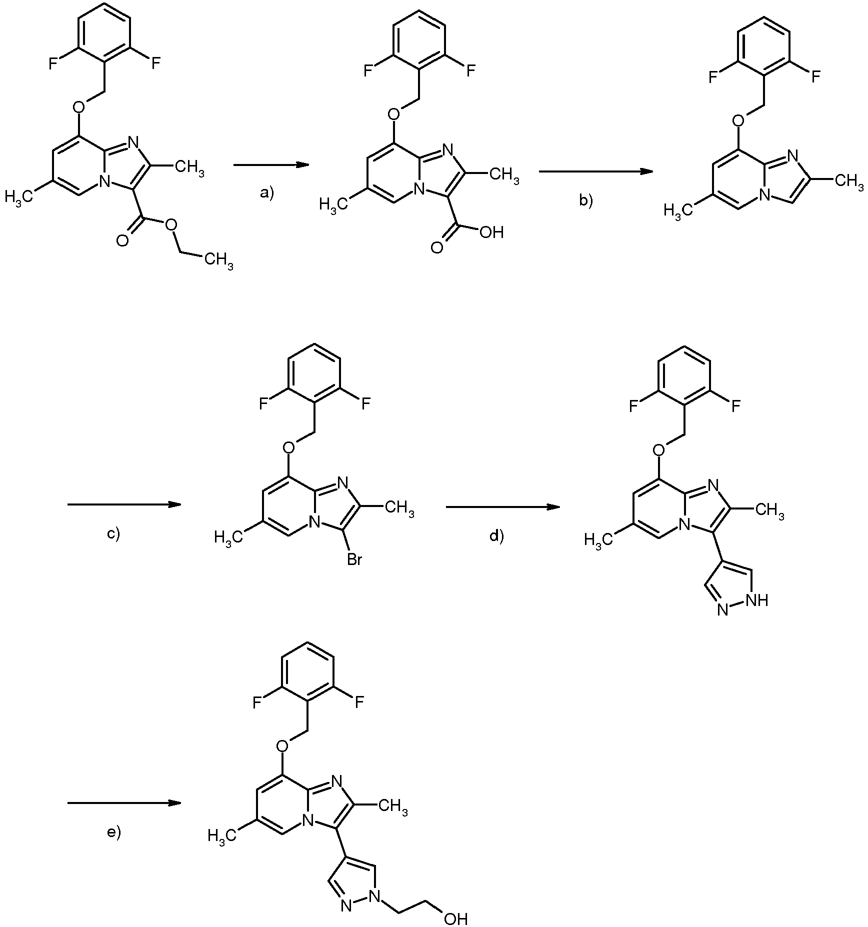 [a): Lithiumhydroxid, THF/Methanol/ H₂O, RT; b): 6 N Salzsäure, 100°C; c): N-Bromsuccinimid, Ethanol, RT; d): 1H-Pyrazol-4-ylboronsäure oder [1-(tert.-Butoxycarbonyl)-1H-pyrazol-4-yl]boronsäure, Bis(tri-tert.-butyl-phosphin)palladium(0), K₃PO₄, Ethanol/Wasser/Toluol, 120°C; e): Cäsiumcarbonat, Kaliumiodid, Iodethanol, DMF, 70°C]. 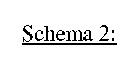 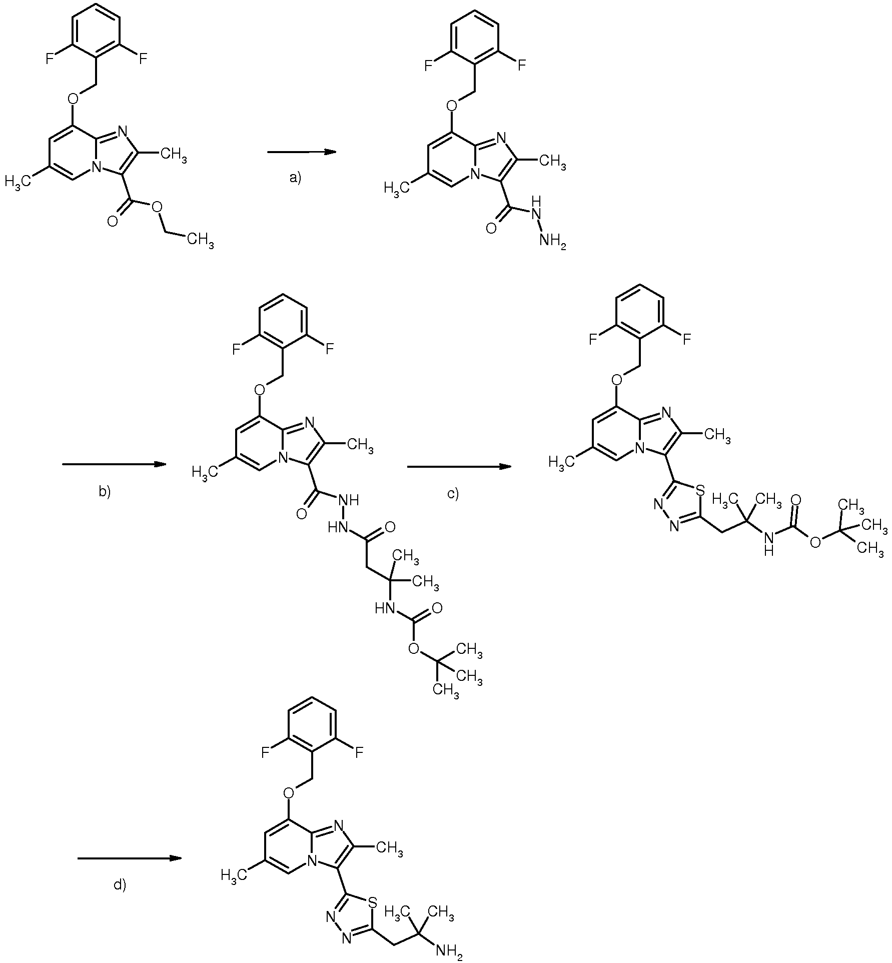 [a): Hydrazinhydrat, Ethanol, 80°C; b): 3-[(tert.-Butoxycarbonyl)amino]-3-methylbutansäure, EDCI, HOBT, DMF, RT; c): 2,4-Bis(4-methoxyphenyl)-1,3,2,4-dithiadiphosphetan-2,4-disulfid [Lawesson-Reagenz], THF, 100°C Mikrowelle; d): TFA, Dichlormethan, RT].

Die Verbindungen der Formeln (VI), (VIII) und (X) sind kommerziell erhältlich, literaturbekannt oder können in Analogie zu literaturbekannten Verfahren hergestellt werden.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Geeignetes Lösungsmittel für den Verfahrensschritt (III) → (IV) ist Wasser.

Geeignete Säuren für den Verfahrensschritt (III) → (IV) sind Chlorwasserstoff/ Salzsäure, Bromwasserstoff/Bromwasserstoffsäure, Schwefelsäure, Essigsäure, .... oder deren Gemische gegebenenfalls unter Zusatz von Wasser. Bevorzugt wird Salzsäure verwendet.

Die Decarboxylierung (III) → (IV) wird im Allgemeinen in einem Temperaturbereich von +20°C bis +100°C, bevorzugt bei 75°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Als Lösungsmittel eignen sich für den Verfahrensschritt (IV) → (V) Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Bevorzugt werden Methanol und/oder Ethanol eingesetzt.

Als Halogen-Quelle bei der Umsetzung (IV) → (V) eignen sich beispielsweise N-Bromsuccinimid, N-Chlorsuccinimid, N-Iod-succinimid, Chlor, Brom oder Iod. Bevorzugt wird N-Bromsuccinimid verwendet.

Die Reaktion (IV) → (V) erfolgt im Allgemeinen in einem Temperaturbereich von +20°C bis +100°C, bevorzugt im Bereich von +20°C bis +80°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (V) + (VI) → (I-A) erfolgt in einem unter den Reaktionsbedingungen inerten Lösungmittel. Geeignete Lösungsmittel sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmitteln wie 1,2-Dimethoxyethan (DME), Dimethylformamid (DMF), Dimethylsulfoxid (DMSO), *N,N'-*Dimethylpropylenharnstoff (DMPU), *N*-Methylpyrrolidon (NMP), Pyridin, Acetonitril, Toluol oder auch Wasser. Ebenso ist es möglich, Gemische der genannten Lösungsmittel einzusetzen. Bevorzugt sind Methanol, Ethanol, Toluol und Wasser.

Gegebenenfalls kann die Umsetzung (V) + (VI) → (I-A) in Gegenwart eines geeigneten Palladium- und/oder Kupferkatalysators erfolgen. Als Palladium-Katalysator ist beispielsweise Palladium(II)-acetat, Tetrakis-(triphenylphosphin)-palladium(0), Bis(tri-tert.-butyl-phosphin)palladium(0), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise (2-Biphenyl)di*-tert.*-butylphosphin, 2-Dicyclohexylphosphino-2',6'-dimethoxybiphenyl (SPHOS) Dicyclohexyl[2',4',6'-tris(1-methylethyl)biphenyl-2-yl]phosphan (XPHOS), Bis(2-phenyl-phosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] geeignet.

Die Umsetzung (V) + (VI) → (I-A) erfolgt gegenbenenfalls in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N-*Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}) oder Kaliumphosphat. Bevorzugt wird Kaliumphosphat verwendet.

Die Reaktion (V) + (VI) → (I-A) wird im Allgemeinen in einem Temperaturbereich von 0°C bis +200°C, bevorzugt bei +100°C bis +150°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (I-A) + (VIII) → (I-B) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Essigsäureethylester, Acetonitril, *N,N*-Dimethylformamid, *N,N*-Dimethylacetamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Dimethylformamid oder Dimethylsulfoxid verwendet.

Als Basen für den Verfahrensschritt (I-A) + (VIII) → (I-B) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N,N*-Diisopropylethylamin, Pyridin, 4-(*N,N*-Dimethylamino)-pyridin (DMAP), 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Geeignete Lösungsmittel für den Verfahrensschritt (II) → (IX) sind Chloroform oder Alkohole wie Methanol, Ethanol, n-Propanol, Isopropanol, n-Butanol, n-Pentanol oder tert.-Butanol. Ebenso ist es möglich, Gemische der genannten Lösungsmittel einzusetzen. Bevorzugt wird Ethanol verwendet.

Als Reagenz für den Verfahrensschritt (II) → (IX) können Hydrazin oder Hydrazinhydrat verwendet werden. Bevorzugt ist Hydrazinhydrat.

Die Reaktion (II) → (IX) wird im Allgemeinen in einem Temperaturbereich von 0°C bis +200°C, bevorzugt bei +70°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Verfahrensschritte (IX) + (X) → (XI) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Essigsäureethylester, Acetonitril, Pyridin, Dimethylsulfoxid, *N,N*-Dimethylformamid, *N,N*-Dimethylacetamid, *N,N'-*Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung in den Verfahrensschritte (IX) + (X) → (XI) eignen sich beispielsweise Carbodiimide wie *N,N'*-Diethyl-, *N,N'*-Dipropyl-, *N,N'*-Diisopropyl-, *N,N'-*Dicyclohexylcarbodiimid (DCC) oder *N*-(3-Dimethylaminopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazoliumperchlorat, Acylaminoverbindungen wie 2-Ethoxy-1-ethoxycarbonyl-1,2-dihydrochinolin, oder Isobutylchlorformiat, Propanphosphonsäureanhydrid (T3P), 1-Chlor-*N,N,*2-trimethylpropl-en-1-amin, Cyanophosphonsäurediethylester, Bis-(2-oxo-3-oxazolidinyl)-phosphorylchlorid, Benzotriazol-1-yloxy-tris(dimethylamino)phosphonium-hexafluorphosphat, Benzotriazol-1-yloxy-tris(pyrrolidino)phosphonium-hexafluorphosphat (PyBOP), *O*-(Benzotriazol-1-yl)-*N,N,N',N'*-tetramethyluronium-tetrafluorborat (TBTU), *O*-(Benzotriazol-1-yl)-*N,N,N',N'*-tetramethyluronium-hexafluorphosphat (HBTU), 2-(2-Oxo-1-(2*H*)-pyridyl)-1,1,3,3-tetramethyluronium-tetrafluorborat (TPTU), *O*-(7-Azabenzotriazol-1-yl)-*N,N,N',N'*-tetramethyluronium-hexafluorphosphat (HATU) oder *O-*(1*H*-6-Chlorbenzotriazol-1-yl)-1,1,3,3-tetramethyluronium-tetrafluorborat (TCTU), gegebenenfalls in Kombination mit weiteren Hilfsstoffen wie 1-Hydroxybenzotriazol (HOBt) oder *N-*Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N*-Diisopropylethylamin. Bevorzugt wird TBTU in Verbindung mit N-Methylmorpholin, HATU in Verbindung mit *N,N*-Diisopropylethylamin oder 1-Chlor-*N,N*,2-trimethylprop-1-en-1amin verwendet.

Die Kondensationen (IX) + (X) → (XI) wird im Allgemeinen in einem Temperaturbereich von - 20°C bis +100°C, bevorzugt bei 0°C bis +60°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Geeignete Lösungsmittel für den Verfahrensschritt (XI) → (I-C) sind Ether wie Diethylether, Dioxan, Tetrahydrofuran, Glykoldimethylether oder Diethylenglykoldimethylether oder andere Lösungsmittel wie Aceton, Dichlormethan, Essigsäureethylester, Acetonitril, Dimethylsulfoxid, *N,N*-Dimethyl-formamid, *N,N*-Dimethylacetamid,*N*-Methylpyrrolidon (NMP), Toluol oder Pyridin. Ebenso ist es möglich, Gemische der genannten Lösungsmittel zu verwenden. Bevorzugt sind Diethylether und Tetrahydrofuran oder Gemische dieser Lösungsmittel.

Als Reagenz für den Verfahrensschritt (XI) → (I-C) sind 2,4-Bis(4-methoxyphenyl)-1,3,2,4-dithiadiphosphetan-2,4-disulfid [Lawesson-Reagenz], Diphosphorpentasulfid oder Tetraphosphordecasuilfid geeignet. Bevorzugt ist 2,4-Bis(4-methoxyphenyl)-1,3,2,4-dithiadiphosphetan-2,4-disulfid [Lawesson-Reagenz].

Die Reaktion (XI) → (I-C) wird im Allgemeinen in einem Temperaturbereich von 0°C bis +200°C, bevorzugt bei +70°C bis +120°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Bevorzugt erfolgt die Reaktion in der Mikrowelle.

Die Verbindungen der Formel (II) sind literaturbekannt oder können hergestellt werden, indem eine Verbindung der Formel (XII) 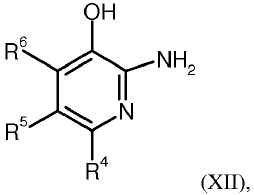 in welcher R⁴, R⁵ und R⁶ die oben angegebene Bedeutung hat,
in einem inerten Lösungsmittel in Gegenwart einer geeigneten Base mit einer Verbindung der Formel (XIII) 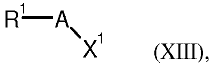 in welcher A und R¹ die oben angegebene Bedeutung hat und
- X¹: für eine geeignete Abgangsgruppe, insbesondere Chlor, Brom, Iod, Mesylat, Triflat oder Tosylat, steht,
zu einer Verbindung der Formel (XIV) 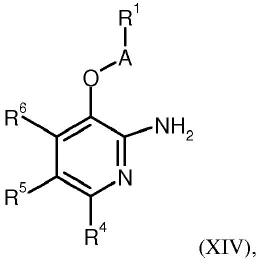 in welcher R¹, R⁴, R⁵ und R⁶ jeweils die oben angegebenen Bedeutungen haben,
umgesetzt wird, und diese anschliessend in einem inerten Lösungsmittel mit einer Verbindung der Formel (XV) 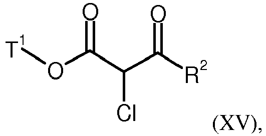 in welcher R² und T¹ jeweils die oben angegebenen Bedeutungen haben,
umgesetzt wird.

Das beschriebene Verfahren wird durch das nachfolgende Schema (Schema 3) beispielhaft verdeutlicht:

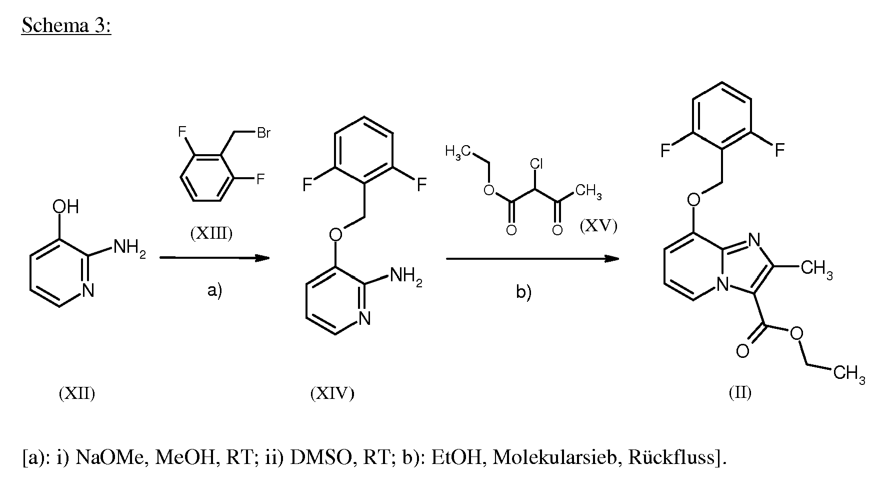

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 4 gezeigt.

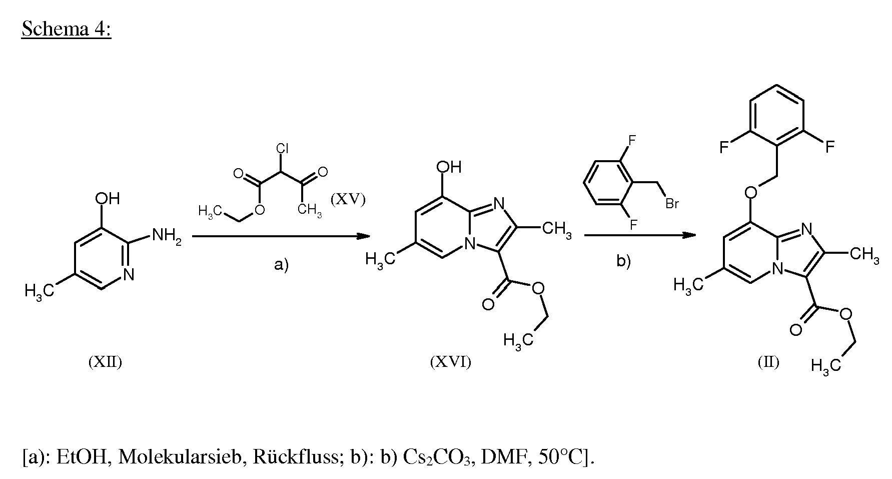

Inerte Lösungsmittel für den Verfahrensschritt (XII) + (XIII) → (XIV) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, *tert*-Butanol, oder andere Lösungsmittel wie Aceton, Methylethylketon, Essigsäureethylester, Acetonitril, *N,N*-Dimethylformamid, Dimethylsulfoxid, *N,N'*-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Methanol, Dimethylformamid oder Dimethylsulfoxid verwendet.

Als Basen für den Verfahrensschritt (XII) + (XIII) → (XIV) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyridin-Grundgerüst (XIV) + (XV) → (II) bzw. (XII) + (XV) → (XVI) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (XIV) + (XV) → (II) bzw. (XII) + (XV) → (XVI) erfolgt optional in Gegenwart wasserziehender Reaktionszusätze, beispielsweise in Gegenwart von Molekularsieb (4Å Porengröße) oder mittels Wasserabscheider. Die Umsetzung (XIV) + (XV) → (II) bzw. (XII) + (XV) → (XVI) erfolgt unter Verwendung eines Überschusses des Reagenzes der Formel (XV), beispielsweise mit 1 bis 20 Äquivalenten des Reagenzes (XV), gegebenenfalls unter Zusatz von Basen (wie z.B. Natriumhydrogencarbonat) wobei die Zugabe dieses Reagenzes einmalig oder in mehreren Portionen erfolgen kann.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung offenbart auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weitere Offenbarung der vorliegenden Erfindung ist die Verwendung der erfindungsgemäßen Verbindungen zur Behandlung und/oder Prophylaxe von Erkrankungen, insbesondere der zuvor genannten Erkrankungen.

Weitere Offenbarung der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weitere Offenbarung der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weitere Offenbarung der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: berechnet
- br.: Verbreitertes Signal (NMR Kupplungsmuster)
- CAS-Nr.: Chemical Abstracts Service Nummer
- δ: Verschiebung im NMR Spektrum (Angabe in)
- d: Dublett (NMR-Kupplungsmuster)
- DC: Dünnschichtchromatographie
- DCI: direkte chemische Ionisation (bei MS)
- DMAP: 4-*N,N*-Dimethylaminopyridin
- DMF: Dimethylformamid
- DMSO: Dimethylsulfoxid
- EDCI: *N*-[3-(Dimethylamino)propyl]-N'-ethylcarbodiimid
- d. Th.: der Theorie (bei Ausbeute)
- eq.: Äquivalent(e)
- ESI: Elektrospray-Ionisation (bei MS)
- Et: Ethyl
- gef.: gefunden
- h: Stunde(n)
- HATU: N-[(Dimethylamino)(3H-[1,2,3]triazolo[4,5-b]-pyridin-3-yloxy)methylen]-N-methylmethanaminiumhexafluorophosphat
- HOBT: 1H-Benzotriazol-1-ol
- HPLC: Hochdruck-, Hochleistungsflüssigchromatographie
- HRMS: hochaufgelöste Massenspektrometrie
- ID: Innendurchmesser
- konz.: konzentriert
- LC-MS: Flüssigchromatographie-gekoppelte Massenspektrometrie
- LiHMDS: Lithiumhexamethyldisilazid
- m: Multiplett
- Me: Methyl
- min: Minute(n)
- MS: Massenspektrometrie
- NMR: Kernresonanzspektrometrie
- PDA: Photodiodenarraydetektor
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- R_{F}: Retentionsfaktor (bei Dünnschichtchromatographie)
- RT: Raumtemperatur
- Rₜ: Retentionszeit (bei HPLC)
- s: Singulett (NMR Kupplungsmuster)
- t: Triplett (NMR Kupplungsmuster)
- THF: Tetrahydrofuran
- TBTU: (Benzotriazol-1-yloxy)bisdimethylaminomethyliumfluoroborat
- UPLC-MS: Ultradruck-Flüssigchromatographiegekoppelte Massenspektrometrie
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Angaben zu Kopplungsmustern in NMR-Spektren sind beschreibender Natur, Kopplungsmuster höherer Ordnung werden nicht als solche beschrieben.

### LC/MS- und HPLC-Methoden:

### Methode 1 (LC-MS):

Instrument: Waters ACQUITY SQD UPLC System; Säule: Waters Acquity UPLC HSS T3 1,8µ 50 x 1mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Instrument: Micromass Quattro Premier mit Waters UPLC Acquity; Säule: Thermo Hypersil GOLD 1.9 µ 50 mm x 1 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD; 210 nm.

### Methode 5 (LC-MS):

Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A - 0.2 min 95% A - 1.8 min 25% A - 1.9 min 10% A - 2.0 min 5% A - 3.2 min 5% A - 3.21 min 100% A - 3.35 min 100% A; Ofen: 40°C; Fluss: 3.0 ml/min; UV-Detektion: 210 nm.

### Methode 6 (präparative HPLC):

Säule: Macherey-Nagel VP 50/21 Nucleosil 100-5 C18 Nautilus. Flußrate: 25 ml/min. Gradient: A = Acetonitril, B = Wasser + 0.1% Ameisensäure, 0 min 10% A ; 2.00 min 10% A ; 6.00 min 90% A ; 7.00 min 90% A ; 7.10 min 10% A ; 8 min 10% A; UV-Detektion: 220 nm

### Methode 7 (präparative HPLC):

Säule: Phenomenex Gemini C18; 110A, AXIA, 5 µm, 21.2 X 50 mm 5 micron; Gradient: A = Wasser + 0.1 % konz. Ammoniak , B = Acetonitril, 0 min = 10% B, 2 min = 10% B, 6 min = 90% B, 7 min = 90% B, 7.1 min = 10% B, 8 min = 10% B, Flußrate 25 ml/min, UV-Detektion 220 nm.

### Methode 8 (präparative HPLC):

Säule: Axia Gemini 5 µ C18 110 A, 50 x 21.5 mm, P/NO: 00B-4435-P0-AX, S/NO: 35997-2, Gradient: A=Wasser + 0.1 % konz. aq. Ammoniak, B = Acetonitril, 0 min = 30 % B, 2 min = 30% B, 6 min = 100% B, 7 min = 100% B, 7,1 Min = 30% B, 8 Min=30% B, Flußrate 25 ml/min, UV-Detektion 220 nm.

### Methode 9 (präparative HPLC):

Säule: Macherey-Nagel VP 50/21 Nucleosil 100-5 C18 Nautilus. Flussrate: 25 ml/min. Gradient: A = Wasser + 0.1 % Ameisensäure, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 10 (präparative HPLC):

Säule: Macherey-Nagel VP 50/21 Nucleosil 100-5 C18 Nautilus. Flussrate: 25 ml/min. Gradient: A = Wasser + 0.1 % konz. aq. Ammoniak, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 11 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, Gradient: 0.0 min 95% A - 0.15 min 95% A - 8.0 min 5% A - 9.0 min 5% A; Fluss: 40 ml/min; UV-Detektion: DAD; 210 - 400 nm).
und

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäuren, Eluent B: Acetonitril (ULC) + 0.05% Ameisensäure, Gradient: 0.0 min 95%A - 0.15 min 95%A - 8.0 min 5%A - 9.0 min 5%A; Fluss: 40 ml/min; UV-Detektion: DAD; 210 - 400 nm).

### Methode 12 (LC-MS):

Instrument MS: Waters SQD; Instrument HPLC: Waters UPLC; Saeule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensaeure; Gradient: 0.0 min 98% A - 0.9 min 25% A - 1.0 min 5% A - 1.4 min 5% A -1.41 min 98% A -1.5 min 98% A; Ofen: 40°C; Fluss: 0.600 ml/min; UV-Detektion: DAD; 210 nm.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

### Methode 13 (DCI-MS):

Gerät: DSQ II; Thermo Fisher-Scientific; DCI mit NH₃, Fluss: 1.1 ml/min; Quellentemeperatur: 200°C; Ionisierungsenergie 70 eV; DCI-Heizfaden bis 800°C aufheizen; Mass-Range 80-900.

### Methode 14 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 15 (MS):

Gerät: Waters ZQ; Ionisierungsart: ESI (+); Laufmittel; Acetonitril/Wasser.

### Methode 16 (LCMS):

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure, Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 17 (LC-MS):

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 18 (präparative HPLC):

Chromatorex C18 10 µ 250 x 20 mm Gradient: A = Wasser + 0,5% Ameisensäure, B = Acetonitril, 0 min = 5% B, 3 min = 5% B vorspülen ohne Substanz, dann Injektion, 5 min = 5% B, 25min = 30% B, 38 min = 30% B, 38,1 min = 95% B, 43 min= 95% B, 43,01 min= 5% B, 48,0 min= 5% B Flussrate 20 ml/min, Wellenlänge 210 nm.

### Methode 19 (präparative HPLC):

Chromatorex C18 10µ 250x20mm Gradient: A = Wasser + 0,5% Ameisensäure, B= Acetonitril, 0min = 5% B, 3min = 5% B vorspülen ohne Substanz, dann Injektion, 5 min = 5% B, 25min = 50% B, 38min = 50% B, 38,1min = 95% B, 43min= 95% B, 43,01min= 5% B, 48,0min= 5% B Flussrate 20 ml/min, Wellenlänge 210 nm.

### Methode 20 (präparative HPLC):

XBridge Prep. C18 5µ 50x19mm Gradient: A = Wasser + 0.5% Ammoniumhydroxid, B= Acetonitril, 0 min = 5% B, 3 min = 5% B vorspülen ohne Substanz, dann Injektion, 5 min = 5% B, 25 min = 50% B, 38 min = 50% B, 38.1 min = 95% B, 43 min= 95% B, 43.01 min= 5% B, 48.0 min = 5% B Flussrate 15 ml/min, Wellenlänge 210 nm.

### Methode 21 (präparative HPLC):

Chromatorex 10 µ 250 x 20 mm Gradient: A = Wasser, B = Acetonitril, 0 min = 5 % B, 3min = 5% B vorspülen ohne Substanz, dann Injektion, 5 min = 5% B, 25 min = 95% B, 38 min = 95% B, 38,1 min = 5% B, 40 min = 5% B, Flussrate 20 ml/min, Wellenlänge 210 nm.

### Methode 22 (LC-MS):

Instrument: Acquity UPLC gekoppelt mit Quattro Micro Massenspektrometer; Säule: Acquity UPLC BEH C18 (50 mm x 2,1 mm ID, 1,7 µm Packungsdiameter); mobile Phase A: 10 mM wässrige Ammoniumhydrogencarbonatlösung (mit Ammoniak auf einen pH-Wert von 10 eingestellt), mobile Phase B: Acetonitril; Gradient: 0,0 min 97 % A, 3 % B, Fließgeschwindigkeit 1 ml/min; 1,5 min 100 % B, Fließgeschwindigkeit 1 ml/min; 1,9 min 100 % B, Fließgeschwindigkeit 1 ml/min; 2,0 min 97 % A, 3 % B, Fließgeschwindigkeit 0,05 ml/min; Säulentemperatur: 40°C; UV-Detektion: von 210 nm bis 350 nm; MS-Bedingungen: Ionisierungs-Modus: alternierende Scans Positives und Negatives Elektrospray (ES+/ES-); Scan-Bereich: 100 bis 1000 AMU.

### Methode 23 (LC-MS):

Instrument: Acquity UPLC gekoppelt mit Quattro Micro Massenspektrometer; Säule: Acquity UPLC BEH C18 (50 mm x 2,1 mm ID, 1,7 µm Packungsdiameter); mobile Phase A: 0,1% Ameisensäure in Wasser, mobile Phase B: 0,1 % Ameisensäure in Acetonitril; Gradient: 0,0 min 97 % A, 3 % B, Fließgeschwindigkeit 1 ml/min; 1,5 min 100 % B, Fließgeschwindigkeit 1 ml/min; 1,9 min 100 % B, Fließgeschwindigkeit 1 ml/min; 2,0 min 97 % A, 3 % B, Fließgeschwindigkeit 0,05 ml/min; Säulentemperatur: 40°C; UV-Detektion: von 210 nm bis 350 nm; MS-Bedingungen: Ionisierungs-Modus: alternierende Scans Positives und Negatives Elektrospray (ES+/ES-); Scan-Bereich: 100 bis 1000 AMU.

### Methode 24 (LC-MS):

Instrument: Waters 2690, PDA-Detektor Waters 2996 gekoppelt mit Quattro Micro Massen-MS-Detektor; Säule: Waters SunFire C18 3,5 µm, 2,1x50 mm; mobile Phase A: 10 mM wässrige Ammoniumhydrogencarbonatlösung (mit Ammoniak auf einen pH-Wert von 10 eingestellt), mobile Phase B: Acetonitril; Gradient: 0,0 min 95 % A, 5 % B, Fließgeschwindigkeit 0,5 ml/min; 3,0 min 95 % A, 5 % B, Fließgeschwindigkeit 0,5 ml/min; 17,50 min 5 % A, 95 % B, Fließgeschwindigkeit 0,5 ml/min; 19,00 min 5 % A, 95 % B, Fließgeschwindigkeit 0,5 ml/min; 19,50 min 95 % A, 5 % B, Fließgeschwindigkeit 0,5 ml/min; 20,00 min 95 % A, 5 % B, Fließgeschwindigkeit 0,5 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich: 130 bis 1100 AMU.

### Methode 25 (LC-MS):

Instrument: Waters 2690, PDA-Detektor Waters 2996 gekoppelt mit Quattro Micro Massen-MS-Detektor; Säule: Waters SunFire C18 3,5 µm, 2,1x50 mm; mobile Phase A: 0,1% Ameisensäure in Wasser, mobile Phase B: 0,1% Ameisensäure in Acetonitril; Gradient: 0,0 min 95 % A, 5 % B, Fließgeschwindigkeit 0,5 ml/min; 3,0 min 95 % A, 5 % B, Fließgeschwindigkeit 0,5 ml/min; 17,50 min 5 % A, 95 % B, Fließgeschwindigkeit 0,5 ml/min; 19,00 min 5 % A, 95 % B, Fließgeschwindigkeit 0,5 ml/min; 19,50 min 95 % A, 5 % B, Fließgeschwindigkeit 0,5 ml/min; 20,00 min 95 % A, 5 % B, Fließgeschwindigkeit 0,5 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich:130 bis 1100 AMU.

### Methode 26 (präp. HPLC):

Instrument: Waters 2690, PDA-Detektor Waters 2996 gekoppelt mit Quattro Micro Massen-MS-Detektor; Säule: XBridge Prep. MS C18 OBD (150 mm x 30mm ID 5 µm Korngröße) bei Raumtemperatur; mobile Phase A: 10 mM NH₄HCO₃, mit Ammoniak auf einen pH-Wert von 10 eingestellt, mobile Phase B: Acetonitril; Gradient: 0,0 min 97 % A, 3 % B; 1,0 min 97 % A, 3 % B; 30 min 0 % A, 100 % B; 35 min 0 % A, 100 % B, Fließgeschwindigkeit 50 ml/min; Säulentemperatur: 30 °C; UV-Detektion: von 210 nm bis 400 nm; MS-Bedingungen: Ionisierungs-Modus: Scans Positives und Negatives Elektrospray (ES+/ES-); Scan-Bereich:100 bis 1000 AMU.

### Ausgangsverbindungen und Intermediate:

### Beispiel 1A

### 3-[(2,6-Difluorbenzyl)oxy]pyridin-2-amin

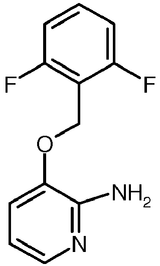

51 g Natriummethanolat (953 mmol, 1.05 Äquivalente) wurden in 1000 ml Methanol bei RT vorgelegt, mit 100 g 2-Amino-3-hydroxypyridin (908 mmol, 1 Äquivalent) versetzt und 15 min bei RT gerührt. Die Reaktionsmischung wurde am Vakuum eingeengt, der Rückstand in 2500 ml DMSO aufgenommen und mit 197 g 2,6-Difluorbenzylbromid (953 mmol, 1.05 Äquivalente) versetzt. Nach 4 h bei RT wurde das Reaktionsgemisch auf 20 L Wasser gegeben, für 15 min nachgerührt und der Feststoff abfiltriert. Der Feststoff wurde mit 1 L Wasser sowie 100 ml Iso-Propanol und 500 ml Petrolether gewaschen und im Hochvakuum getrocknet. Es wurden 171 g der Titelverbindung (78% d. Th) erhalten.
¹H-NMR (400 MHz, DMSO-d₆): δ = 5.10 (s, 2 H), 5.52 (br. s, 2 H), 6.52 (dd, 1 H), 7.16 - 7.21 (m, 3 H), 7.49 - 7.56 (m, 2 H).

### Beispiel 2A

### Ethyl-8-[(2,6-difluorbenzyl)oxy]-2-methylimidazo[1,2-a]pyridin-3-carboxylat

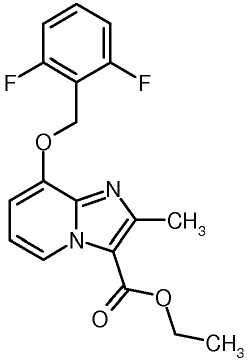

170 g 3-[(2,6-Difluorbenzyl)oxy]pyridin-2-amin (Beispiel 1A; 719 mmol, 1 Äquivalent) wurden in 3800 ml Ethanol vorgelegt, mit 151 g gepulvertem Molekularsieb 3Å und 623 g Ethyl-2-chloracetoacetat (3.6 mol, 5 Äquivalente) versetzt. Die Reaktionsmischung wurde für 24 h zum Rückfluß erhitzt, anschließend über Kieselgel abfiltriert und am Vakuum aufkonzentriert. Es wurde 48 h bei RT belassen und der enstandene Feststoff filtriert. Dann wurde dreimal mit wenig Iso-Propanol gerührt und anschließend abfiltriert und mit Diethylether gewaschen. Es wurden 60.8 g (23% d. Th.) der Titelverbindung erhalten. Die vereinigten Filtrate der Filtrationsschritte wurden eingeengt und der Rückstand an Kieselgel mit Cyclohexan/Diethylether als Eluent chromatographiert. Man erhielt weitere 46.5 g (18% d. Th.; Gesamtausbeute: 41% d. Th.) der Titelverbindung.
LC-MS (Methode 1): Rₜ = 1.01 min
MS (ESpos): m/z = 347 (M+H)⁺
¹H-NMR (400 MHz, DMSO-d₆): δ = 1.36 (t, 3 H), 2.54 (s, 3 H; verdeckt durch DMSO-Signal), 4.36 (q, 2 H), 5.33 (s, 2 H), 7.11 (t, 1 H), 7.18 - 7.27 (m, 3 H),7.59 (quint, 1 H), 8.88 (d, 1 H).

### Beispiel 3A

### 8-[(2,6-Difluorbenzyl)oxy]-2-methylimidazo[1,2-a]pyridin-3-carbonsäure

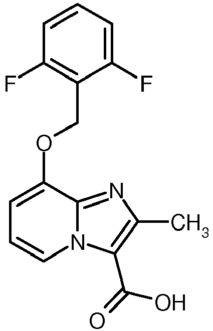

107 g Ethyl-8-[(2,6-difluorbenzyl)oxy]-2-methylimidazo[1,2-a]pyridin-3-carboxylat (Beispiel 2A; 300 mmol, 1 Äquivalent) wurden in 2.8 L THF/Methanol (1:1) gelöst, mit 1.5 L 1 N wässriger Lithiumhydroxid-Lösung (1.5 mol, 5 Äquivalente) versetzt und 16 h bei RT gerührt. Die organischen Lösemittel wurden am Vakuum entfernt und die resultierende wässrige Lösung im Eisbad mit 1 N wässriger Salzsäure auf pH 3-4 eingestellt. Der resultierende Feststoff wurde abfiltriert, mit Wasser und Iso-Propanol nachgewaschen und im Vakuum getrocknet. Es wurden 92 g (95% d. Th.) der Titelverbindung erhalten.
LC-MS (Methode 1): Rₜ = 0.62 min
MS (ESpos): m/z = 319.1 (M+H)⁺
¹H-NMR (400 MHz, DMSO-d₆): δ = 2.55 (s, 3 H; überlagert durch DMSO-Signal), 5.32 (s, 2 H); 7.01 (t, 1 H), 7.09 (d, 1 H), 7.23 (t, 2 H), 7.59 (quint, 1 H), 9.01 (d, 1 H).

### Beispiel 4A

### 3-(Cyclohexylmethoxy)pyridin-2-amin

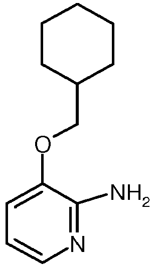

96 g Natriumhydroxid 45%ig in Wasser (1081 mmol, 1 Äquivalent) wurden in 1170 ml Methanol bei RT vorgelegt, mit 119 g 2-Amino-3-hydroxypyridin (1080 mmol, 1 Äquivalent) versetzt und 10 min bei RT weitergerührt. Die Reaktionsmischung wurde am Vakuum eingeengt, der Rückstand in 2900 ml DMSO aufgenommen und mit 101 g Cyclohexylmethylbromid (1135 mmol, 1.05 Äquivalente) versetzt. Nach 16 h bei RT wurde das Reaktionsgemisch langsam zu 6 L Wasser gegeben und die wässrige Lösung zweimal mit je 2 L Essigsäureethylester extrahiert. Die vereinigten organischen Phasen wurden mit je 1 L gesättigter, wässriger Natriumhydrogencarbonat-Lösung und Wasser gewaschen, getrocknet, filtriert und eingeengt. Der Rückstand wurde mit 500 ml n-Pentan gerührt, filtriert und am Vakuum getrocknet. Es wurden 130 g (58% d. Th.) der Titelverbindung erhalten.
LC-MS (Methode 3): Rₜ = 1.41 min
MS (ESpos): m/z = 207.1 (M+H)⁺

### Beispiel 5A

### Ethyl-8-(cyclohexylmethoxy)-2-methylimidazo[1,2-a]pyridin-3-carboxylat

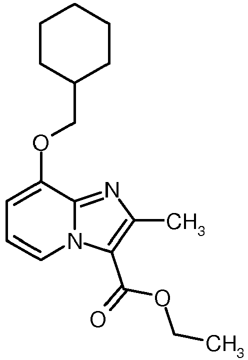

130 g 3-(Cyclohexylmethoxy)pyridin-2-amin (Beispiel 4A; 630 mmol, 1 Äquivalent) wurden in 3950 ml Ethanol vorgelegt und mit 436 ml Ethyl-2-chloracetoacetat (3.2 mol, 5 Äquivalente) versetzt. Es wurde 24 h am Rückfluß erhitzt und anschließend im Vakuum eingeengt. Das so erhaltene Rohprodukt wurde an Kieselgel mit Cyclohexan/Diethylether als Eluent chromatographiert und lieferte 66.2 g (33% d. Th.) der Titelverbindung.
LC-MS (Methode 1): Rₜ = 1.17 min
MS (ESpos): m/z = 317.1 (M+H)⁺
¹H-NMR (400 MHz, DMSO-d₆): δ = 1.02-1.31 (m, 5 H), 1.36 (t, 3 H), 1.64 - 1.77 (m, 3 H), 1.79 - 1.90 (m, 3 H), 2.60 (s, 3 H),3.97 (d, 2 H), 4.35 (q, 2 H), 6.95 (d, 1 H), 7.03 (t, 1 H), 8.81 (d, 1 H).

### Beispiel 6A

### 8-(Cyclohexylmethoxy)-2-methylimidazo[1,2-a]pyridin-3-carbonsäure

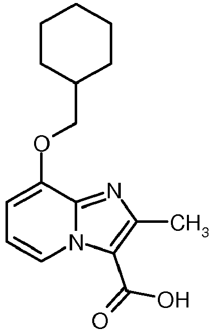

50 g Ethyl-8-(cyclohexylmethoxy)-2-methylimidazo[1,2-a]pyridin-3-carboxylat (Beispiel 5A; 158 mmol, 1 Äquivalent) wurde in 600 ml 1,4-Dioxan gelöst, mit 790 ml 2 N wässriger Natronlauge (1.58 mol, 10 Äquivalente) versetzt und 16 h bei RT gerührt. Es wurde mit 316 ml 6 N wässriger Salzsäure versetzt und auf ca. 1/5 des Gesamtvolumens eingeengt. Der resultierende Feststoff wurde abfiltriert, mit Wasser und tert.-Butylmethylether nachgewaschen und im Vakuum getrocknet. Es wurden 35 g (74% d. Th.) der Titelverbindung erhalten.
LC-MS (Methode 1): Rₜ = 0.81 min
MS (ESpos): m/z = 289.0 (M+H)⁺
¹H-NMR (400 MHz, DMSO-d₆): δ = 1.03-1.44 (m, 5 H), 1.64 - 1.78 (m, 3 H), 1.81 - 1.92 (m, 3 H), 2.69 (s, 3 H), 4.07 (d, 2 H), 7.30 - 7.36 (m, 2 H), 9.01 (d, 1 H).

### Beispiel 7A

### 5-Chlor-2-nitropyridin-3-ol

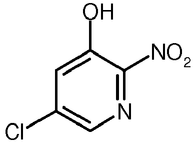

30 g 5-Chlorpyridin-3-ol (232 mmol, 1 Äquivalent) wurden unter Eiskühlung in 228 ml konzentrierter Schwefelsäure gelöst und bei 0 °C langsam mit 24 ml konzentrierter Salpetersäure versetzt. Es wurde auf RT erwärmt, über Nacht gerührt und anschließend in ein Eis/WasserGemisch eingerührt und für 30 min nachgerührt. Der Feststoff wurde abfiltriert, mit kaltem Wasser nachgewaschen und an der Luft getrocknet. Es wurden 33 g (82% d. Th.) der Titelverbindung erhalten und ohne weitere Aufreinigung in die Folgereaktion eingesetzt.
LC-MS (Methode 1): Rₜ = 0.60 min
MS (ESneg): m/z = 172.9/174.9 (M-H)⁻
¹H-NMR (400 MHz, DMSO-d₆): δ = 7.71 (d, 1 H), 8.10 (d, 1 H), 12.14 (br. 1 H).

### Beispiel 8A

### 5-Chlor-3-[(2,6-difluorbenzyl)oxy]-2-nitropyridin

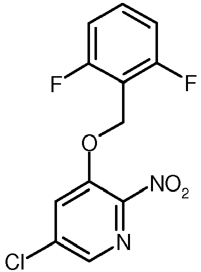

33 g 5-Chlor-2-nitropyridin-3-ol (Beispiel 7A; 189 mmol, 1 Äquivalent) und 61.6 g Cäsiumcarbonat (189 mmol, 1 Äquivalent) wurden in 528 ml DMF vorgelegt, mit 40.4 g 2,6-Difluorbenzylbromid (189 mmol, 1 Äquivalent) versetzt und bei RT über Nacht gerührt. Das Reaktionsgemisch wurde in Wasser/1N wässrige Salzsäure eingerührt. Der Feststoff wurde abfiltriert, mit Wasser gewaschen und an der Luft getrocknet. Es wurden 54.9 g (97% d. Th.) der Titelverbindung erhalten.
¹H-NMR (400 MHz, DMSO-d₆): δ = 5.46 (s, 2 H), 7.22 (t, 2 H), 7.58 (q, 1 H), 8.28 (d, 1 H), 8.47 (d, 1H).

### Beispiel 9A

### 5-Chlor-3-[(2,6-difluorbenzyl)oxy]pyridin-2-amin

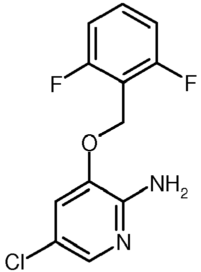

59.7 g 5-Chlor-3-[(2,6-difluorbenzyl)oxy]-2-nitropyridin (Beispiel 8A; 199 mmol, 1 Äquivalent) wurden in 600 ml Ethanol vorgelegt, mit 34.4 g Eisenpulver (616 mmol, 3.1 Äquivalente) versetzt und zum Rückfluß erhitzt. Es wurden langsam 152 ml konzentrierte Salzsäure zugetropft und weitere 30 min am Rückfluß gekocht. Das Reaktionsgemisch wurde abgekühlt und in ein Eis-Wassergemisch eingerührt. Das resultierende Gemisch wurde mit Natriumacetat auf pH 5 eingestellt. Der Feststoff wurde abfiltriert, mit Wasser nachgewaschen und an der Luft und anschließend im Vakuum bei 50°C getrocknet. Es wurden 52.7 g (98% d. Th.) der Titelverbindung erhalten.
LC-MS (Methode 1): Rₜ = 0.93 min
MS (ESpos): m/z = 271.1/273.1 (M+H)⁺
¹H-NMR (400 MHz, DMSO-d₆): δ = 5.14 (s, 2 H), 5.82 (br. s, 2 H),; 7.20 (t, 2 H),7.35 (d, 1 H), 7.55 (q, 1 H),7.56 (d, 1 H).

### Beispiel 10A

### Ethyl-6-chlor-8-[(2,6-difluorbenzyl)oxy]-2-methylimidazo[1,2-a]pyridin-3-carboxylat

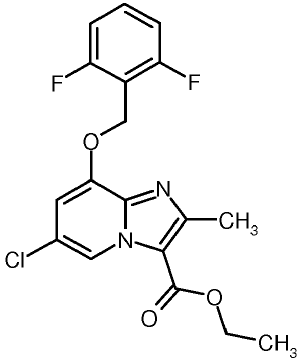

40 g 5-Chlor-3-[(2,6-difluorbenzyl)oxy]pyridin-2-amin (Beispiel 9A; 147.8 mmol; 1 Äquivalent) wurden in 800 ml Ethanol vorgelegt, mit 30 g gepulvertem Molekularsieb 3Å und 128 g Ethyl-2-chloracetoacetat (739 mmol, 5 Äquivalente) versetzt und über Nacht zum Rückfluß erhitzt. Das Reaktionsgemisch wurde eingeengt, der Rückstand in Essigsäureethylester aufgenommen und filtriert. Die Essigsäureethylester-Phase wurde mit Wasser gewaschen, getrocknet, filtriert und eingeengt. Es wurden 44 g (78% d. Th.) der Titelverbindung erhalten.
LC-MS (Methode 1): Rₜ = 1.27 min
MS (ESpos): m/z = 381.2/383.2 (M+H)⁺
¹H-NMR (400 MHz, DMSO-d₆): δ = 1.36 (t, 3 H), 2.54 (s, 3 H; verdeckt durch DMSO-Signal); 4.37 (q, 2 H), 5.36 (s, 2 H), 7.26 (t, 2 H), 7.38 (d, 1 H), 7.62 (q, 1 H), 8.92 (d, 1 H).

### Beispiel 11A

### 6-Chlor-8-[(2,6-difluorbenzyl)oxy]-2-methylimidazo[1,2-a]pyridin-3-carbonsäure

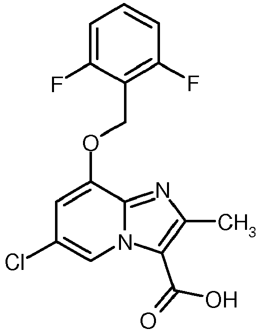

44 g Ethyl-6-chlor-8-[(2,6-difluorbenzyl)oxy]-2-methylimidazo[1,2-a]pyridin-3-carboxylat (Beispiel 10A; 115 mmol, 1 Äquivalent) wurden in 550 ml THF und 700 ml Methanol gelöst, mit 13.8 g Lithiumhydroxid (gelöst in 150 ml Wasser; 577 mmol, 5 Äquivalente) versetzt und über Nacht bei RT gerührt. Es wurde mit 1 N wässriger Salzsäure versetzt und im Vakuum eingeengt. Der erhaltene Feststoff wurde abfiltriert und mit Wasser nachgewaschen. Es wurden 34 g der Titelverbindung (84% d. Th.) erhalten.
LC-MS (Methode 2): Rₜ = 1.03 min
MS (ESpos): m/z = 353.0/355.0 (M+H)⁺
¹H-NMR (400 MHz, DMSO-d₆): δ = 2.54 (s, 3 H; überlagert von DMSO-Signal), 5.36 (s, 2 H), 7.26 (t, 2 H), 7.34 (d, 1 H), 7.61 (q, 1 H), 8.99 (d, 1 H), 13.36 (br. s, 1 H).

### Beispiel 12A

### 5-Brom-3-[(2,6-difluorbenzyl)oxy]pyridin-2-amin

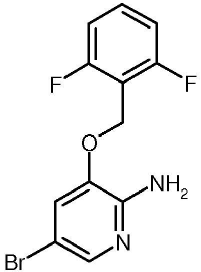

32.6 g 3-[(2,6-Difluorbenzyl)oxy]pyridin-2-amin (Beispiel 1A; 138 mmol, 1 Äquivalent) wurden in 552 ml 10%iger Schwefelsäure suspendiert und auf 0°C gekühlt. 8.5 ml Brom (165 mmol, 1.2 Äquivalente) wurde in 85 ml Essigsäure gelöst und dann innerhalb von 90 min zur eisgekühlten, Reaktionslösung getropft. Nach erfolgter Zugabe wurde 90 min bei 0°C -gerührt, anschließend mit 600 ml Essigsäureethylester verdünnt und die wässrige Phase abgetrennt. Die wässrige Phase wurde mit Essigsäureethylester extrahiert. Die organischen Phasen wurden vereinigt, mit gesättigter, wässriger Natriumhydrogencarbonat-Lösung gewaschen, getrocknet und eingeengt. Der Rückstand wurde in Dichlormethan gelöst und an Kieselgel chromatographiert (Petrolether/Essigsäureethylester Gradient als Eluent). Es wurden 24 g (55% d. Th.) der Titelverbindung erhalten.
LC-MS (Methode 1): Rₜ = 0.96 min
MS (ESpos): m/z = 315.1/317.1 (M+H)⁺
¹H-NMR (400 MHz, DMSO-d₆): δ = 5.14 (s, 2 H),5.83 (br. s, 2 H), 7.20 (t, 2 H), 7.42 (d, 1 H), 7.54 (q, 1 H), 7.62 (d, 1 H).

### Beispiel 13A

### Ethyl-6-brom-8-[(2,6-difluorbenzyl)oxy]-2-methylimidazo[1,2-a]pyridin-3-carboxylat

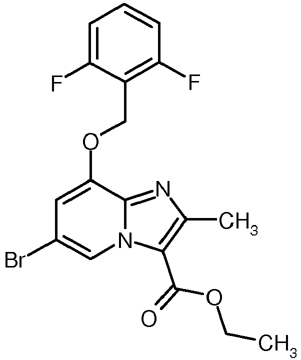

24 g 5-Brom-3-[(2,6-difluorbenzyl)oxy]pyridin-2-amin (Beispiel 12A; 76.2 mmol; 1 Äquivalent) in 400 ml Ethanol wurden mit 16 g gepulvertem Molekularsieb 3Å und 52.7 ml Ethyl-2-chloracetoacetat (380.8 mmol; 5 Äquivalente) versetzt und über Nacht zum Rückfluß erhitzt. Es wurden 8 g Molekularsieb zugegeben und für weitere 24 h zum Rückfluß erhitzt. Das Reaktionsgemisch wurde im Vakuum eingeengt, der Rückstand in Dichlormethan aufgenommen und an Kieselgel chromatographiert (Dichlormethan/Methanol 20:1 als Eluent). Die produkthaltigen Fraktionen wurden eingeengt und der Rückstand mit 100 ml Diethylether 30 min gerührt. Dann wurde abfiltriert, mit wenig Diethylether gewaschen und getrocknet. Es wurden 15 g (45% d. Th.) der Titelverbindung erhalten.
LC-MS (Methode 2): Rₜ = 1.43 min
MS (ESpos): m/z = 414.9/416.8 (M+H)⁺
¹H-NMR (400 MHz, DMSO-d₆): δ = 1.36 (t, 3 H), 2.54 (s, 3 H; verdeckt durch DMSO-Signal), 4.37 (q, 2 H), 5.36 (s, 2 H), 7.25 (t, 2 H), 7.42 (d, 1 H), 7.61 (q, 1 H),9.00 (d, 1 H).

### Beispiel 14A

### 6-Brom-8-[(2,6-difluorbenzyl)oxy]-2-methylimidazo[1,2-a]pyridin-3-carbonsäure

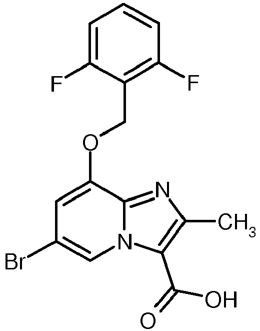

1.5 g Ethyl-6-brom-8-[(2,6-difluorbenzyl)oxy]-2-methylimidazo[1,2-a]pyridin-3-carboxylat (Beispiel 13A; 3.5 mmol, 1 Äquivalent) wurden in 72 ml THF/Methanol 5:1 gelöst, mit 17.6 ml IN wässriger Lithiumhydroxid-Lösung (17.6 mmol, 5 Äquivalente) versetzt, auf 40°C erwärmt und für 6 h bei dieser Temperatur gerührt. Dann wurde mit 6 N wässriger Salzsäure auf pH 4 gestellt und im Vakuum eingeengt. Der enstandene Feststoff wurde mit Wasser versetzt, -gerührt, abfiltriert, mit Wasser nachgewaschen und im Vakuum getrocknet. Es wurden 1.24 g der Titelverbindung (88% d. Th.) erhalten.
LC-MS (Methode 1): Rₜ = 0.93 min
MS (ESpos): m/z = 397.0/399.1 (M+H)⁺
¹H-NMR (400 MHz, DMSO-d₆): δ = 2.54 (s, 3 H; überlagert von DMSO-Signal); 5.36 (s, 2 H); 7.25 (t, 2 H); 7.40 (d, 1 H); 7.61 (q, 1 H); 9.06 (d, 1 H); 13.35 (br. s, 1 H).

### Beispiel 15A

### Ethyl-8-[(2,6-difluorbenzyl)oxy]-2,6-dimethylimidazo[1,2-a]pyridin-3-carboxylat

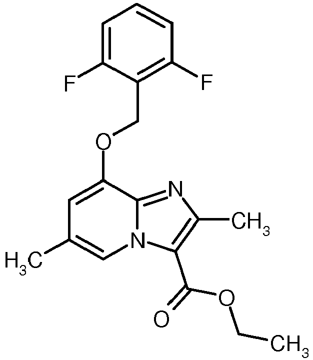

### Methode 1:

600 mg Ethyl-6-brom-8-[(2,6-difluorbenzyl)oxy]-2-methylimidazo[1,2-a]pyridin-3-carboxylat (Beispiel 13A; 1.4 mmol, 1 Äquivalent) und 230 mg 1,1'-Bis(diphenylphosphino)ferrocene-palladium(II)dichlorid dichloromethan Komplex (0.282 mmol, 20 mol%) wurden in 25 ml THF gelöst und mit 0.88 ml (1.76 mmol, 1.2 Äquivalente) einer 2 M Lösung von Methylzinkchlorid in THF versetzt. Die Reaktionsmischung wurde in der Mikrowelle für 40 min auf 100°C erhitzt. Die Reaktionsmischung wurde über Celite filtriert und anschließend im Vakuum eingeengt. Der Rückstand wurde chromatographiert (Biotage Isolera Four; Cyclohexan:Essigsäurethylester). Es wurden 225 mg (38% d. Th.) der Titelverbindung erhalten.

### Methode 2:

20.00 g (85.38 mmol) Ethyl-8-hydroxy-2,6-dimethylimidazo[1,2-a]pyridin-3-carboxylat aus Beispiel 20A, 19.44 g (93.91 mmol), 2,6-Difluorbenzylbromid und 61.20 g (187.83 mmol) Cäsiumcarbonat in 1.18 L DMF wurden 5 h bei 60°C gerührt. Dann wurde das Reaktionsgemisch auf 6.4 L 10%ige wässrige Natriumchlorid-Lösung gegeben und anschließend zweimal mit Essigsäureethylester extrahiert. Die vereinigten organischen Phasen wurden mit 854 ml 10%iger wässriger Natriumchlorid-Lösung gewaschen, getrocknet, eingeengt und über Nacht im Hochvakuum bei RT getrocknet. Es wurden 28.2 g (92% d. Th.; Reinheit 90%) der Titelverbindung erhalten.
LC-MS (Methode 1): Rₜ = 1.05 min
MS (ESpos): m/z = 361.1 (M+H)⁺
¹H-NMR (400 MHz, DMSO-d₆): δ = 1.38 (t, 3 H), 2.36 (s, 3 H), 4.35 (q, 2 H), 5.30 (s, 2 H), 7.10 (s, 1 H), 7.23 (t, 2 H), 7.59 (q, 1 H), 8.70 (s, 1 H).

### Beispiel 16A

### 8-[(2,6-Difluorbenzyl)oxy]-2,6-dimethylimidazo[1,2-a]pyridin-3-carbonsäure

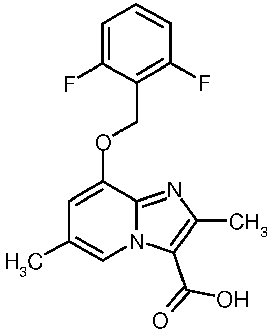

220 mg Ethyl-8-[(2,6-difluorbenzyl)oxy]-2,6-dimethylimidazo[1,2-a]pyridin-3-carboxylat (Beispiel 15A; 0.524 mmol, 1 Äquivalent) wurden in 7 ml THF/Methanol 1:1 gelöst, mit 2.6 ml 1 N wässriger Lithiumhydroxid-Lösung (2.6 mmol, 5 Äquivalente) versetzt und für 16 h bei RT gerührt. Es wurde im Vakuum eingeengt, der Rückstand mit IN wässriger Salzsäure sauer gestellt und 15 min gerührt. Der Feststoff wurde abfiltriert, mit Wasser nachgewaschen und im Vakuum getrocknet. Es wurden 120 mg der Titelverbindung (60% d. Th.) erhalten.
LC-MS (Methode 1): Rₜ = 0.68 min
MS (ESpos): m/z = 333.1 (M+H)⁺
¹H-NMR (400 MHz, DMSO-d₆): δ = 2.34 (s, 3 H), 5.28 (s, 2 H), 7.09 (s, 1 H), 7.23 (t, 2 H), 7.58 (q, 1 H), 8.76 (s, 1 H), 13.1 (br. s, 1 H).

### Beispiel 17A

### 3-(Benzyloxy)-5-brompyridin-2-amin

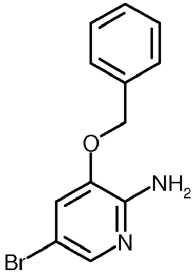

Die Zielverbindung ist literaturbekannt und beschrieben:
1) Palmer, A.M. et al. J Med. Chem. 2007, 50, 6240-6264.
2) ALTANA WO2005/58325
3) ALTANA WO2005/90358
4) Cui, J.T. et al. J Med. Chem. 2011, 54, 6342-6363

### Weitere Herstellungsmethode:

200 g (1 mol) 2-Amino-3-benzyloxypyridin wurden in 4 1 Dichlormethan vorgelegt und bei 0°C innerhalb von 30 min mit einer Lösung aus 62 ml (1.2 mol) Brom in 620 ml Dichlormethan versetzt. Nach beendeter Zugabe wurde die Reaktionslösung 60 min bei 0°C gerührt. Dann wurde das Gemisch mit ca. 4 1 gesättigter, wässriger Natriumhydrogencarbonat-Lösung versetzt. Die organische Phase wurde abgetrennt und eingeengt. Der Rückstand wurde mittels Kieselgelsäulechromatographie (Petrolether:Essigsäurethylester 6:4) gereinigt und die Produktfraktionen wurden eingeengt. Man erhielt 214 g (77% d. Th.) der Titelverbindung.
LC-MS (Methode 1): Rₜ = 0.92 min
MS (ESpos): m/z = 279 (M+H)⁺
¹H-NMR (400 MHz, DMSO-d₆): δ = 5.16 (s, 2H), 5.94 - 6.00 (m, 2H), 7.26 - 7.29 (m, 1H), 7.31 - 7.36 (m, 1H), 7.37 - 7.43 (m, 2H), 7.47-7.52 (m, 2H), 7.57 - 7.59 (m, 1H).

### Beispiel 18A

### Ethyl-8-(benzyloxy)-6-brom-2-methylimidazo[1,2-a]pyridin-3-carboxylat

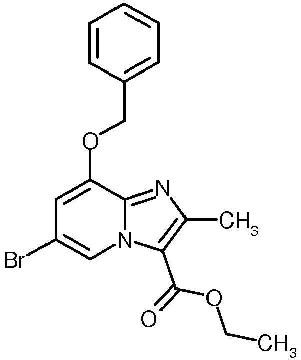

Unter Argon wurden 200 g (0.72 mol) 3-(Benzyloxy)-5-brompyridin-2-amin aus Beispiel 17A, 590 g (3.58 mol) Ethyl-2-chloracetoacetat und 436 g 3A Molekularsieb in 6 1 Ethanol suspendiert und 72 h bei Rückfluß gerührt. Die Reaktionsmischung wurde über Kieselgel abfiltriert und eingeengt. Der Rückstand wurde mittels Kieselgelchromatographie (Petrolether:Essigsäureethylester 9:1, anschließend 6:4) gereinigt und die Produktfraktionen wurden eingeengt. Man erhielt 221 g (79% d. Th.) der Zielverbindung.
LC-MS (Methode 16): Rₜ = 1.31 min
MS (ESpos): m/z = 389 (M+H)⁺
¹H-NMR (400 MHz, DMSO-d₆): δ = 1.36 (t, 3 H), 2.58 (s, 3 H), 4.32 - 4.41 (m, 2 H), 5.33 (s, 2 H), 7.28 - 7.32 (m, 1 H), 7.36 - 7.47 (m, 3 H), 7.49 - 7.54 (m, 2 H), 8.98 (d, 1 H).

### Beispiel 19A

### Ethyl-8-(benzyloxy)-2,6-dimethylimidazo[1,2-a]pyridin-3-carboxylat

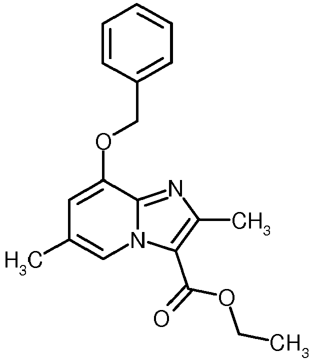

105 g (270 mmol) Ethyl-8-(benzyloxy)-6-brom-2-methylimidazo[1,2-a]pyridin-3-carboxylat aus Beispiel 18A wurden unter Argon in 4.2 1 1,4-Dioxan suspendiert und nacheinander mit 135.4 g (539 mmol, Reinheit 50%) Trimethylboroxin, 31.2 g (27 mmol) Tetrakis-(triphenylphosphin)palladium(0) sowie 78.3 g (566 mmol) Kaliumcarbonat versetzt und 8 h unter Rückfluss gerührt. Die auf RT abgekühlte Reaktionsmischung wurde über Kieselgel vom Niederschlag abfiltriert und das Filtrat wurde eingeengt. Der Rückstand wurde in Dichlormethan gelöst und mittels Kieselgelchromatographie (Dichlormethan:Essigsäureethylester = 9:1) gereinigt. Man erhielt 74 g (84.6% d. Th.; Reinheit 100%) der Zielverbindung.
LC-MS (Methode 16): Rₜ = 1.06 min
MS (ESpos): m/z = 325 (M+H)⁺
¹H-NMR (400 MHz, DMSO-d₆): δ = 1.35 (t, 3 H), 2.34 (br. s, 3 H), 2.56 (s, 3 H), 4.31 - 4.38 (m, 2 H), 5.28 (br. s, 2 H), 6.99 - 7.01 (m, 1 H), 7.35 - 7.47 (m, 3 H), 7.49 - 7.54 (m, 2 H), 8.68 - 8.70 (m, 1 H).

### Beispiel 20A

### Ethyl-8-hydroxy-2,6-dimethylimidazo[1,2-a]pyridin-3-carboxylat

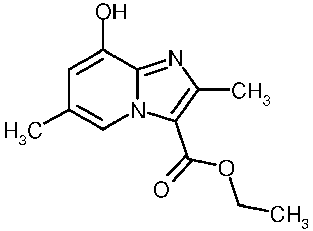

74 g (228 mmol) Ethyl-8-(benzyloxy)-2,6-dimethylimidazo[1,2-a]pyridin-3-carboxylat aus Beispiel 19A wurden in 1254 ml Dichlormethan und 251 ml Ethanol vorgelegt und unter Argon mit 20.1 g 10%igem Palladium auf Aktivkohle (wasserfeucht 50%) versetzt. Das Reaktionsgemisch wurde über Nacht bei RT und Normaldruck hydriert. Die Reaktionsmischung wurde über Kieselgel abfiltriert und eingeengt. Der Rohprodukt wurde mittels Kieselgel-chromatographie (Dichlormethan:Methanol = 95:5) gereinigt. Man erhielt 50.4 g (94% d. Th.) der Zielverbindung.
DCI-MS: (Methode 13) (ESpos): m/z = 235.2 (M+H)⁺
¹H-NMR (400 MHz, DMSO-d₆): δ = 1.35 (t, 3 H), 2.27 (s, 3 H), 2.58 (s, 3 H), 4.30 - 4.38 (m, 2 H), 6.65 (d, 1 H), 8.59 (s, 1 H), 10.57 (br. s, 1H).

### Beispiel 21A

### Ethyl-2,6-dimethyl-8-[(2,3,6-trifluorbenzyl)oxy]imidazo[1,2-a]pyridin-3-carboxylat

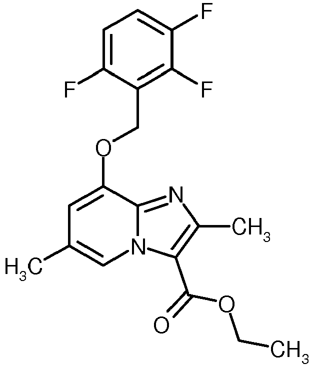

3.00 g (12.81 mmol) Ethyl-8-hydroxy-2,6-dimethylimidazo[1,2-a]pyridin-3-carboxylat Beispiel 20A, 3.27 g (14.1 mmol) 2-(Brommethyl)-1,3,4-trifluorbenzol und 9.18 g (28.17 mmol) Cäsiumcarbonat wurden in 183 ml trockenem DMF vorgelegt und für 30 min in einem auf 60°C erwärmten Ölbad erhitzt. Dann wurde mit ca. 1.8 1 Wasser versetzt und 30 min gerührt. Der Feststoff wurde abfiltriert, mit Wasser gewaschen und im Vakuum getrocknet. Es wurden 5.07 g der Titelverbindung (99% d. Th.) erhalten.
LC-MS (Methode 1): Rₜ = 1.14 min
MS (ESpos): m/z = 379 (M+H)⁺
¹H-NMR (400 MHz, DMSO-d₆): δ = 1.35 (t, 3 H), 2.36 (s, 3 H); 2.55 (s, 3 H; überlagert durch DMSO-Signal), 4.36 (q, 2 H), 5.35 (s, 2 H), 7.09 (s, 1 H), 7.22 - 7.32 (m, 1 H), 7.60 - 7.73 (m, 1 H), 8.72 (s, 1 H).

### Beispiel 22A

### 2,6-Dimethyl-8-[(2,3,6-trifluorbenzyl)oxy]imidazo[1,2-a]pyridin-3-carbonsäure

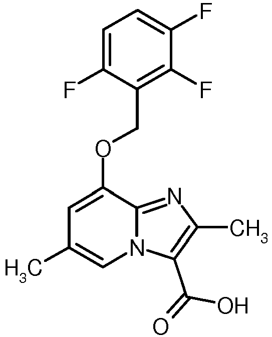

5.07 g (12.87 mmol) Ethyl-2,6-dimethyl-8-[(2,3,6-trifluorbenzyl)oxy]imidazo[1,2-a]pyridin-3-carboxylat Beispiel 21A wurden in 275 ml THF/Methanol (5/1) gelöst, mit 64.4 ml 1 N wässriger Lithiumhydroxid-Lösung versetzt und 3.5 h bei 40°C gerührt. Es wurde bei 0°C mit 6 N wässriger Salzsäure auf ca. pH 4 gebracht und dann eingeengt. Der erhaltene Feststoff wurde abfiltriert, mit Wasser nachgewaschen und im Vakuum getrocknet. Es wurden 4.77 g (98% d. Th.; Reinheit ca. 93%) der Titelverbindung erhalten.
LC-MS (Methode 1): Rₜ = 0.72 min
MS (ESpos): m/z = 351 (M+H)⁺
¹H-NMR (400 MHz, DMSO-d₆): δ = 2.37 (s, 3 H), 2.54 (s, 3 H; überlagert durch DMSO-Signal), 5.36 (s, 2 H), 7.11 (s, 1 H), 7.25 - 7.33 (m, 1 H), 7.61 - 7.73 (m, 1 H), 8.78 (s, 1 H), 13.10 (br. s, 1 H).

### Beispiel 23A

### Ethyl-8-[(3-fluorpyridin-2-yl)methoxy]-2,6-dimethylimidazo[1,2-a]pyridin-3-carboxylat

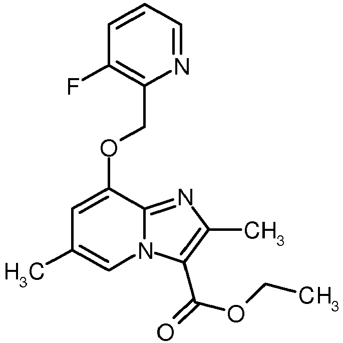

16.92 g (72.2 mmol) Ethyl-8-hydroxy-2,6-dimethylimidazo[1,2-a]pyridin-3-carboxylat aus Beispiel 20A wurden in 956 ml DMF vorgelegt und mit 15.78 g (86.7 mmol) 2-(Chlormethyl)-3-fluorpyridinhydrochlorid (beschrieben in: US5593993 A1, 1997; WO2007/2181 A2, 2007) sowie 94.06 g (288.9 mmol) Cäsiumcarbonat versetzt. Das Reaktionsgemisch wurde über Nacht bei 60°C gerührt. Das auf RT abgekühlte Reaktionsgemisch wurde filtriert, mit Essigsäureethylester gewaschen und das Filtrat eingeengt. Der Rückstand wurde mit ca. 500 ml Wasser versetzt, der Feststoff abfiltriert und im Hochvakuum getrocknet. Man erhielt 24.1 g (93% d. Th.) der Zielverbindung.
LC-MS (Methode 1): Rₜ = 0.84 min
MS (ESpos): m/z = 344 (M+H)⁺
¹H-NMR (400 MHz, DMSO-d₆): δ = 1.35 (t, 3H), 2.35 (s, 3H), 2.54 (s, 3H, verdeckt vom DMSO-Signal), 4.35 (q, 2H), 5.40 (s, 2H), 7.08 (s, 1H), 7.55 - 7.62 (m, 1H), 7.82 - 7.89 (m, 1H), 8.48 - 8.52 (m, 1H), 8.70 (s, 1H).

### Beispiel 24A

### 8-[(3-Fluorpyridin-2-yl)methoxy]-2,6-dimethylimidazo[1,2-a]pyridin-3-carbonsäure

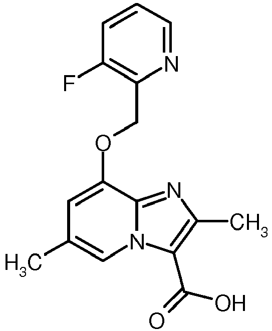

24.06 g (70.1 mmol) Ethyl-8-[(3-fluorpyridin-2-yl)methoxy]-2,6-dimethylimidazo[1,2-a]pyridin-3-carboxylat aus Beispiel 23A wurden in 1.5 1 THF/Methanol (5:1) vorgelegt, mit 350.4 ml (350.4 mmol) 1 N wässriger Lithiumhydroxid-Lösung versetzt und 2.5 h bei 40°C gerührt. Nach dem Abkühlen wurde mit 1 N wässriger Salzsäure auf ca. pH 4 gebracht und die Lösung wurde im Vakuum von THF/Methanol befreit. Der Rückstand wurde abgekühlt, der Feststoff abfiltriert und im Vakuum getrocknet. Es wurden 22.27 g (100% d. Th.) der Titelverbindung erhalten.
LC-MS (Methode 1): Rₜ = 0.55 min
MS (ESpos): m/z = 316 (M+H)⁺
¹H-NMR (400 MHz, DMSO-d₆): δ = 2.34 (s, 3H), 2.53 (s, 3H, verdeckt vom DMSO-Signal), 5.38 - 5.42 (m, 2H), 7.06 (s, 1H), 7.56 - 7.62 (m, 1H), 7.82 - 7.89 (m, 1H), 8.48 - 8.52 (m, 1H), 8.74 (s, 1H), 13.02 (br. s, 1H).

### Beispiel 25A

### 8-[(2,6-Difluorbenzyl)oxy]-2-methylimidazo[1,2-a]pyridin-3-carbonylchlorid-Hydrochlorid

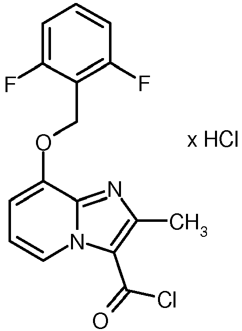

Zu 2.0 g 8-[(2,6-Difluorbenzyl)oxy]-2-methylimidazo[1,2-a]pyridin-3-carbonsäure (6.28 mmol, 1 Äquivalent) aus Beispiel 3A in 25 ml trockenem THF wurden 4 Tropfen DMF und anschließend 3.19 g Oxalsäuredichlorid (25.14 mmol, 4 Äquivalente) getropft. Das Reaktionsgemisch wurde 3 h bei RT gerührt. Es wurden nochmals 0.80 g Oxalsäuredichlorid (6.28 mmol, 1 Äquivalent) zugegeben und die Reaktion wurde weitere 4 h bei RT gerührt. Das Reaktionsgemisch wurde eingeengt, dreimal mit Toluol abgedampft und der Rückstand wurde im Hochvakuum getrocknet. Es wurden 2.43 g der Titelverbindung (103% d. Th.) erhalten.
DCI-MS (Methode 13): MS (ESpos): m/z = 437 (M-HCl+H)⁺

### Beispiel 26A

### 8-(Cyclohexylmethoxy)-2-methylimidazo[1,2-a]pyridin-3-carbonylchlorid

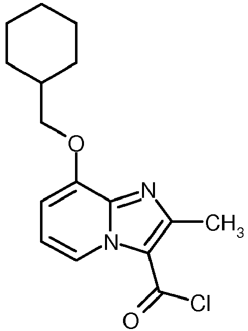

2.8 g 8-(Cyclohexylmethoxy)-2-methylimidazo[1,2-a]pyridin-3-carbonsäure (Beispiel 6A, 9.6 mmol) wurden in 60 ml Thionylchlorid gelöst und über Nach bei 80°C gerührt. Dann wurde im Vakuum eingeengt, in Toluol gelöst und erneut eingeengt. Der Rückstand wurde am Hochvakuum getrocknet. Man erhielt 2.9 g (98% d. Th.) der Titelverbindung. Das erhaltene Rohprodukt wurde ohne Reinigung weiter umgesetzt.

### Beispiel 27A

### 8-[(2,6-Difluorbenzyl)oxy]-2-methylimidazo[1,2-a]pyridin

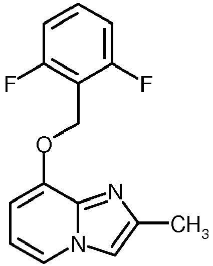

12 g 3-[(2,6-Difluorbenzyl)oxy]pyridin-2-amin (Beispiel 1A, 50.8 mmol, 1 Äquivalent) und 8 g 1-Chloraceton (86.4 mmol, 1.7 Äquivalente) in 90 ml Ethanol wurden über Nacht bei 80°C gerührt. Das Reaktionsgemisch wurde mit Kieselgel versetzt und eingeengt. Den Rückstand wurde mittels Kieselgelchromatographie (Laufmittelgemisch Dichlormethan/Ethanol = 50:1) gereinigt. Das erhaltene Produktgemisch wurde anschließend mittels Kieselgelchromatographie (Laufmittelgemisch Dichlormethan/Ethanol/Diethylamin = 50:1:0.1, 40:1:0.5, 30:1:0.5) gereinigt. Man erhielt 6.3 g (45% d. Th.) der Titelverbindung.
LC-MS (Methode 1): Rₜ = 0.58 min
MS (ESpos): m/z = 274 (M+H)⁺
¹H-NMR (400 MHz, DMSO-d₆): δ = 2.27 (s, 3 H), 5.27 (s, 2 H), 6.69 - 6.80 (m, 2 H), 7.23 (s, 2 H), 7.51 - 7.62 (m, 1 H), 7.65 (s, 1 H), 8.03 - 8.12 (m, 1 H).

### Beispiel 28A

### 3-Brom-8-[(2,6-difluorbenzyl)oxy]-2-methylimidazo[1,2-a]pyridin

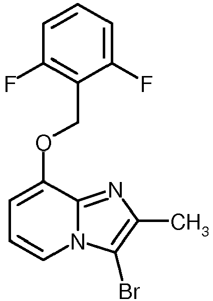

193 g 8-[(2,6-Difluorbenzyl)oxy]-2-methylimidazo[1,2-a]pyridin (Beispiel 27A, 0.7 mmol, 1 Äquivalent) wurden in 2.2 L Ethanol vorgelegt und mit 150.3 g N-Bromsuccinimid (0.8 mmol, 1.2 Äquivalente) versetzt. Nach 1.5 h bei RT wurde bei RT im Vakuum eingeengt. Der Rückstand wurde mit Essigsäureethylester verdünnt, die organische Phase wurde mit gesättigter wässriger Natriumhydrogencarbonat-Lösung gewaschen, über Natriumsulfat getrocknet, filtriert und einrotiert. Den Rückstand wurde mittels Kieselgelchromatographie (Laufmittelgemisch Cyclohexan/Essigsäureethylester = 98:2, 96:4, 92:8, 9:1, 8:2 und 7:3) gereinigt. Das erhaltene Produkt wurde mit 600 ml Essigsäureethylester gerührt und es wurde abdekantiert. Der Rückstand wurde im Vakuum getrocknet. Es wurden 23.4 g (9% d. Th.) der Titelverbindung erhalten.
Das Filtrat wurde im Vakuum eingeengt und der Rückstand mit 100 ml Essigsäureethylester gerührt. Die Essigsäureethylester Phase wurde abdekantiert und der Rückstand im Vakuum getrocknet. Es wurden weitere 6.1g (2.3% d. Th.) der Titelverbindung erhalten.
LC-MS (Methode 1): Rₜ = 0.90 min
MS (ESpos): m/z = 353 (M+H)⁺
¹H-NMR (400 MHz, DMSO-d₆): δ = 2.27 (s, 3 H), 5.27 (s, 2 H), 6.70 - 6.80 (m, 2 H), 7.23 (t, 2 H), 7.52 - 7.62 (m, 1 H), 7.65 (s, 1 H), 8.09 (d, 1 H).

### Beispiel 29A

### 8-[(2,6-Difluorbenzyl)oxy]-2,6-dimethylimidazo[1,2-a]pyridin

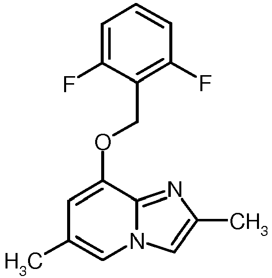

10.0 g (30.09 mmol) 8-[(2,6-Difluorbenzyl)oxy]-2,6-dimethylimidazo[1,2-a]pyridin-3-carbonsäure aus Beispiel 16A wurden in 228 ml Dioxan vorgelegt, mit 25.1 ml 6 N wässriger Salzsäure-Lösung versetzt und 2 h bei 100°C gerührt. Nach Abkühlen wurde Dioxan im Vakuum entfernt und der wässrige Rückstand mit 2 N wässriger Natronlauge auf pH 8 gebracht. Der erhaltene Feststoff wurde abfiltriert, mit Wasser gewaschen und im Hochvakuum getrocknet. Es wurden 8.97g der Zielverbindung (97% d. Th., Reinheit 94%) erhalten.
LC-MS (Methode 1): Rₜ = 0.70 min
MS (ESpos): m/z = 289 (M+H)⁺
¹H-NMR (400 MHz, DMSO-d₆): δ = 2.22-2.30 (m, 6 H); 5.27 (s, 2 H); 6.67 (s, 1 H); 7.21 (t, 2 H); 7.53-7.63 (m, 2 H); 7.89 (s, 1 H).

### Beispiel 30A

### 3-Brom-8-[(2,6-difluorbenzyl)oxy]-2,6-dimethylimidazo[1,2-a]pyridin

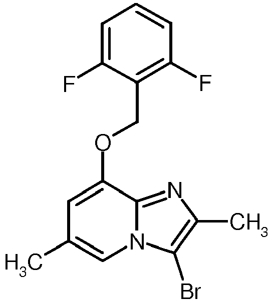

3.865 g (13.41 mmol) 8-[(2,6-Difluorbenzyl)oxy]-2,6-dimethylimidazo[1,2-a]pyridin aus Beispiel 29A wurden unter Argon und Lichtausschluss in 42 ml Ethanol vorgelegt, mit 2.625 g (14.75 mmol) *N*-Bromsuccinimid versetzt und 4 h bei Raumtemperatur gerührt. Das Reaktionsgemisch wurde eingeengt. Der Rückstand wurde mit ca. 100 ml Wasser gerührt und die entstandene Suspension anschließend 30 min bei RT gerührt. Der entstandene Niederschlag wurde abfiltriert, mit Wasser gewaschen und im Hochvakuum getrocknet. Es wurden 4.48 g der Zielverbindung (91% d. Th., Reinheit 100%) erhalten.
LC-MS (Methode 1): Rₜ = 0.93 min
MS (ESpos): m/z = 267 (M+H)⁺
¹H-NMR (400 MHz, DMSO-d₆): δ = 2.28 (s, 3H), 2.33 (s, 3 H); 5.30 (s, 2 H); 6.89 (s, 1 H); 7.22 (t, 2 H); 7.53-7.63 (m, 1 H); 7.75 (s, 1 H).

### Beispiel 31A

### 2,6-Dimethyl-8-[(2,3,6-trifluorbenzyl)oxy]imidazo[1,2-a]pyridin

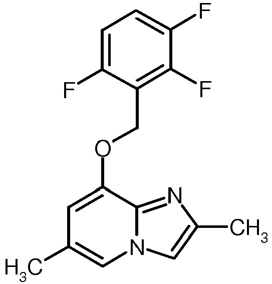

6.48 g (18.50 mmol) 2,6-Dimethyl-8-[(2,3,6-trifluorbenzyl)oxy]imidazo[1,2-a]pyridin-3-carbonsäure aus Beispiel 22A wurden in 140 ml Dioxan vorgelegt, mit 15.4 ml 6 N wässriger Salzsäure-Lösung versetzt und 4 h bei 100°C gerührt. Nach Abkühlen wurde Dioxan im Vakuum entfernt und der wässrige Rückstand mit 1 N Natronlauge auf pH 8 gebracht. Der entstandene Feststoff wurde abfiltriert, mit Wasser gewaschen und im Hochvakuum getrocknet. Es wurden 5.57g der Zielverbindung (96% d. Th.) erhalten.
LC-MS (Methode 1): Rₜ = 0.65 min
MS (ESpos): m/z = 307 (M+H)⁺
¹H-NMR (400 MHz, DMSO-d₆): δ = 2.20 - 2.29 (m, 6 H), 5.29 (s, 2 H), 6.69 (s, 1 H), 7.23 - 7.33 (m, 1 H), 7.57 (s, 1 H), 7.60-7.73 (m, 1 H), 7.91 (s, 1 H).

### Beispiel 32A

### 3-Brom-2,6-dimethyl-8-[(2,3,6-trifluorbenzyl)oxy]imidazo[1,2-a]pyridin

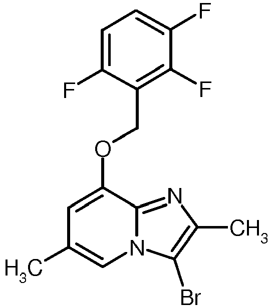

2.28 g (7.45 mmol) 2,6-Dimethyl-8-[(2,3,6-trifluorbenzyl)oxy]imidazo[1,2-a]pyridin aus Beispiel 31A wurden unter Argon und Lichtausschluss in 23.4 ml Ethanol vorgelegt, mit 1.46 g (8.20 mmol) *N*-Bromsuccinimid versetzt und 1.5 h bei Raumtemperatur gerührt. Das Reaktionsgemisch wurde im Vakuum eingeengt. Der Rückstand wurde mit 200 ml Wasser gerührt und die entstandene Suspension anschließend 2 h bei RT gerührt. Der entstandene Niederschlag wurde abfilrtiert, mit Wasser gewaschen und im Hochvakuum getrocknet. Es wurden 2.47 g der Zielverbindung (86% d. Th.) erhalten.
LC-MS (Methode 1): Rₜ = 0.97 min
MS (ESpos): m/z = 385 (M+H)⁺
¹H-NMR (400 MHz, DMSO-d₆): δ = 2.28 (s, 3 H), 2.33 (s, 3 H); 5.32 (s, 2 H); 6.87 (s, 1 H); 7.24 - 7.33 (m, 1 H); 7.62-7.73 (m, 1 H); 7.76 (s, 1 H).

### Beispiel 33A

### 8-[(3-Fluorpyridin-2-yl)methoxy]-2,6-dimethylimidazo[1,2-a]pyridin

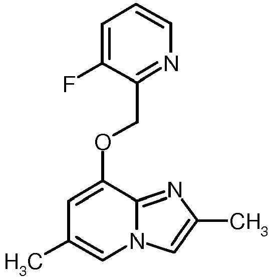

2.30 g (7.29 mmol) 8-[(3-Fluorpyridin-2-yl)methoxy]-2,6-dimethylimidazo[1,2-a]pyridin-3-carbonsäure aus Beispiel 24A wurden in 55.2 ml Dioxan vorgelegt, mit 12.2 ml 6 N wässriger Salzsäure-Lösung versetzt und über Nacht bei 100°C gerührt. Nach dem Abkühlen wurde Dioxan im Vakuum entfernt und der wässrige Rückstand mit 2 N wässriger Natronlauge auf pH 8 gebracht. Der entstandene Feststoff wurde abfiltriert, gut mit Wasser gewaschen und im Hochvakuum getrocknet. Es wurden 2.53 g der Zielverbindung (125% d. Th.) erhalten.
LC-MS (Methode 1): Rₜ = 0.58 min
MS (ESpos): m/z = 272 (M+H)⁺
¹H-NMR (400 MHz, DMSO-d₆): δ = 2.24 (s, 3 H), 2.26 (s, 3 H), 5.40 (s, 2 H), 6.87 (s, 1 H), 7.54-7.70 (m, 2 H), 7.85 (t, 1 H), 7.99 (s, 1 H), 8.47 - 8.53 (m, 1 H).

### Beispiel 34A

### 3-Brom-8-[(3-fluorpyridin-2-yl)methoxy]-2,6-dimethylimidazo[1,2-a]pyridin Trifluoracetat

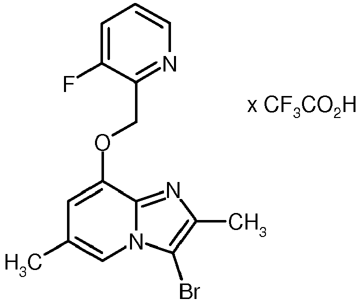

916 mg (3.38 mmol) 8-[(3-Fluorpyridin-2-yl)methoxy]-2,6-dimethylimidazo[1,2-a]pyridin aus Beispiel 33A wurden unter Argon in 10.6 ml Dichlormethan vorgelegt, auf -78°C abgekühlt, mit 631 mg (3.55 mmol) N-Bromsuccinimid versetzt und 1 h bei -78°C gerührt. Das Reaktionsgemisch wurde im Vakuum eingeengt. Der Rückstand wurde in Acetonitril aufgenommen, mit Wasser/TFA versetzt und mittels präparativer HPLC gereinigt (RP18 Säule, Laufmittel: Acetonitril/Wasser-Gradient unter Zusatz von 0.1% TFA). Es wurden 508 mg der Zielverbindung (30% d. Th., Reinheit 94%) erhalten.
LC-MS (Methode 1): Rₜ = 0.71 min
MS (ESpos): m/z = 350 (M+H)⁺
¹H-NMR (400 MHz, DMSO-d₆): δ = 2.29 (s, 3 H), 2.34 (s, 3 H); 5.39 (s, 2 H); 6.85 (s, 1 H); 7.54-7.62 (m, 1 H); 7.72 (s, 1H), 7.85 (t, 1 H), 8.49 (d, 1 H).

### Beispiel 35A

### 8-[(2,6-Difluorbenzyl)oxy]-2-methylimidazo[1,2-a]pyridin-3-carboxamid

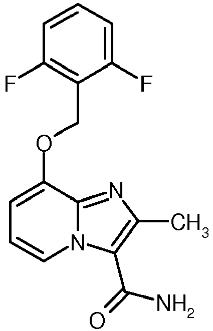

Unter Argon wurden 5 g 8-[(2,6-Difluorbenzyl)oxy]-2-methylimidazo[1,2-a]pyridin-3-carbonsäure (Beispiel 3A, 15.7 mmol, 1 Äquivalent) in 300 ml Dichlormethan vorgelegt und bei RT nacheinander mit 4.5 g 1-(3-Dimethylaminopropyl)-3-ethylcarbodiimidhydrochlorid (23.6 mmol, 1.5 Äquivalente) sowie 3.6 g 1-Hydroxy-1H-benzotriazol Hydrat (HOBT, 23.6 mmol, 1.5 Äquivalente) versetzt und 10 min bei RT gerührt. Dann wurden 4.2 g Ammoniumchlorid (78.5 mmol, 5 Äquivalente) und 19.2 ml *N,N*-Diisopropylethylamin (109.9 mmol, 7 Äquivalente) zugegeben und über Nacht bei RT gerührt. Es wurde einrotiert, der Rückstand mit Dichlormethan versetzt, filtriert, mit Dichlormethan nachgewaschen und über Nacht im Vakuum getrocknet. Es wurden 5.38 g (108% d. Th.) der Titelverbindung erhalten und ohne Reinigung weiter umgesetzt.
LC-MS (Methode 1): Rₜ = 0.65 min
MS (ESpos): m/z = 318.2 (M+H)⁺

### Beispiel 36A

### 8-[(2,6-Difluorbenzyl)oxy]-2-methylimidazo[1,2-a]pyridin-3-carbonitril

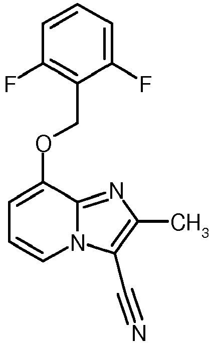

912 mg 8-[(2,6-Difluorbenzyl)oxy]-2-methylimidazo[1,2-a]pyridin-3-carboxamid (Beispiel 35A, 2.9 mmol, 1 Äquivalent) wurde in 13 ml THF vorgelegt und mit 0.6 ml Pyridin (7.4 mmol, 2.56 Äquivalente) versetzt. Dann wurden 1.04 ml Trifluoressigsäureanhydrid (7.4 mmol, 2.56 Äquivalente) zugetropft und der Ansatz über Nacht bei RT gerührt. Anschließend wurde auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure und einmal mit gesättigter Natriumchlorid-Lösung gewaschen, über Natriumsulfat getrocknet und einrotiert. Der Rückstand wurde über Nacht im Vakuum getrocknet. Es wurden 787 mg (91% d. Th.) der Titelverbindung erhalten.
LC-MS (Methode 1): Rₜ = 0.97 min
MS (ESpos): m/z = 300.1 (M+H)⁺
¹H-NMR (400 MHz, DMSO-d₆): δ = 2.44 (s, 3 H), 5.33 (s, 2 H), 7.10 - 7.16 (m, 1 H), 7.18 - 7.28 (m, 3 H), 7.54 - 7.64 (m, 1 H), 8.22 (d, 1 H).

### Beispiel 37A

### 8-[(2,6-Difluorbenzyl)oxy]-2-methylimidazo[1,2-a]pyridin-3-carboximidamid

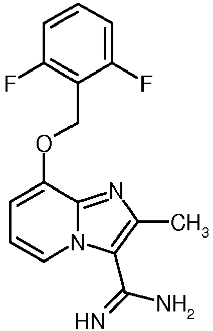

135 mg Ammoniumchlorid (2.5 mmol, 2.52 Äquivalente) wurden unter Argon in 3.9 ml Toluol vorgelegt und auf 0°C abgekühlt. Bei dieser Temperatur wurden 1.26 ml 2 M Trimethylaluminium in Toluol (2.5 mmol, 2.52 Äquivalente) zugegeben und die Lösung wurde 2 h bei RT gerührt. In einem anderen Kolben wurden 300 mg 8-[(2,6-Difluorbenzyl)oxy]-2-methylimidazo[1,2-a]pyridin-3-carbonitril (Beispiel 36A, 1.0 mmol, 1 Äquivalent) in 3.3 ml Toluol vorgelegt, bei RT mit 2 ml der zuvor hergestellten Lösung versetzt und 1 h bei 110°C gerührt. Dieser Vorgang wurde viermal wiederholt. Dann wurde der Ansatz abgekühlt, bei RT mit Kieselgel und einem 1:1 Gemisch aus Dichlormethan/Methanol versetzt und 30 min bei RT gerührt. Das Kieselgel wurde über eine Fritte abfilrtiert. Es wurde mit Methanol gewaschen und das Filtrat wurde im Vakuum eingeengt. Der Rückstand wurde mittels Kieselgel-chromatographie (Eluent: Dichlormethan; Dichlormethan :Methanol = 10:2) gereinigt. Es wurden 137.5 mg (43% d. Th.) der Titelverbindung erhalten.
LC-MS (Methode 1): Rₜ = 0.51 min
MS (ESpos): m/z = 317.1 (M+H)⁺
¹H-NMR (400 MHz, DMSO-d₆): δ = 2.46 (s, 3 H), 5.32 (s, 2 H), 7.04 (t, 1 H), 7.14 (d, 1 H), 7.24 (t, 2 H), 7.53 - 7.66 (m, 1 H), 8.17 (d, 1 H), 9.31 (d, 3 H).

### Beispiel 38A

### 8-[(2,6-Difluorbenzyl)oxy]-N-hydroxy-2-methylimidazo[1,2-a]pyridin-3-carboximidamid

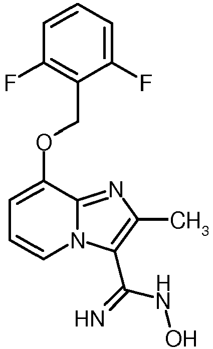

50.0 g (148.9 mmol) 8-[(2,6-Difluorbenzyl)oxy]-2-methylimidazo[1,2-a]pyridin-3-carbonitril aus Beispiel 36A wurden in Ethanol (1.5 1) suspendiert, mit 51.75 g (744.6 mmol) Hydroxylamin Hydrochlorid sowie 103.0 ml (744.6 mmol) Triethylamin versetzt und über Nacht bei RT gerührt. Anschliessend wurde im Vakuum eingeengt, mit Wasser (2.0 1) und Ethanol (100 ml) versetzt und 1 h gerührt. Der entstandene Feststoff wurde abfiltriert, mit Wasser gewaschen und über Nacht am Hochvakuum getrocknet. Es wurden 38.5 g (78 % d. Th.) der Titelverbindung erhalten.
LC-MS (Methode 1): Rₜ = 0.56 min
MS (ESpos): m/z = 333.2 (M+H)⁺

### Beispiel 39A

### 8-[(2,6-Difluorbenzyl)oxy]-2-methylimidazo[1,2-a]pyridin-3-carboximidamid Hydrochlorid

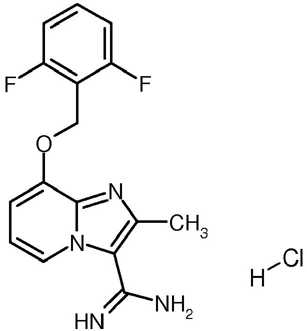

37.5 g (98.4 mmol, 87 % Reinheit) 8-[(2,6-Difluorbenzyl)oxy]-N-hydroxy-2-methylimidazo[1,2-a]pyridin-3-carboximidamid aus Beispiel 38A wurden in Essigsäure (1.01) vorgelegt und mit 11.14 ml (118.08 mmol) Essigsäureanhydrid versetzt. Danach wurden 7.5 g Palladium/Kohle (10%ig, feucht) zugegeben und es wurde 16 h bei Normaldruck hydriert. Es wurde über Kieselgur filtriert und mit Ethanol gewaschen. Nach Einengen wurde der Rückstand dreimal mit je 500 ml Toluol versetzt und im Vakuum eingeengt. Der Rückstand wurde mit 200 ml Essigsäureethylester gerührt, filtriert und im Hochvakuum getrocknet. Es wurden 22.0 g (59% d. Th.) der Titelverbindung erhalten.
LC-MS (Methode 1): Rₜ = 0.51 min
MS (ESpos): m/z = 317.2 (M+H)⁺
¹H-NMR (400 MHz, DMSO-d₆): δ = 1.82 (s, 3H), 2.46 (s, 3 H), 5.31 (s, 2 H), 6.93 (t, 1 H), 7.01 (d, 1 H), 7.21-7.25 (m, 2 H), 7.55 - 7.63 (m, 1 H), 8.55 (br d, 1 H).

### Beispiel 40A

### 8-[(2,6-Difluorbenzyl)oxy]-2,6-dimethylimidazo[1,2-a]pyridin-3-carboxamid

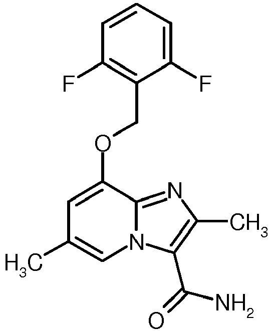

7.0 g (21.07 mmol) 8-[(2,6-Difluorbenzyl)oxy]-2,6-dimethylimidazo[1,2-a]pyridin-3-carbonsäure aus Beispiel 16A wurden in 403 ml Dichlormethan vorgelegt, mit 6.06 g (31.60 mmol) 1-(3-Dimethylaminopropyl)-3-ethylcarbodiimidhydrochlorid und 4.27 g (31.60 mmol) 1-Hydroxy-1H-benzotriazol-Hydrat versetzt und 10 min bei Raumtemperatur gerührt. Anschließend wurden 5.63 g (105.32 mmol) Ammoniumchlorid und 25.68 ml (147.5 mmol) *N,N*-Diisopropylethylamin zugegeben und über Nacht bei Raumtemperatur gerührt. Das Reaktionsgemisch wurde mit Wasser versetzt, der enthaltene Feststoff wurde abfiltriert, anschließend mit Wasser 30 min bei 50°C verrührt, erneut abfiltriert und mit Wasser gewaschen. Es wurden 4.59 g (65% d. Th.) der Titelverbindung erhalten. Von den vereinten Filtratfraktionen (Dichlormethan/Wasser) wurden die Phasen getrennt. Die Dichlormethanphase wurde je einmal mit gesättigter, wässriger Natriumhydrogencarbonatlösung und mit gesättigter, wässriger Natriumchloridlösung gewaschen. Die organische Phase wurde über Natriumsulfat getrocknet, filtriert und im Vakuum eingeengt Der Rückstand wurde mit wenig Acetonitril gerührt und abfiltriert. Es wurden weitere 1.29 g (17% d. Th.; Reinheit 93%) der Titelverbindung erhalten.
LC-MS (Methode 1): Rₜ = 0.64 min
MS (ESpos): m/z = 332 (M+H)⁺
¹H-NMR (400 MHz, DMSO-d₆): δ = 2.31 (s, 3H), 2.50 (s, 3 H; verborgen unter DMSO-Signal), 5.28 (s, 2 H), 6.92 (s, 1 H), 7.22 (t, 2 H), 7.35 (br. s, 2 H), 7.53-7.63 (m, 1 H); 8.62 (s, 1 H).

### Beispiel 41A

### 8-[(2,6-Difluorbenzyl)oxy]-2,6-dimethylimidazo[1,2-a]pyridin-3-carbonitril

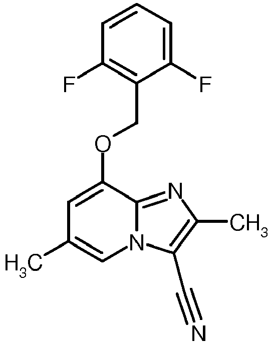

5.7 g (17.20 mol) 8-[(2,6-Difluorbenzyl)oxy]-2,6-dimethylimidazo[1,2-a]pyridin-3-carboxamid Beispiel 40A wurden in 77 ml THF vorgelegt und mit 3.56 ml (44.0 mmol) Pyridin versetzt. Dann wurden bei RT 6.22 ml (44.0 mmol) Trifluoressigsäureanhydrid zugetropft und die Reaktionsmischung rührte 3 h bei RT. Nach beendeter Reaktionszeit wurde auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure sowie einmal mit gesättigter Natriumchlorid-Lösung gewaschen, über Natriumsulfat getrocknet und im Vakuum eingeengt. Der Rückstand wurde über Nacht im Vakuum getrocknet. Es wurden 5.47 g (90% d. Th.) der Titelverbindung erhalten.
LC-MS (Methode 1): Rₜ = 1.12 min
MS (ESpos): m/z = 314 (M+H)⁺
¹H-NMR (400 MHz, DMSO-d₆): δ = 2.37 (s, 3 H), 2.41 (s, 3 H), 5.31 (s, 2 H), 7.12 (s, 1 H), 7.23 (t, 2H), 7.54 - 7.63 (m, 1 H), 8.09 (s, 1 H).

### Beispiel 42A

### 8-[(2,6-Difluorbenzyl)oxy]-2,6-dimethylimidazo[1,2-a]pyridin-3-carboximidamid

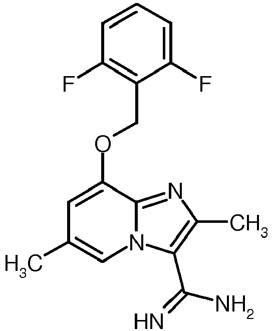

5.47 g (17.46 mmol) 8-[(2,6-Difluorbenzyl)oxy]-2,6-dimethylimidazo[1,2-a]pyridin-3-carbonitril aus Beispiel 41A wurden in Analogie zu Beispiel 37A umgesetzt. Es wurden 1.28 g (22 % d. Th.) der Titelverbindung erhalten.
LC-MS (Methode 1): Rₜ = 0.60 min
MS (ESpos): m/z = 331.3 (M+H)⁺
¹H-NMR (400 MHz, DMSO-d₆): δ = 2.35 (s, 3 H), 2.43 (s, 3 H), 5.31 (s, 2 H), 7.06 (s, 1 H), 7.24 (t, 2 H), 7.54 - 7.65 (m, 1 H), 8.02 (s, 1 H), 9.25 (br. s, 3 H).

### Beispiel 43A

### 8-[(2,6-Difluorbenzyl)oxy]-2,6-dimethylimidazo[1,2-a]pyridin-3-carboximidohydrazid

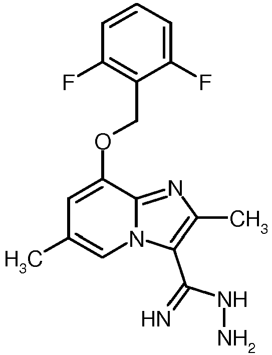

600 mg (1.82 mmol) 8-[(2,6-Difluorbenzyl)oxy]-2,6-dimethylimidazo[1,2-a]pyridin-3-carboximidamid aus Beispiel 42A wurden in Ethanol (15 ml) vorgelegt, mit 2.025 ml (14.53 mmol) Triethylamin und danach mit 220 µl (3.63 mmol) Hydrazinhydrat (80 %ig) versetzt. Es über Nacht bei 50°C gerührt und dann im Vakuum eingeengt. Es wurden 681 mg Rohprodukt erhalten.
LC-MS (Methode 1): Rₜ = 0.55 min
MS (ESpos): m/z = 346.2 (M+H)⁺

### Beispiel 44A

### 8-[(2,6-Difluorbenzyl)oxy]-2-methylimidazo[1,2-a]pyridin-3-carbohydrazid

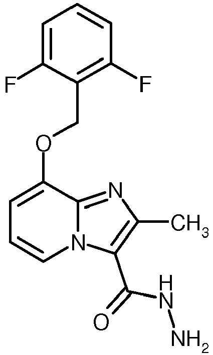

3 g 8-[(2,6-Difluorbenzyl)oxy]-2-methylimidazo[1,2-a]pyridin-3-carbonsäure (Beispiel 16A, 9.4 mmol), 5.4 g *N*-[3-(Dimethylamino)propyl]-*N*'-ethylcarbodiimidhydrochlorid (28.3 mmol, 3 Äquivalente) und 3.8 g 1H-Benzotriazol-1-ol (28.3 mmol, 3 Äquivalente) wurden bei RT in DMF vorgelegt. Nach 30 min wurden 1.4 ml Hydrazinhydrat (28.3 mmol, 1.4 g, 3 Äquivalente) sowie 3.9 ml Triethylamin (28.3 mmol, 2.9 g, 3 Äquivalente) zugegeben und 6 h bei RT gerührt. Dann wurde das Reaktionsgemisch mit Wasser und Essigsäureethylester versetzt. Die organische Phase wurde abgetrennt, mit gesättigter wässriger Natriumchlorid-Lösung gewaschen, über Magnesiumsulfat getrocknet und im Vakuum eingeengt. Es wurden 3.1 g (85% d. Th., Reinheit: 85%) der Titelverbindung erhalten.
LC-MS (Methode 1): Rₜ = 0.58 min
MS (ESpos): m/z = 333 (M+H)⁺

### Beispiel 45A

### 5-{8-[(2,6-Difluorbenzyl)oxy]-2-methylimidazo[1,2-a]pyridin-3-yl}-1,3,4-oxadiazol-2(3H)-on

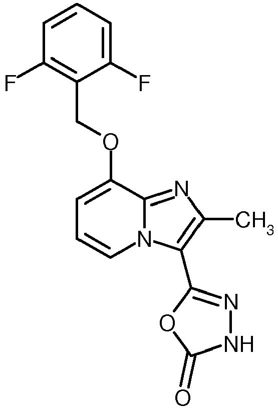

3.1 g 8-[(2,6-Difluorbenzyl)oxy]-2-methylimidazo[1,2-a]pyridin-3-carbohydrazid (Beispiel 44A, 7.9 mmol, 1 Äquivalent) wurden bei RT in 23.7 ml DMF vorgelegt und mit 1.35 g Di-1H-imidazol-1-ylmethanon (CDI; 8.3 mmol, 1.05 Äquivalente) versetzt. Es wurde über Nacht bei RT gerührt und dann mit Wasser versetzt. Der enstandene Feststoff wurde abfiltriert und im Vakuum getrocknet. Es wurden 0.71 g (23% d. Th.) der Titelverbindung erhalten.
LC-MS (Methode 2): Rₜ = 1.01 min
MS (ESpos): m/z = 359 (M+H)⁺
¹H-NMR (400 MHz, DMSO-d₆): δ = 2.55 (s, 3 H; verdeckt durch DMSO-Signal), 5.32 (s, 2 H), 7.09 - 7.15 (m, 2 H), 7.24 (t, 2 H), 7.54 - 7.64 (m, 1 H), 8.60 (d, 1 H), 12.61 (br. s, 1 H).

### Beispiel 46A

### 6-Chlor-8-[(2,6-difluorbenzyl)oxy]-N-[(2R)-1-hydroxyhexan-2-yl]-2-methylimidazo[1,2-a]pyridin-3-carboxamid

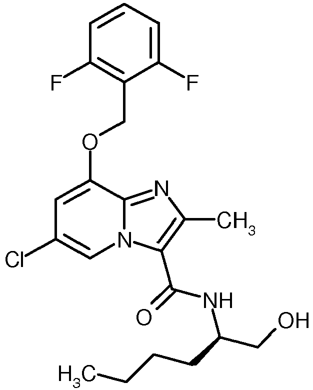

80 mg 6-Chlor-8-[(2,6-difluorbenzyl)oxy]-2-methylimidazo[1,2-a]pyridin-3-carbonsäure (Beispiel 11 A, 0.23 mmol, 1 Äquivalent) in 0.72 ml DMF wurden nacheinander mit 37.2 mg (2R)-2-Aminohexan-1-ol (0.32 mmol, 1.4 Äquivalente), 112 mg HATU (0.3 mmol, 1.3 Äquivalente) und 0.112 ml *N,N*-Diisopropylethylamin (0.68 mmol, 3 Äquivalente) versetzt und über Nacht bei RT gerührt. Der enstandene Fesstoff wurde abfiltriert, mit Wasser gewaschen und im Vakuum getrocknet. Man erhielt 88 mg (82% d. Th.) der Titelverbindung.
LC-MS (Methode 1): Rₜ = 1.05 min
MS (ESpos): m/z = 452.1 (M+H)⁺
¹H-NMR (400 MHz, DMSO-d₆): δ = 0.88 (s, 3 H), 1.25 - 1.40 (m, 4 H), 1.42 - 1.53 (m, 1 H), 1.54 - 1.67 (m, 1 H), 3.39 - 3.56 (m, 2 H), 3.92 - 4.04 (m, 1 H), 4.67 - 4.79 (m, 1 H), 5.35 (s, 2 H), 7.13 - 7.32 (m, 3 H), 7.53 - 7.66 (m, 2 H), 8.59 - 8.67 (m, 1 H), [weitere Signale unter den Lösungsmittelpeaks verborgen].

### Beispiel 47A

### 6-Chlor-N-[(2R)-1-chlorhexan-2-yl]-8-[(2,6-difluorbenzyl)oxy]-2-methylimidazo[1,2-a]pyridin-3-carboxamidhydrochlorid

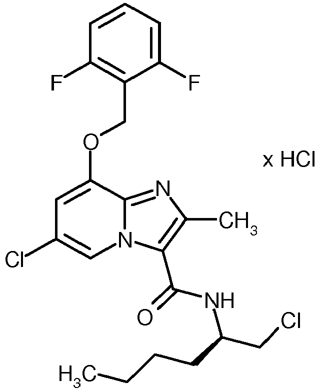

270 mg 6-Chlor-8-[(2,6-difluorbenzyl)oxy]-N-[(2*R*)-1-hydroxyhexan-2-yl]-2-methylimidazo[1,2-a]pyridin-3-carboxamid (Beispiel 46A, 0.6 mmol, 1 Äquivalent) wurden in 2.5 ml Dichlormethan vorgelegt. Bei 0°C wurden 0.13 ml Thionylchorid (1.79 mmol, 3 Äquivalente) zugetropft und es wurde 1 h bei 0°C und dann über Nacht bei RT gerührt. Danach wurde im Vakuum eingeengt, dreimal mit Dichlormethan versetzt und im Vakkum wieder eingeengt und abschließend im Vakuum getrocknet. Es wurden 295 mg (97% d. Th.) der Titelverbindungen erhalten.
LC-MS (Methode 1): Rₜ = 1.33 min
MS (ESpos): m/z = 470.3 (M+H)⁺

### Beispiel 48A

### 3-[(4R)-4-Butyl-4,5-dihydro-1,3-oxazol-2-yl]-6-chlor-8-[(2,6-difluorbenzyl)oxy]-2-methylimidazo[1,2-a]pyridin

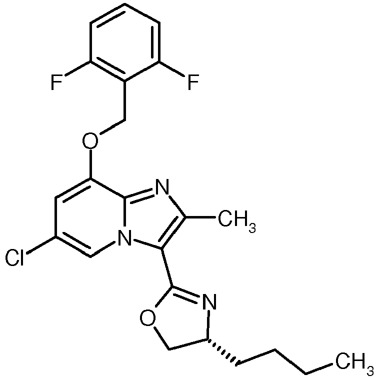

393 mg 6-Chlor-*N*-[(2*R*)-1-chlorhexan-2-yl]-8-[(2,6-difluorbenzyl)oxy]-2-methylimidazo[1,2-a]pyridin-3-carboxamidhydrochlorid (Beispiel 47A, 0.78 mmol, 1 Äquivalent) wurden in 79 ml DMF vorgelegt, mit 1 g Natriumazid (15.5 mmol, 20 Äquivalente) versetzt und 6 h bei 60°C gerührt. Dann wurden 65 ml Wasser zum Reaktionsgemisch gegeben und das Gemisch wurde dreimal mit Essigsäureethylester extrahiert. Die vereinten organischen Phasen wurden über Natriumsulfat getrocknet, filtriert und im Vakuum eingeengt. Der Rückstand mittels Kieselgelchromatographie (Cyclohexan/Essigsäureethylester = 9:1, 7:3) gereinigt. Es wurden 167 mg (50% d. Th.) der Titelverbindung erhalten.
LC-MS (Methode 1): Rₜ = 1.52 min
MS (ESpos): m/z = 434.3 (M+H)⁺
¹H-NMR (400 MHz, DMSO-d₆): δ = 0.91 (t, 3 H), 1.21 - 1.50 (m, 5 H), 1.52 - 1.70 (m, 2 H), 4.01 (m, 7.90 Hz, 1 H), 4.21 - 4.36 (m, 1 H), 4.43 - 4.57 (m, 1 H), 5.36 (s, 2 H), 7.16 - 7.35 (m, 3 H), 7.52 - 7.70 (m, 1 H), 9.29 (s, 1 H); [weiter Signale unter Lösungsmittel Peaks verborgen].

### Beispiel 49A

### 8-[(2,6-Difluorbenzyl)oxy]-2,6-dimethyl-3-[(trimethylsilyl)ethinyl]imidazo[1,2-a]pyridin

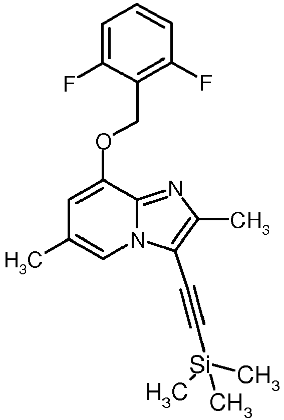

2.0 g (5.27 mmol) 3-Brom-8-[(2,6-difluorbenzyl)oxy]-2,6-dimethylimidazo[1,2-a]pyridin aus Beispiel 30A wurden in 16 ml Acetonitril vorgelegt, mit 1.04 g (10.55 mmol) Ethinyl(trimethyl)silan, 152 mg (0.13 mmol) Bis-(Triphenylphosphin)Palladium-(II)-chlorid, 36 mg (0.19 mmol) Kupfer-(I)-iodid sowie 1.04 ml (7.38 mmol) Diisopropylamin versetzt und über Nacht unter Rückfluss gerührt. Das Gemisch wurde im Vakuum eingeengt, der Rückstand in Essigsäureethylester aufgenommen und mit Wasser extrahiert. Hier erfolgte keine Phasentrennung. Das Gemisch wurde über Celite abfiltriert und das Filtrat mit wenig gesättigter, wässriger Natriumchloridlösung versetzt. Die beiden Phasen wurden anschließend getrennt. Die organische Phase wurde über Natriumsulfat getrocknet, filtriert und im Vakuum eingeengt. Das Rohprodukt wurde mittels Kieselgelchromatographie gereinigt (Laufmittel: Cyclohexan/Ethyacetat 5/1 bis 7/3). Es wurden 1.1 g der Zielverbindung (35% d. Th.; Reinheit ca. 64%) erhalten.
LC-MS (Methode 1): Rₜ = 1.25 min
MS (ESpos): m/z = 385 (M+H)⁺

### Beispiel 50A

### 8-[(2,6-Difluorbenzyl)oxy]-3-ethinyl-2,6-dimethylimidazo[1,2-a]pyridin

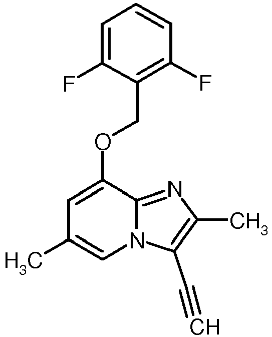

1.1 g (1.83 mmol; Reinheit 64%) 8-[(2,6-Difluorbenzyl)oxy]-2,6-dimethyl-3-[(trimethylsilyl)ethinyl]imidazo[1,2-a]pyridin aus Beispiel 49A wurden in 9.3 ml Methanol vorgelegt, mit 25 mg (0.18 mmol) Kaliumcarbonat versetzt und 1 h bei RT gerührt. Der Rückstand wurde abfiltriert und mit Methanol gewaschen. Das Fitrat wurde im Vakuum eingeengt t und der Rückstand im Hochvakuum getrocknet. Es wurden 0.97 g der Zielverbindung (99% d. Th.; Reinheit ca. 60%) erhalten.
LC-MS (Methode 1): Rₜ = 0.91 minpo
MS (ESpos): m/z = 313 (M+H)⁺

### Beispiel 51A

### 2-Methyl-2-nitropropyltrifluormethansulfonat

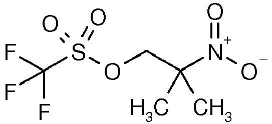

1.0 g (8.40 mmol) 2-Methyl-2-nitropropan-1-ol wurden in 20 ml Dichlormethan vorgelegt, mit 1.0 ml (12.59 mmol) Pyridin versetzt, auf 0°C abgekühlt und langsam mit 1.85 ml (10.91 mmol) Trifluormethansulfonsaeureanhydrid versetzt. Anschließend wurde 1 h bei 0°C gerührt. Der Reaktionsverlauf wurde durch DC kontrolliert (Cyclohexan/Essigsäureethylester 7/3, Anfärbereagenz Kaliumpermangantfärbereagenz). Die Reaktionslösung wurde je einmal mit Wasser und gesättigter, wässriger Natriumchloridlösung gewaschen. Die organische Phase wurde über Natriumsulfat getrocknet, filtriert und das Filtrat eingeengt. Es wurden 2.18 g der Zielverbindung (99% d. Th.) erhalten. Die Zielverbindung wurde bei -18°C gelagert und ohne weitere Reinigung eingesetzt.
MS (Methode 13):
MS (ESpos): m/z = 269 (M+NH₄)⁺
¹H NMR (400 MHz, DMSO-*d*₆) δ = 1.64 (s, 6 H), 5.13 (s, 2 H).

### Beispiel 52A

### 8-[(2,6-Difluorbenzyl)oxy]-2-methyl-3- [1-(2-methyl-2-nitropropyl)-1H-pyrazol-4-yl]imidazo[1,2-a]pyridin

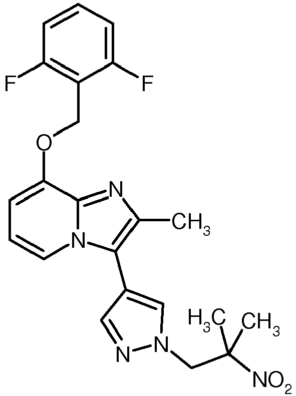

417 mg (1.23 mmol) 8-[(2,6-Difluorbenzyl)oxy]-2-methyl-3-(1H-pyrazol-4-yl)imidazo[1,2-a]pyridin aus Beispiel 45 in 7.5 ml DMF wurden mit 479 mg (1.47 mmol) Cäsiumcarbonat und mit 435 mg (1.73 mmol) 2-Methyl-2-nitropropyltrifluormethansulfonat Beispiel 51A versetzt und es wurde über Nacht bei 100°C gerührt. Anschließend wurden 242 mg (0.96 mmol) 2-Methyl-2-nitropropyltrifluormethansulfonat Beispiel 51A hinzugegeben und es wurde 6 h bei 100°C gerührt. Das Reaktionsgemisch wurde filtriert, der Feststoff mit Essigsäureethylester gewaschen und das Filtrat im Vakuum eingeengt. Der Rückstand wurde mittels präparativer HPLC gereinigt (RP18 Säule, Laufmittel: Acetonitrill/Wasser-Gradient unter Zusatz von 0.1% TFA). Das Rohprodukt wurde mit gesättigter wässriger Natrium-hydrogencarbonat-Lösung versetzt und dreimal mit Dichlormethan extrahiert. Die vereinten organischen Phasen wurden über Natriumsulfat getrocknet und filtriert. Das Filtrat wurde eingeengt, der Rückstand im Hochvakuum getrocknet und mittels Kieselgel-Chromatographie gereinigt (Laufmittel: erst Cyclohexan/Essigsäureethylester 1/1, dann Dichlormethan/2 N methanolischer Ammoniak-Lösung 20/1). Es wurden 193 mg der Zielverbindung (33% d. Th., Reinheit 93%) erhalten.
LC-MS (Methode 1): Rₜ = 0.79 min
MS (ESpos): m/z = 442 (M+H)⁺
¹H NMR (400 MHz, DMSO-d₆) δ = 1.58 (s, 6 H), 2.31 (s, 3 H), 4.79 (s, 2 H), 5.31 (s, 2 H), 6.80 - 6.89 (m, 2 H), 7.23 (t, 2 H), 7.55 - 7.64 (m, 1 H), 7.82 - 7.86 (m, 2 H), 8.08 (s, 1 H).

### Beispiel 53A

### 8-[(2,6-Difluorbenzyl)oxy]-2,6-dimethyl-3-[1-(2-methyl-2-nitropropyl)-1H-pyrazol-4-yl]imidazo[1,2-a]pyridin

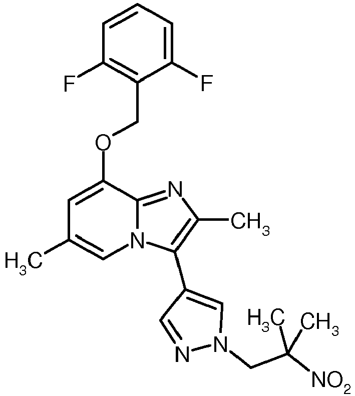

1.30 g (3.67 mmol) 8-[(2,6-Difluorbenzyl)oxy]-2,6-dimethyl-3-(1H-pyrazol-4-yl)imidazo[1,2-a]pyridinaus Beispiel 88 wurden in 22.5 ml DMF vorgelegt, mit 1.43 g (4.40 mmol) Cäsiumcarbonat sowie mit 2.53 g (10.07 mmol) 2-Methyl-2-nitropropyltrifluormethansulfonat Beispiel 51A versetzt und über Nacht bei 100°C gerührt. Das Reaktionsgemisch wurde filtriert, der Niederschlag mit Essigsäureethylester gewaschen und das Filtrat im Vakuum eingeengt. Der Rückstand wurde mit Wasser und Essigsäureethylester versetzt, die wässrige Phase wurde zweimal mit Essigsäureethylester extrahiert und die vereinten organischen Phasen wurden über Natriumsulfat getrocknet. Nach Filtration wurde das Filtrat im Vakuum eingeengt und der Rückstand mittels Kieselgel-Chromatographie gereinigt (Laufmittel: Dichlormethan/Methanol 60/1). Das Rohprodukt wurde erneut mittels Kieselgelchromatographie gereinigt (Laufmittel: Dichlormethan/Methanol 80/1). Es wurden 412 mg der Zielverbindung (24% d. Th.) erhalten.
LC-MS (Methode 1): Rₜ = 0.82 min
MS (ESpos): m/z = 456 (M+H)⁺
¹H NMR (400 MHz, DMSO-d₆) δ = 1.57 (s, 6 H), 2.25 - 2.32 (m, 6 H), 4.78 (s, 2 H), 5.28 (s, 2 H), 6.73 (s, 1 H), 7.22 (t, 2 H), 7.54 - 7.64 (m, 1 H), 7.68 (s, 1 H), 7.82 (s, 1 H), 8.06 (s, 1 H).

### Beispiel 54A

### 2,6-Dimethyl-3-[1-(2-methyl-2-nitropropyl)-1H-pyrazol-4-yl]-8-[(2,3,6-trifluorbenzyl)oxy]imidazo[1,2-a]pyridin

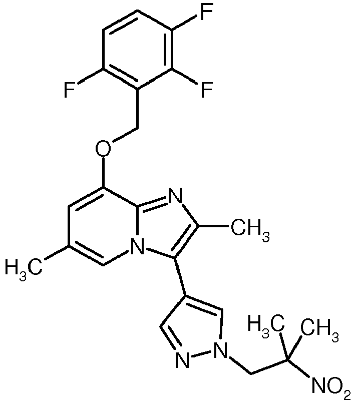

100 mg (0.27 mmol) 2,6-Dimethyl-3-(1H-pyrazol-4-yl)-8-[(2,3,6-trifluorbenzyl)oxy]imidazo[1,2-a]pyridin aus Beispiel 86 wurden in 3.8 ml THF vorgelegt, mit 12 mg (0.32 mmol) Natriumhydrid (65%ig) versetzt, 5 min bei Raumtemperatur gerührt und anschließend mit 213 mg (0.81 mmol) 2-Methyl-2-nitropropyltrifluormethansulfonat Beispiel 51A in 0.3 ml DMF versetzt. Das Reaktionsgemisch wurde 1 h bei Raumtempertaur gerührt. Dann wurde mit Essigsäureethylester verdünnt und zweimal mit Wasser gewaschen. Die organische Phase wurde über Natriumsulfat getrocknet, filtriert, das Filtrat eingeengt und der Rückstand mittels Kieselgelchromatographie gereinigt (Laufmittel Dichlormethan/Methanol 80/1 nach 40/1). Es wurden 94 mg der Zielverbindung (65% d. Th., Reinheit 89%) erhalten.
LC-MS (Methode 1): Rₜ = 0.87 min
MS (ESpos): m/z = 474 (M+H)⁺
¹H NMR (400 MHz, DMSO-*d*₆) δ = 1.58 (s, 6 H), 2.39 (s, 3 H), 2.43 (s, 3 H), 4.82 (s, 2 H), 5.49 (s, 2 H), 6.78 - 6.87 (m, 1 H), 7.59 (br. s, 1 H), 7.65 - 7.76 (m, 1 H), 7.93 (s, 1 H), 8.02 (s, 1 H), 8.22 (s, 1 H).

### Beispiel 55A

### 8-[(3-Fluorpyridin-2-yl)methoxy]-2,6-dimethyl-3-[1-(2-methyl-2-nitropropyl)-1H-pyrazol-4-yl]imidazo[1,2-a]pyridin

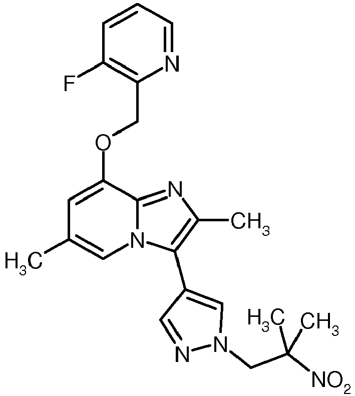

95 mg (0.28 mmol) 8-[(3-Fluorpyridin-2-yl)methoxy]-2,6-dimethyl-3-(1H-pyrazol-4-yl)imidazo[1,2-a]pyridin aus Beispiel 84 wurden in 1.73 ml THF vorgelegt, mit 8.5 mg (0.34 mmol) Natriumhydrid (95%ig) versetzt, 5 min bei Raumtemperatur gerührt und anschließend mit 185 mg (0.70 mmol) 2-Methyl-2-nitropropyltrifluormethansulfonat Beispiel 51A versetzt. Das Reaktionsgemisch wurde 1 h bei Raumtempertaur gerührt. Dann wurde nochmals mit 2 mg (0.09 mmol) Natriumhydrid (95%ig) versetzt, 15 min bei Raumtemperatur gerührt. Es wurden 74 mg (0.28 mmol) 2-Methyl-2-nitropropyltrifluormethansulfonat zugegeben und über Nacht bei Raumtemperatur gerührt. Das Reaktionsgemisch wurde mit Essigsäureethylester verdünnt und zweimal mit Wasser gewaschen. Die organische Phase wurde über Natriumsulfat getrocknet, filtriert, das Filtrat eingeengt und der Rückstand mittels Kieselgelchromatographie gereinigt (Laufmittel Dichlormethan/2 N methanolischer Ammoniak-Lösung (60/1)). Es wurden 66 mg der Zielverbindung (50% d. Th., Reinheit 93%) erhalten.
LC-MS (Methode 1): Rₜ = 0.75 min
MS (ESpos): m/z = 439 (M+H)⁺

### Beispiel 56A

### 8-[(2,6-Difluorbenzyl)oxy]-2,6-dimethyl-3-[2-(2-methyl-2-nitropropyl)-2H-1,2,3-triazol-4-yl]imidazo[1,2-a]pyridin Trifluoracetat

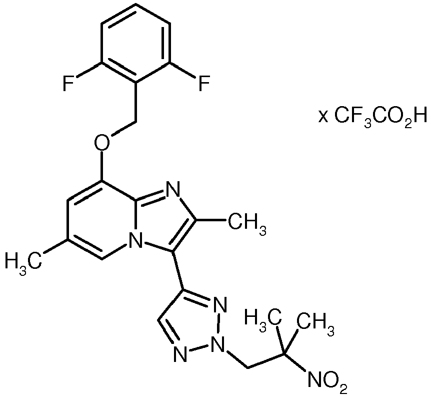

70 mg (0.13 mmol; Reinheit ca. 84%) 8-[(2,6-Difluorbenzyl)oxy]-2,6-dimethyl-3-(2H-1,2,3-triazol-4-yl)imidazo[1,2-a]pyridin aus Beispiel 82 in 1.8 ml THF und 0.45 ml DMF wurden mit 6.1 mg (0.15 mmol) Natriumhydrid (60%ig) versetzt und 10 min bei Raumtemperatur gerührt. Anschließend wurden 95 mg (0.38 mmol) 2-Methyl-2-nitropropyltrifluormethansulfonat aus Beispiel 51A hinzugegeben und 1.5 h bei Raumtemperatur gerührt. Es wurde mit wenig Wasser/TFA versetzt und mittels präparativer HPLC gereinigt (RP18 Säule, Laufmittel: Acetonitril/Wasser-Gradient unter Zusatz von 0.1% TFA). Es wurden 46 mg der Zielverbindung (48% d. Th., Reinheit ca. 75%) erhalten und ohne weitere Reinigung weiter umgesetzt.
LC-MS (Methode 1): Rₜ = 0.93 min
MS (ESpos): m/z = 457 (M+H)⁺

### Beispiel 57A

### 2-(4-{8-[(2,6-Difluorbenzyl)oxy]-2,6-dimethylimidazo[1,2-a]pyridin-3-yl}-1H-pyrazol-1-yl)ethylmethansulfonat

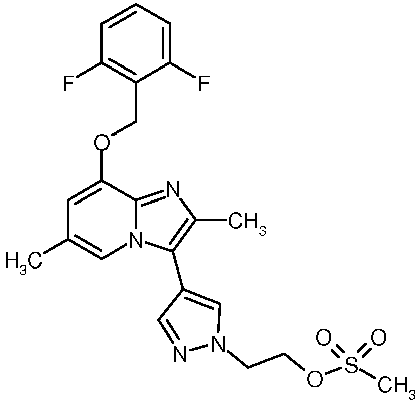

780 mg (1.72 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethanol aus Beispiel 91 wurden unter Argon in 4.0 ml Dichlormethan gelöst und mit 0.72 ml (5.17 mmol) Triethylamin versetzt. Unter Eiskühlung wurden 0.16 ml (2.08 mmol) Methansulfonsäurechlorid zugetropft und die Reaktionsmischung wurde langsam auf Raumtemperatur kommend 30 min gerührt. Bei RT wurden 0.08 ml (1.04 mmol) Methansulfonsäurechlorid zugegeben und dann 30 min gerührt. Das Reaktionsgemisch wurde mit Dichlormethan verdünnt und einmal mit gesättigter, wässriger Natriumchloridlösung gewaschen. Die organische Phase wurde über Natriumsulfat getrocknet, filtriert, das Filtrat eingeengt und mittels Kieselgelchromatographie gereinigt (Lösungsmittel: Essigsäureethylester /Cyclohexan = 2/1). Es wurden 608 mg (74% d. Th.) der Zielverbindung erhalten.
LC-MS (Methode 1): Rₜ = 0.78 min
MS (ESpos): m/z = 477 (M+H)⁺
¹H NMR (400 MHz, DMSO-*d*₆) δ = 2.27 - 2.35 (m, 6 H), 3.12 (s, 3 H), 4.52 - 4.69 (m, 4 H), 5.28 (s, 2 H), 6.73 (s, 1 H), 7.22 (t, 2 H), 7.53 - 7.65 (m, 1 H), 7.72 (s, 1 H), 7.83 (s, 1 H), 8.20 (s, 1 H).

### Beispiel 58A

### 8-[(2,6-Difluorbenzyl)oxy]-2,6-dimethyl-N-(prop-2-in-1-yl)imidazo[1,2-a]pyridin-3-carboxamid

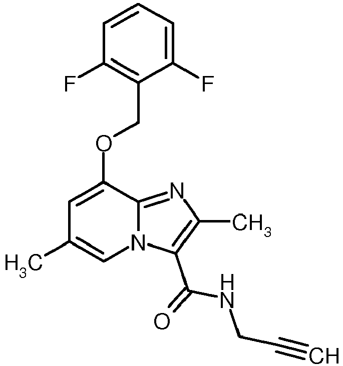

Eine Mischung von 1.00 g 8-[(2,6-Difluorbenzyl)oxy]-2,6-dimethylimidazo[1,2-a]pyridin-3-carbonsäure (3.01 mmol) aus Beispiel 16A, 0.29 ml Propargylamin (4.5 mmol) und 2.6 ml *N,N-*Diisopropylethylamin (15.0 mmol) in 6.0 ml DMF wurden mit 1.49 g HATU (3.91 mmol) versetzt und für 1 h bei Raumtemperatur gerührt. Anschließend wurden 70 ml Wasser zugegeben, der ausgefallene Feststoff wurde gerührt, abfiltriert, mit Wasser nachgewaschen und im Hochvakuum getrocknet. Es wurden 933 mg (81 % d. Th.) der Titelverbindung erhalten.
LC-MS (Methode 1): Rₜ = 0.79 min
MS (ESpos): m/z = 370 (M+H)⁺
¹H-NMR (400 MHz, DMSO-d₆): δ [ppm] = 2.32 (s, 3 H), 2.49 (s, 3 H), 3.13 - 3.17 (m, 1 H), 4.08 (dd, 2 H), 5.28 (s, 2 H), 6.94 (s, 1 H), 7.20 - 7.28 (m, 2 H), 7.53 - 7.65 (m, 1 H), 8.21 - 8.28 (m, 1 H), 8.48 (s, 1 H).

### Beispiel 59A

### 8-[(2,6-Difluorbenzyl)oxy]-N-methoxy-N,2,6-trimethylimidazo[1,2-a]pyridin-3-carboxamid

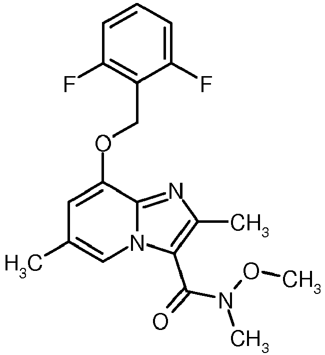

Eine Lösung von 2.50 g 8-[(2,6-Difluorbenzyl)oxy]-2,6-dimethylimidazo[1,2-a]pyridin-3-carbonsäure (7.52 mmol) aus Beispiel 16A in 100 ml Dichlormethan wurde mit 2.16 g EDCI (11.3 mmol) und 1.73 g HOBT (11.3 mmol) versetzt und 10 min bei Raumtemperatur gerührt. Anschließend wurden 9.2 ml *N,N*-Diisopropylethylamin (52.7 mmol) und 3.67 g *N,O-*Dimethylhydroxylamin Hydrochlorid (37.6 mmol) zugegeben und das Gemsich wurde über Nacht bei Raumtemperatur gerührt. Es wurde eingeengt, der Rückstand zunächst mit 200 ml Wasser und anschließend mit 150 ml tert.-Butylmethylether gerührt und dann abfiltriert. Der Feststoff wurde in Essigsäureethylester aufgenommen und dreimal mit gesättigter wässriger Natriumhydrogencarbonat-Lösung, sowie Wasser und gesättigter wässriger Natriumchlorid-Lösung gewaschen, mit Magnesiumsulfat getrocknet, filtriert und eingeengt. Das Rohprodukt wurde mit Diisopropylether gerührt, abfiltriert und im Hochvakuum getrocknet. Es wurden 1.75 g (61 % d. Th.) der Titelverbindung erhalten.
LC-MS (Methode 1): Rₜ = 0.79 min
MS (ESpos): m/z = 376 (M+H)⁺
¹H-NMR (400 MHz, DMSO-d₆): δ [ppm] = 2.31 (s, 3 H), 2.33 (s, 3 H), 3.31 (s, 3 H), 3.50 (s, 3 H), 5.28 (s, 2 H), 6.90 - 6.92 (m, 1 H), 7.20 - 7.29 (m, 2 H), 7.55 - 7.65 (m, 1 H), 7.96 - 7.99 (m, 1 H).

### Beispiel 60A

### 1-{8-[(2,6-Difluorbenzyl)oxy]-2,6-dimethylimidazo[1,2-a]pyridin-3-yl}ethanon

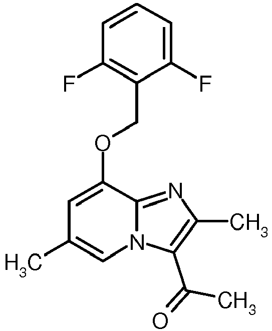

1.97 ml 3 N Methylmagnesiumbromid Lösung in THF (5.9 mmol) wurden langsam zu einer auf 0°C gekühlten Lösung von 1.70 g 8-[(2,6-Difluorbenzyl)oxy]-N-methoxy-N,2,6-trimethylimidazo-[1,2-a]pyridin-3-carboxamid (4.53 mmol) aus Beispiel 59A in 45 ml THF getropft. Anschließend wurde für 15 min bei 0°C sowie 2 h bei RT gerührt. Es wurden 150 ml Wasser zugetropft und dreimal mit Essigsäureethylester extrahiert. Die vereinigten organischen Phasen wurden mit gesättigter wässriger Natriumchlorid-Lösung gewaschen, über Magnesiumsulfat getrocknet, filtriert und eingeengt. Der Rückstand wurde mit n-Pentan ausgrührt, der entstandene Feststoff abfiltriert und im Hochvakuum getrocknet. Es wurden 1.24 g (78% d. Th.) der Titelverbindung erhalten.
DC (Cyclohexan/Essigsäureethylester 1:1): R_{F} = 0.32
LC-MS (Methode 1): Rₜ = 0.96 min
MS (ESpos): m/z = 331 (M+H)⁺
¹H-NMR (400 MHz, DMSO-d₆): δ [ppm] = 2.37 (s, 3 H), 2.56 (s, 3 H), 2.65 (s, 3 H), 5.31 (s, 2 H), 7.16 (s, 1 H), 7.20 - 7.29 (m, 2 H), 7.54 - 7.65 (m, 1 H), 9.11 (s, 1 H).

### Beispiel 61A

### 2-Brom-1-{8-[(2,6-difluorbenzyl)oxy]-2,6-dimethylimidazo[1,2-a]pyridin-3-yl}ethanon

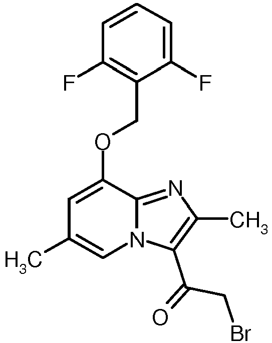

170 µl Brom (3.30 mmol) wurden bei Raumtemperatur zu einer Suspension von 990 mg 1-{8-[(2,6-Difluorbenzyl)oxy]-2,6-dimethylimidazo[1,2-a]pyridin-3-yl}ethanon (3.00 mmol) aus Beispiel 60A in 10 ml Bromwasserstoff (33% in Essigsäure) getropft und für 1 h bei Raumtemperatur gerührt. Anschließend wurden 40 ml Diisopropylether zugegeben, es wurde gerührt und der Feststoff anschließend abfiltriert. Der Feststoff wurde mittels Biotage Isolera (100 g Kieselgelkartusche, Cyclohexan/Essigsäureethylester-Gradient gefolgt von Dichlormethan /Methanol 5:1) gereinigt. Es wurden 317 mg (23% d. Th., Reinheit 90%) sowie 819 mg (47% d. Th., Reinheit 70%) der Titelverbindung isoliert.
LC-MS (Methode 1): Rₜ = 1.13 min
MS (ESpos): m/z = 409 (M+H)⁺

### Beispiel 62A

### Ethyl-4-{8-[(2,6-difluorbenzyl)oxy]-2,6-dimethylimidazo[1,2-a]pyridin-3-yl}-2,4-dioxobutanoat

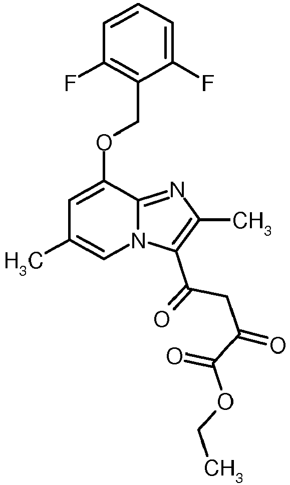

Zu einer Lösung von 800 mg 1-{8-[(2,6-Difluorbenzyl)oxy]-2,6-dimethylimidazo[1,2-a]pyridin-3-yl}ethanon (2.42 mmol) aus Beispiel 61A und 493 µl Oxalsäurediethylester (3.63 mmol) in 80 ml THF wurden bei -40°C 2.66 ml 1 N Lithiumhexamethylsilazan-Lösung in THF (2.66 mmol) getropft und es wurde 30 min bei -40°C sowie 1.5 h bei Raumtemperatur gerührt. Anschließend wurden 250 ml Wasser zugetropft, mehrfach mit Essigsäureethylester extrahiert. Die vereinigten organischen Phasen wurden mit gesättigter wässriger Natriumchlorid-Lösung gewaschen, mit Magnesiumsulfat getrocknet, filtriert und eingeengt. Es wurden 814 mg (62% d. Th., Reinheit 80%) der Titelverbindung erhalten.
LC-MS (Methode 1): Rₜ = 1.25 min
MS (ESpos): m/z = 431 (M+H)⁺

### Beispiel 63A

### 8-[(2,6-Difluorbenzyl)oxy]-2,6-dimethylimidazo[1,2-a]pyridin-3-carbohydrazid

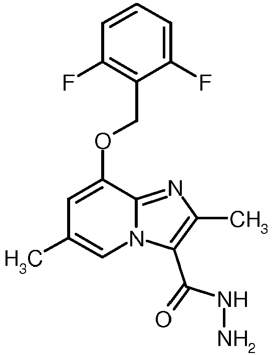

1.474 g (4.09 mmol) Ethyl-8-[(2,6-difluorbenzyl)oxy]-2,6-dimethylimidazo[1,2-a]pyridin-3-carboxylat aus Beispiel 15A wurden in Ethanol (30 ml) vorgelegt und mit 7.60 ml (195.11 mmol) Hydrazinhydrat (80 %ig) versetzt. Es wurde 2 Tage bei Rückfluss gerührt, anschließend mit 3.80 ml (97.5 mmol) Hydrazinhydrat (80%ig) versetzt und 6 h unter Rückfluss gerührt. Das Reaktionsgemisch wurde mit Wasser versetzt und im Eisbad abgekühlt. Der Feststoff wurde abfiltriert, gut mit Wasser nachgewaschen und über Nacht im Hochvakuum getrocknet. Es wurden 998 mg (70% d. Th.) der Titelverbindung erhalten.
LC-MS (Methode 1): Rₜ = 0.60 min
MS (ESpos): m/z = 347 (M+H)⁺
¹H NMR (400 MHz, DMSO-*d*₆) δ = 2.31 (s, 3 H), 2.44 (s, 3 H), 4.50 - 4.54 (m, 2 H), 5.28 (s, 2 H), 6.91 (s, 1 H), 7.23 (t, 2 H), 7.54 - 7.64 (m, 1 H), 8.38 (s, 1 H), 9.18 (br. s, 1 H).

### Beispiel 64A

### tert.-Butyl-{4-[2-({8-[(2,6-difluorbenzyl)oxy]-2,6-dimethylimidazo[1,2-a]pyridin-3-yl}carbonyl)hydrazino]-2-methyl-4-oxobutan-2-yl}carbamat

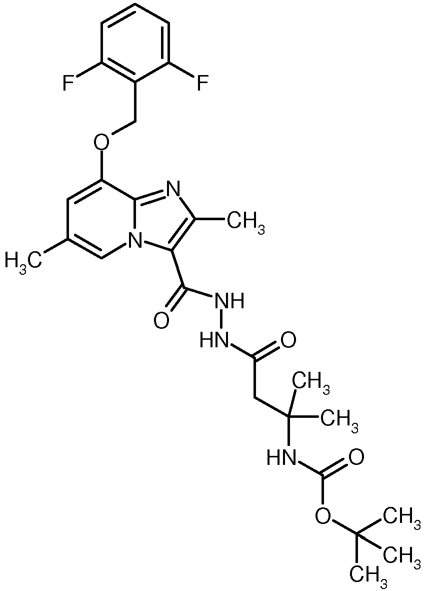

125.5 mg (0.58 mmol) 3-[(tert.-Butoxycarbonyl)amino]-3-methylbutansäure aus Beispiel 63A wurden in 4 ml DMF vorgelegt, mit 266 mg (1.39 mmol) EDCI und 212 mg (1.39 mmol) HOBT versetzt, 30 min bei RT gerührt. Dann wurde mit 200 mg (0.58 mmol) 8-[(2,6-Difluorbenzyl)oxy]-2,6-dimethylimidazo[1,2-a]pyridin-3-carbohydrazid aus Beispiel 63A und 0.24 ml (1.39 mmol) *N,N*-Diisopropylethylamin versetzt und über Nacht bei RT gerührt. Das Reaktionsgemisch wurde mittels präparativer HPLC gereinigt (RP18 Säule, Laufmittel: Acetonitril/Wasser-Gradient unter Zusatz von 0.05% Ameisensäure). Es wurden 128 mg (40% d. Th.) der Titelverbindung erhalten.
LC-MS (Methode 1): Rₜ = 0.95 min
MS (ESpos): m/z = 546 (M+H)⁺
¹H NMR (400 MHz, DMSO-*d*₆) δ = 1.34 (s, 6 H), 1.38 (s, 9 H), 2.32 (s, 3 H), 5.29 (s, 2 H), 5.76 (s, 1 H), 6.57 (br. s, 1 H), 6.97 (s, 1 H), 7.24 (t, 2 H), 7.54 - 7.64 (m, 1 H), 8.37 (br. s, 1 H), 9.74 (br. s, 1 H), 10.00 (br. s, 1 H) [weitere peaks unter Lösungsmittel Signalen].

### Beispiel 65A

### tert.-Butyl-[1-(5-{8-[(2,6-difluorbenzyl)oxy]-2,6-dimethylimidazo[1,2-a]pyridin-3-yl}-1,3,4-thiadiazol-2-yl)-2-methylpropan-2-yl]carbamat

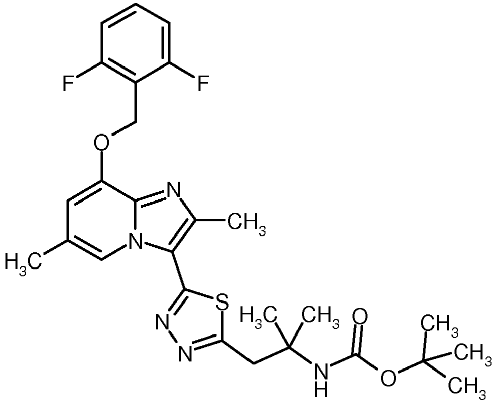

126 mg (0.13 mmol; Reinheit 55%) tert.-Butyl-{4-[2-({8-[(2,6-difluorbenzyl)oxy]-2,6-dimethylimidazo[1,2-a]pyridin-3-yl}carbonyl)hydrazino]-2-methyl-4-oxobutan-2-yl}carbamat aus Beispiel 64A in 3 ml THF wurden mit 77 mg (0.19 mmol) 2,4-Bis(4-methoxyphenyl)-1,3,2,4-dithiadiphosphetan-2,4-disulfid [Lawesson-Reagenz] versetzt und 2 h bei 100°C in der Mikrowelle gerührt. Es wurden 77 mg (0.19 mmol) 2,4-Bis(4-methoxyphenyl)-1,3,2,4-dithiadiphosphetan-2,4-disulfid [Lawesson-Reagenz] hinzugegeben und zunächst 8 h bei 100°C in der Mikrowelle und anschließend 11 h bei 120°C in der Mikrowelle gerührt. Das Reaktionsgemisch wurde mittels präparativer HPLC gereinigt (RP18 Säule, Laufmittel: Acetonitril /Wasser-Gradient unter Zusatz von 0.05% Ameisensäure). Es wurden 12 mg (18% d. Th.) der Titelverbindung erhalten.
LC-MS (Methode 1): Rₜ = 1.28 min
MS (ESpos): m/z = 544 (M+H)⁺

### Beispiel 66A

### tert.-Butyl-[1-(5-{8-[(2,6-difluorbenzyl)oxy]-2,6-dimethylimidazo[1,2-a]pyridin-3-yl}-1,3,4-oxadiazol-2-yl)-2-methylpropan-2-yl]carbamat

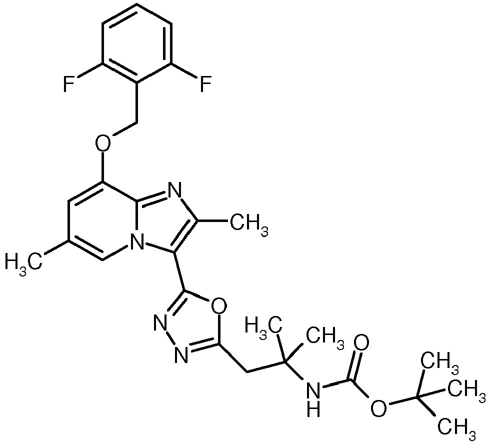

129 mg (0.24 mmol) tert.-Butyl-{4-[2-({8-[(2,6-difluorbenzyl)oxy]-2,6-dimethylimidazo[1,2-a]pyridin-3-yl}carbonyl)hydrazino]-2-methyl-4-oxobutan-2-yl}carbamat aus Beispiel 64A wurden in 4 ml THF vorgelegt, 169 mg (0.71 mmol) 3,3,3-Triethyl-1-(methoxycarbonyl)diazathian-3-ium-1-id-2,2-dioxid (Burgess-Reagenz) zugegeben und das Reaktionsgemisch 15 min bei 80°C in der Mikrowelle gerührt. Nach Einengen im Vakuum wurden 125 mg der Zielverbindung (quantitative Ausbeute) erhalten.
LC-MS (Methode 1): Rₜ = 1.23 min
MS (ESpos): m/z = 528 (M+H)⁺

### Beispiel 67A

### 8-[(2,6-Difluorbenzyl)oxy]-N-hydroxy-2,6-dimethylimidazo[1,2-a]pyridin-3-carboximidamid

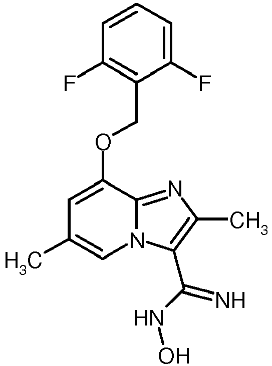

500 mg (1.43 mmol) 8-[(2,6-Difluorbenzyl)oxy]-2,6-dimethylimidazo[1,2-a]pyridin-3-carbonitril aus Beispiel 41A wurden in 15 ml Ethanol vorgelegt, mit 0.43 ml (7.15 mmol) 50%iger Hydroxylamin-Lösung in Wasser veresetzt und 16 h bei RT gerührt. Das Reaktionsgemisch wurde im Vakuum eingeengt und der Rückstand mit 20 ml Wasser und 1 ml Ethanol versetzt. Der entstandene Feststoff wurde abfiltriert, mit 10 ml Wasser nachgewaschen und über Nacht am Hochvakuum getrocknet. Es wurden 512 mg der Zielverbindung (90 % d. Th., Reinheit 87%) erhalten.
LC-MS (Methode 1): Rₜ = 0.58 min
MS (ESpos): m/z = 347 (M+H)⁺
¹H-NMR (400 MHz, DMSO-d₆): δ = 2.28 (s, 3 H), 2.36 (s, 3 H), 5.27 (s, 2 H), 5.87 (s, 2 H), 6.78 (s, 1 H), 7.19 - 7.28 (m, 2 H), 7.54 - 7.64 (m, 1 H), 8.15 (s, 1 H), 9.77 (s, 1 H).

### Beispiel 68A

### tert.-Butyl-[4-({[(Z)-amino{8-[(2,6-difluorbenzyl)oxy]-2,6-dimethylimidazo[1,2-alpyridin-3-yl}methylen]amino}oxy)-2-methyl-4-oxobutan-2-yl]carbamat

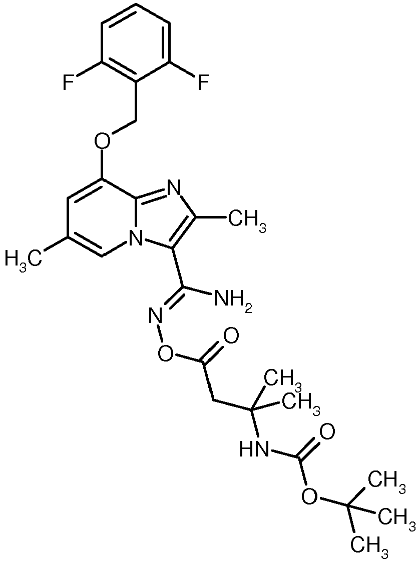

137 mg (0.63 mmol) Boc-3-amino-3-methyl-buttersäure in 5 ml DMF vorgelegt wurden mit 121 mg (0.63 mmol) 1-(3-Dimethylaminopropyl)-3-ethylcarbodiimidhydrochlorid und 96.4 mg (0.63 mmol) 1-Hydroxy-1H-benzotriazol Hydrat versetzt und es wurde 30 min bei RT gerührt. 250 mg (0.63 mmol, 87%ig) 8-[(2,6-Difluorbenzyl)oxy]-N'-hydroxy-2,6-dimethylimidazo[1,2-a]pyridin-3-carboximidamid aus Beispiel 67A wurden in 3 ml DMF suspendiert, zum Reaktionsgemisch zugegeben und es wurde 48 Stunden bei RT gerührt. Dann wurde über präparative HPLC (RP18 Säule, Laufmittel: Acetonitril/Wasser-Gradient unter Zusatz von 0.05% Ameisensäure) gereinigt, und die Produktfraktionen wurden am Rotationsverdampfer eingeengt. Es wurden 168 mg der Zielverbindung (49% d. Th.,) erhalten.
LC-MS (Methode 1): Rₜ = 0.99 min
MS (ESpos): m/z = 546 (M+H)⁺
¹H-NMR (400 MHz, DMSO-d₆): δ = 1.32 (s, 6 H), 1.39 (s, 9 H), 2.29 (s, 3 H), 2.39 (s, 3 H), 2.83 - 2.89 (m, 2 H), 5.26 - 5.31 (m, 2 H), 6.76 - 6.81 (m, 2 H), 6.84 - 6.88 (m, 1 H), 7.19 - 7.28 (m, 2 H), 7.54 - 7.64 (m, 1 H), 8.25 - 8.30 (m, 1 H).

### Beispiel 69A

### tert.-Butyl-[1-(3-{8-[(2,6-difluorbenzyl)oxy]-2,6-dimethylimidazo[1,2-a]pyridin-3-yl}-1,2,4-oxadiazol-5-yl)-2-methylpropan-2-yl]carbamat

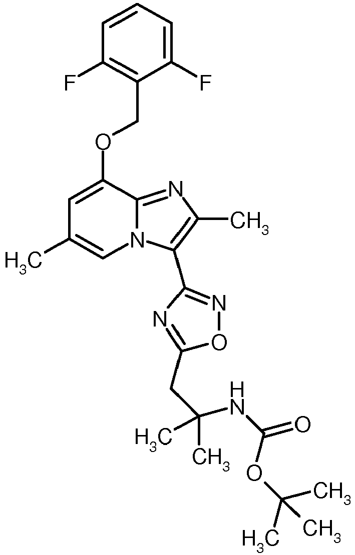

50 mg (0.09 mmol) *tert*.-Butyl-[4-({[(Z)-amino{8-[(2,6-difluorbenzyl)oxy]-2,6-dimethylimidazo[1,2-a]pyridin-3-yl}methylen]amino}oxy)-2-methyl-4-oxobutan-2-yl]carbamat aus Beispiel 68A in 3 ml THF wurden mit 0.092 ml (0.09 mmol) Tetra-n-butylammoniumfluorid-Lösung (1 M in THF) versetzt und es wurde über Nacht bei RT gerührt. Das Reaktionsgemisch wurde bis zur Trockene eingeengt und im Hochvakuume getrocknet. Es wurden 54 mg der Zielverbindung erhalten. Das Produkt wurde ohne weitere Reinigung weiterverwendet.
LC-MS (Methode 1): Rₜ = 1.26 min
MS (ESpos): m/z = 528 (M+H)⁺

### Beispiel 70A

### Benzyl-[4-(hydroxyamino)-2-methylpentan-2-yl]carbamat

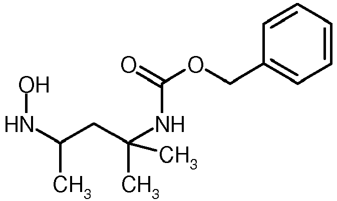

1.20 g (5.17 mmol) Benzyl-(1-cyan-2-methylpropan-2-yl)carbamat in 10 ml Ethanol wurden mit 1.58 ml (25.83 mmol) 50%iger wässriger Hydroxylamin-Lösung versetzt und über Nacht bei RT gerührt. 1.58 ml (25.83 mmol) 50%ige wässrige Hydroxylamin-Lösung wurden nachgegeben und es wurde 5 Tage gerührt. Das Reaktionsgemisch wurde eingeengt, der Rückstand in 20 ml Essigsäureethylester aufgenommen und dreimal mit gesättigter, wässriger Natriumchlorid-Lösung gewaschen. Die organische Phase wurde über Natriumsulfat getrocknet, abfiltriert und im Vakuum eingeengt. Es wurden 1.39 g der Zielverbindung (quantitativ) erhalten. Das Produkt wurde ohne weitere Reinigung weiterverwendet.
LC-MS (Methode 1): Rₜ = 0.60 min
MS (ESpos): m/z = 266 (M+H)⁺
¹H-NMR (400 MHz, DMSO-d₆): δ = 1.27 (s, 6 H), 2.24 (s, 2 H), 4.97 (s, 2 H), 5.36 (s, 2 H), 6.89 - 6.95 (m, 1 H), 7.28 - 7.40 (m, 5 H), 8.96 (s, 1 H).

### Beispiel 71A

### Benzyl-[(4Z)-4-amino-4-{[({8-[(2,6-difluorbenzyl)oxy]-2,6-dimethylimidazo[1,2-a]pyridin-3-yl}carbonyl)oxy]imino}-2-methylbutan-2-yl]carbamat

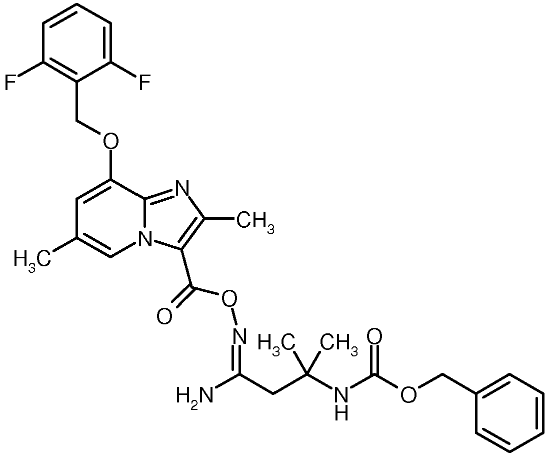

313 mg (0.94 mmol) 8-[(2,6-Difluorbenzyl)oxy]-2,6-dimethylimidazo[1,2-a]pyridin-3-carbonsäure aus Beispiel 16A in 5 ml DMF wurden mit 180.6 mg (0.9 mmol) 1-(3-Dimethylaminopropyl)-3-ethylcarbodiimidhydrochlorid und 144 mg (0.94 mmol) 1-Hydroxy-1H-benzotriazol Hydrat versetzt und die Reaktionsmischung wurde 30 min bei RT gerührt. 250 mg (0.94 mmol) Benzyl-[4-(hydroxyamino)-2-methylpentan-2-yl]carbamat aus Beispiel 70A wurden in 3 mL DMF suspendiert und zur Reaktionsmischung getropft und es wurde über Nacht bei RT gerührt. Die Reaktionslösung wurde über präparative HPLC (RP18 Säule, Laufmittel: Acetonitril/Wasser-Gradient unter Zusatz von 0.05% Ameisensäure) gereinigt und die Produktfraktionen wurden am Rotationsverdampfer eingeengt. Es wurden 317 mg der Zielverbindung (58% d. Th.) erhalten.
LC-MS (Methode 1): Rₜ = 1.15 min
MS (ESpos): m/z = 580 (M+H)⁺
¹H-NMR (400 MHz, DMSO-d₆): δ = 1.37 (s, 6 H), 2.36 (s, 3 H), 2.58 (s, 3 H), 5.01 (s, 2 H), 5.31 (s, 2 H), 6.33 (s, 2 H), 7.06 - 7.13 (m, 2 H), 7.21 - 7.27 (m, 2 H), 7.28 - 7.40 (m, 5 H), 7.55 - 7.65 (m, 1 H), 8.71 (s, 1 H).

### Beispiel 72A

### Benzyl-[1-(5-{8-[(2,6-difluorbenzyl)oxy]-2,6-dimethylimidazo[1,2-a]pyridin-3-yl}-1,2,4-oxadiazol-3-yl)-2-methylpropan-2-yl]carbamat

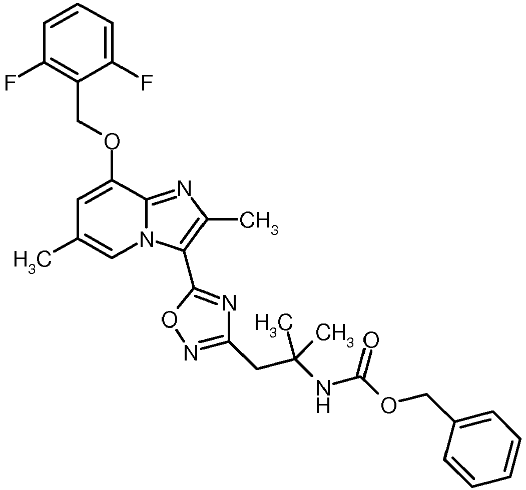

50 mg (0.09 mmol) Benzyl-[(4Z)-4-amino-4-{[({8-[(2,6-difluorbenzyl)oxy]-2,6-dimethylimidazo[1,2-a]pyridin-3-yl}carbonyl)oxy]imino}-2-methylbutan-2-yl]carbamat aus Beispiel 71A in 3 ml THF wurden mit 0.09 ml (0.09 mmol) Tetra-n-butylammoniumfluorid-Lösung (1 M in THF) versetzt und über Nacht bei RT gerührt. Das Reaktionsgemisch wurde im Vakuum eingeengt und am Hochvakuum getrocknet. Es wurden 58 mg der Zielverbindung (quantitativ) erhalten. Das Produkt wurde ohne weitere Reinigung weiterverwendet.
LC-MS (Methode 1): Rt= 1.36 min
MS (ESpos): m/z = 562 (M+H)⁺

### Beispiel 73A

### 5-Methyl-1-(2-methyl-2-nitropropyl)-4-(4,4,5,5-tetramethyl-1,3,2-dioxaborolan-2-yl)-1H-pyrazol

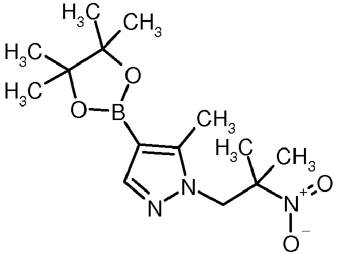

300 mg (1.44 mmol) 5-Methyl-4-(4,4,5,5-tetramethyl-1,3,2-dioxaborolan-2-yl)-1H-pyrazol in 4 ml THF und 2 ml DMF wurden mit 69 mg (1.72 mmol) 60%igem Natriumhydrid versetzt, 10 min bei RT gerührt und mit 435 mg (1.73 mmol) 2-Methyl-2-nitropropyltrifluormethansulfonat aus Beispiel 51A versetzt. Das Reaktionsgemisch wurde anschließend 5 Tage bei RT gerührt. Dann wurden 29 mg (0.72 mmol) 60%iges Natriumhydrid zugegeben, 5 min bei RT gerührt und es wurde anschließend mit 181 mg (0.72 mmol) 2-Methyl-2-nitropropyltrifluormethansulfonat aus Beispiel 51A versetzt. Das Gemisch wurde über Nacht bei RT gerührt. Das Reaktionsgemisch wurde mit 1 ml gesättigter, wässriger Ammoniumchlorid-Lösung versetzt. Acetonitril und Wasser wurde hinzugegeben und das Gemisch wurde mittels präparativer HPLC gereinigt (RP18 Säule, Laufmittel: Acetonitril/Wasser-Gradient unter Zusatz von 0.05% Ameisensäure). Die Produktfraktionen wurden eingeengt und am Hochvakuum getrocknet. Es wurden 174 mg der Zielverbindung (39% d. Th.) erhalten.
LC-MS (Methode 1): Rₜ = 1.10 min
MS (ESpos): m/z = 310 (M+H)⁺
¹H-NMR (400 MHz, DMSO-d₆): δ = 1.24 (s, 18 H), 2.18 (s, 3 H), 4.58 (s, 2 H), 7.70 (s, 1 H).

### Beispiel 74A

### 8-[(2,6-Difluorbenzyl)oxy]-2,6-dimethyl-3-[3-methyl-1-(2-methyl-2-nitropropyl)-1H-pyrazol-4-yl]imidazo[1,2-a]pyridin

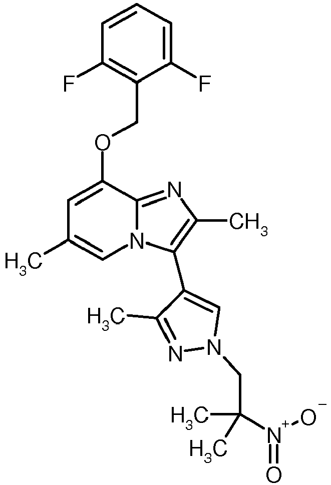

186 mg (0.51 mmol) 3-Brom-8-[(2,6-difluorbenzyl)oxy]-2,6-dimethylimidazo[1,2-a]pyridin aus Beispiel 30A und 172 mg (0.56 mmol) 5-Methyl-1-(2-methyl-2-nitropropyl)-4-(4,4,5,5-tetramethyl-1,3,2-dioxaborolan-2-yl)-1H-pyrazol aus Beispiel 73A wurden unter Argon in 10 ml Acetonitril mit 30 mg (0.04 mmol) Bis-(diphenylphosphino)ferrocen-Palladium(II)chlorid Dichlormethan-Komplex und 2 ml (2.0 mmol) wässriger 1 M Kaliumcarbonat-Lösung versetzt und es wurde über Nacht bei 90°C erhitzt. Es wurde filtriert, das Filtrat mit 3 Tropfen Wasser versetzt und über die präparative HPLC (RP18 Säule, Laufmittel: Acetonitril/Wasser-Gradient unter Zusatz von 0.05% Ameisensäure) gereinigt. Die Produktfraktionen wurden am Rotationsverdampfer eingeengt. Es wurden 74 mg der Zielverbindung (31% d. Th.) erhalten.
LC-MS (Methode 1): Rₜ = 0.92 min
MS (ESpos): m/z = 470 (M+H)⁺
¹H-NMR (400 MHz, DMSO-d₆): δ = 1.54 - 1.61 (m, 6 H), 1.96 (s, 3 H), 2.11 - 2.16 (m, 3 H), 2.25 (s, 3 H), 4.66 - 4.71 (m, 2 H), 5.28 (s, 2 H), 6.74 (s, 1 H), 7.24 (m, 2 H), 7.29 - 7.33 (m, 1 H), 7.55 - 7.64 (m, 1 H), 7.82 (s, 1 H).

### Beispiel 75A

### 8-[(2,6-Difluorbenzyl)oxy]-2,6-dimethyl-3-[1-(2-methyl-2-nitropropyl)-1H-1,2,4-triazol-3-yl]imidazo[1,2-a]pyridin

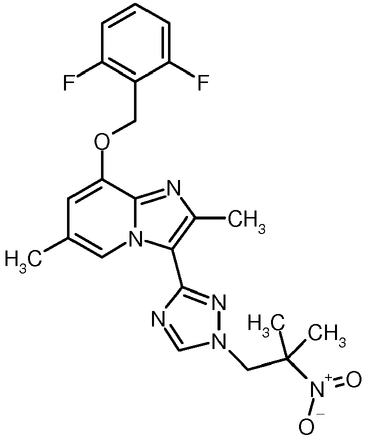

200 mg (0.56 mmol) 8-[(2,6-Difluorbenzyl)oxy]-2,6-dimethyl-3-(1H-1,2,4-triazol-3-yl)imidazo[1,2-a]pyridin aus Beispiel 128 in 8 ml THF und 2 ml DMF wurden mit 27 mg 60%igem Natriumhydrid (0.68 mmol) versetzt. Das Reaktionsgemisch wurde 10 min bei RT gerührt. Anschließend wurden 424 mg (1.69 mmol) 2-Methyl-2-nitropropyltrifluormethansulfonat aus Beispiel 51A zugegeben und die Reaktion 30 min bei RT gerührt. Das Reaktionsgemisch wurde mit gesättigter, wässriger Ammoniumchlorid-Lösung und 2 ml Wasser versetzt und das THF am Rotationsverdampfer abdestilliert. 5 ml Acetonitril wurden zugegeben, entstandene Feststoff wurde abfiltriert, mit Acetonitril gewaschen und am Hochvakuum getrocknet. Das Filtrat wurde eingeengt, mit 10 ml Wasser und 2 ml Acetonitril gerührt, der Rückstand abfiltriert, mit Acetonitril gewaschen und am Hochvakuum getrocknet. Es wurden 287 mg der Zielverbindung (quantitativ) erhalten.
LC-MS (Methode 1): Rₜ = 0.86 min
MS (ESpos): m/z = 457 (M+H)⁺
¹H-NMR (400 MHz, DMSO-d₆): δ = 1.59 (s, 6 H), 2.43 (s, 3 H), 2.64 (s, 3 H), 4.95 (s, 2 H), 5.38 (s, 2 H), 7.21 - 7.30 (m, 3 H), 7.61 (quin, 1 H), 8.75 - 8.86 (m, 2 H).

### Beispiel 76A

### 8-[(2,6-Difluorbenzyl)oxy]-2-methyl-3-[(trimethylsilyl)ethinyl]imidazo[1,2-a]pyridin

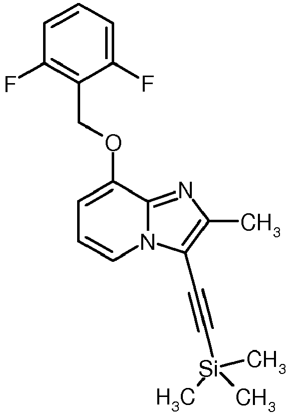

Zu 2.60 g (7.08 mmol, Reinheit 96%) 3-Brom-8-[(2,6-difluorbenzyl)oxy]-2-methylimidazo[1,2-a]pyridin aus Beispiel 28A, 202 mg (1.06 mmol) Kupfer(I)iodid, 0.50 g (0.71 mmol) Bis(triphenylphosphin)palladium(II)chlorid und 3.12 ml (22.41 mmol) Triethylamin in 3.1 ml THF wurden langsam 2 ml (14.15 mmol) Trimethylsilylacethylen getropft und das Reaktionsgemisch 8 Stunden unter Argon bei Rückfluss gerührt. Es wurde eingeengt, der Rückstand in Dichlormethan aufgenommen und mittels Kieselgelchromatographie gereinigt (Laufmittel: Dichlormethan). Die Produktfraktionen wurden eingeengt und im Hochvakuum getrocknet. Es wurden 1.46 g der Zielverbindung (56% d. Th.) erhalten.
LC-MS (Methode 1): Rₜ = 1.23 min
MS (ESpos): m/z = 371 (M+H)⁺
¹H-NMR (400 MHz, DMSO-d₆): δ = 0.29 (s, 9 H), 2.34 (s, 3 H), 5.30 (s, 2 H), 6.93 - 7.03 (m, 2 H), 7.23 (quin, 2 H), 7.54 - 7.63 (m, 1 H), 7.90 - 7.97 (m, 1 H).

### Beispiel 77A

### 8-[(2,6-Difluorbenzyl)oxy]-3-ethinyl-2-methylimidazo[1,2-a]pyridin

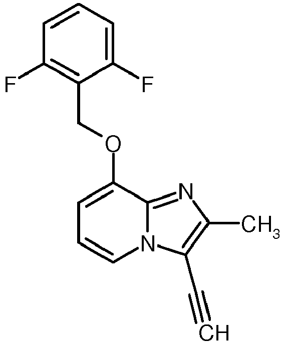

1.46 g (3.93 mmol) 8-[(2,6-Difluorbenzyl)oxy]-2-methyl-3-[(trimethylsilyl)ethinyl]imidazo[1,2-a]pyridin aus Beispiel 76A und 54 mg (0.39 mmol) Kaliumcarbonat in 20 ml Methanol wurden 30 min unter Argon bei RT gerührt. Dann wurde filtriert, der Rückstand mit Methanol gewaschen, das Filtrat eingeengt und im Hochvakuum getrocknet. Es wurden 1.31 g der Zielverbindung (84% d. Th.; Reinheit 76%) erhalten.
LC-MS (Methode 1): Rₜ = 0.86 min
MS (ESpos): m/z = 299 (M+H)⁺
¹H-NMR (400 MHz, DMSO-d₆): δ = 2.30 (s, 1 H), 2.35 (s, 3 H), 5.30 (s, 2 H), 6.93 - 7.02 (m, 2 H), 7.23 (quin, 2 H), 7.54 - 7.64 (m, 1 H), 8.00 (dd, 1 H).

### Beispiel 78A

### Pyrimidin-2-carbohydrazid

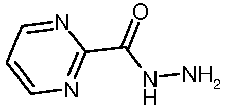

Die Darstellung der Titelverbindung kann über folgende Vorschriften erfolgen: 1.) WOCKHARDT RESEARCH CENTRE; TRIVEDI, Bharat Kalidas; PATEL, Mahesh Vithalbhai, WO2010/136971 A1, 2010 oder 2.) GLAXO GROUP LIMITED; DEAN, David Kenneth; MUNOZ-MURIEDAS, Jorge; SIME, Mairi; STEADMAN, Jon Graham Anthony; THEWLIS, Rachel Elizabeth Anne; TRANI, Giancarlo; WALTER, Daryl Simon, WO2010/125102 A1, 2010.

### Beispiel 79A

### (6-{8-[(2,6-Difluorbenzyl)oxy]-2,6-dimethylimidazo[1,2-a]pyridin-3-yl}-2-methyl-4,6-dioxohexan-2-yl)carbamidsäure-tert.-butylester

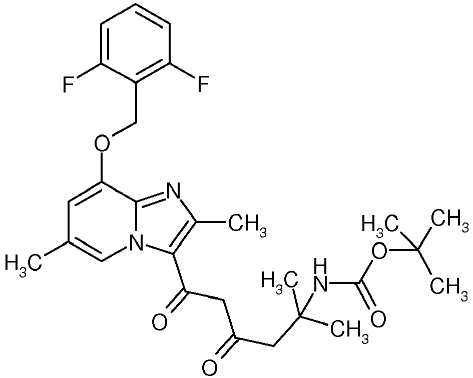

Eine Mischung von 98.6 mg (0.454 mmol) 3-[(tert.-Butoxycarbonyl)amino]-3-methylbutansäure (CAS 129765-95-3) und 73.6 mg (0.454 mmol) 1,1'-Carbonyldiimidazol in 2 ml trockenem THF wurde 3 h bei Raumtemperatur gerührt. Die erhaltene Lösung wurde tropfenweise zu einer frisch hergestellten Lösung von 150 mg (0.454 mmol) 1-{8-[(2,6-Difluorbenzyl)oxy]-2,6-dimethylimidazo[1,2-a]pyridin-3-yl}ethanon Beispiel 60A und 0.454 ml (0.454 mmol) Lithiumhexamethyldisilazid (1M in Tetrahydrofuran) in 5 ml trockenem Tetrahydrofuran gegeben, die unter Argon bei -40°C gerührt wurde. Nach 30 min bei -40°C und 30 min bei Raumtemperatur wurde die Reaktionsmischung zwischen Wasser (20 ml) und Essigsäureethylester (30 ml) verteilt. Die Phasen wurden getrennt und die wässrige Phase wurde zusätzlich mit Essigsäureethylester (2x15 ml) extrahiert. Die vereinigten organischen Phasen wurden im Vakuum eingeengt, wodurch man 200 mg das Zielprodukt enthaltendes Rohmaterial in einer Ausbeute von 8.4% als Nebenprodukt in einer Mischung mit Ausgangsmaterial erhielt. Ohne weitere Aufreinigung in den nächsten Schritt eingesetzt.
LC-MS (Methode 23): Rₜ = 1.39 min; m/z= 530.36 (M+H)⁺

### Beispiel 80A

### [1-(3-{8-[(2,6-Difluorbenzyl)oxy]-2,6-dimethylimidazo[1,2-a]pyridin-3-yl}-1H-pyrazol-5-yl)-2-methylpropan-2-yl]carbamidsäure-tert.-butylester

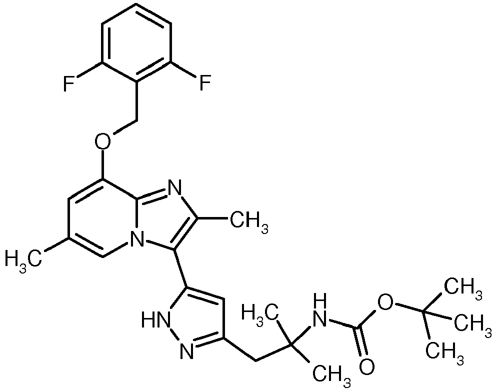

Eine Mischung von 200 mg (0.032 mmol, 8,4% Ausbeute in einer Mischung) von (6-{8-[(2,6-Difluorbenzyl)oxy]-2,6-dimethylimidazo[1,2-a]pyridin-3-yl}-2-methyl-4,6-dioxohexan-2-yl)carbamidsäure-tert.-butylester Beispiel 79A und 21.7 mg (0.317 mmol) Hydrazinmonohydrochlorid in 5 ml Ethanol wurde unter Mikrowellenbestrahlung 30 min auf 120°C erhitzt. Die Reaktionsmischung wurde auf Raumtemperatur abgekühlt, das Lösungsmittel wurde im Vakuum abgedampft und der Rückstand wurde zwischen Essigsäureethylester (15 ml) und Wasser (10 ml) verteilt. Die Phasen wurden getrennt und die organische Phasen wurde im Vakuum zur Trockne eingedampft, wodurch man 120 mg das Zielprodukt enthaltendes Rohmaterial in einer Ausbeute von 8% als Nebenprodukt in einer Mischung mit Ausgangsmaterial aus dem vorherigen Schritt erhielt. Die rohe Mischung wurde ohne weitere Aufreinigung verwendet.
LC-MS (Methode 23): Rₜ = 1.00 min; m/z= 526.38 (M+H)⁺

### Ausführungsbeispiele:

### Beispiel 1

### 3-(1-Benzyl-1H-pyrazol-4-yl)-8-[(2,6-difluorbenzyl)oxy]-2-methylimidazo[1,2-a]pyridin

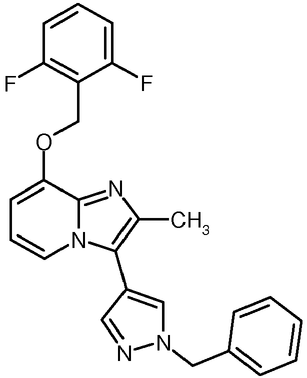

28 mg 1-Benzyl-4-(4,4,5,5-tetramethyl-1,3,2-dioxaborolan-2-yl)-1H-pyrazol (0.1 mmol, 1 Äquivalent) in 0.6 ml 1,4-Dioxan wurden mit 35 mg 3-Brom-8-[(2,6-difluorbenzyl)oxy]-2-methylimidazo[1,2-a]pyridin (Beispiel 28A), 5.8 mg Tetrakis(triphenylphosphine)palladium(0) (0.005 mmol, 0.05 Äquivalente), 21 mg Natriumcarbonat (0.2 mmol, 2 Äquivalente) und 0.2 ml Wasser versetzt und es wurde bei 85°C über Nacht geschüttelt. Nach beendeter Reaktionszeit wurde die Reaktionslösung filtriert, 1,4-Dioxan im Vakuum entfernt, der Rückstand in wenig DMSO gelöst und über präparative HPLC (Methode 11) gereinigt. Es wurden 0.6 mg (1.4% d. Th.) der Titelverbindung erhalten.
LC-MS (Methode 12): Rₜ = 0.90 min
MS (ESpos): m/z = 431 (M+H)⁺

In Analogie zu Beispiel 1 wurden die in Tabelle 1 gezeigten Beispielverbindungen hergestellt, indem 3-Brom-8-[(2,6-difluorbenzyl)oxy]-2-methylimidazo[1,2-a]pyridin (Beispiel 28A) mit den entsprechenden Boronsäuren oder Boronsäureestern umgesetzt wurde.

**Tabelle 1:**

| **Beispiel Nr.** | **IUPAC-Name** | **Analytische Daten** |
|---|---|---|
| | **Struktur** | |
| | **(Ausbeute)** | |
| **2** | 1-(3-{8-[(2,6-Difluorbenzyl)oxy]-2-methylimidazo[1,2-a]pyridin-3-yl}phenyl)ethanon | LC-MS (Methode 12): Rₜ = 0.87 min |
| | 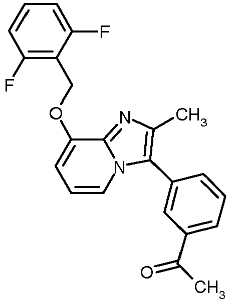 | MS (ESpos): m/z = 393.2 (M+H)⁺ |
| | (35% d. Th., Reinheit 82%) | |
| **3** | 8-[(2,6-Difluorbenzyl)oxy]-2-methyl-3-(2-methylpyridin-4-yl)imidazo[1,2-a]pyridin | LC-MS (Methode 12): Rₜ = 0.77 min |
| | 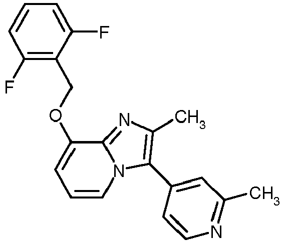 | MS (ESpos): m/z = 366.1 (M+H)⁺ |
| | (21% d. Th., Reinheit 77%) | |
| **4** | *N*-(3-{8-[(2,6-Difluorbenzyl)oxy]-2-methylimidazo[1,2-a]pyridin-3-yl}phenyl)acetamid | LC-MS (Methode 12): Rₜ = 0.83 min |
| | 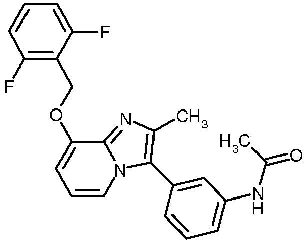 | MS (ESpos): m/z = 408.1 (M+H)⁺ |
| | (5% d. Th.) | |
| **5** | *N*-(3-{8-[(2,6-Difluorbenzyl)oxy]-2-methylimidazo[1,2-a]pyridin-3-ylbenzyl)-N-methylethanamin | LC-MS (Methode 12): Rₜ = 0.64 min |
| | 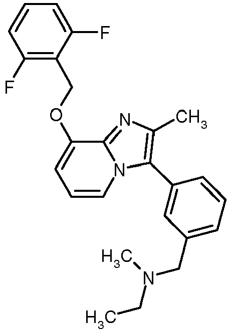 | MS (ESpos): m/z = 422.3 (M+H)⁺ |
| | (31% d. Th.) [1] | |
| **6** | 8-[(2,6-Difluorbenzyl)oxy]-3-[3-(ethylsulfonyl)phenyl]-2-methylimidazo[1,2-a]pyridin | LC-MS (Methode 12): Rₜ = 0.85 min |
| | 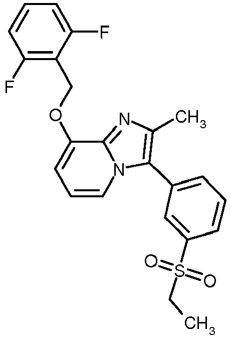 | MS (ESpos): m/z = 443.1 (M+H)⁺ |
| | (32% d. Th., Reinheit 83%) | |
| | 3-{8-[(2,6-Difluorbenzyl)oxy]-2-methylimidazo[1,2-a]pyridin-3-yl}benzamid | LC-MS (Methode 12): Rt = 0.78 min |
| | 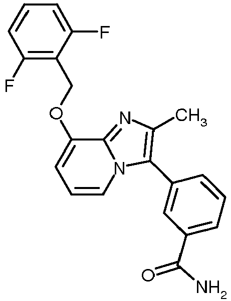 | MS (ESpos): m/z = 394.1 (M+H)⁺ |
| | Es wurde der Boronsäure-Pinakolester eingesetzt. | |
| | (27% d. Th.) | |
| **8** | 3-{8-[(2,6-Difluorbenzyl)oxy]-2-methylimidazo[1,2-a]pyridin-3-yl}phenol | LC-MS (Methode 12): Rₜ = 0.83 min |
| | 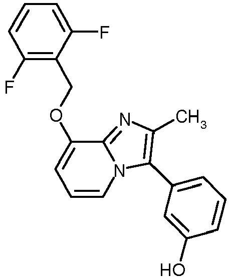 | MS (ESpos): m/z = 367.1 (M+H)⁺ |
| | (33% d. Th., Reinheit 89%) | |
| **9** | *N*-Cyclopropyl-4-{8-[(2,6-difluorbenzyl)oxy]-2-methylimidazo[1,2-a]pyridin-3-yl}benzamid | LC-MS (Methode 12): Rₜ = 0.84 min |
| | 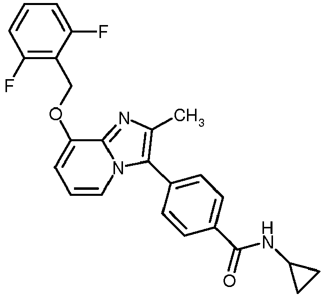 | MS (ESpos): m/z = 434.2 (M+H)⁺ |
| | (12% d. Th.) | |
| **10** | 8-[(2,6-Difluorbenzyl)oxy]-3-(4-ethoxyphenyl)-2-methylimidazo[1,2-a]pyridin | LC-MS (Methode 12): Rₜ = 0.93 min |
| | 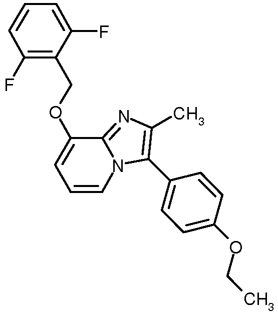 | MS (ESpos): m/z = 395.2 (M+H)⁺ |
| | (45% d. Th., Reinheit 80%) | |
| **11** | 8-[(2,6-Difluorbenzyl)oxy]-2-methyl-3-(1-methyl-1H-pyrazol-5-yl)imidazo[1,2-a]pyridin | LC-MS (Methode 12): Rₜ = 0.94 min |
| | 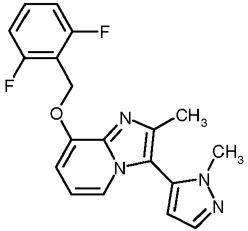 | MS (ESpos): m/z = 355.0 (M+H)⁺ |
| | (18% d. Th.) | |
| **12** | 3-(6-Chlor-5-methylpyridin-3-yl)-8-[(2,6-difluorbenzyl)oxy]-2-methylimidazo[1,2-a]pyridin | LC-MS (Methode 12): Rₜ = 0.94 min |
| | 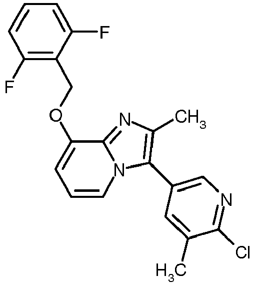 | MS (ESpos): m/z = 400.1 (M+H)⁺ |
| | (4% d. Th., Reinheit 84%) | |
| **13** | 3-(3-Bromphenyl)-8-[(2,6-difluorbenzyl)oxy]-2-methylimidazo[1,2-a]pyridin | LC-MS (Methode 12): Rₜ = 0.86 min |
| | 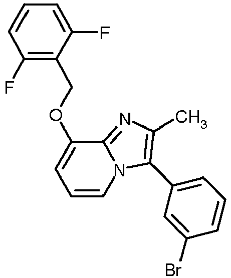 | MS (ESpos): m/z = 430.0 (M+H)⁺ |
| | (12% d. Th.) | |
| **14** | 8-[(2,6-Difluorbenzyl)oxy]-2-methyl-3-(3-thienyl)imidazo [1,2-a]pyridin | LC-MS (Methode 12): Rₜ = 0.87 min |
| | 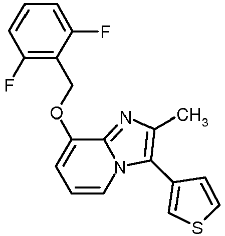 | MS (ESpos): m/z = 357.0 (M+H)⁺ |
| | Es wurde der Boronsäure-Pinakolester eingesetzt. (26% d. Th., Reinheit 80%) | |
| **15** | 8-[(2,6-Difluorbenzyl)oxy]-2-methyl-3-[3-(pyrrolidin-1-ylmethyl)phenyl]imidazo[1,2-a]pyridin | LC-MS (Methode 12): Rₜ = 0.65 min |
| | 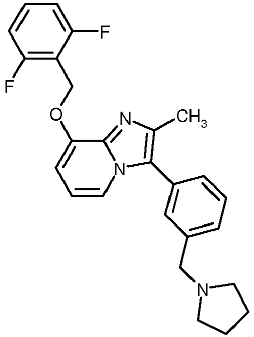 | MS (ESpos): m/z = 434.3 (M+H)⁺ |
| | (11% d. Th.) [2] | |
| **16** | 8-[(2,6-Difluorbenzyl)oxy]-3-(4-fluorphenyl)-2-methylimidazo[1,2-a]pyridin | LC-MS (Methode 12): Rₜ = 0.89 min |
| | 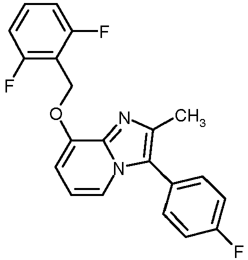 | MS (ESpos): m/z = 369.1 (M+H)⁺ |
| | (31% d. Th.) | |
| **17** | 4-{8-[(2,6-Difluorbenzyl)oxy]-2-methylimidazo[1,2-a]pyridin-3-yl}benzonitril | LC-MS (Methode 12): Rₜ = 0.89 min |
| | 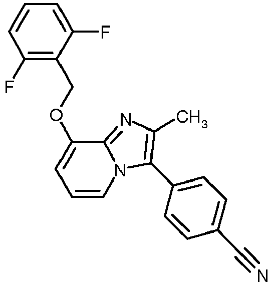 | MS (ESpos): m/z = 376.1 (M+H)⁺ |
| | Es wurde der Boronsäure-Pinakolester eingesetzt. (6% d. Th., Reinheit 87%) | |
| **18** | Ethyl-3-{8-[(2,6-difluorbenzyl)oxy]-2-methylimidazo[1,2-a]pyridin-3-yl}benzoat | LC-MS (Methode 12): Rₜ = 0.93 min |
| | 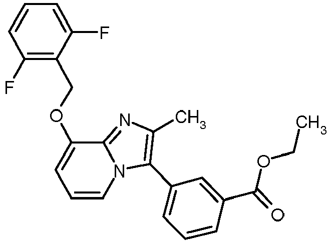 | MS (ESpos): m/z = 423.2 (M+H)⁺ |
| | (39% d. Th., Reinheit 82%) | |
| **19** | 3-{8-[(2,6-Difluorbenzyl)oxy]-2-methylimidazo[1,2-a]pyridin-3-yl}chinolin | LC-MS (Methode 12): Rₜ = 0.93 min |
| | 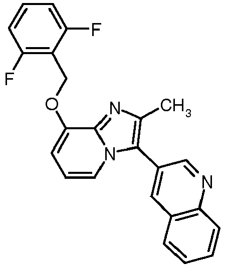 | MS (ESpos): m/z = 423.2 (M+H)⁺ |
| | (16% d. Th., Reinheit 77%) | |
| **20** | 8-[(2,6-Difluorbenzyl)oxy]-2-methyl-3-(5-methyl-2-furyl)imidazo[1,2-a]pyridin | LC-MS (Methode 12): Rₜ = 0.94 min |
| | 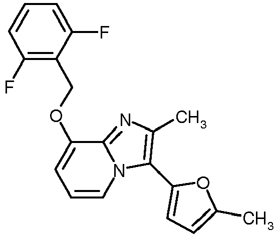 | MS (ESpos): m/z = 353.0/356.0 (M+H)⁺ |
| | (26% d. Th., Reinheit 84%) [3] | |
| **21** | 8-[(2,6-Difluorbenzyl)oxy]-2-methyl-3-[3-(morpholin-4-ylmethyl)phenyl]imidazo[1,2-a]pyridin | LC-MS (Methode 12): Rₜ = 0.96 min |
| | 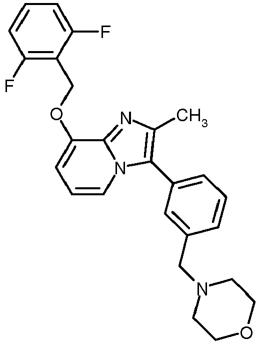 | MS (ESpos): m/z = 419.1 (M+H)⁺ |
| | (32% d. Th.) | |
| **22** | 8-[(2,6-Difluorbenzyl)oxy]-2-methyl-3-(6-propoxypyridin-3-yl)imidazo[1,2-a]pyridin | LC-MS (Methode 12): Rₜ = 0.94 min |
| | 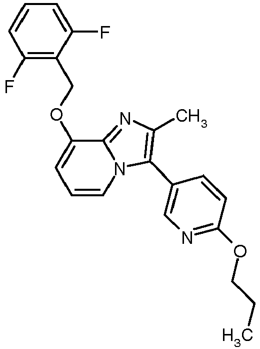 | MS (ESpos): m/z = 410.1(M+H)⁺ |
| | (4% d. Th.) | |
| **23** | 5-{8-[(2,6-Difluorbenzyl)oxy]-2-methylimidazo[1,2-a]pyridin-3-yl}-2-benzofuran-1(3H)-on | LC-MS (Methode 12): Rₜ = 0.85 min |
| | 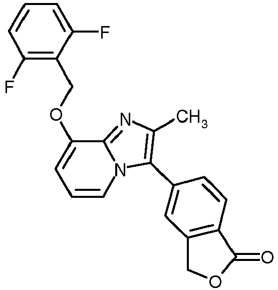 | MS (ESpos): m/z = 407.0 (M+H)⁺ |
| | Es wurde der Boronsäure-Pinakolester eingesetzt. (8% d. Th.) [4] | |
| **24** | 8-[(2,6-Difluorbenzyl)oxy]-2-methyl-3-[3-(trifluormethoxy)phenyl]imidazo[1,2-a]pyridin | LC-MS (Methode 12): Rₜ = 0.97 min |
| | 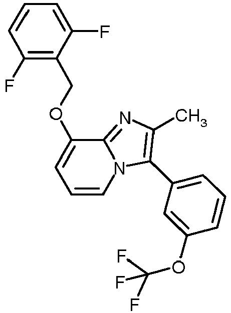 | MS (ESpos): m/z = 435.0 (M+H)⁺ |
| | (20% d. Th., Reinheit 78%) | |
| **25** | 8-[(2,6-Difluorbenzyl)oxy]-3-(4-methoxyphenyl)-2-methylimidazo[1,2-a]pyridin | LC-MS (Methode 12): Rₜ = 0.89 min |
| | 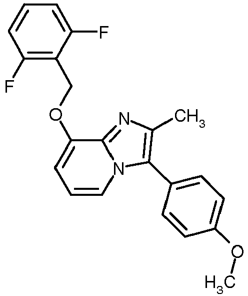 | MS (ESpos): m/z = 400.1 (M+H)⁺ |
| | (4% d. Th., Reinheit 84%) | |
| **26** | 1-(4-{8-[(2,6-Difluorbenzyl)oxy]-2-methylimidazo[1,2-a]pyridin-3-yl}phenyl)-*N,N-*dimethylmethanamin | LC-MS (Methode 12): Rₜ = 0.90 min |
| | 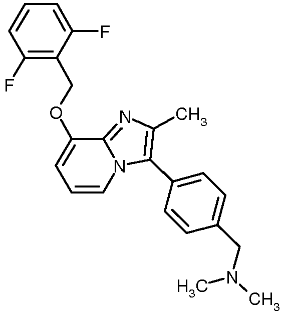 | MS (ESpos): m/z = 381.2 (M+H)⁺ |
| | (39% d. Th., Reinheit 82%) [5] | |
| **27** | *N*-(5-{8-[(2,6-Difluorbenzyl)oxy]-2-methylimidazo[1,2-a]pyridin-3-yl}-2-methoxybenzyl)-2-methoxy-*N*-methylethanamin | LC-MS (Methode 12): Rₜ = 0.67 min |
| | 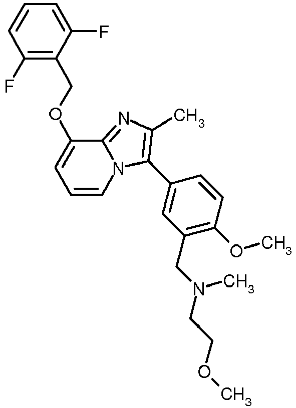 | MS (ESpos): m/z = 482.3(M+H)⁺ |
| | (33% d. Th.) [6] | |
| **28** | *N*-(3-{8-[(2,6-Difluorbenzyl)oxy]-2-methylimidazo[1,2-a]pyridin-3-yl}-4-methoxybenzyl)-*N*-methylethanamin | LC-MS (Methode 12): Rₜ = 0.65 min |
| | 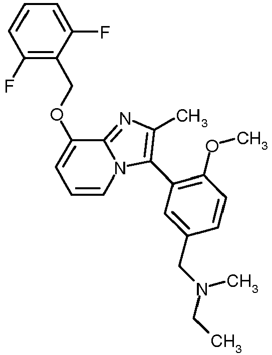 | MS (ESpos): m/z = 452.2 (M+H)⁺ |
| | (25% d. Th.) [7] | |
| **29** | 8-[(2,6-Difluorbenzyl)oxy]-2-methyl-3-(pyridin-4-yl)imidazo[1,2-a]pyridin | LC-MS (Methode 12): Rₜ = 0.79 min |
| | 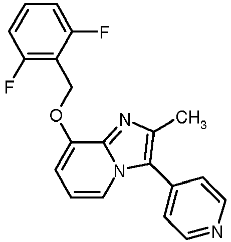 | MS (ESpos): m/z = 352.2 (M+H)⁺ |
| | (13% d. Th., Reinheit 81%) | |
| **30** | 8-[(2,6-Difluorbenzyl)oxy]-2-methyl-3-(4-methylphenyl)imidazo [1,2-a]pyridin | LC-MS (Methode 12): Rₜ = 0.92 min |
| | 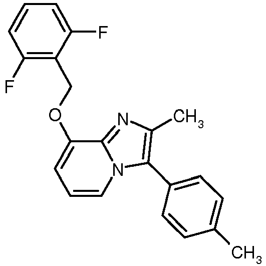 | MS (ESpos): m/z = 365.1 (M+H)⁺ |
| | (27% d. Th.) | |
| **31** | 8-[(2,6-Difluorbenzyl)oxy]-2-methyl-3-(4-phenoxyphenyl)imidazo[1,2-a]pyridin | LC-MS (Methode 12): Rₜ = 1.01 min |
| | 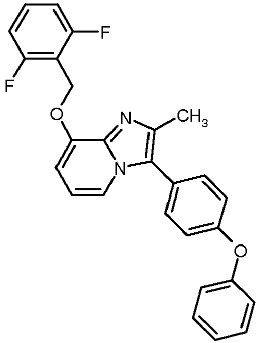 | MS (ESpos): m/z = 443.2 (M+H)⁺ |
| | (7% d. Th.) | |
| **32** | 8-[(2,6-Difluorbenzyl)oxy]-2-methyl-3-(4-vinylphenyl)imidazo[1,2-a]pyridin | LC-MS (Methode 12): Rₜ = 0.95 min |
| | 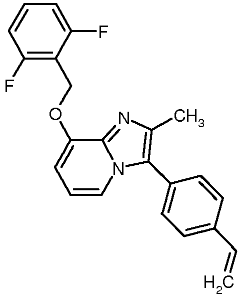 | MS (ESpos): m/z = 377.1 (M+H)⁺ |
| | (3% d. Th.) | |
| **33** | 8-[(2,6-Difluorbenzyl)oxy]-2-methyl-3-[3-(methylsulfanyl)phenyl] imidazo [1,2-a]pyridin | LC-MS (Methode 12): Rₜ = 0.93 min |
| | 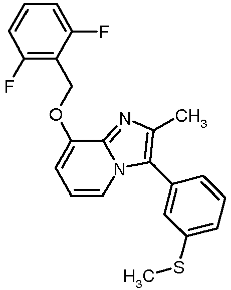 | MS (ESpos): m/z = 397.1 (M+H)⁺ |
| | (45% d. Th.) | |
| **34** | 3-(3-Chlorphenyl)-8-[(2,6-difluorbenzyl)oxy]-2-methylimidazo[1,2-a]pyridin | LC-MS (Methode 12): Rₜ = 0.93 min |
| | 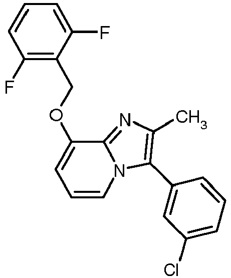 | MS (ESpos): m/z = 385.1 (M+H)⁺ |
| | (29% d. Th., Reinheit 77%) | |
| **35** | 3-(1-Benzothiophen-3-yl)-8-[(2,6-difluorbenzyl)oxy]-2-methylimidazo [1,2-a]pyridin | LC-MS (Methode 12): Rₜ = 0.94 min |
| | 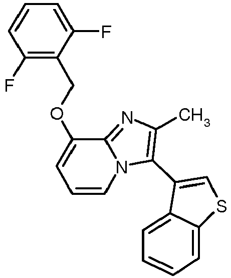 | MS (ESpos): m/z = 407.1 (M+H)⁺ |
| | (10% d. Th.) | |
| **36** | 8-[(2,6-Difluorbenzyl)oxy]-2-methyl-3-(1-methyl-1H-indol-5-yl)imidazo[1,2-a]pyridin | LC-MS (Methode 12): Rₜ = 0.94 min |
| | 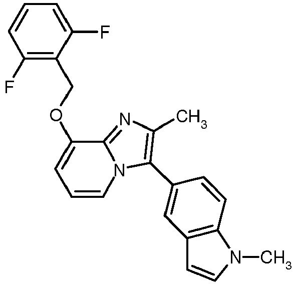 | MS (ESpos): m/z = 404.1 (M+H)⁺ |
| | Es wurde der Boronsäure-Pinakolester eingesetzt. (30% d. Th.) | |
| **37** | 8-[(2,6-Difluorbenzyl)oxy]-3-(2,3-dihydro-1,4-benzodioxin-6-yl)-2-methylimidazo[1,2-a]pyridin | LC-MS (Methode 12): Rₜ = 0.90 min |
| | 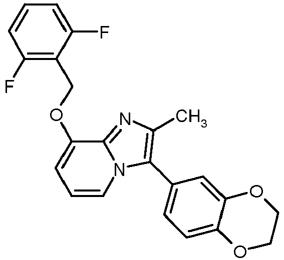 | MS (ESpos): m/z = 409.1 (M+H)⁺ |
| | (28% d. Th., Reinheit 86%) | |
| **38** | 8-[(2,6-Difluorbenzyl)oxy]-2-methyl-3-[3-(piperidin-1-yl)phenyl]imidazo[1,2-a]pyridin | LC-MS (Methode 12): Rₜ = 0.92 min |
| | 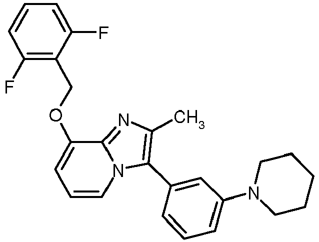 | MS (ESpos): m/z = 434.2 (M+H)⁺ |
| | (31% d. Th., Reinheit 93%) [8] | |
| **39** | 8-[(2,6-Difluorbenzyl)oxy]-3-[3-fluor-4-(morpholin-4-ylmethyl)phenyl]-2-methylimidazo[1,2-a]pyridin | LC-MS (Methode 12): Rₜ = 0.65 min |
| | 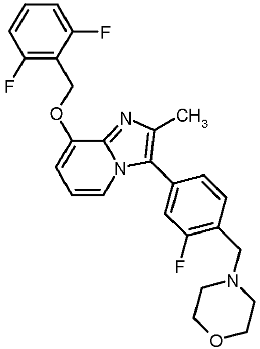 | MS (ESpos): m/z = 468.2 (M+H)⁺ |
| | (42% d. Th.) [9] | |
| **40** | 4-{8-[(2,6-Difluorbenzyl)oxy]-2-methylimidazo[1,2-a]pyridin-3-yl}benzamid | LC-MS (Methode 12): Rₜ = 0.77 min |
| | 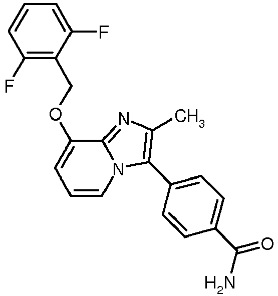 | MS (ESpos): m/z = 394.2 (M+H)⁺ |
| | (35% d. Th.) | |
| **41** | 8-[(2,6-Difluorbenzyl)oxy]-3-[4-methoxy-3-(morpholin-4-ylmethyl)phenyl]-2-methylimidazo[1,2-a]pyridin | LC-MS (Methode 12): Rₜ = 0.66 min |
| | 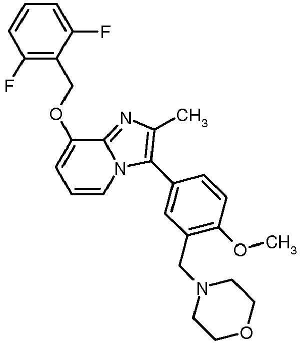 | MS (ESpos): m/z = 480.4 (M+H)⁺ |
| | (39% d. Th.) [10] | |
| **42** | 8-[(2,6-Difluorbenzyl)oxy]-2-methyl-3-[3-(piperidin-1-ylmethyl)phenyl]imidazo[1,2-a]pyridin | LC-MS (Methode 12): Rₜ = 0.69 min |
| | 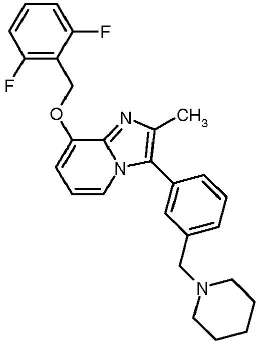 | MS (ESpos): m/z = 448.2 (M+H)⁺ |
| | (18% d. Th.) | |
| **43** | *N*-(4-{8-[(2,6-Difluorbenzyl)oxy]-2-methylimidazo[1,2-a]pyridin-3-yl}-2-fluorbenzyl)-*N-*ethylethanamin | LC-MS (Methode 12): Rₜ = 0.66 min |
| | 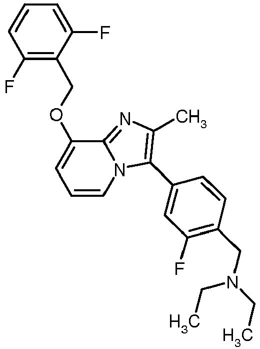 | MS (ESpos): m/z = 454.3 (M+H)⁺ |
| | (23% d. Th.) [11] | |
| **44** | 8-[(2,6-Difluorbenzyl)oxy]-3-[4-fluor-3-(morpholin-4-ylmethyl)phenyl]-2-methylimidazo[1,2-a]pyridin | LC-MS (Methode 12): Rₜ = 0.65 min |
| | 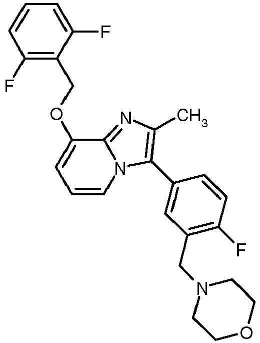 | MS (ESpos): m/z = 468.2 (M+H)⁺ |
| | (50% d. Th.) [12] | |

Nicht kommerziell erhältliche Boronsäuren und Boronsäureester können nach folgenden Literaturvorschriften hergestellt werden:
[1] Darstellung analog Leblanc, Catherine; Pulz, Robert Alexander; Stiefl, Nikolaus Johannes *Patent:* US2009/181941 A1, 2009 aus N-(3-Brombenzyl)-N-methylethanamin.
[2] SIRTRIS PHARMACEUTICALS, INC.; Rebecca, L.; *Patient:* WO2010/101949 A1, 2010.
[3] Florentin et al., Journal of Heterocyclic Chemistry, 1976, Vol. 13, p. 1265, 1266-1268, 1271.
[4] ELI LILLY AND COMPANY; Patent: WO2005/73205 A1, 2005.
[5] Leblanc, Catherine; Pulz, Robert Alexander; Stiefl, Nikolaus Johannes; Patent: US2009/181941 A1, 2009.
[6] Darstellung analog Leblanc, Catherine; Pulz, Robert Alexander; Stiefl, Nikolaus Johannes Patient: US2009/181941 A1, 2009 aus N-(5-Brom-2-methoxybenzyl)-2-methoxy-N-methylethanamin.
[7] Darstellung analog Leblanc, Catherine; Pulz, Robert Alexander; Stiefl, Nikolaus Johannes Patent: US2009/181941 A1, 2009 aus N-(3-Brom-4-methoxybenzyl)-2-methoxy-N-methylethanamin.
[8] Darstellung analog Leblanc, Catherine; Pulz, Robert Alexander; Stiefl, Nikolaus Johannes *Patent:* US2009/181941 A1, 2009 aus 1-(3-Bromphenyl)piperidin.
[9] Darstellung analog NOVARTIS AG; Patent: WO2008/148867 A2, 2008 aus 4-(4-Brom-2-fluorbenzyl)morpholin.
[10] Darstellung analog NOVARTIS AG; Patient: WO2008/148867 A2, 2008 aus 4-(5-Brom-2-methoxybenzyl)morpholin.
[11] Darstellung analog ASTRAZENECA AB; Patient: WO2008/32191 A2, 2008 aus N-(4-Brom-2-fluorbenzyl)-N-ethylethanamin.
[12] Darstellung analog NOVARTIS AG; Patent: WO2008/148867 A2, 2008 aus 4-(5-Brom-2-fluorbenzyl)morpholin.

### Beispiel 45

### 8-[(2,6-Difluorbenzyl)oxy]-2-methyl-3-(1H-pyrazol-4-yl)imidazo[1,2-a]pyridin

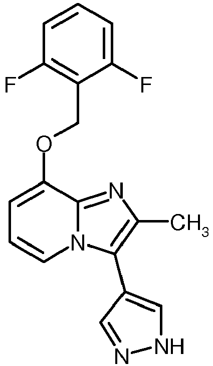

100 mg 3-Brom-8-[(2,6-difluorbenzyl)oxy]-2-methylimidazo[1,2-a]pyridin (Beispiel 28A, 0.28 mmol, 1 Äquivalent) in einem Gemisch aus 2 ml Ethanol, 1 ml Wasser und 1 ml Toluol wurde unter Argon nacheinander mit 95 mg 1H-Pyrazol-4-ylboronsäure (0.85 mmol, 3 Äquivalente), 180 mg Kaliumphosphat und 15 mg Bis(tri-tert-butyl-phosphin)palladium(0) (0.85 mmol, 3 Äquivalente) versetzt. Die Suspension wurde mit Argon entgast und 30 Sekunden gerührt und dann 15 min bei 120°C in einer CEM Discover Mikrowelle gerührt. Nach beendeter Reaktion wurde das Reaktionsgemisch auf Diatomeenerde aufgezogen und per Isolera gereinigt. (Säule: Biotage SNAP Cartridge KP-Sil 10g, Eluent: Gradient: 100%Cyclohexan bis Essigsäureethylester 100%). Es wurden 23 mg (24% d. Th.) der Titelverbindung erhalten.
LC-MS (Methode 1): Rₜ = 0.65 min
MS (ESpos): m/z = 341 (M+H)⁺
¹H NMR (400 MHz, DMSO-*d*₆) δ = 2.32 (s, 3 H), 5.31 (s, 2 H), 6.75 - 6.87 (m, 3 H), 7.18 - 7.29 (m, 3 H), 7.53 - 7.65 (m, 2 H), 7.80 (s, 1 H), 7.87 - 7.92 (m, 2 H), 8.10 - 8.19 (m, 1 H), 13.25 (s, 1 H).

In Analogie zu Beispiel 45 wurden die in Tabelle 2 gezeigten Beispielverbindungen hergestellt, indem 3-Brom-8-[(2,6-difluorbenzyl)oxy]-2-methylimidazo[1,2-a]pyridin (Beispiel 28A mit den entsprechenden, kommerziell erhältlichen Boronsäuren oder Boronsäureestern umgesetzt wurde.

**Tabelle 2:**

| **Beispiel Nr.** | **IUPAC-Name** | **Analytischen Methoden** |
|---|---|---|
| | **Struktur** | |
| | **(Ausbeute)** | |
| **46** | 8-[(2,6-Difluorbenzyl)oxy]-2-methyl-3-[3-(methylsulfonyl)phenyl]imidazo[1,2-a]pyridin | LC-MS (Methode 2): Rₜ = 0.86 min |
| | 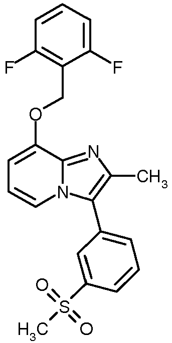 | MS (ESpos): m/z = 429.0 (M+H)⁺ |
| | | ¹H NMR (400 MHz, DMSO-*d*₆) δ = 2.35 (s, 3 H), 5.33 (s, 2 H), 6.83 - 6.89 (m, 1 H), 6.91 - 6.96 (m, 1 H), 7.21 - 7.29 (m, 2 H), 7.55 - 7.65 (m, 1 H), 7.80 - 7.92 (m, 2 H), 7.93 - 8.04 (m, 3 H) [weiteres Signal unter Lösungsmittelpeaks verborgen]. |
| | Das erhaltene Rohprodukt wurde nach der Kieselgelchromatographie mit Acetonitril gerührt, abfiltriert und im Vakuum getrocknet. | |
| | (66% d. Th.) | |
| **47** | 8-[(2,6-Difluorbenzyl)oxy]-2-methyl-3-(1H-pyrazol-5-yl)imidazo[1,2-a]pyridin | LC-MS (Methode 1): Rₜ = 0.69 min |
| | 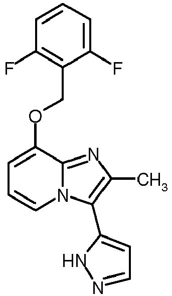 | MS (ESpos): m/z = 341.1 (M+H)⁺ ¹H NMR (400 MHz, DMSO-*d*₆) δ = 5.32 (s, 2 H), 6.63 (s, 1 H), 6.81 - 6.96 (m, 2 H), 7.24 (t, 2 H), 7.50 - 7.69 (m, 1 H), 7.95 (d, 1 H), 8.85 (dd, 2.13 Hz, 1 H), 13.17 (br. s., 1 H) [weiteres Signal unter Lösungsmittelpeaks verborgen]. |
| | | |
| | Das erhaltene Rohprodukt wurde nach der Kieselgelchromatographie mit Acetonitril gerührt, abfiltriert und im Vakuum getrocknet. | |
| | (66% d. Th.) | |
| **48** | *N*-(3-{8-[(2,6-Difluorbenzyl)oxy]-2-methylimidazo[1,2-a]pyridin-3-yl}phenyl)methansulfonamid | LC-MS (Methode 1): Rₜ = 0.76 min |
| | 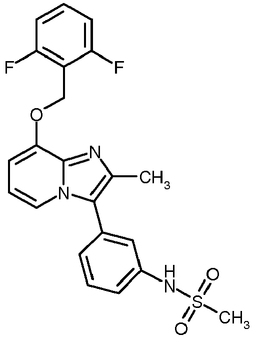 | MS (ESpos): m/z = 444.2 (M+H)⁺ |
| | | ¹H NMR (400 MHz, DMSO-*d*₆) δ = 2.33 (s, 3 H), 3.06 (s, 3 H), 5.32 (s, 2 H), 6.79 - 6.86 (m, 1 H), 6.87 - 6.92 (m, 1 H), 7.20 - 7.35 (m, 5 H), 7.49 - 7.64 (m, 2 H), 7.93 (d, 1 H), 9.92 (s, 1 H). |
| | Es wurde der Boronsäure-Pinakolester eingesetzt. | |
| | Das erhaltene Rohprodukt wurde nach Kieselgelchromatographie per Isolera über präparative HPLC (Methode 10) nachgereinigt. | |
| | (49% d. Th.) | |
| **49** | 8-[(2,6-Difluorbenzyl)oxy]-3-(1-ethyl-1H-pyrazol-4-yl)-2-methylimidazo[1,2-a]pyridin | LC-MS (Methode 2): Rₜ = 0.85 min |
| | 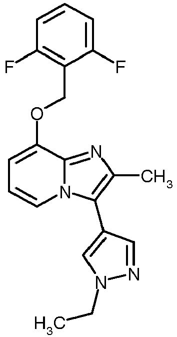 | MS (ESpos): m/z = 369.1 (M+H)⁺ |
| | | ¹H NMR (400 MHz, DMSO-*d*₆) δ = 1.45 (t, 3 H), 2.32 (s, 3 H), 4.23 (m, 2 H), 5.30 (s, 2 H), 6.74 - 6.89 (m, 2 H), 7.24 (s, 2 H), 7.50 - 7.66 (m, 1 H), 7.76 (d, 1 H), 7.89 - 7.97 (m, 1 H), 8.16 (s, 1 H). |
| | (43% d. Th.) | |
| | Es wurde der Boronsäure-Pinakolester eingesetzt. | |
| **50** | 8-[(2,6-Difluorbenzyl)oxy]-2-methyl-3-(1-methyl-1H-pyrazol-4-yl)imidazo[1,2-a]pyridin | LC-MS (Methode 1): Rₜ = 0.63 min |
| | 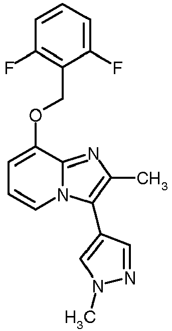 | MS (ESpos): m/z = 355.1 (M+H)⁺ |
| | | ¹H NMR (400 MHz, DMSO-*d*₆) δ = 2.31 (s, 3 H), 3.94 (s, 3 H), 5.29 (s, 2 H), 6.73 - 6.89 (m, 2 H), 7.14 - 7.29 (m, 2 H), 7.54 - 7.64 (m, 1 H), 7.74 (s, 1 H), 7.87 - 7.97 (m, 1 H), 8.10 (s, 1 H). |
| | Es wurde der Boronsäure-Pinakolester eingesetzt. | |
| | (22% d. Th.) | |
| **51** | 6-{8-[(2,6-Difluorbenzyl)oxy]-2-methylimidazo[1,2-a]pyridin-3-yl}-1H-indazol | LC-MS (Methode 1): Rₜ = 0.72 min |
| | 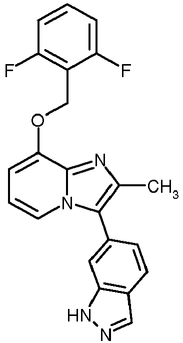 | MS (ESpos): m/z = 391.1 (M+H)⁺ |
| | | ¹H NMR (400 MHz, DMSO-*d*₆) δ = 2.35 (s, 3 H), 5.29 (s, 2 H), 6.74 - 6.97 (m, 2 H), 7.14 - 7.36 (m, 3 H), 7.53 - 7.69 (m, 2 H), 7.86 - 8.03 (m, 2 H), 8.16 (s, 1 H), 13.18 (s, 1 H). |
| | (22% d. Th.) | |
| **52** | 8-[(2,6-Difluorbenzyl)oxy]-3-(1-isopropyl-1H-pyrazol-4-yl)-2-methylimidazo[1,2-a]pyridin | LC-MS (Methode 1): Rₜ = 0.74 min |
| | 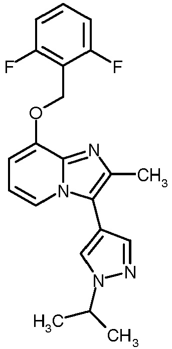 | MS (ESpos): m/z = 383.1 (M+H)⁺ |
| | | ¹H NMR (400 MHz, DMSO-*d*₆) δ = 1.49 (d, 6 H), 2.33 (s, 3 H), 4.54 - 4.65 (m, 1 H), 5.30 (s, 2 H), 6.78 - 6.86 (m, 2 H), 7.19 - 7.28 (m, 2 H), 7.54 - 7.64 (m, 1 H), 7.76 (s, 1 H), 7.91 - 7.96 (m, 1 H), 8.18 (s, 1 H). |
| | Es wurde der Boronsäure-Pinakolester eingesetzt. | |
| | Das erhaltene Rohprodukt wurde nach Kieselgelchromatographie per Isolera über präparative HPLC (Methode 10) nachgereinigt. | |
| | (41% d. Th.) | |
| **53** | 4-{8-[(2,6-Difluorbenzyl)oxy]-2-methylimidazo[1,2-a]pyridin-3-yl}-1H-indazol | LC-MS (Methode 1): Rₜ = 0.72 min |
| | 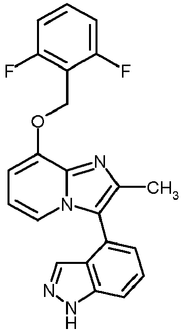 | MS (ESpos): m/z = 391.1 (M+H)⁺ |
| | | ¹H NMR (400 MHz, DMSO-*d*₆) δ = 2.29 (s, 3 H), 5.32 (s, 2 H) 6.73 - 6.83 (m, 1 H), 6.88 - 6.96 (m, 1 H), 7.21 - 7.35 (m, 3 H), 7.47 - 7.75 (m, 5 H), 13.34 (s, 1 H). |
| | Das erhaltene Rohprodukt wurde nach Kieselgelchromatographie per Isolera über präparative HPLC (Methode 10) nachgereinigt. | |
| | (9% d. Th.) | |
| **54** | 5-{8-[(2,6-Difluorbenzyl)oxy]-2-methylimidazo[1,2-a]pyridin-3-yl}-1H-indazol | LC-MS (Methode 2): Rₜ = 0.87 min |
| | 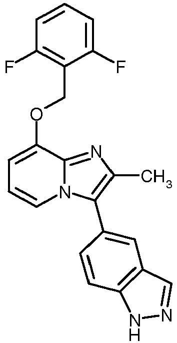 | MS (ESpos): m/z = 391.0 (M+H)⁺ ¹H NMR (400 MHz, DMSO-*d*₆) δ = 2.31 (s, 3 H), 5.32 (s, 2 H), 6.78 (t, 1 H), 6.87 (d, 1 H), 7.21 - 7.29 (m, 2 H), 7.41 - 7.46 (m, 1 H), 7.55 - 7.65 (m, 1 H), 7.72 (d, 1 H), 7.85 (d, 1 H), 7.89 - 7.92 (m, 1 H), 8.16 (s, 1 H), 13.24 (s, 1 H). |
| | | |
| | Das erhaltene Rohprodukt wurde nach Kieselgelchromatographie per Isolera über präparative HPLC (Methode 10) nachgereinigt. | |
| | (30% d. Th.) | |
| **55** | 8-[(2,6-Difluorbenzyl)oxy]-3-(1,3-dimethyl-1H-pyrazol-4-yl)-2-methylimidazo[1,2-a]pyridin | LC-MS (Methode 1): Rₜ = 0.70 min |
| | 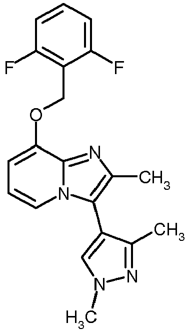 | MS (ESpos): m/z = 369.1 (M+H)⁺ |
| | | ¹H NMR (400 MHz, DMSO-*d*₆) δ = 1.98 (s, 3 H), 2.19 (s, 3 H), 3.85 (s, 3 H), 5.28 (s, 2 H), 6.70 - 6.88 (m, 2 H), 7.18 - 7.29 (m, 2 H), 7.51 - 7.66 (m, 2 H), 7.87 (s, 1 H). |
| | Es wurde der Boronsäure-Pinakolester eingesetzt. | |
| | (25% d. Th.) | |
| **56** | 8-[(2,6-Difluorbenzyl)oxy]-3-(1,5-dimethyl-1H-pyrazol-4-yl)-2-methylimidazo[1,2-a]pyridin | LC-MS (Methode 1): Rₜ = 0.71 min |
| | 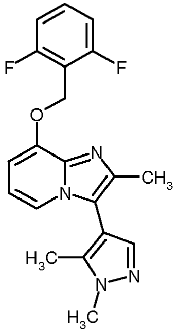 | MS (ESpos): m/z = 369.2 (M+H)⁺ |
| | | ¹H NMR (400 MHz, DMSO-*d*₆) δ = 2.10 (s, 3 H), 2.19 (s, 3 H), 3.84 (s, 3 H), 5.29 (s, 2 H), 6.73 - 6.78 (m, 1 H), 6.84 (d, 1 H), 7.17 - 7.31 (m, 2 H), 7.53 (s, 1 H), 7.55 - 7.64 (m, 2 H). |
| | Es wurde der Boronsäure-Pinakolester eingesetzt. | |
| | (13% d. Th.) | |

### Beispiel 57

### 5-{8-[(2,6-Difluorbenzyl)oxy]-2-methylimidazo[1,2-a]pyridin-3-yl}-N-ethyl-1,3,4-oxadiazol-2-amin

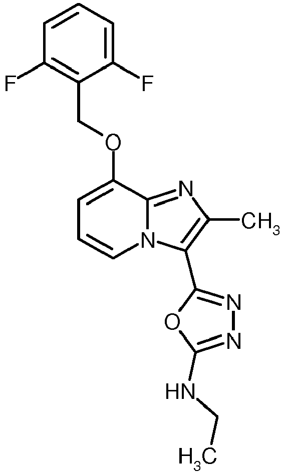

50 mg 5-{8-[(2,6-Difluorbenzyl)oxy]-2-methylimidazo[1,2-a]pyridin-3-yl}-1,3,4-oxadiazol-2(3H)-on Beispiel 45A, 0.13 mmol, 1 Äquivalent) wurden bei RT in 1.3 ml Ethanol suspendiert, mit 0.19 ml 2 M Ethylamin in THF (0.4 mmol, 3 Äquivalente) versetzt und 2.5 h bei 80°C in einer CEM Discover Mikrowelle gerührt. Dann wurde im Vakuum eingeengt, der Rückstand anschließend in einem Gemisch aus 1 ml Acetonitril und 3 ml Dichlormethan gelöst und nacheinander mit 0.09 ml Triethylamin (0.6 mmol, 5 Äquivalente) und 0.04 ml Tetrachlormethan (0.4 mmol, 3 Äquivalente) versetzt. Das Reaktionsgemisch wurde 1.5 h bei 50°C gerührt und dann im Vakuum eingeengt. Das Rohprodukt wurde mit einem Laufmittelgemisch von Cyclohexan/Essigsäureethylester über die Biotage getrennt. Das so erhaltene Produkt wurde über eine präparative HPLC nachgereinigt (Säule: Sunfire C 18, 5 µm, 250 x 20mm, Eluent: 45% Methanol + TFA). Man erhielt 18 mg (36% d. Th.) der Titelverbindung.
LC-MS (Methode 1): Rₜ = 0.82 min
MS (ESpos): m/z = 386.2 (M+H)⁺
¹H-NMR (400 MHz, DMSO-d₆): δ = 1.21 (t, 3 H), 2.59 (s, 3 H), 3.22 - 3.36 (m, 2 H), 5.30 - 5.45 (s, 2 H), 7.19 - 7.34 (m, 4 H), 7.54 - 7.65 (m, 1 H), 7.83 - 7.96 (m, 1 H), 8.85 - 8.97 (d, 1 H).

### Beispiel 58

### 5-{8-[(2,6-Difluorbenzyl)oxy]-2-methylimidazo[1,2-a]pyridin-3-yl}-1,3,4-oxadiazol-2-amin

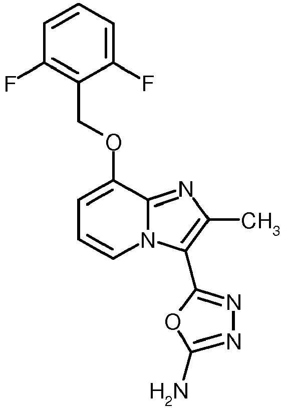

85 mg 8-[(2,6-Difluorbenzyl)oxy]-2-methylimidazo[1,2-a]pyridin-3-carbohydrazid Beispiel 44A, 0.2 mmol, 1 Äquivalent) und 43 mg Bromcyan (0.4 mmol, 2 Äquivalente) in 2.25 ml 1,4-Dioxan wurden mit 2.3 mL 0.1 M wässriger Natriumcarbonat-Lösung (0.23 mmol, 1.1 Äquivalente) versetzt. Es wurde über Nacht bei RT gerührt und dann mit Essigsäureethylester und Wasser extrahiert. Die organische Phase wurde über Magnesiumsulfat getrocknet, filtriert und einrotiert. Der Rückstand wurde über präparative HPLC (Methode 10) gereinigt. Es wurden 32 mg (44% d. Th.) der Titelverbindung erhalten.
LC-MS (Methode 1): Rₜ = 0.70 min
MS (ESpos): m/z = 358.1 (M+H)⁺
¹H-NMR (400 MHz, DMSO-d₆): δ = 2.57 (s, 3 H), 5.34 (s, 2 H), 7.07 - 7.13 (m, 2 H), 7.25 (t, 2 H), 7.31 (s, 2 H), 7.52 - 7.66 (m, 1 H), 8.85 (dd, 2.36 Hz, 1 H).

### Beispiel 59

### 8-[(2,6-Difluorbenzyl)oxy]-3-[2-(4-fluorphenyl)pyridin-4-yl]-2-methylimidazo[1,2-a]pyridin

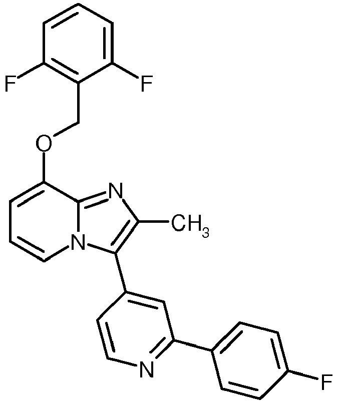

2.2 mg Palladium(II)acetat (0.01 mmol, 0.05 Äquivalente) und 8 mg 2-Dicyclohexylphosphino-2',6'-dimethoxybiphenyl (S-Phos, 0.02 mmol, 0.1 Äquivalent) in 0.35 ml Acetonitril wuden 15 min bei RT gerührt. Dann wurden erst eine Lösung von 82 mg Kaliumcarbonat (0.6 mmol, 3 Äquivalente) in 0.5 ml Wasser, dann eine Lösung von 77 mg 2-(4-Fluorphenyl)-4-(4,4,5,5-tetramethyl-1,3,2-dioxaborolan-2-yl)pyridin (0.3 mmol, 1.3 Äquivalente) in 0.35 ml Acetonitril und anschließend 70 mg 3-Brom-8-[(2,6-difluorbenzyl)oxy]-2-methylimidazo[1,2-a]pyridin (Beispiel 28A, 0.2 mmol, 1 Äquivalent) zugegeben. Das Reaktionsgemisch wurde 8 h unter Rückfluss gerührt. Nach dem Abkühlen wurde das Reaktionsgemisch durch einen Milliporefilter filtriert und das Filtrat mittels präparativer HPLC gereinigt (RP18 Säule, Laufmittel: Acetonitril/Wasser-Gradient unter Zusatz von 0.1 % TFA). Die produkthaltigen Fraktionen wurden eingeengt, der Rückstand in Essigsäureethylester aufgenommen, zweimal mit wässriger, gesättigter Natriumhydrogencarbonat-Lösung gewaschen, die organische Phase über Natriumsulfat getrocknet, filtriert, eingeengt und lyophilisiert. Es wurden 56 mg (63% d. Th.) der Titelverbindung erhalten.
LC-MS (Methode 1): Rₜ = 0.95 min
MS (ESpos): m/z = 446.3 (M+H)⁺
¹H-NMR (400 MHz, DMSO-d₆): δ = 2.43 (s, 3 H), 5.34 (s, 2 H), 6.88 (t, 1 H), 6.97 (d, 1 H), 7.25 (t, 2 H), 7.34 (t, 2 H), 7.50 - 7.54 (m, 1 H), 7.56 - 7.65 (m, 1 H), 8.08 (s, 1 H), 8.15 (d, 1 H), 8.19 - 8.26 (m, 2 H), 8.73 - 8.86 (m, 1 H).

### Beispiel 60

### 8-[(2,6-Difluorbenzyl)oxy]-2-methyl-3-(pyrazin-2-yl)imidazo[1,2-a]pyridin

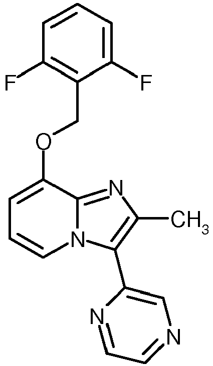

350 mg 3-Brom-8-[(2,6-difluorbenzyl)oxy]-2-methylimidazo[1,2-a]pyridin (Beispiel 28A, 0.99 mmol, 1 Äquivalent) in 10.5 ml DMF wurden mit 600 mg 2-(Tributylstannyl)pyrazin (1.6 mmol, 1.7 Äquivalente) und 60 mg Dichlorpalladium-ditriphenylphosphan (0.09 mmol, 0.125 Äquivalente) versetzt. Der Ansatz wurde in 4 Ansätze geteilt und je 1 h bei 120°C in einer CEM Discover Mikrowelle gerührt. Dann wurde mit Wasser versetzt und dreimal mit Essigsäureethylester extrahiert. Die organische Phase wurde mit gesättigter wässriger Natriumchlorid-Lösung gewaschen, über Magnesiumsulfat getrocknet und einrotiert. Der Rückstand wurde auf Diatomeenerde absorbiert und über Isolera (Säule: Biotage SNAP Cartridge KP-Sil 50g, Eluent: Gradient Cyclohexan 100% bis Essigsäureethylester 100%) gereinigt. Der erhaltene Feststoff wurde mit Methanol gerührt, abfiltriert und am Hochvakuum getrocknet. Das Filtrat wurde einrotiert und per präparativer HPLC gereinigt (Methode 10). Es wurden 56 mg (16% d. Th.) der Titelverbindung erhalten.
LC-MS (Methode 2): Rₜ = 0.93 min
MS (ESpos): m/z = 353.1 (M+H)⁺
¹H-NMR (400 MHz, DMSO-d₆): δ = 2.58 (s, 3 H), 5.34 (s, 2 H), 6.95 (t, 1 H), 7.04 (d, 1 H), 7.25 (m, 2 H), 7.55 - 7.65 (m, 1 H), 8.59 (d, 1 H), 8.77 - 8.80 (m, 1 H), 8.85 - 8.90 (m, 1 H), 8.97 (d, Hz, 1 H).

### Beispiel 61

### 3-(4-Butyl-1,3-oxazol-2-yl)-6-chlor-8-[(2,6-difluorbenzyl)oxy]-2-methylimidazo[1,2-a]pyridin Trifluoracetat

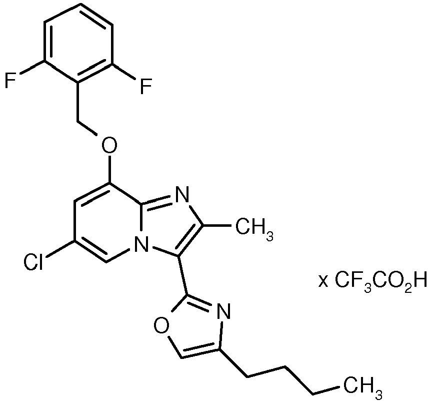

47 mg 3-[(4*S*)-4-Butyl-4,5-dihydro-1,3-oxazol-2-yl]-6-chlor-8-[(2,6-difluorbenzyl)oxy]-2-methylimidazo[1,2-a]pyridin (Beispiel 48A, 0.1 mmol, 1 Äquivalent) in 2.35 ml Toluol wurden mit 44 mg 2,3-Dichlor-5,6-dicyano-1,4-benzochinon (DDQ, 0.2 mmol, 1.8 Äquivalente) versetzt und 45 min bei 150°C in der Mikrowelle gerührt. Dann wurde im Vakuum eingeengt und der Rückstand per präparativer Dünnschichtchromatographie (Laufmittel: Cyclohexan /Essigsäureethylester = 7:3) gereinigt. Das erhaltene Produkt wurde über präparative HPLC nachgereinigt (Säule: Nucleodur C 18, 5 µm, Gravity 21x100, Eluent: Acetonitril/ Wasser + TFA 50% bis 70%). Man erhielt 6 mg (10% d. Th.) der Titelverbindung.
¹H-NMR (400 MHz, DMSO-d₆): δ = 0.94 (s, 3 H), 1.32 - 1.48 (m, 2 H), 1.57 - 1.71 (m, 2 H), 2.58 - 2.65 (m, 5 H), 5.38 (s, 2 H), 7.16 - 7.37 (m, 3 H), 7.54 - 7.68 (m, 1 H), 7.95 - 8.06 (m, 1 H), 9.14 - 9.26 (m, 1 H).

### Beispiel 62

### 8-(Cyclohexylmethoxy)-2-methyl-3-[5-(pyrimidin-2-yl)-1,3,4-oxadiazol-2-yl]imidazo[1,2-a]pyridin

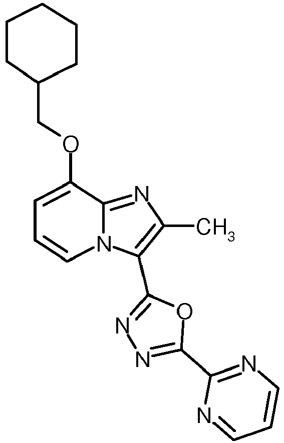

15 mg Pyrimidin-2-carbohydrazid (Beispiel 78A, 0.11 mmol, 1.1 Äquivalente) wurden vorgelegt und mit 8-(Cyclohexylmethoxy)-2-methylimidazo[1,2-a]pyridin-3-carbonylchlorid (Beispiel 26A, 0.1 mmol, 1 Äquivalent), gelöst in 0.6 ml Methylenchlorid, zugegeben. Dann wurde das Gemisch mit 0.02 mg Pyridin (0.3 mmol, 3 Äquivalente) versetzt und über Nacht bei RT geschüttelt. Nach dieser Zeit wurde der Ansatz mit 0.6 ml Methylenchlorid verdünnt und unter Eisbadkühlung nacheinander mit 0.05 mg Pyridin (0.6 mmol, 6 Äquivalente) und 0.112 mg Trifluormethansulfonsäure (0.4 mmol, 4 Äquivalente) versetzt und zuerst 1h bei 0°C und dann über Nacht bei RT geschüttelt. Das entstandene Produkt wurde über die präparative HPLC (Methode 11) gereinigt. Es wurden 13 mg (30% d. Th.; Reinheit 92%) der Titelverbindung erhalten.
LC-MS (Methode 12): Rₜ = 1.15 min
MS (ESpos): m/z = 391.2 (M+H)⁺

In Analogie zu Beispiel 62 wurden die in Tabelle 3 gezeigten Beispielverbindungen hergestellt, indem 8-(Cyclohexylmethoxy)-2-methylimidazo[1,2-a]pyridin-3-carbonylchlorid (Beispiel 26A) mit den entsprechenden Hydraziden umgesetzt wurde.

**Tabelle 3:**

| **Bei-spiel Nr.** | **IUPAC-Name** | **Analytischen Methoden** |
|---|---|---|
| | **Struktur** | |
| | **(Ausbeute)** | |
| **63** | {5-[8-(Cyclohexylmethoxy)-2-methylimidazo[1,2-a]pyridin-3-yl]-1,3,4-oxadiazol-2-yl}(phenyl)methanol | LC-MS (Methode 12): Rₜ = 1.16 min |
| | 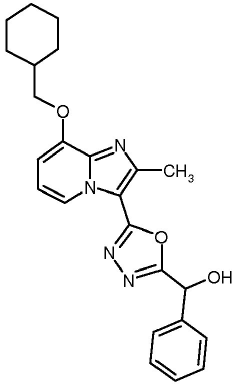 | MS (ESpos): m/z = 319.2 (M+H)⁺ |
| | (3% d. Th.) | |
| **64** | 8-(Cyclohexylmethoxy)-2-methyl-3-[5-(pyridin-3-yl)-1,3,4-oxadiazol-2-yl]imidazo[1,2-a] pyridin | LC-MS (Methode 12): Rₜ = 1.25 min |
| | 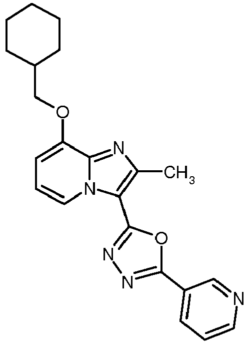 | MS (ESpos): m/z = 390.2 (M+H)⁺ |
| | (23% d. Th.) | |
| **65** | 1-{5-[8-(Cyclohexylmethoxy)-2-methylimidazo[1,2-a]pyridin-3-yl]-1,3,4-oxadiazol-2-yl}ethanol | LC-MS (Methode 12): Rₜ = 1.03 min |
| | 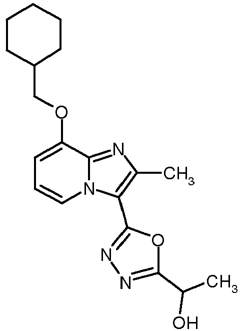 | MS (ESpos): m/z = 357.2 (M+H)⁺ |
| | (4% d. Th.) | |
| **66** | 8-(Cyclohexylmethoxy)-3-[5-(ethoxymethyl)-1,3,4-oxadiazol-2-yl]-2-methylimidazo[1,2-a]pyridin | LC-MS (Methode 12): Rₜ = 1.18 min |
| | 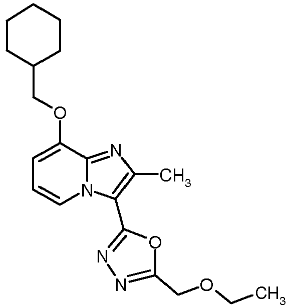 | MS (ESpos): m/z = 371.2 (M+H)⁺ |
| | (18% d. Th.) | |
| **67** | 8-(Cyclohexylmethoxy)-3-[5-(methoxymethyl)-1,3,4-oxadiazol-2-yl]-2-methylimidazo[1,2-a]pyridin | LC-MS (Methode 12): Rₜ = 1.14 min |
| | 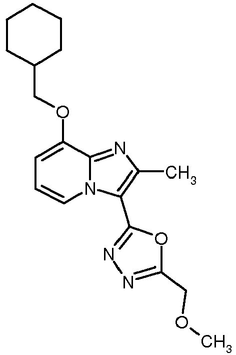 | MS (ESpos): m/z = 357.2 (M+H)⁺ |
| | (23% d. Th.; Reinheit 91%) | |
| **68** | 8-(Cyclohexylmethoxy)-3-[5-(2-methoxyethyl)-1,3,4-oxadiazol-2-yl]-2-methylimidazo[1,2-a]pyridin | LC-MS (Methode 12): Rₜ = 1.12 min |
| | 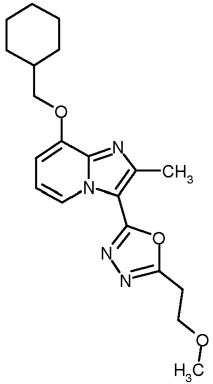 | MS (ESpos): m/z = 371.2 (M+H)⁺ |
| | (25% d. Th.) | |
| **69** | 8-(Cyclohexylmethoxy)-3-[5-(2-methoxyethyl)-1,3,4-oxadiazol-2-yl]-2-methylimidazo[1,2-a]pyridin | LC-MS (Methode 12): Rₜ = 1.23 min |
| | 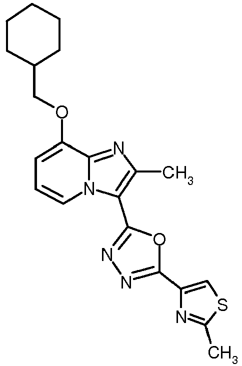 | MS (ESpos): m/z = 410.2 (M+H)⁺ |
| | (13% d. Th.) | |
| **70** | 3-(5-sec-Butyl-1,3,4-oxadiazol-2-yl)-8-(cyclohexylmethoxy)-2-methylimidazo[1,2-a]pyridin | LC-MS (Methode 12): Rₜ = 1.25 min |
| | 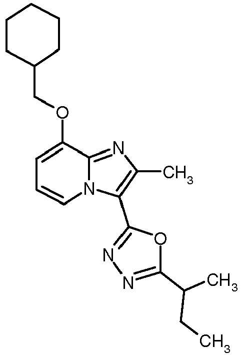 | MS (ESpos): m/z = 369.2 (M+H)⁺ |
| | (32% d. Th.) | |
| **71** | 1-({5-[8-(Cyclohexylmethoxy)-2-methylimidazo[1,2-a]pyridin-3-yl]-1,3,4-oxadiazol-2-yl}methyl)pyrrolidin-2-on | LC-MS (Methode 12): Rₜ = 1.07 min |
| | 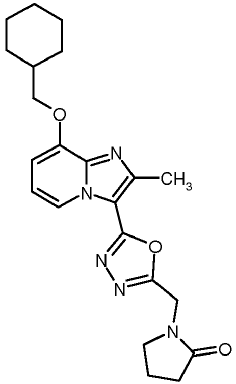 | MS (ESpos): m/z = 410.2 (M+H)⁺ |
| | (12% d. Th.) | |
| **72** | 8-(Cyclohexylmethoxy)-3-{5-[(3,5-dimethyl-1H-1,2,4-triazol-1-yl)methyl]-1,3,4-oxadiazol-2-yl}-2-methylimidazo[1,2-a]pyridin | LC-MS (Methode 12): Rₜ = 1.12 min |
| | 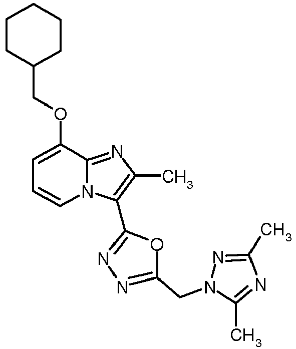 | MS (ESpos): m/z = 422.2 (M+H)⁺ |
| | (1% d. Th.) | |
| **73** | *rac*-8-(Cyclohexylmethoxy)-3-[5-(1,1-dioxidotetrahydrothiophen-3-yl)-1,3,4-oxadiazol-2-yl]-2-methylimidazo[1,2-a]pyridin | LC-MS (Methode 12): Rₜ = 1.08 min |
| | 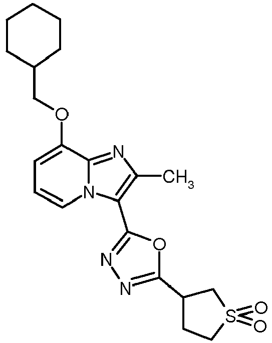 | MS (ESpos): m/z = 431.2 (M+H)⁺ |
| | (21% d. Th.) | |
| **74** | 8-(Cyclohexylmethoxy)-3-(5-cyclopropyl-1,3,4-oxadiazol-2-yl)-2-methylimidazo [1,2-a]pyridin | LC-MS (Methode 12): Rₜ = 1.18 min |
| | 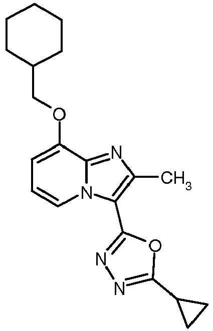 | MS (ESpos): m/z = 353.2 (M+H)⁺ |
| | (36% d. Th.; Reinheit 90%) | |

### Beispiel 75

### Ethyl-5-{8-[(2,6-difluorbenzyl)oxy]-2,6-dimethylimidazo[1,2-a]pyridin-3-yl}nicotinat Trifluoracetat

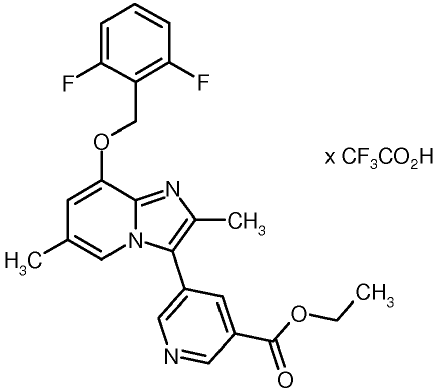

200 mg (0.55 mmol) 3-Brom-8-[(2,6-difluorbenzyl)oxy]-2,6-dimethylimidazo[1,2-a]pyridin aus Beispiel 30A, 166 mg (0.60 mmol) Ethyl-5-(4,4,5,5-tetramethyl-1,3,2-dioxaborolan-2-yl)nicotinat und 33 mg (0.04 mmol) Bis-(diphenylphosphino)ferrocen-Palladium(II)chlorid Dichlormethan-Komplex in 10.8 ml Acetonitril wurden unter Argon mit 2.16 ml 1 M wässriger Kaliumcarbonat-Lösung versetzt und über Nacht bei 90°C gerührt. Die Reaktionslösung wurde mit Wasser und TFA versetzt und in zwei Portionen mittels präparativer HPLC gereinigt (RP18 Säule, Laufmittel: Acetonitril/Wasser-Gradient unter Zusatz von 0.1% TFA). Es wurden 134 mg der Zielverbindung (42% d. Th., Reinheit 94%) erhalten.
LC-MS (Methode 1): Rₜ = 0.85 min
MS (ESpos): m/z = 438 (M-TFA+H)⁺
¹H-NMR (400 MHz, DMSO-d₆): δ = 2.28 (s, 3H), 2.33 (s, 3 H); 5.30 (s, 2 H); 6.89 (s, 1 H); 7.22 (t, 2 H); 7.53-7.63 (m, 1 H); 7.75 (s, 1 H).

### Beispiel 76

### 5-{8-[(2,6-Difluorbenzyl)oxy]-2,6-dimethylimidazo[1,2-a]pyridin-3-yl}nicotinsäure Trifluoracetat

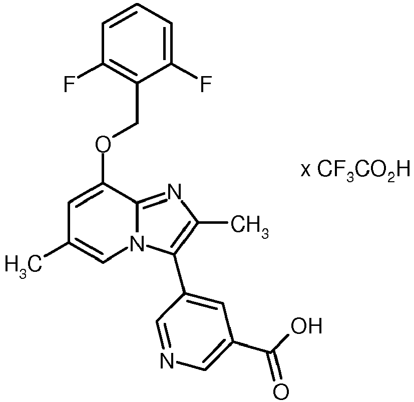

152 mg (0.28 mmol) Ethyl-5-{8-[(2,6-difluorbenzyl)oxy]-2,6-dimethylimidazo[1,2-a]pyridin-3-yl}nicotinat Trifluoracetat aus Beispiel 75 in 5.9 ml THF/Ethanol (5/1) wurden mit 1.38 ml IN wässriger Lithiumhydroxid-Lösung versetzt und 4 h bei Raumtemperatur gerührt. Die Mischung wurde unter Eiskühlung mit 1 N wässriger Salzsäure-Lösung auf pH = 4 gebracht und anschließend am Rotationsverdampfer vom Lösemittel befreit. Es wurden 189 mg des Rohproduktes erhalten. 80 mg dieses Rohproduktes wurden in Acetonitril/Wasser/TFA gelöst und mittels präparativer HPLC gereinigt (RP18 Säule, Laufmittel: Acetonitril/Wasser-Gradient unter Zusatz von 0.1% TFA). Es wurden 55 mg der Titelverbindung erhalten.
LC-MS (Methode 1): Rₜ = 0.73 min
MS (ESpos): m/z = 410 (M-TFA+H)⁺
¹H NMR (400 MHz, DMSO-*d*₆) δ = 2.38 (s, 3 H), 2.40 (s, 3 H), 5.48 (s, 2 H), 7.29 (t, 2 H), 7.52 - 7.69 (m, 2 H), 8.09 (s, 1 H), 8.48 (s, 1 H), 8.98 (d, 1 H), 9.27 (d, 1 H), 13.78 (br. s, 1H).

### Beispiel 77

### 5-{8-[(2,6-Difluorbenzyl)oxy]-2,6-dimethylimidazo[1,2-a]pyridin-3-yl}nicotinamid

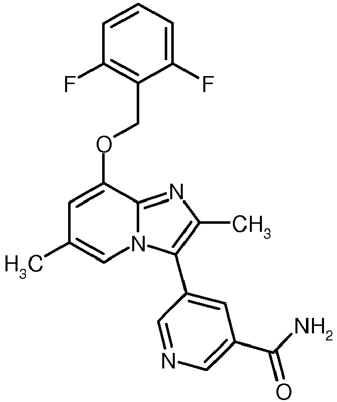

50 mg (0.09 mmol) 5-{8-[(2,6-Difluorbenzyl)oxy]-2,6-dimethylimidazo[1,2-a]pyridin-3-yl}nicotinsäure Trifluoracetat aus Beispiel 76 in 1.8 ml Dichlormethan wurden mit 54 mg (0.28 mmol) 1-(3-Dimethylaminopropyl)-3-ethylcarbodiimidhydrochlorid und 38 mg (0.28 mmol) 1-Hydroxy-1H-benzotriazol-Hydrat versetzt und 10 min bei Raumtemperatur gerührt. Anschließend wurden 50 mg (0.94 mmol) Ammoniumchlorid und 158 mg (1.22 mmol) *N,N-*Diisopropylethylamin zugegeben und es wurde über Nacht bei Raumtemperatur gerührt. Das Reaktionsgemisch wurde eingeengt und der Rückstand wurde auf Kieselgel aufgezogen und mittels Kieselgelchromatographie (Laufmittel: Dichlormethan/2 N Ammoniak in Methanol 50/1, 20/1) gereinigt. Es wurden 25 mg (66% d. Th.) der Titelverbindung erhalten.
LC-MS (Methode 1): Rₜ = 0.69 min
MS (ESpos): m/z = 409 (M+H)⁺
¹H NMR (400 MHz, DMSO-*d*₆) δ = 2.29 (s, 3 H), 2.31 (s, 3 H), 5.30 (s, 2 H), 6.83 (s, 1 H), 7.23 (t, 2 H), 7.55 - 7.64 (m, 1 H), 7.72 (br. s, 1 H), 7.78 (s, 1 H), 8.26 (br. s, 1 H), 8.29-8.33 (m, 1 H), 8.82 (d, 1 H), 9.08 (d, 1 H).

### Beispiel 78

### N-Cyclopropyl-5-{8-[(2,6-difluorbenzyl)oxy]-2,6-dimethylimidazo[1,2-a]pyridin-3-yl}nicotinamid Trifluoracetat

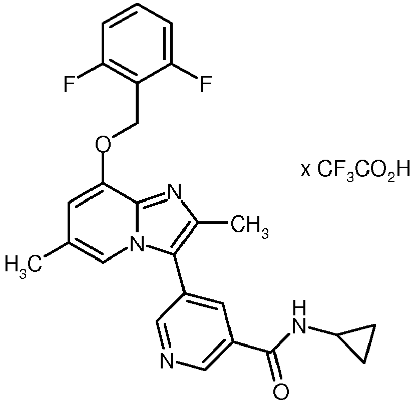

54 mg (0.10 mmol) 5-{8-[(2,6-Difluorbenzyl)oxy]-2,6-dimethylimidazo[1,2-a]pyridin-3-yl}nicotinsäure Trifluoracetat aus Beispiel 76, 49 mg (0.15 mmol) (Benzotriazol-1-yloxy)bisdimethylaminomethyliumfluoroborat und 0.056 ml (0.51 mmol) 4-Methylmorpholin in 0.65 ml DMF wurden mit 0.012 m (0.12 mmol) Cyclopropylamin versetzt und über Nacht bei Raumtemperatur gerührt. Die Reaktionslösung wurde mit Wasser und TFA verdünnt und mittels präparativer HPLC gereinigt (RP18 Säule, Laufmittel: Acetonitril/Wasser-Gradient unter Zusatz von 0.1% TFA). Es wurden 35 mg (60% d. Th;) der Titelverbindung erhalten.
LC-MS (Methode 1): Rₜ = 0.75 min
MS (ESpos): m/z = 449 (M-TFA+H)⁺
¹H NMR (400 MHz, DMSO-*d*₆) δ = 0.58-0.62 (m, 2 H), 0.72-0.79 (m, 2 H), 2.37 (s, 3 H), 2.39 (s, 3 H), 2.85-2.93 (m, 1 H), 5.48 (s, 2 H), 7.29 (t, 2 H), 7.40 - 7.68 (m, 2 H), 8.04 (br. s, 1 H), 8.38 (s, 1 H), 8.79 (d, 1 H), 8.88 (d, 1 H), 9.17 (s, 1 H).

### Beispiel 79

### Methyl-2-{8-[(2,6-difluorbenzyl)oxy]-2,6-dimethylimidazo[1,2-a]pyridin-3-yl}pyrimidin-4-carboxylat

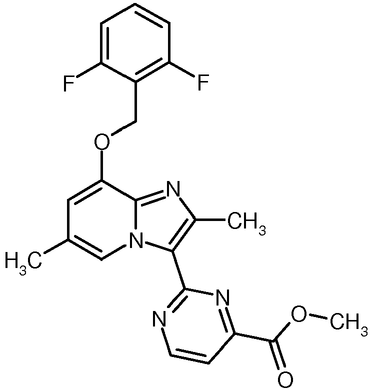

200 mg (0.61 mmol) 8-[(2,6-Difluorbenzyl)oxy]-2,6-dimethylimidazo[1,2-a]pyridin-3-carboximidamid aus Beispiel 42A wurden in 7 ml Ethanol unter Rückfluss gelöst. Anschließend wurde die Lösung auf 50°C abgekühlt und mit 51 mg (0.76 mmol) Natriumethylat versetzt. Dann wurden 207 mg (1.21 mmol) Ethyl-4-(dimethylamino)-2-oxobut-3-enoat gelöst in 0.26 ml Ethanol zu der Reaktionslösung gegeben und 4 Tage bei 50°C gerührt. Es wurden nochmals 52 mg (0.24 mmol) Ethyl-4-(dimethylamino)-2-oxobut-3-enoat und 10 mg (0.15mmol) Natriumethylat zu der Reaktionslösung gegeben und 3 Tage bei 50°C gerührt. Das Reaktionsgemisch wurde über Molsieb abfiltriert, mit Ethanol gewaschen und das Filtrat wurde im Vakuum eingeengt. Das Filtrat wurde mittels präparativer HPLC gereinigt (RP18 Säule, Laufmittel: Acetonitril/Wasser-Gradient unter Zusatz von 0.1% TFA). Es wurden 19 mg der Zielverbindung (6% d. Th., Reinheit 80%%) erhalten.
LC-MS (Methode 17): Rₜ = 2.12 min
MS (ESpos): m/z = 425 (M+H)⁺

### Beispiel 80

### 2-{8-[(2,6-Difluorbenzyl)oxy]-2,6-dimethylimidazo[1,2-a]pyridin-3-yl}pyrimidin-4-carboxamid Trifluoracetat

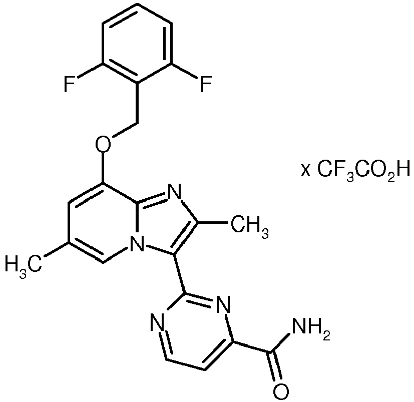

6.7 mg (0.016 mmol) Methyl-2-{8-[(2,6-difluorbenzyl)oxy]-2,6-dimethylimidazo[1,2-a]pyridin-3-yl}pyrimidin-4-carboxylat aus Beispiel 79 wurden mit 0.29 ml (2.05 mmol) 7 N Ammoniak-Lösung in Methanol versetzt und über Nacht bei 50°C gerührt. Das Reaktionsgemisch wurde im Vakuum eingeengt und der Rückstand mittels Dickschichtchromatographie gereinigt (Laufmittel: Dichlormethan/Methanol = 20/1). Die produkthaltigen Fraktionen wurden nochmals gereinigt [Säule: Sunfire C18, 5 µm, 250 x 20 mm; Eluent: 52% Wasser, 35% Acetonitril + 13% 1%ige wässrige TFA; Fluß: 25 ml/min; 40°C; Detektion: 210 nm], eingeengt und lyophilisiert. Es wurden 1.2 mg der Zielverbindung (14% d. Th.) erhalten.
LC-MS (Methode 1): Rₜ = 0.80 min
MS (ESpos): m/z = 410 (M+H)⁺
¹H NMR (400 MHz, DMSO-*d*₆) δ = 2.40 (s, 3 H), 2.74 (s, 3 H), 5.32 (s, 2 H), 7.03 (s, 1 H), 7.23 (t, 2 H), 7.53 - 7.65 (m, 1 H), 7.73 (d, 1 H), 7.98 - 8.10 (m, 2 H), 9.13 (d, 1 H), 9.30 (s, 1 H).

### Beispiel 81

### N-Cyclopropyl-2-{8-[(2,6-difluorbenzyl)oxy]-2,6-dimethylimidazo[1,2-a]pyridin-3-yl}pyrimidin-4-carboxamid Trifluoracetat

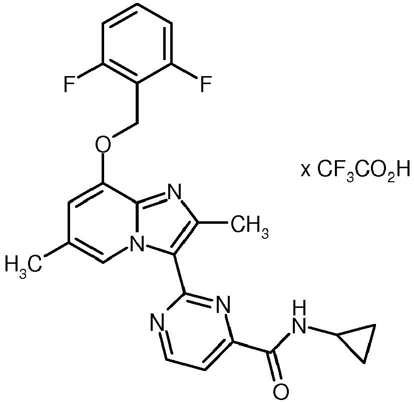

15 mg (0.028 mmol) Methyl-2-{8-[(2,6-difluorbenzyl)oxy]-2,6-dimethylimidazo[1,2-a]pyridin-3-yl}pyrimidin-4-carboxylat Beispiel 79 wurden mit 0.256 ml (3.70 mmol) Cyclopropylamin versetzt und über Nacht bei 50°C gerührt. Das Reaktionsgemisch wurde im Vakuum eingeengt und der Rückstand mittels Dickschichtchromatographie gereinigt (Laufmittel: Dichlormethan/Methanol = 20/1). Die produkthaltigen Fraktionen wurden nochmals gereinigt [Säule: Shield RP18, 5 µm, 19 x 100 mm; Eluent (Gradient): Wasser/Acetonitril/1%ige wässrige TFA; Fluß: 40 ml/min; 25°C; Detektion: 210 nm], eingeengt und lyophilisiert. Es wurde 1 mg der Zielverbindung (6% d. Th., Reinheit 90%) erhalten.
LC-MS (Methode 1): Rₜ = 0.89 min
MS (ESpos): m/z = 450 (M+H)⁺
¹H NMR (400 MHz, DMSO-*d*₆) δ = 0.59 - 0.65 (m, 2 H), 0.69 - 0.74 (2 H), 2.33 (s, 3 H), 2.70 (s, 3 H), 2.82 - 2.94 (m, 1 H), 5.30 (s, 2 H), 7.05 - 7.22 (m, 3 H), 7.51 - 7.62 (m, 1 H), 7.67 - 7.72 (m, 1 H), 8.53 - 8.62 (m, 1 H), 9.08 (d, 1 H), 9.30 (s, 1 H).

### Beispiel 82

### 8-[(2,6-Difluorbenzyl)oxy]-2,6-dimethyl-3-(2H-1,2,3-triazol-4-yl)imidazo[1,2-a]pyridin

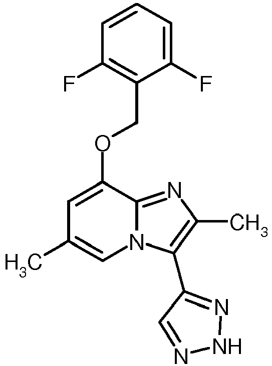

762 mg (1,44 mmol; Reinheit ca. 60%) 8-[(2,6-Difluorbenzyl)oxy]-3-ethinyl-2,6-dimethylimidazo[1,2-a]pyridin aus Beispiel 77A wurden in einem trockenen Reaktionskolben in 1.44 ml DMF/Methanol (4/1) vorgelegt, mit 14 mg (0.07 mmol) Kupfer(I)iodid und 249 mg (2.16 mmol) Azido(trimethyl)silan versetzt und unter Argon anschließend über Nacht bei 100°C gerührt. Die Reaktionslösung wurde mit Wasser/Acetonitril/TFA verdünnt und mittels präparativer HPLC gereinigt (RP18 Säule, Laufmittel: Acetonitril/Wasser-Gradient unter Zusatz von 0.1% TFA). Es wurden 471 mg der Zielverbindung als TFA-Salz (59% d. Th., Reinheit 84%) erhalten. 20 mg von dieser Fraktion wurden mittels Dickschlichtchromatographie gereinigt (Laufmittel: Dichlormethan/2N Ammoniak in Methanol 20/1). Hieraus wurden 6.4 mg der Zielverbindung erhalten.
LC-MS (Methode 1): Rₜ = 0.74 min
MS (ESpos): m/z = 356 (M+H)⁺
¹H NMR (400 MHz, DMSO-*d*₆) δ = 2.33 (s, 3 H), 2.43 (s, 3 H), 5.30 (s, 2 H), 6.83 (s, 1 H), 7.23 (t, 2 H), 7.54 - 7.66 (m, 1 H), 8.29 (br. s, 1 H), 8.45 (s, 1 H), 15.38 (br. s, 1 H).

### Beispiel 83

### 1-(4-{8-[(2,6-Difluorbenzyl)oxy]-2,6-dimethylimidazo[1,2-a]pyridin-3-yl}-2H-1,2,3-triazol-2-yl)-2-methylpropan-2-amin

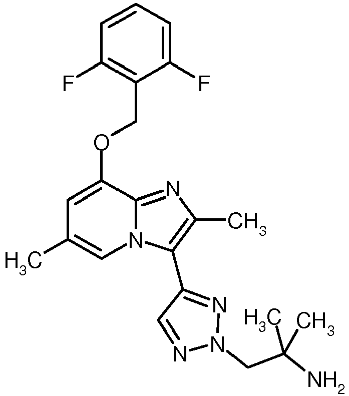

46 mg (0.06 mmol; Reinheit ca. 75%) 8-[(2,6-Difluorbenzyl)oxy]-2,6-dimethyl-3-[2-(2-methyl-2-nitropropyl)-2H-1,2,3-triazol-4-yl]imidazo[1,2-a]pyridin Trifluoracetat aus Beispiel 56A in 0.25 ml Ethanol wurden mit ca. 80 mg Raney-Nickel (50%ige wässrige Aufschlämmung) versetzt und über Nacht unter Normaldruck bei Raumtemperatur hydriert. Das Reaktionsgemisch wurde über Celite filtriert und gut mit Ethanol sowie einem Gemisch Dichlormethan/2 N Ammoniak-Lösung in Methanol (20/1) nachgewaschen. Das Filtrat wurde im Vakuum eingeengt. Der Rückstand wurde mittels Dickschichtchromatographie gereinigt (Laufmittel: Dichlormethan/2N Ammoniak in Methanol 20/1). Es wurden 19 mg der Zielverbindung (73% d. Th.) erhalten.
LC-MS (Methode 1): Rₜ = 0.61 min
MS (ESpos): m/z = 427 (M+H)⁺
¹H NMR (400 MHz, DMSO-*d*₆) δ = 1.08 (s, 6 H), 1.68 (br. s, 2 H), 2.32 (s, 3 H), 2.45 (s, 3 H), 4.41 (s, 2 H), 5.31 (s, 2 H), 6.87 (s, 1 H), 7.22 (t, 2 H), 7.54 - 7.64 (m, 1 H), 8.18 (s, 1 H), 8.39 (s, 1 H).

### Beispiel 84

### 8-[(3-Fluorpyridin-2-yl)methoxy]-2,6-dimethyl-3-(1H-pyrazol-4-yl)imidazo[1,2-a]pyridin

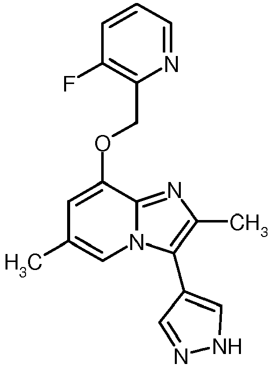

0.943 g (2.03 mmol) 3-Brom-8-[(3-fluorpyridin-2-yl)methoxy]-2,6-dimethylimidazo[1,2-a]pyridin Trifluoracetat aus Beispiel 34A in Toluol/Ethanol/Wasser (7.2 ml/14.4 ml/7.2 ml) wurden unter Argon mit 1.08 g (5.08 mmol) [1-(tert.-Butoxycarbonyl)-1H-pyrazol-4-yl]boronsäure [herstellbar aus dem korrespondierenden Pinacol-Boronsäueester nach literatubekannten Methoden, z. B. WO2009/155527; WO2012/6760], 1.29 g (6.09 mmol) Kaliumphosphat und 104 mg (0.20 mmol) Bis(tri-tert.-butyl-phosphin)palladium(0) versetzt und 30 min in einem auf 120°C vorgeheizten Ölbad gerührt. Das Reaktionsgemisch wurde eingeengt und der Rückstand in Essigsäureethylester/Wasser aufgenommen. Es bildete sich ein unlöslicher Feststoff. Das Lösungsmittel wurde vom Feststoff abdekantiert und der Feststoff wurde mit Acetonitril/Wasser gerührt. Anschließend wurde das Acetonitril abdestilliert und das wässrige Gemisch wurde gekühlt. Der Niederschlag wurde abfiltriert und im Hochvakuum getrocknet. Es wurden 581 mg der Zielverbindung (84% d. Th.) erhalten.
LC-MS (Methode 1): Rₜ = 0.66 min
MS (ESpos): m/z = 338 (M+H)⁺
¹H NMR (400 MHz, DMSO-*d*₆) δ = 2.27 (s, 3 H), 2.30 (s, 3 H), 5.38 (s, 2 H), 6.72 (s, 1 H), 7.55 - 7.63 (m, 1 H), 7.70 (s, 1 H), 7.75 - 7.89 (m, 2 H), 8.12 (br. s, 1 H), 8.50 (d, 1 H), 13.28 (br. s, 1 H).

### Beispiel 85

### 1-(4-{8-[(3-Fluorpyridin-2-yl)methoxy]-2,6-dimethylimidazo[1,2-a]pyridin-3-yl}-1H-pyrazol-1-yl)-2-methylpropan-2-amin

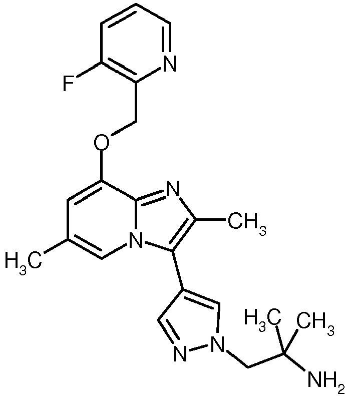

65 mg (0.148 mmol) 8-[(3-Fluorpyridin-2-yl)methoxy]-2,6-dimethyl-3-[1-(2-methyl-2-nitropropyl)-1H-pyrazol-4-yl]imidazo[1,2-a]pyridin aus Beispiel 55A in 1.5 ml Ethanol wurden mit ca. 197 mg Raney-Nickel (50%ige wässrige Aufschlämmung) versetzt und über Nacht unter Normaldruck bei Raumtemperatur hydriert. Das Reaktionsgemisch wurde über Celite filtriert und mit Dichlormethan/2 N Ammoniak-Lösung in Methanol (20/1) gewaschen. Das Filtrat wurde eingeengt und der Rückstand mittels präparativer Dickschichtchromatographie gereinigt (Laufmittel: Dichlormethan/2 N Ammoniak-Lösung in Methanol (20/1)). Es wurden 42 mg der Zielverbindung (69% d. Th.) erhalten.
LC-MS (Methode 1): Rₜ = 0.46 min
MS (ESpos): m/z = 409 (M+H)⁺
¹H NMR (400 MHz, DMSO-*d*₆) δ = 1.02 (s, 6 H), 1.61 (br. s, 2 H), 2.28 (s, 3 H), 2.31 (s, 3 H), 4.05 (s, 2 H), 5.38 (s, 2 H), 6.72 (s, 1 H), 7.55 - 7.62 (m, 1 H), 7.71 (s, 1 H), 7.78 (s, 1 H), 7.86 (t, 1 H), 8.10 (s, 1 H), 8.49 (d, 1 H).

### Beispiel 86

### 2,6-Dimethyl-3-(1H-pyrazol-4-yl)-8-[(2,3,6-trifluorbenzyl)oxy]imidazo[1,2-a]pyridin

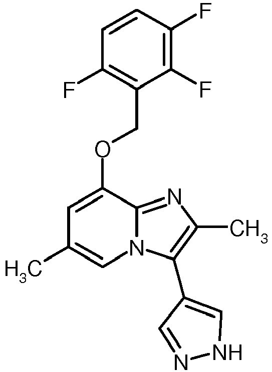

2.47 g (6.42 mmol) 3-Brom-2,6-dimethyl-8-[(2,3,6-trifluorbenzyl)oxy]imidazo[1,2-a]pyridin aus Beispiel 32A in Toluol/Ethanol/Wasser (22.7 ml/45.3 ml/22.7 ml) wurden unter Argon mit 3.40 g (16.04 mmol) [1-(tert.-Butoxycarbonyl)-1H-pyrazol-4-yl]boronsäure [herstellbar aus dem korrespondierenden Pinacol-Boronsäueester nach literatubekannten Methoden, z. B. WO2009/155527; WO2012/6760], 4.09 g (19.25 mmol) Kaliumphosphat und 328 mg (0.64 mmol) Bis(tri-tert.-butyl-phosphin)palladium(0) versetzt und 45 min in einem auf 120°C vorgeheizten Ölbad gerührt. Das Reaktionsgemisch wurde eingeengt und der Rückstand wurde in Dichlormethan/Wasser aufgenommen. Es wurde vom unlöslichen Feststoff abfiltriert und dieser wurde im Hochvakuum getrocknet. Die wässrige Phase wurde zweimal mit Dichlormethan extrahiert und die vereinten organischen Phasen wurden über Natriumsulfat getrocknet und filtriert. Das Filtrat wurde eingeengt und der Rückstand zusammen mit dem Feststoff mittels KieselgelChromatographie gereinigt (Laufmittel: Dichlormethan/Methanol 50/1). Es wurden 1.61 g der Zielverbindung (66% d. Th.) erhalten.
LC-MS (Methode 1): Rₜ = 0.67 min
MS (ESpos): m/z = 373 (M+H)⁺
¹H NMR (400 MHz, DMSO-*d*₆) δ = 2.29 (s, 3 H), 2.30 (s, 3 H), 5.32 (s, 2 H), 6.72 (s, 1 H), 7.27 - 7.34 (m, 1 H), 7.61 - 7.73 (m, 2 H), 7.80 (s, 1 H), 8.14 (s, 1 H), 13.28 (br. s, 1 H).

### Beispiel 87

### 1-(4-{2,6-Dimethyl-8-[(2,3,6-trifluorbenzyl)oxy]imidazo[1,2-a]pyridin-3-yl}-1H-pyrazol-1-yl)-2-methylpropan-2-amin

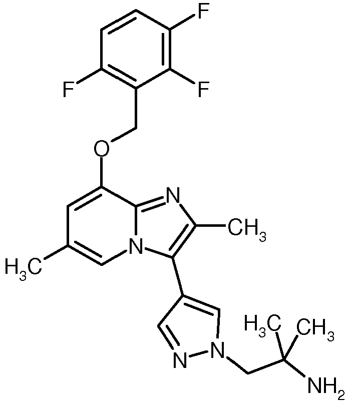

140 mg (0.26 mmol) 2,6-Dimethyl-3-[1-(2-methyl-2-nitropropyl)-1H-pyrazol-4-yl]-8-[(2,3,6-trifluorbenzyl)oxy]imidazo[1,2-a]pyridin aus Beispiel 54A in 1.1 ml Ethanol wurden mit ca. 349 mg Raney-Nickel (50%ige wässrige Aufschlämmung) versetzt und über Nacht unter Normaldruck bei Raumtemperatur hydriert. Das Reaktionsgemisch wurde über Celite filtriert und mit Dichlormethan/2 N Ammoniak-Lösung in Methanol (20/1) nachgewaschen. Das Filtrat wurde eingeengt und der Rückstand mittels Kieselgelchromatographie gereinigt (Laufmittel: Dichlormethan/2 N Ammoniak-Lösung in Methanol (60/1)). Es wurden 73 mg der Zielverbindung (61% d. Th.) erhalten.
LC-MS (Methode 1): Rₜ = 0.59 min
MS (ESpos): m/z = 444 (M+H)⁺
¹H NMR (400 MHz, DMSO-*d*₆) δ = 1.02 (s, 6 H), 1.61 (br. s, 2 H), 2.25 - 2.35 (m, 6 H), 4.05 (s, 2 H), 5.32 (s, 2 H), 6.72 (s, 1 H), 7.25 - 7.33 (m, 1 H), 7.62 - 7.72 (m, 1 H), 7.73 (s, 1 H), 7.78 (s, 1 H), 8.11 (s, 1 H).

### Beispiel 88

### 8-[(2,6-Difluorbenzyl)oxy]-2,6-dimethyl-3-(1H-pyrazol-4-yl)imidazo[1,2-a]pyridin

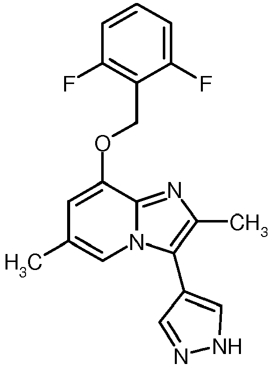

1.0 g (2.72 mmol) 3-Brom-8-[(2,6-difluorbenzyl)oxy]-2,6-dimethylimidazo[1,2-a]pyridin aus Beispiel 30A in Toluol/Ethanol/Wasser (9.6 ml/19.2 ml/9.6 ml) wurden unter Argon mit 1.44 g (6.81 mmol) [1-(tert.-Butoxycarbonyl)-1H-pyrazol-4-yl]boronsäure [herstellbar aus dem korrespondierenden Pinacol-Boronsäueester nach literatubekannten Methoden, z. B. WO2009/155527; WO2012/6760], 1.73 g (8.17 mmol) Kaliumphosphat und 139 mg (0.27 mmol) Bis(tri-tert.-butyl-phosphin)palladium(0) versetzt und 30 min in einem auf 120°C vorgeheizten Ölbad gerührt. Das Reaktionsgemisch wurde eingeengt, der Rückstand in Essigsäureethylester/Wasser aufgenommen und die wässrige Phase zweimal mit Essigsäureethylester extrahiert. Die vereinten organischen Phasen wurden über Natriumsulfat getrocknet und filtriert. Das Filtrat wurde eingeengt und der Rückstand mittels KieselgelChromatographie gereinigt (Laufmittel: Dichlormethan/Methanol 50/1 nach 20/1). Es wurden 889 mg der Zielverbindung (89% d. Th.) erhalten.
LC-MS (Methode 1): Rₜ = 0.73 min
MS (ESpos): m/z = 355 (M+H)⁺
¹H NMR (400 MHz, DMSO-*d*₆) δ = 2.26 - 2.30 (m, 6 H), 5.28 (s, 2 H), 6.72 (s, 1 H), 7.22 (t, 2 H), 7.54 - 7.63 (m, 1 H), 7.70 (s, 1 H), 7.80 (s, 1 H), 8.14 (s, 1 H), 13.28 (br. s, 1 H).

### Beispiel 89

### 1-(4-{8-[(2,6-Difluorbenzyl)oxy]-2,6-dimethylimidazo[1,2-a]pyridin-3-yl}-1H-pyrazol-1-yl)-2-methylpropan-2-amin

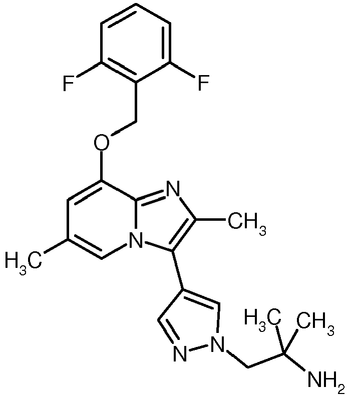

412 mg (0.91 mmol) 8-[(2,6-Difluorbenzyl)oxy]-2,6-dimethyl-3-[1-(2-methyl-2-nitropropyl)-1H-pyrazol-4-yl]imidazo[1,2-a]pyridin aus Beispiel 53A in 3.75 ml Ethanol wurden mit ca. 1200 mg Raney-Nickel (50%ige wässrige Aufschlämmung) versetzt und über Nacht unter Normaldruck bei Raumtemperatur hydriert. Das Reaktionsgemisch wurde über Celite filtriert und mit Ethanol, Dichlormethan, Ethanol und THF gewaschen. Das Filtrat wurde eingeengt und der Rückstand mittels Kieselgelchromatographie gereinigt (Laufmittel: Dichlormethan/2 N Ammoniak-Lösung in Methanol (60/1)). Es wurden 263 mg der Zielverbindung (68% d. Th.) erhalten.
LC-MS (Methode 1): Rₜ = 0.59 min
MS (ESpos): m/z = 426 (M+H)⁺
¹H NMR (400 MHz, DMSO-*d*₆) δ = 1.04 (s, 6 H), 1.59 (br. s, 2 H), 2.27 - 2.32 (m, 6 H), 4.07 (s, 2 H), 5.28 (s, 2 H), 6.72 (s, 1 H), 7.22 (t, 2 H), 7.54 - 7.64 (m, 1 H), 7.71 (s, 1 H), 7.78 (s, 1 H), 8.09 (s, 1 H).

### Beispiel 90

### 1-(4-{8-[(2,6-Difluorbenzyl)oxy]-2-methylimidazo[1,2-a]pyridin-3-yl}-1H-pyrazol-1-yl)-2-methylpropan-2-amin

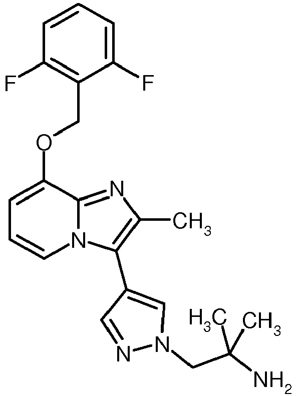

151 mg (0.32 mmol; Reinheit 93%) 8-[(2,6-Difluorbenzyl)oxy]-2-methyl-3-[1-(2-methyl-2-nitropropyl)-1H-pyrazol-4-yl]imidazo[1,2-a]pyridin aus Beispiel 52A in 3.3 ml DMF wurden mit ca. 150 mg Raney-Nickel (50%ige wässrige Aufschlämmung) versetzt und über Nacht unter Normaldruck bei Raumtemperatur hydriert. Dann wurden 150 mg Raney-Nickel (50%ige wässrige Aufschlämmung) zugegeben und wieder über Nacht unter Normaldruck hydriert. Das Reaktionsgemisch wurde über Celite filtriert, mit Dichlormethan gewaschen, das Filtrat eingeengt und der Rückstand mittels präparativer HPLC gereinigt (RP18 Säule, Laufmittel: Acetonitril/Wasser-Gradient unter Zusatz von 0.1% TFA). Die Produktfraktionen wurden mit Dichlormethan und gesättigter wässriger Natriumhydrogencarbonat-Lösung versetzt und die wässrige Phase wurde dreimal mit Dichlormethan extrahiert. Die vereinten organischen Phasen wurden über Natriumsulfat getrocknet, filtriert, das Filtrat wurde eingeengt und lyophilisiert. Das Produkt wurde erneut in Dichlormethan gelöst, mit gesättigter wässriger Natriumhydrogencarbonat-Lösung versetzt und die wässrige Phase dreimal mit Dichlormethan extrahiert. Die vereinten organischen Phasen wurden über Natriumsulfat getrocknet, filtriert, das Filtrat wurde eingeengt und lyophilisiert. Es wurden 28 mg der Zielverbindung (21% d. Th.) erhalten.
LC-MS (Methode 1): Rₜ = 0.48 min
MS (ESpos): m/z = 412 (M+H)⁺
¹H NMR (400 MHz, DMSO-*d*₆) δ = 1.04 (s, 6 H), 1.68 (br. s, 2 H), 2.31 (s, 3 H), 4.05 (s, 2 H), 5.31 (s, 2 H), 6.78 - 6.84 (m, 2 H), 7.22 (t, 2 H), 7.54 - 7.63 (m, 1 H), 7.78 (s, 1 H), 7.88 - 7.92 (m, 1 H), 8.11 (s, 1 H).

### Beispiel 91

### 2-(4-{8-[(2,6-Difluorbenzyl)oxy]-2,6-dimethylimidazo[1,2-a]pyridin-3-yl}-1H-pyrazol-1-yl)ethanol

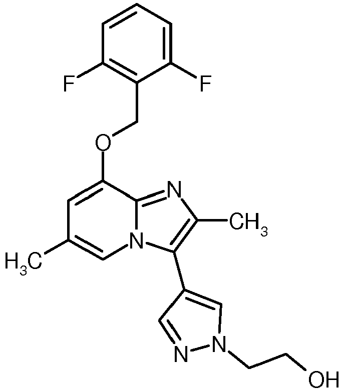

1.40 g (3.95 mmol) 8-[(2,6-Difluorbenzyl)oxy]-2,6-dimethyl-3-(1H-pyrazol-4-yl)imidazo[1,2-a]pyridin aus Beispiel 88 in 21.6 DMF wurden mit 3.35 g (10.27 mmol) Cäsiumcarbonat, 66 mg (0.40 mmol) Kaliumiodid und 0.40 ml (5.14 mmol) Iodethanol versetzt und über Nacht bei 70°C gerührt. Nach dem Abkühlen wurde der Feststoff abfiltriert, mit Dichlormethan/Methanol (20/1) gewaschen, das Filtrat eingeengt und der Rückstand mittels Kieselgelchromatographie gereinigt (Lösungsmittel: Dichlormethan/Methanol = 80/1). Es wurden 962 mg der Zielverbindung (54% d. Th.; Reinheit ca. 90%) erhalten. Die Mischfraktionen der Kieselgelchromatographie wurden eingeengt und der Rückstand mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt und die wässrige Phase zweimal mit Dichlormethan extrahiert. Die vereinten organischen Phasen wurden über Natriumsulfat getrocknet, filtriert, das Filtrat eingeengt und lyophilisiert. Es wurden nochmals 211 mg der Zielverbindung (10% d. Th.; Reinheit ca. 80%) erhalten.
LC-MS (Methode 1): Rₜ = 0.71 min
MS (ESpos): m/z = 399 (M+H)⁺
¹H NMR (400 MHz, DMSO-*d*₆) δ = 2.25 - 2.33 (m, 6 H), 3.80 (q, 2 H), 4.23 (t, 2 H), 4.96 (t, 1 H), 5.28 (s, 2 H), 6.73 (s, 1 H), 7.22 (t, 2 H), 7.54 - 7.65 (m, 1 H), 7.72 (s, 1 H), 7.78 (s, 1 H), 8.10 (s, 1 H).

### Beispiel 92

### 8-[(2,6-Difluorbenzyl)oxy]-3-{1-[2-(4,4-difluorpiperidin-1-yl)ethyl]-1H-pyrazol-4-yl}-2,6-dimethylimidazo[1,2-a]pyridin

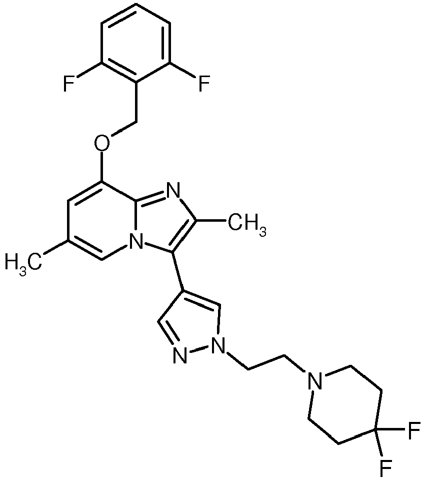

106 mg (0.20 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethylmethansulfonat aus Beispiel 57A wurden mit 0.07 ml (0.40 mmol) *N,N*-Diisopropylethylamin, 0.08 ml (0.60 mmol) Triethylamin, 2.4 mg (0.02 mmol) 4-Dimethylaminopyridin, 60 mg (0.40 mmol) Natriumiodid und 157 mg (1.0 mmol) 4,4-Difluorpiperidinhydrochlorid in 2 ml THF versetzt. Das Gemisch wurde über Nacht bei Rückfluss gerührt. Nach dem Abkühlen wurde das Reaktionsgemisch mit Essigsäureethylester verdünnt und mit Wasser/gesättigter wässriger Natriumchlorid-Lösung (1/1) gewaschen. Die wässrige Phase wurde zweimal mit Essigsäureethylester extrahiert, die vereinten organischen Phasen wurden über Natriumsulfat getrocknet und filtriert. Das Filtrat wurde eingeengt und im Hochvakuum getrocknet. Der Rückstand wurde mittels Dickschichtchromatographie gereinigt (Lösungsmittel: Essigsäureethylester/Cyclohexan = 4/1). Es wurden 16 mg der Zielverbindung (16% d. Th.) erhalten.
LC-MS (Methode 1): Rₜ = 0.67 min
MS (ESpos): m/z = 502 (M+H)⁺
¹H NMR (400 MHz, DMSO-*d*₆) δ = 1.85 - 2.00 (m, 4 H), 2.25 - 2.32 (m, 6 H), 2.50 - 2.64 (m, 4 H), 2.87 (t, 2 H), 4.30 (t, 2 H), 5.28 (s, 2 H), 6.73 (s, 1 H), 7.23 (t, 2 H), 7.54 - 7.63 (m, 1 H), 7.69 (s, 1 H), 7.76 (s, 1 H), 8.17 (s, 1 H).

### Beispiel 93

### 8-[(2,6-Difluorbenzyl)oxy]-3-{1-[2-(1,1-dioxidothiomorpholin-4-yl)ethyl]-1H-pyrazol-4-yl}-2,6-dimethylimidazo[1,2-a]pyridin

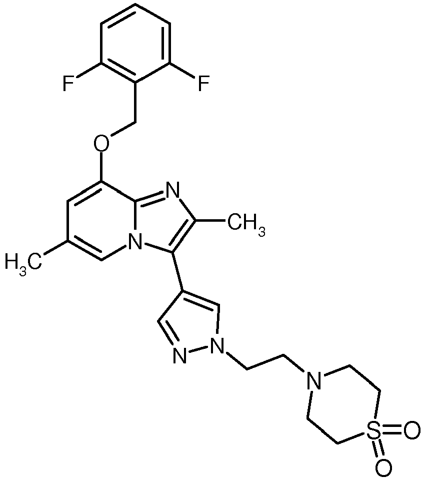

130 mg (0.25 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethylmethansulfonat aus Beispiel 57A, 0.17 ml (0.98 mmol) *N,N-*Diisopropylethylamin, 3 mg (0.025 mmol) 4-Dimethylaminopyridin und 74 mg (0.49 mmol) Natriumiodid in 2.4 ml THF wurden mit 135 mg (0.98 mmol) Thiomorpholin-1,1-dioxid versetzt. Das Gemisch wurde über Nacht bei Rückfluss gerührt. Nach dem Abkühlen wurde das Reaktionsgemisch mit Essigsäureethylester verdünnt und mit Wasser/gesättigter wässriger Natriumchlorid-Lösung (1/1) gewaschen. Die wässrige Phase wurde zweimal mit Essigsäureethylester extrahiert, die vereinten organischen Phasen wurden über Natriumsulfat getrocknet, filtriert und das Filtrat eingeengt. Der Rückstand wurde mittels Kieselgelchromatographie gereinigt (Lösungsmittel: Dichlormethan/Methanol = 80/1 nach 40/1). Es wurden 85 mg der Zielverbindung (64% d. Th.) erhalten.
LC-MS (Methode 1): Rₜ = 0.76 min
MS (ESpos): m/z = 516 (M+H)⁺
¹H NMR (400 MHz, DMSO-*d*₆) δ = 2.25 - 2.32 (m, 6 H), 2.93 - 3.11 (m, 10 H), 4.30 (t, 2 H), 5.29 (s, 2 H), 6.69 - 6.81 (m, 1 H), 7.23 (t, 2 H), 7.54 - 7.64 (m, 1 H), 7.70 (br. s, 1 H), 7.78 (s, 1 H), 8.18 (s, 1 H).

### Beispiel 94

### 8-[(2,6-Difluorbenzyl)oxy]-2,6-dimethyl-3-{1-[2-(4-methylpiperazin-1-yl)ethyl]-1H-pyrazol-4-yl}imidazo[1,2-a]pyridin

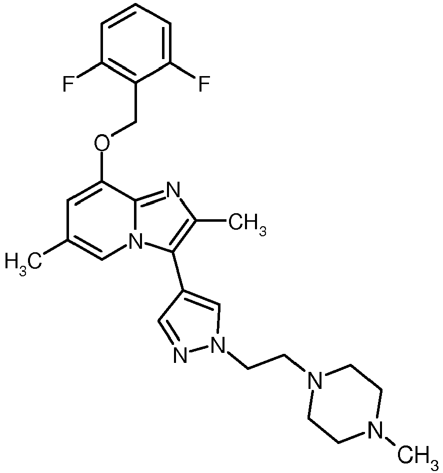

106 mg (0.20 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethylmethansulfonat aus Beispiel 57A, 0.13 ml (0.77 mmol) *N,N-*Diisopropylethylamin, 2.4 mg (0.02 mmol) 4-Dimethylaminopyridin und 58 mg (0.39 mmol) Natriumiodid in 1.9 ml THF wurden mit 77 mg (0.77 mmol) 1-Methylpiperazin versetzt. Das Gemisch wurde über Nacht bei Rückfluss gerührt. Nach dem Abkühlen wurde das Reaktionsgemisch mit Essigsäureethylester verdünnt und mit Wasser/gesättigter wässriger Natriumchlorid-Lösung (1/1) gewaschen. Die wässrige Phase wurde zweimal mit Essigsäureethylester extrahiert, die vereinten organischen Phasen wurden über Natriumsulfat getrocknet, filtriert und das Filtrat wurde eingeengt. Der Rückstand wurde mittels präparativer HPLC gereinigt (RP18 Säule, Laufmittel: Acetonitril/Wasser-Gradient unter Zusatz von 0.1% TFA). Die produkthaltigen Fraktionen wurden eingeengt und der Rückstand zwischen Dichlormethan und gesättigert, wässriger Natriumhydrogencarbonatlösung verteilt. Die wässrige Phase wurde zweimal mit Dichlormethan extrahiert, die vereinten organischen Phasen über Natriumsulfat getrocknet und filtriert. Das Filtat wurde eingeengt und der Rückstand mittels Dickschichtchromatographie (Dichlormethan/Methanol 10/1) gereinigt (das Kieselgel der DC-Platte wurde mit Dichlormethan/2 N Ammoniak-Lösung in Methanol (10/1) extrahiert). Es wurden 19 mg der Zielverbindung (20% d. Th.) erhalten.
LC-MS (Methode 1): Rₜ = 0.53 min
MS (ESpos): m/z = 481 (M+H)⁺
¹H NMR (400 MHz, DMSO-*d*₆) δ = 2.18 (s, 3 H), 2.25 - 2.58 (m, 14 H), 2.75 (t, 2 H), 4.29 (t, 2 H), 5.28 (s, 2 H), 6.72 (m, 1 H), 7.22 (t, 2 H), 7.55 - 7.65 (m, 1 H), 7.70 (s, 1 H), 7.75 (s, 1 H), 8.13 (s, 1 H).

### Beispiel 95

### 1-[2-(4-{8-[(2,6-Difluorbenzyl)oxy]-2,6-dimethylimidazo[1,2-a]pyridin-3-yl}-1H-pyrazol-1-yl)ethyl]azetidin-3-ol Trifluoracetat

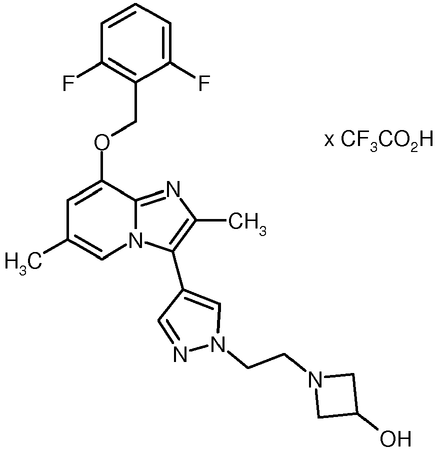

56 mg (0.76 mmol) Azetidin-3-ol in 1.47 ml abs. THF wurden mit 80 mg (0.15 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethylmethansulfonat aus Beispiel 57A, 0.13 ml (0.76 mmol) *N,N*-Diisopropylethylamin, 1.8 mg (0.015 mmol) 4-Dimethylaminopyridin und 45 mg (0.30 mmol) Natriumiodid versetzt. Das Gemisch wurde über Nacht bei Rückfluss gerührt. Es wurden nochmals 56 mg (0.76 mmol) Azetidin-3-ol hinzugegeben und die Reaktionsmischung wurde 3 Tage unter Rückfluss gerührt. Anschließend wurde das Reaktionsgemisch eingeengt, der Rückstand mit Wasser/TFA versetzt und mittels präparativer HPLC gereinigt (RP18 Säule, Laufmittel: Acetonitril/Wasser-Gradient unter Zusatz von 0.1% TFA). Es wurden 6.2 mg (7% d. Th.) der Zielverbindung erhalten.
LC-MS (Methode 1): Rₜ = 0.60 min
MS (ESpos): m/z = 454 (M-TFA+H)⁺
¹H NMR (400 MHz, DMSO-*d*₆) δ = 2.36 - 2.44 (m, 6 H), 3.69 - 3.96 (m, 4 H), 4.12 - 4.20 (m, 1 H), 4.28 - 4.38 (m, 1 H), 4.41 - 4.60 (m, 3 H), 5.46 (s, 2 H), 6.09 - 6.25 (m, 1 H), 7.27 (t, 2 H), 7.52 - 7.66 (m, 2 H), 7.97 - 8.08 (m, 2 H), 8.31 (d, 1 H).

### Beispiel 96

### 8-[(2,6-Difluorbenzyl)oxy]-2,6-dimethyl-3-{1-[2-(methylsulfonyl)ethyl]-1H-pyrazol-4-yl}imidazo[1,2-a]pyridin

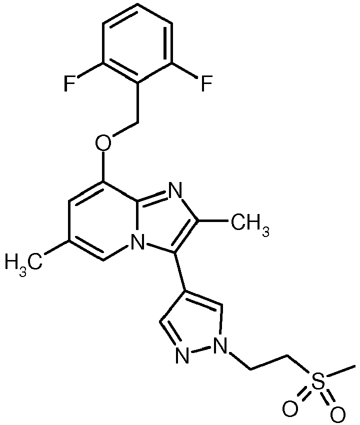

130 mg (0.25 mmol; Reinheit ca. 90%) 2-(4-{8-[(2,6-Difluorbenzyl)oxy]-2,6-dimethylimidazo[1,2-a]pyridin-3-yl}-1H-pyrazol-1-yl)ethylmethansulfonat aus Beispiel 57A und 295 mg (2.46 mmol) Natriummethansulfinat in 2.4 ml DMF wurden über Nacht bei 100°C gerührt. Das Reaktionsgemisch wurde mit Essigsäureethylester verdünnt und einmal mit gesättigter, wässriger Natriumchloridlösung/Wasser (1/1) gewaschen. Die wässrige Phase wurde zweimal mit Essigsäureethylester extrahiert, die vereinten organischen Phasen wurden eingeengt und der Rückstand mittels Kieselgelchromatographie gereinigt (Dichlormethan/Methanol = 40/1). Die Produktfraktionen wurden nochmals mittels präperativer HPLC gereinigt (RP18 Säule, Laufmittel: Acetonitril/Wasser-Gradient unter Zusatz von 0.1 % TFA). Die produkthaltigen Fraktionen wurden eingeengt, der Rückstand zwischen Dichlormethan und gesättigter, wässriger Natriumhydrogencarbonatlösung verteilt und die wässrige Phase zweimal mit Dichlormethan extrahiert. Die vereinten organischen Phasen wurden über Natriumsulfat getrocknet, filtriert, das Filtat eingeengt und der Rückstand lyophilisiert. Das Rohprodukt wurde erneut mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand zwischen Dichlormethan und gesättigter, wässriger Natriumhydrogencarbonatlösung verteilt und die wässrige Phase zweimal mit Dichlormethan extrahiert. Die vereinten organischen Phasen wurden über Natriumsulfat getrocknet, filtriert, das Filtat eingeengt und der Rückstand lyophilisiert. Es wurden 51 mg der Zielverbindung (42% d. Th., Reinheit 94%) erhalten.
LC-MS (Methode 1): Rₜ = 0.68 min
MS (ESpos): m/z = 461 (M+H)⁺
¹H NMR (400 MHz, DMSO-*d*₆) δ = 2.26 - 2.35 (m, 6 H), 2.97 (s, 3 H), 3.80 (t, 2 H), 4.68 (t, 1 H), 5.30 (s, 2 H), 6.83 (br. s, 1 H), 7.23 (t, 2 H), 7.55 - 7.64 (m, 1 H), 7.78 (br. s, 1 H), 7.85 (s, 1 H), 8.23 (s, 1 H).

### Beispiel 97

### 2-(4-{8-[(2,6-Difluorbenzyl)oxy]-2,6-dimethylimidazo[1,2-a]pyridin-3-yl}-1H-pyrazol-1-yl)ethylcarbamat

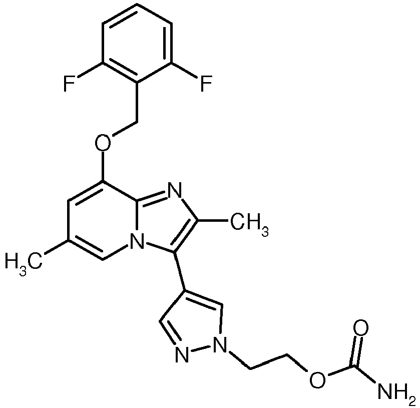

180 mg (0.45 mmol) 2-(4-{8-[(2,6-Difluorbenzyl)oxy]-2,6-dimethylimidazo[1,2-a]pyridin-3-yl}-1H-pyrazol-1-yl)ethanol aus Beispiel 91 in 4.6 ml Dichlormethan wurden bei -15°C mit 128 mg (0.90 mmol) Isocyanatosulfurylchlorid versetzt und langsam auf Raumtemperatur kommend 1 h gerührt. Das Reaktionsgemisch wurde eingeengt und der Rückstand mittels präparativer DC gereinigt (Laufmittel: Dichlormethan/Methanol = 10/1). Das Rohprodukt wurde erneut mittels präparativer DC gereinigt (Laufmittel: Dichlormethan/2 N methanolische Ammoniaklösung = 20/1). Es wurden 19 mg der Zielverbindung (9% d. Th.) erhalten.
LC-MS (Methode 1): Rₜ = 0.73 min
MS (ESpos): m/z = 442 (M+H)⁺
¹H NMR (400 MHz, DMSO-*d*₆) δ = 2.25 - 2.32 (m, 6 H), 4.29 - 4.44 (m, 4 H), 5.27 (s, 2 H), 6.40 - 6.79 (m, 3 H), 7.23 (t, 2 H), 7.54 - 7.65 (m, 1 H), 7.73 (s, 1 H), 7.79 (s, 1 H), 8.12 (s, 1 H).

### Beispiel 98

### 8-[(2,6-Difluorbenzyl)oxy]-2,6-dimethyl-3-{1-[(5-methyl-1,2-oxazol-3-yl)methyl]-1H-pyrazol-4-yl}imidazo[1,2-a]pyridin

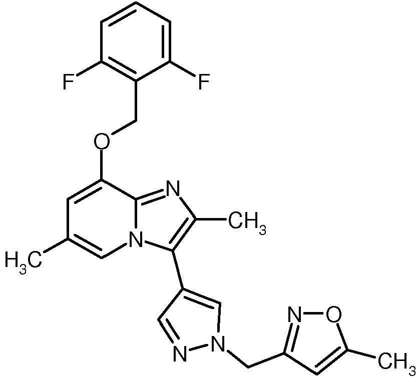

100 mg (0.28 mmol) 8-[(2,6-Difluorbenzyl)oxy]-2,6-dimethyl-3-(1H-pyrazol-4-yl)imidazo[1,2-a]pyridin aus Beispiel 88 in 1.4 ml DMF wurden mit 0.65 ml (0.65 mmol) Kalium-tert-butylat-Lösung (1 N in THF) versetzt, 5 min bei Raumtemperatur gerührt, anschließend mit 77 mg (0.42 mmol) 3-(Brommethyl)-5-methyl-1,2-oxazol und 4.7 mg (0.03 mmol) Kaliumiodid versetzt und über Nacht bei 70°C gerührt. Das Reaktionsgemisch wurde eingeengt, der Rückstand in Essigsäureethylester aufgenommen und zweimal mit Wasser gewaschen. Die organische Phase wurde über Natriumsulfat getrocknet, filtriert, das Filtrat eingeengt und der Rückstand mittels präparativer HPLC gereinigt (RP18 Säule, Laufmittel: Acetonitril/Wasser-Gradient unter Zusatz von 0.1% TFA).

Die produkthaltigen Fraktionen wurden eingeengt, der Rückstand in Dichlormethan aufgenommen und einmal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filriert und das Filtrat eingeengt. Das Rohprodukt wurde nochmals mittels Kieselgelchromatographie gereinigt (Laufmittel: Dichlormethan/Methanol = 80/1). Es wurden 39 mg der Zielverbindung (30% d. Th.) erhalten.
LC-MS (Methode 1): Rₜ = 0.77 min
MS (ESpos): m/z = 450 (M+H)⁺
¹H NMR (400 MHz, DMSO-*d*₆) δ = 2.25 - 2.34 (m, 6 H), 2.39 (s, 3 H), 5.28 (s, 2 H), 5.48 (s, 2 H), 6.20 (s, 1 H), 6.77 (br. s, 1 H), 7.23 (t, 2 H), 7.54 - 7.64 (m, 1 H), 7.73 (s, 1 H), 7.85 (s, 1 H), 8.30 (s, 1 H).

### Beispiel 99

### 8-[(2,6-Difluorbenzyl)oxy]-3-{1-[2-(3,5-dimethyl-1H-pyrazol-4-yl)ethyl]-1H-pyrazol-4-yl}-2,6-dimethylimidazo[1,2-a]pyridin

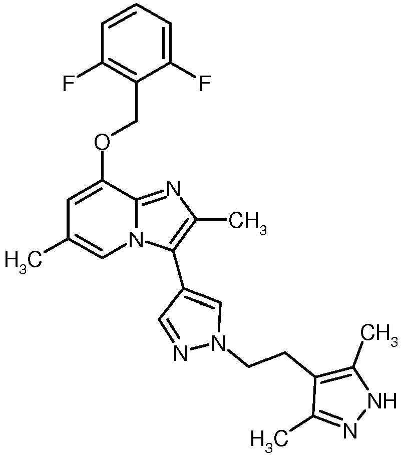

100 mg (0.28 mmol) 8-[(2,6-Difluorbenzyl)oxy]-2,6-dimethyl-3-(1H-pyrazol-4-yl)imidazo[1,2-a]pyridin aus Beispiel 88 in 1.4 ml DMF wurden mit 0.37 ml (0.37 mmol) Kalium-tert-butylat-Lösung (1 N in THF) versetzt, 5 min bei Raumtemperatur gerührt, anschließend mit 90 mg (0.42 mmol) 4-(2-Bromethyl)-3,5-dimethyl-1H-pyrazol und 4.7 mg (0.03 mmol) Kaliumiodid versetzt und über Nacht bei 70°C gerührt. Das Reaktionsgemisch wurde eingeengt, der Rückstand in Essigsäureethylester aufgenommen und zweimal mit Wasser gewaschen. Die organische Phase wurde über Natriumsulfat getrocknet, filtriert, das Filtrat eingeengt und der Rückstand mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und einmal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filriert, das Filtrat eingeengt und der Rückstand lyophilisiert. Es wurden 85 mg der Zielverbindung (62% d. Th.) erhalten.
LC-MS (Methode 1): Rₜ = 0.73 min
MS (ESpos): m/z = 477 (M+H)⁺
¹H NMR (400 MHz, DMSO-*d*₆) δ = 1.93 (s, 6 H), 2.26 (s, 3 H), 2.31 (s, 3 H), 2.82 (t, 2 H), 4.23 (t, 2 H), 5.29 (s, 2 H), 6.83 (br. s, 1 H), 7.23 (t, 2 H), 7.53 - 7.66 (m, 2 H), 7.74 - 7.83 (m, 2 H), 12.08 (br. s, 1 H).

### Beispiel 100

### 4-(4-{8-[(2,6-Difluorbenzyl)oxy]-2,6-dimethylimidazo[1,2-a]pyridin-3-yl}-1H-pyrazol-1-yl)butanonitril

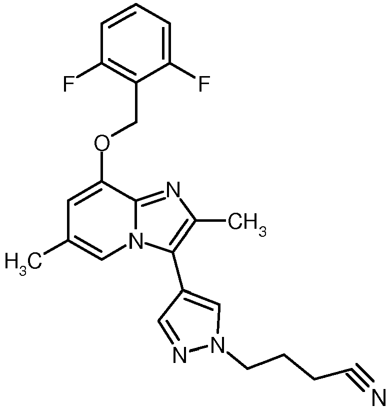

100 mg (0.28 mmol) 8-[(2,6-Difluorbenzyl)oxy]-2,6-dimethyl-3-(1H-pyrazol-4-yl)imidazo[1,2-a]pyridin aus Beispiel 88 in 1.4 ml DMF wurden mit 0.37 ml (0.37 mmol) Kalium-tert-butylat-Lösung (1 N in THF) versetzt, 5 min bei Raumtemperatur gerührt, anschließend mit 63 mg (0.42 mmol) 4-Brombutanonitril und 4.7 mg (0.03 mmol) Kaliumiodid versetzt und über Nacht bei 70°C gerührt. Das Reaktionsgemisch wurde eingeengt und der Rückstand mittels präparativer HPLC gereinigt (RP18 Säule, Laufmittel: Acetonitril/Wasser-Gradient unter Zusatz von 0.1% TFA). Die produkthaltigen Fraktionen wurden eingeengt, der Rückstand in Dichlormethan aufgenommen und einmal mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filriert und das Filtrat eingeengt. Das Rohprodukt wurde mittels Kieselgelchromatographie gereinigt (Laufmittel: Dichlormethan/Methanol = 20/1). Es wurden 47 mg der Zielverbindung (40% d. Th.) erhalten.
LC-MS (Methode 1): Rₜ = 0.77 min
MS (ESpos): m/z = 422 (M+H)⁺
¹H NMR (400 MHz, DMSO-*d*₆) δ = 2.18 (q, 2 H), 2.25 - 2.32 (m, 6 H), 2.57 (t, 2 H; überlagert mit DMSO-Signal), 4.29 (t, 2 H), 5.28 (s, 2 H), 6.73 (s, 1 H), 7.23 (t, 2 H), 7.55 - 7.65 (m, 1 H), 7.73 (s, 1 H), 7.79 (s, 1 H), 8.19 (s, 1 H).

### Beispiel 101

### 8-[(2,6-Difluorbenzyl)oxy]-2,6-dimethyl-3-{1-[3-(1H-tetrazol-5-yl)propyl]-1H-pyrazol-4-yl}imidazo[1,2-a]pyridin

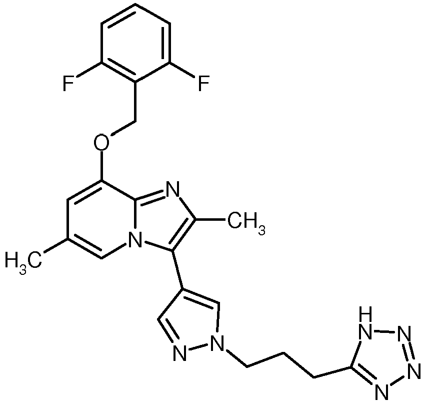

20 mg (0.05 mmol) 4-(4-{8-[(2,6-Difluorbenzyl)oxy]-2,6-dimethylimidazo[1,2-a]pyridin-3-yl}-1H-pyrazol-1-yl)butanonitril aus Beispiel 100 in 0.5 ml DMF wurden mit 12.3 mg (0.19 mmol) Natriumazid und 41 mg (0.76 mmol) Ammoniumchlorid versetzt und 8 h bei 150°C in der Mikrowelle bestrahlt. Das Reaktionsgemisch wurde mit Wasser/TFA versetzt und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, dreimal mit Dichlormethan/Ameisensäure (10/1) und dreimal mit Dichlormethan eingeengt und der Rückstand anschließend lyophilisiert. Es wurden 8.4 mg der Zielverbindung (35% d. Th.) erhalten.
LC-MS (Methode 1): Rₜ = 0.69 min
MS (ESpos): m/z = 465 (M+H)⁺
¹H NMR (400 MHz, DMSO-*d*₆) δ = 2.22 - 2.38 (m, 8 H), 2.92 (t, 2 H), 4.30 (t, 2 H), 5.32 (s, 2 H), 6.93 (br. s, 1 H), 7.23 (t, 2 H), 7.55 - 7.64 (m, 1 H), 7.76 - 7.90 (m, 2 H), 8.18 (s, 1 H), 16.05 (br. s, 1 H).

### Beispiel 102

### Methyl-3-(4-{8-[(2,6-difluorbenzyl)oxy]-2,6-dimethylimidazo[1,2-a]pyridin-3-yl}-1H-pyrazol-1-yl)propanoat

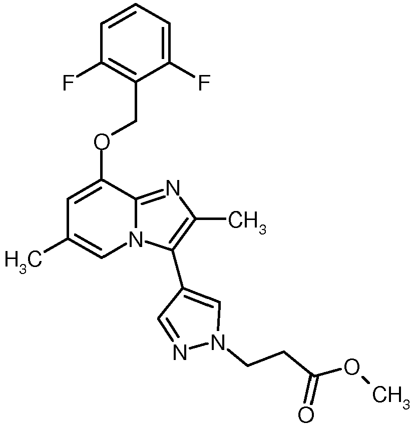

218 mg (0.61 mmol) 8-[(2,6-Difluorbenzyl)oxy]-2,6-dimethyl-3-(1H-pyrazol-4-yl)imidazo[1,2-a]pyridin aus Beispiel 88 in 3.3 ml DMF wurden mit 520 mg (1.60 mmol) Cäsiumcarbonat, 10.2 mg (0.06 mmol) Kaliumiodid und 133 mg (0.80 mmol) Methyl-3-brompropanoat versetzt und 2.5 h bei 70°C gerührt. Nach dem Abkühlen wurde der Feststoff abfiltriert, mit THF/Methanol gewaschen, das Filtrat eingeengt und der Rückstand mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt. Die wässrige Phase wurde zweimal mit Dichlormethan extrahiert, die vereinten organischen Phasen über Natriumsulfat getrocknet, filtriert und das Filtrat eingeengt. Das Rohprodukt wurde mittels Kieselgelchromatographie gereinigt (Lösungsmittel: Dichlormethan/Methanol = 80/1). Es wurden 179 mg der Zielverbindung (65% d. Th.) erhalten.
LC-MS (Methode 1): Rₜ = 0.78 min
MS (ESpos): m/z = 441 (M+H)⁺
¹H NMR (400 MHz, DMSO-*d*₆) δ = 2.25 - 2.32 (m, 6 H), 2.98 (t, 2 H), 3.62 (s, 3 H), 4.44 (t, 2 H), 5.28 (s, 2 H), 6.74 (s, 1 H), 7.23 (t, 2 H), 7.55 - 7.65 (m, 1 H), 7.72 (s, 1 H), 7.78 (s, 1 H), 8.14 (s, 1 H).

Die in Tabelle 4 gezeigten Beispiele wurden in Analogie zu Beispiel 102 hergestellt, indem 8-[(2,6-Difluorbenzyl)oxy]-2,6-dimethyl-3-(1H-pyrazol-4-yl)imidazo[1,2-a]pyridin aus Beispiel 88 mit den entsprechenden kommerziell erhältlichen Bromiden (1.1-2.5 Äquivalente), Cäsiumcarbonat (2-4 Äquivalente) und Kaliumiodid (0.1-0.5 Äquivalente) unter den beschriebenen Reaktionsbedingungen (Reaktionszeit: 2 - 24 h; Temperatur: 70°C) umgesetzt wurde.

### Beispielhafte Aufarbeitung der Reaktionsmischung:

Nach dem Abkühlen wurde der Feststoff abfiltriert, gut mit THF/Methanol gewaschen, das Filtrat eingeengt und der Rückstand mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt. Die wässrige Phase wurde zweimal mit Dichlormethan extrahiert, die vereinten organischen Phasen über Natriumsulfat getrocknet, filtriert und das Filtrat eingeengt. Das Rohprodukt wurde gegebenenfalls nochmal mittels Kieselgelchromatographie gereinigt (Lösungsmittel: Dichlormethan/Methanol = 80/1 bis 20/1).

**Tabelle 4:**

| **Beispiel** | **IUPAC-Name / Struktur (Ausbeute)** | **Analytische Daten** |
|---|---|---|
| **103** | Methyl-(4-{8-[(2,6-difluorbenzyl)oxy]-2,6-dimethylimidazo[1,2-a]pyridin-3-yl}-1H-pyrazol-1-yl)acetat | LC-MS (Methode 1): Rₜ = 0.70 min |
| | | MS (ESpos): m/z = 427 (M+H)⁺ |
| | 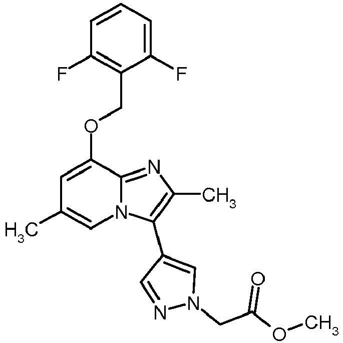 | ¹H-NMR (400 MHz, DMSO-d₆): δ = 2.26 - 2.32 (m, 6 H), 3.27 (s, 3 H), 5.18 (s, 2 H), 5.28 (s, 2 H), 6.75 (s, 1 H), 7.23 (t, 2 H), 7.55 - 7.65 (m, 1 H), 7.71 (s, 1 H), 7.83 (s, 1 H), 8.18 (s, 1 H). |
| | (46% d. Th.) | |
| **104** | *rac*-Methyl-2-(4-{8-[(2,6-difluorbenzyl)oxy]-2,6-dimethylimidazo[1,2-a]pyridin-3-yl}-1H-pyrazol-1-yl)propanoat | LC-MS (Methode 1): Rₜ = 0.77 min |
| | | MS (ESpos): m/z = 441 (M+H)⁺ |
| | 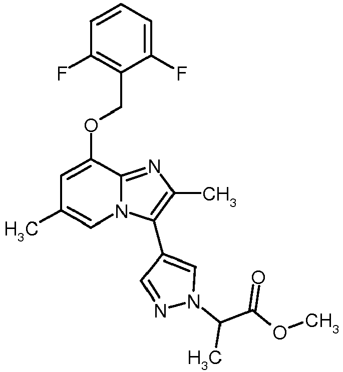 | ¹H-NMR (400 MHz, DMSO-d₆): δ = 1.74 (d, 3 H), 2.25 - 2.32 (m, 6 H), 3.70 (s, 3 H), 5.28 (s, 2 H), 5.38 (q, 1 H), 6.75 (s, 1 H), 7.23 (t, 2 H), 7.55-7.65 (m, 1 H), 7.72 (s, 1 H), 7.83 (s, 1 H), 8.25 (s, 1 H). |
| | (42% d. Th.) | |
| **105** | *rac*-Methyl-3-(4-{8-[(2,6-difluorbenzyl)oxy]-2,6-dimethylimidazo[1,2-a]pyridin-3-yl}-1H-pyrazol-1-yl)butanoat | LC-MS (Methode 1): Rₜ = 0.80 min |
| | | MS (ESpos): m/z = 455 (M+H)⁺ |
| | 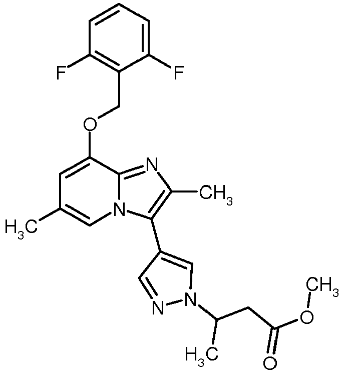 | ¹H-NMR (400 MHz, DMSO-d₆): δ = 1.52 (d, 3 H), 2.25 - 2.32 (m, 6 H), 2.88 - 2.97 (m, 1 H), 2.98 - 3.08 (m, 1 H), 3.58 (s, 3 H), 4.80 - 4.92 (m, 1 H), 5.28 (s, 2 H), 6.73 (s, 1 H), 7.23 (t, 2 H), 7.55 - 7.65 (m, 1 H), 7.72 (s, 1 H), 7.78 (s, 1 H), 8.18 (s, 1 H). |
| | (47% d. Th.) | |
| **106** | *rac*-Methyl-2-(4-{8-[(2,6-difluorbenzyl)oxy]-2,6-dimethylimidazo[1,2-a]pyridin-3-yl}-1H-pyrazol-1-yl)cyclopropancarboxylat | LC-MS (Methode 1): Rₜ = 0.77 min |
| | | MS (ESpos): m/z = 453 (M+H)⁺ |
| | 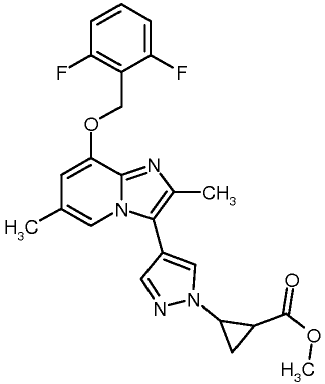 | ¹H-NMR (400 MHz, DMSO-d₆): δ = 1.57 - 1.63 (m, 1 H), 1.95 - 2.02 (m, 1 H), 2.26 - 2.32 (m, 6 H), 2.33 - 2.40 (m, 1 H), 3.32 (s, 3 H), 4.23 - 4.30 (m, 1 H), 5.28 (s, 2 H), 6.73 (s, 1 H), 7.23 (t, 2 H), 7.55 - 7.65 (m, 1 H), 7.73 (s, 1 H), 7.79 (s, 1 H), 8.30 (s, 1 H). |
| | (% d. Th.) | |

### Beispiel 107

### 3-(4- {8-[(2,6-Difluorbenzyl)oxy]-2,6-dimethylimidazo[1,2-a]pyridin-3-yl}-1H-pyrazol-1-yl)propansäure

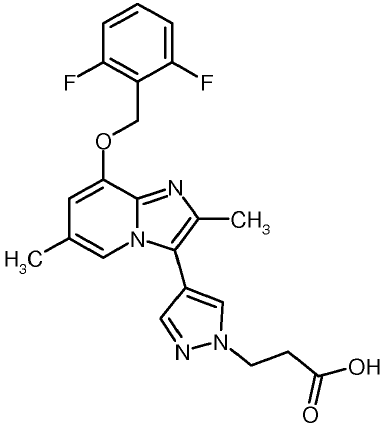

50 mg (0.11 mmol) Methyl-3-(4-{8-[(2,6-difluorbenzyl)oxy]-2,6-dimethylimidazo[1,2-a]pyridin-3-yl}-1H-pyrazol-1-yl)propanoat aus Beispiel 102 in 2.45 ml THF/Methanol (5/1) wurden mit 0.23 ml (0.23 mmol) 1 N wässriger Lithiumhydroxidlösung versetzt und 2 h bei Raumtemperatur gerührt. Die Reaktionslösung wurde mit 1 N wässriger Salzsäure auf pH=2 gebracht, eingeengt und der Rückstand mittels Kieselgelchromatographie gereinigt (Laufmittel: Dichlormethan/Methanol = 10/1). Das Rohprodukt wurde mittels präparativer HPLC gereinigt (RP18 Säule, Laufmittel: Acetonitril/Wasser-Gradient unter Zusatz von 0.1 % TFA). Die produkthaltigen Fraktionen wurden eingeengt und der Rückstand dreimal mit Dichlormethan/Ameisensäure (10/1) und danach dreimal mit Dichlormethan eingeengt. Es wurden 30.5 mg der Zielverbindung (63% d. Th.) erhalten.
LC-MS (Methode 1): Rₜ = 0.72 min
MS (ESpos): m/z = 427 (M+H)⁺
¹H NMR (400 MHz, DMSO-*d*₆) δ = 2.23 - 2.32 (m, 6 H), 2.88 (t, 2 H), 4.40 (t, 2 H), 5.27 (s, 2 H), 6.73 (s, 1 H), 7.23 (t, 2 H), 7.56 - 7.65 (m, 1 H), 7.72 (s, 1 H), 7.78 (s, 1 H), 8.13 (s, 1 H), 12.48 (br. s, 1 H).

Die in Tabelle 5 gezeigten Beispiele wurden in Analogie zu Beispiel 107 hergestellt, indem die entsprechenden Carbonsäureester mit Lithiumhydroxid (2-5 Äquivalente) unter den beschriebenen Reaktionsbedingungen (Reaktionszeit: 2 - 5 h; Temperatur: RT) umgesetzt wurden.

### Beispielhafte Aufarbeitung der Reaktionsmischung:

Die Reaktionslösung wurde mit wässriger Salzsäure (1 N bis 6 N) oder TFA auf pH=2-4 eingestellt, eingeengt und der Rückstand mittels Kieselgelchromatographie gereinigt (Laufmittel: Dichlormethan/Methanol = 10/1). Das Rohprodukt wurde zusätzlich oder alternativ mittels präparativer HPLC gereinigt (RP18 Säule, Laufmittel: Acetonitril/Wasser-Gradient unter Zusatz von 0.1 % TFA). Die produkthaltigen Fraktionen wurden eingeengt und der Rückstand dreimal mit Dichlormethan/Ameisensäure (10/1) und danach dreimal mit Dichlormethan eingeengt.

**Tabelle 5:**

| **Beispiel** | **IUPAC-Name / Struktur (Ausbeute)** | **Analytische Daten** |
|---|---|---|
| **108** | (4-{8-[(2,6-Difluorbenzyl)oxy]-2,6-dimethylimidazo[1,2-a]pyridin-3-yl}-1H-pyrazol-1-yl)essigsäure | LC-MS (Methode 1): Rₜ = 0.70 min |
| | | MS (ESpos): m/z = 413 (M+H)⁺ |
| | 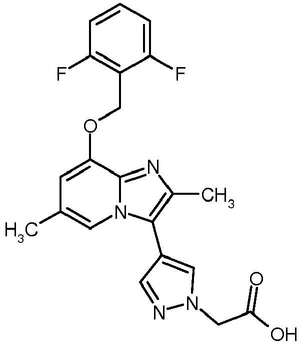 | ¹H-NMR (400 MHz, DMSO-d₆): δ = 2.33 - 2.46 (m, 6 H), 5.12 (s, 2 H), 5.46 (s, 2 H), 7.27 (t, 2 H), 7.44 - 7.68 (m, 2 H), 7.92 (s, 1 H), 8.02 (br. s, 1 H), 8.31 (s, 1 H), 13.28 (br. s, 1 H). |
| | (29% d. Th.) | |
| **109** | *rac*-2-(4-{8-[(2,6-Difluorbenzyl)oxy]-2,6-dimethylimidazo[1,2-a]pyridin-3-yl}-1H-pyrazol-1-yl)propansäure | LC-MS (Methode 1): Rₜ = 0.72 min |
| | | MS (ESpos): m/z = 427 (M+H)⁺ |
| | 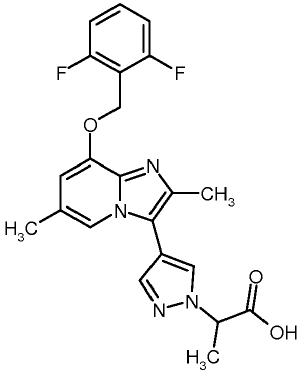 | ¹H-NMR (400 MHz, DMSO-d₆): δ = 1.73 (d, 3 H), 2.25 - 2.32 (m, 6 H), 5.22 (q, 1 H), 5.28 (s, 2 H), 6.74 (s, 1 H), 7.23 (t, 2 H), 7.55 - 7.65 (m, 1 H), 7.72 (s, 1 H), 7.80 (s, 1 H), 8.12 (s, 1 H), 13.12 (br. s, 1 H). |
| | (98% d. Th.) | |
| **110** | *rac*-3-(4-{8-[(2,6-Difluorbenzyl)oxy]-2,6-dimethylimidazo[1,2-a]pyridin-3-yl}-1H-pyrazol-1-yl)butansäure | LC-MS (Methode 1): Rₜ = 0.78 min |
| | | MS (ESpos): m/z = 441 (M+H)⁺ |
| | 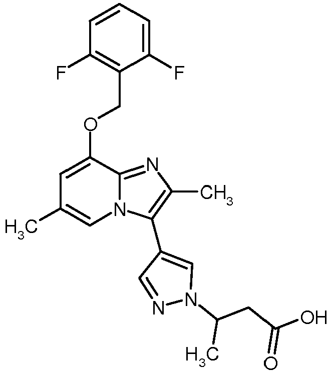 | ¹H-NMR (400 MHz, DMSO-d₆): δ = 1.50 (d, 3 H), 2.25 - 2.32 (m, 6 H), 2.78 - 2.37 (m, 1 H), 2.89 - 2.99 (m, 1 H), 4.78 - 4.88 (m, 1 H), 5.28 (s, 2 H), 6.73 (s, 1 H), 7.23 (t, 2 H), 7.55 - 7.65 (m, 1 H), 7.72 (s, 1 H), 7.78 (s, 1 H), 8.18 (s, 1 H), 12.40 (br. s, 1 H). |
| | (87% d. Th.) | |
| **111** | *rac*-2-(4-{8-[(2,6-Difluorbenzyl)oxy]-2,6-dimethylimidazo[1,2-a]pyridin-3-yl}-1H-pyrazol-1-yl)cyclopropancarbonsäure | LC-MS (Methode 1): Rₜ = 0.76 min |
| | | MS (ESpos): m/z = 439 (M+H)⁺ |
| | 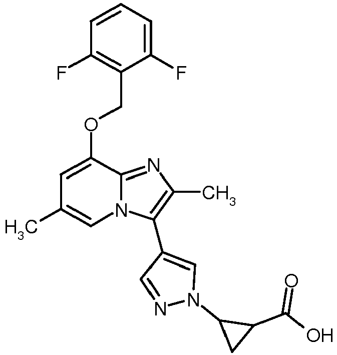 | ¹H-NMR (400 MHz, DMSO-d₆): δ = 1.53 - 1.60 (m, 1 H), 1.87 - 1.94 (m, 1 H), 2.20 - 2.39 (m, 7 H), 4.20 - 4.28 (m, 1 H), 5.33 (s, 2 H), 7.04 (br. s, 1 H), 7.22 (t, 2 H), 7.55 - 7.65 (m, 1 H), 7.81 (s, 1 H), 7.88 (br. s, 1 H), 8.37 (s, 1 H), 12.70 (br. s, 1 H). |
| | (88% d. Th.) | |

### Beispiel 112

### rac-N-[2-(Diethylamino)ethyl]-3-(4-{8-[(2,6-difluorbenzyl)oxy]-2,6-dimethylimidazo[1,2-a]pyridin-3-yl}-1H-pyrazol-1-yl)butanamid

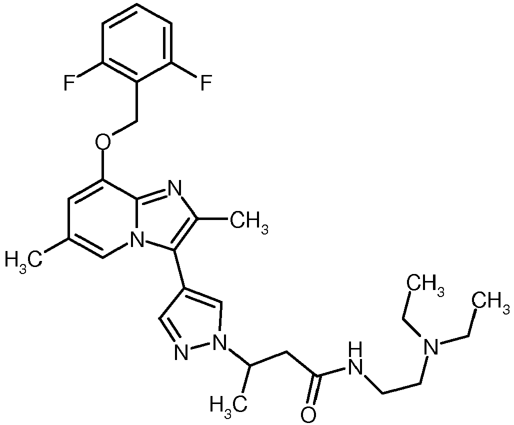

44 mg (0.10 mmol) *rac*-3-(4-{8-[(2,6-Difluorbenzyl)oxy]-2,6-dimethylimidazo[1,2-a]pyridin-3-yl}-1H-pyrazol-1-yl)butansäure aus Beispiel 110, 49.4 mg (0.13 mmol) HATU und 0.07 ml (0.40 mmol) *N,N*-Diisopropylethylamin in 0.33 ml DMF wurden 10 min bei RT gerührt, mit 15 mg (0.13 mmol) *N,N*-Diethylethan-1,2-diamin versetzt und über Nacht bei RT gerührt. Anschließend wurden 19 mg (0.05 mmol) HATU, 0.035 ml (0.20 mmol) *N,N*-Diisopropylethylamin und 17.4 mg (0.15 mmol) *N,N*-Diethylethan-1,2-diamin zur Reaktionsmischung hinzugegeben und das Gemisch wurde 30 min bei RT gerührt. Die Reaktionslösung wurde mit TFA versetzt und anschließend mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt. Die wässrige Phase wurde zweimal mit Dichlormethan extrahiert, die vereinten organischen Phasen über Natriumsulfat getrocknet, filtriert und das Filtrat eingeengt und lyophilisiert. Es wurden 33 mg der Zielverbindung (60% d. Th.) erhalten.
LC-MS (Methode 1): Rₜ = 0.58 min
MS (ESpos): m/z = 539 (M+H)⁺
¹H NMR (400 MHz, DMSO-*d*₆) δ = 0.78 - 0.98 (m, 6 H), 1.50 (d, 3 H), 2.23 - 2.47 (m, 10 H), 2.58 - 2.69 (m, 1 H), 2.70 - 2.80 (m, 1 H), 2.98 - 3.14 (m, 2 H), 4.79 - 4.89 (m, 1 H), 5.28 (s, 2 H), 6.72 (s, 1 H), 7.23 (t, 2 H), 7.55 - 7.65 (m, 1 H), 7.72 (s, 1 H), 7.76 (s, 1 H), 7.84 (br. s, 1 H), 8.09 (s, 1 H).

### Beispiel 113

### N-Cyclopropyl-2-(4-{8-[(2,6-difluorbenzyl)oxy]-2,6-dimethylimidazo[1,2-a]pyridin-3-yl}-1H-pyrazol-1-yl)acetamid

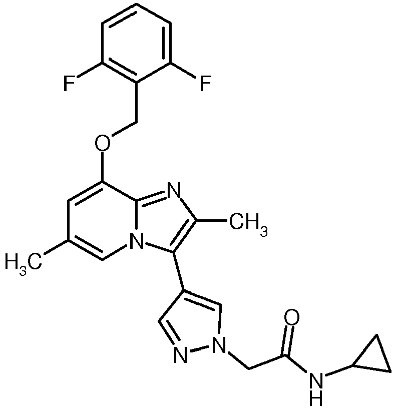

55 mg (0.13 mmol) (4-{8-[(2,6-Difluorbenzyl)oxy]-2,6-dimethylimidazo[1,2-a]pyridin-3-yl}-1H-pyrazol-1-yl)essigsäure aus Beispiel 108, 56 mg (0.15 mmol) HATU und 0.09 ml (0.53 mmol) *N,N*-Diisopropylethylamin in 0.85 ml DMF wurden 20 min bei RT gerührt, mit 9 mg (0.15 mmol) Cyclopropylamin versetzt und über Nacht bei RT gerührt. Die Reaktionslösung wurde eingeengt und anschließend zweimal mittels präparativer DC gereinigt (1. Laufmittel: Dichlormethan/Methanol = 10/1; 2. Laufmittel: Dichlormethan/2 N Ammoniaklösung in Methanol = 20/1). Es wurden 5.1 mg der Zielverbindung (8% d. Th.) erhalten.
LC-MS (Methode 1): Rₜ = 0.76 min
MS (ESpos): m/z = 452 (M+H)⁺
¹H NMR (400 MHz, DMSO-*d*₆) δ = 0.42 - 0.48 (m, 2 H), 0.62 - 0.69 (m, 2 H), 2.25 - 2.34 (m, 6 H), 2.63 - 2.70 (m, 1 H), 4.80 (s, 2 H), 5.28 (s, 2 H), 6.73 (s, 1 H), 7.23 (t, 2 H), 7.55 - 7.65 (m, 1 H), 7.72 (s, 1 H), 7.78 (s, 1 H), 8.10 (s, 1 H), 8.34 (d, 1 H).

### Beispiel 114

### N-Cyclopropyl-3-(4-{8-[(2,6-difluorbenzyl)oxy]-2,6-dimethylimidazo[1,2-a]pyridin-3-yl}-1H-pyrazol-1-yl)propanamid

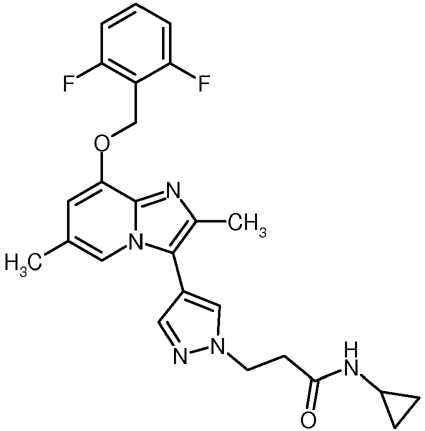

50 mg (0.11 mmol) Methyl-3-(4-{8-[(2,6-difluorbenzyl)oxy]-2,6-dimethylimidazo[1,2-a]pyridin-3-yl}-1H-pyrazol-1-yl)propanoat aus Beispiel 102 wurden mit 843 mg (14.76 mmol) Cyclopropylamin bei 50°C gerührt. Die Reaktionslösung wurde eingeengt, der Rückstand zweimal mit Dichlormethan eingeengt und anschließend mittels Kieselgelchromatographie gereinigt (Lösungsmittel: Dichlormethan pur; Dichlormethan/Methanol 100/1 nach 40/1). Die eingeengten Produktfraktionen wurden nochmals mittels präparativer HPLC gereinigt (RP18 Säule, Laufmittel: Acetonitril/Wasser-Gradient unter Zusatz von 0.1 % TFA). Die produkthaltigen Fraktionen wurden eingeengt und der Rückstand dreimal mit Dichlormethan/Ameisensäure (10/1) und danach dreimal mit Dichlormethan eingeengt. Es wurden 32 mg der Zielverbindung (58% d. Th.) erhalten.
LC-MS (Methode 1): Rₜ = 0.75 min
MS (ESpos): m/z = 466 (M+H)⁺
¹H NMR (400 MHz, DMSO-*d*₆) δ = 0.29 - 0.36 (m, 2 H), 0.55 - 0.62 (m, 2 H), 2.24 - 2.39 (m, 6 H), 2.54 - 2.72 (m, 3 H), 4.42 (t, 2 H), 5.32 (s, 2 H), 6.99 (br. s, 1 H), 7.23 (t, 2 H), 7.55 - 7.65 (m, 1 H), 7.73 - 7.88 (m, 2 H), 8.07 (d, 1 H), 8.10 (s, 1 H).

### Beispiel 115

### 2-(4- {8-[(2,6-Difluorbenzyl)oxy]-2,6-dimethylimidazo[1,2-a]pyridin-3-yl}-1H-pyrazol-1-yl)acetamid

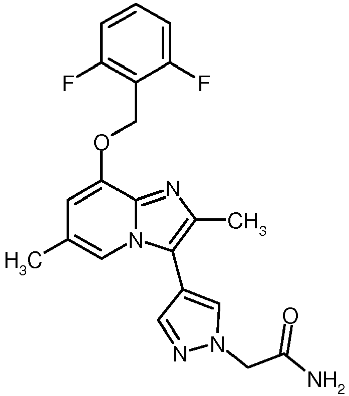

70 mg (0.29 mmol) 8-[(2,6-Difluorbenzyl)oxy]-2,6-dimethyl-3-(1H-pyrazol-4-yl)imidazo[1,2-a]pyridin aus Beispiel 88 in 1.1 ml DMF wurden mit 29 mg (0.26 mmol) Kalium-tert.-butylat, 3.3 mg (0.02 mmol) Kaliumiodid und 44 mg (0.32 mmol) 2-Bromacetamid versetzt und über Nacht bei 70°C gerührt. Nach dem Abkühlen wurde der Feststoff abfiltriert, gut mit THF gewaschen, das Filtrat eingeengt und der Rückstand mittels präparativer HPLC gereinigt (RP18 Säule, Laufmittel: Acetonitril/Wasser-Gradient unter Zusatz von 0.1% TFA). Die eingeengten Produktfraktionen wurden zwischen Dichlormethan und gesättigter, wässriger Natriumhydrogencarbonatlösung verteilt. Die wässrige Phase wurde zweimal mit Dichlormethan extrahiert, die vereinten organischen Phasen über Natriumsulfat getrocknet, filtriert und das Filtrat eingeengt und lyophilisiert. Es wurden 32 mg der Zielverbindung (37% d. Th.) erhalten.
LC-MS (Methode 1): Rₜ = 0.66 min
MS (ESpos): m/z = 412 (M+H)⁺
¹H NMR (400 MHz, DMSO-*d*₆) δ = 2.25 - 2.32 (m, 6 H), 4.86 (s, 2 H), 5.29 (s, 2 H), 6.75 (s, 1 H), 7.23 (t, 2 H), 7.30 (br. s, 1 H), 7.48 - 7.65 (m, 2 H), 7.73 (s, 1 H), 7.79 (s, 1 H), 8.12 (s, 1 H).

### Beispiel 116

### 1-(4-{8-[(2,6-Difluorbenzyl)oxy]-2,6-dimethylimidazo[1,2-a]pyridin-3-yl}-1H-pyrazol-1-yl)-2-methylpropan-2-ol

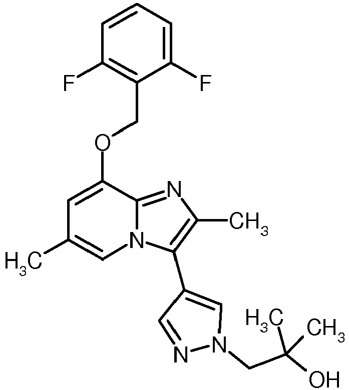

100 mg (0.24 mmol) Methyl-(4-{8-[(2,6-difluorbenzyl)oxy]-2,6-dimethylimidazo[1,2-a]pyridin-3-yl}-1H-pyrazol-1-yl)acetat aus Beispiel 103 in 2.3 ml trockenem THF wurden bei 0°C unter Argon mit 0.27 ml (0.82 mmol) einer Methylmagnesium-Lösung (3 M in Diethylether) versetzt und 15 min bei dieser Temperatur gerührt. Anschließend wurde das Gemisch langsam auf RT kommend über Nacht bei Raumtemperatur gerührt. Das Reaktionsgemisch wurde vorsichtig mit gesättigter, wässriger Ammoniumchloridlösung versetzt. Die Suspension wurde mit Celite versetzt, der Feststoff abfiltriert, gut mit THF gewaschen, das Filtrat eingeengt und der Rückstand mittels Kieselgel-Chromatographie gereinigt (Laufmittel: Dichlormethan/Methanol 20/1). Die Produktfraktionen wurden eingeengt und mittels präparativer DC gereinigt (Laufmittel: Dichlormethan/Methanol = 10/1). Es wurden 30.5 mg der Zielverbindung (31% d. Th.) erhalten.
LC-MS (Methode 1): Rₜ = 0.76 min
MS (ESpos): m/z = 427 (M+H)⁺
¹H NMR (400 MHz, DMSO-*d*₆) δ = 1.12 (s, 6 H), 2.26 - 2.32 (m, 6 H), 4.12 (s, 2 H), 4.77 (s, 1 H), 5.28 (s, 2 H), 6.73 (s, 1 H), 7.23 (t, 2 H), 7.55 - 7.65 (m, 1 H), 7.73 (s, 1 H), 7.77 (s, 1 H), 8.04 (s, 1 H).

### Beispiel 117

### 1-(4-{8-[(2,6-Difluorbenzyl)oxy]-2,6-dimethylimidazo[1,2-a]pyridin-3-yl}-1H-pyrazol-1-yl)-2-methylpropan-2-amin Hydrochlorid

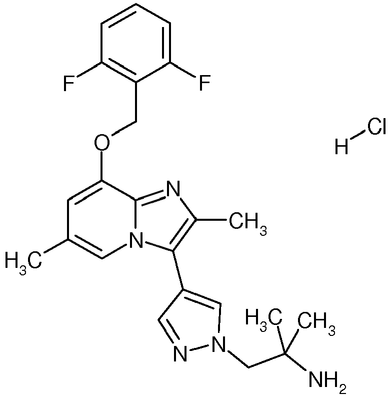

70 mg (0.17 mmol) 1-(4-{8-[(2,6-Difluorbenzyl)oxy]-2,6-dimethylimidazo[1,2-a]pyridin-3-yl}-1H-pyrazol-1-yl)-2-methylpropan-2-amin aus Beispiel 89 in 1.3 ml Diethylether wurden mit 0.1 ml (0.20 mmol) Chlorwasserstofflösung (2 N in Diethylether) versetzt und 30 min bei Raumtemperatur gerührt. Das Lösungsmittel wurde am Rotationsverdampfer entfernt. Es wurden 78 mg der Zielverbindung (98% d. Th.) erhalten.
LC-MS (Methode 1): Rₜ = 0.53 min
MS (ESpos): m/z = 426 (M+H)⁺
¹H NMR (400 MHz, DMSO-*d*₆) δ = 1.28 (s, 6 H), 2.26 - 2.32 (m, 6 H), 4.38 (s, 2 H), 5.28 (s, 2 H), 6.76 (s, 1 H), 7.22 (t, 2 H), 7.54 - 7.64 (m, 1 H), 7.80 - 8.12 (m, 4 H), 7.78 (s, 1 H), 8.20 (s, 1 H).

### Beispiel 118

### 8-[(2,6-Difluorbenzyl)oxy]-2,6-dimethyl-3-{1-[2-(morpholin-4-yl)ethyl]-1H-pyrazol-4-yl}imidazo[1,2-a]pyridin

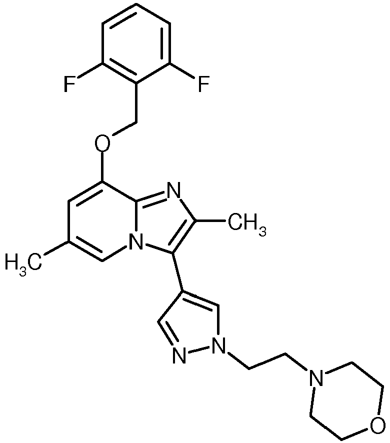

Zu 125 mg (0.33 mmol) 3-Brom-8-[(2,6-difluorbenzyl)oxy]-2,6-dimethylimidazo[1,2-a]pyridin aus Beipiel 30A, 112 mg (0.36 mmol) 4-{2-[4-(4,4,5,5-Tetramethyl-1,3,2-dioxaborolan-2-yl)-1H-pyrazol-1-yl]ethyl}morpholin, 111 mg (1.32 mmol) Natriumhydrogencarbonat und 13.5 mg (0.02 mmol) Bis-(diphenylphosphino)ferrocen-Palladium(II)chlorid-Dichlormethan-Komplex wurden unter Argon 1.9 ml einer entgasten 3:1-Mischung aus 1,2-Dimethoxyethan und Wasser gegeben. Die Reaktionsmischung wurde über Nacht bei 80°C gerührt. Das Reaktionsgemisch wurde mit Wasser/TFA verdünnt und mittels präparativer HPLC gereinigt (RP18 Säule, Laufmittel: Acetonitril/Wasser-Gradient unter Zusatz von 0.1 % TFA). Die produkthaltigen Fraktionen wurden eingeengt, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingeengt und lyophilisiert. Es wurden 44 mg der Zielverbindung (27% d. Th., Reinheit 94%) erhalten.
LC-MS (Methode 1): Rₜ = 0.60 min
MS (ESpos): m/z = 468 (M+H)⁺
¹H NMR (400 MHz, DMSO-*d*₆) δ = 2.26 - 2.32 (m, 6 H), 2.38 - 2.50 (m, 4 H), 2.78 (t, 2 H), 3.48-3.60 (m, 4 H), 4.32 (t, 2 H), 5.28 (s, 2 H), 6.74 (s, 1 H), 7.23 (t, 2 H), 7.54 - 7.65 (m, 1 H), 7.71 (s, 1 H), 7.77 (s, 1 H), 8.16 (s, 1 H).

In Analogie zu Beispiel 118 wurden die in Tabelle 6 gezeigten Beispielverbindungen hergestellt, indem 3-Brom-8-[(2,6-difluorbenzyl)oxy]-2-methylimidazo[1,2-a]pyridin aus Beispiel 28A oder 3-Brom-8-[(2,6-difluorbenzyl)oxy]-2,6-dimethylimidazo[1,2-a]pyridin aus Beipiel 30A mit den entsprechenden, kommerziell erhältlichen Pinacol-Boronsäureestern, Natriumhydrogencarbonat oder Kaliumcarbonat (4 Äquivalente) und Bis-(diphenylphosphino)ferrocen-Palladium(II)chlorid-Dichlormethan-Komplex (0.02 - 0.1 Äquivalente) in 1,2-Dimethoxyethan/Wasser (3/1) oder Acetonitril unter den beschriebenen Reaktionsbedingungen (Reaktionszeit: 12 - 24 h; Temperatur: 80°C) umgesetzt wurden.

### Beispielhafte Aufarbeitung der Reaktionsmischung:

Das Reaktionsgemisch wurde mit Wasser/TFA verdünnt und mittels präparativer HPLC gereinigt (RP18 Säule, Laufmittel: Acetonitril/Wasser-Gradient unter Zusatz von 0.1% TFA). Das Rohprodukt wurde zusätzlich oder alternativ mittels Kieselgelchromatographie gereinigt (Laufmittel: Dichlormethan/Methanol = 20/1 bis 10/1). Die produkthaltigen Fraktionen wurden eingeengt, der Rückstand in Dichlormethan aufgenommen und mit gesättigter, wässriger Natriumhydrogencarbonatlösung gewaschen. Die wässrige Phase wurde zweimal mit Dichlormethan extrahiert, die vereinigten organischen Phasen über Natriumsulfat getrocknet, filtriert, eingeengt und lyophilisiert.

**Tabelle 6:**

| **Beispiel Nr**. | **IUPAC**-**Name**/**Struktur** | **Analytische Methoden** |
|---|---|---|
| | **(Ausbeute)** | |
| **119** | 8-[(2,6-Difluorbenzyl)oxy]-2-methyl-3-{1-[2-(morpholin-4-yl)ethyl]-1H-pyrazol-4-yl}imidazo[1,2-a]pyridin | LC-MS (Methode 1): Rₜ = 0.53 min |
| | | MS (ESpos): m/z = 454 (M+H)⁺ |
| | 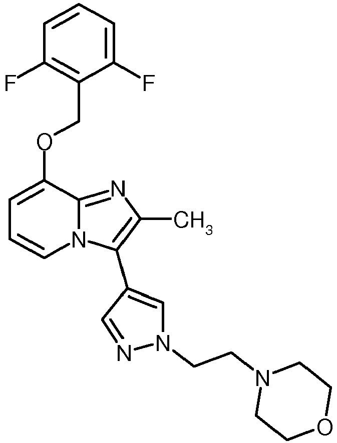 | ¹H NMR (400 MHz, DMSO-*d*₆) δ = 2.32 (s, 3 H), 2.39 - 2.50 (m, 4 H), 2.78 (t, 2 H), 3.54 - 3.60 (m, 4 H), 4.32 (t, 2 H), 5.30 (s, 2 H), 6.79 - 6.85 (m, 2 H), 7.22 (t, 2 H), 7.54 - 7.64 (m, 1 H), 7.77 (s, 1 H), 7.90 (d, 1 H), 8.18 (s, 1 H). |
| | (33% d. Th.) | |
| **120** | 8-[(2,6-Difluorbenzyl)oxy]-2,6-dimethyl-3-{3-[(4-methylpiperazin-1-yl)methyl]phenyl}imidazo[1,2-a]pyridin | LC-MS (Methode 1): Rₜ = 0.61 min |
| | | MS (ESpos): m/z = 477 (M+H)⁺ |
| | 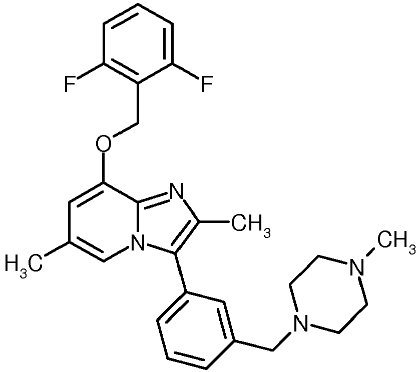 | ¹H NMR (400 MHz, DMSO-*d*₆) δ = 2.18 (s, 3 H), 2.22 - 2.55 (m, 14 H), 3.57 (s, 2 H), 5.29 (s, 2 H), 6.78 (s, 1 H), 7.24 (t, 2 H), 7.32-7.43 (m, 3 H), 7.52 (t, 1 H), 7.55 - 7.65 (m, 1 H), 7.69 (s, 1 H). |
| | (10% d. Th.) | |
| **121** | 8-[(2,6-Difluorbenzyl)oxy]-2-methyl-3-[1-(pyridin-3-yl)-1H-pyrazol-4-yl] imidazo [1,2-a]pyridin | LC-MS (Methode 1): Rₜ = 0.70 min |
| | 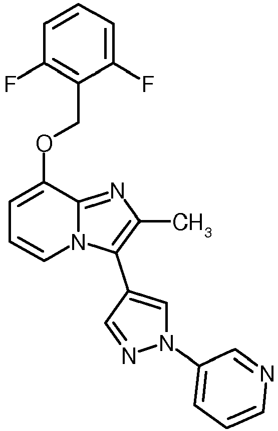 | MS (ESpos): m/z = 418 (M+H)⁺ |
| | | ¹H NMR (400 MHz, DMSO-*d*₆) δ = 2.40 (s, 3 H), 5.32 (s, 2 H), 6.83 - 6.91 (m, 2 H), 7.23 (t, 2 H), 7.55 - 7.64 (m, 2 H), 8.11 (d, 1 H), 8.20 (s, 1 H), 8.32 - 8.38 (m, 1 H), 8.58 (d, 1 H), 9.01 (s, 1 H), 9.24 (d, 1 H). |
| | (13% d. Th.) | |
| **122** | 8-[(2,6-Difluorbenyl)oxy]-3-[1-(5-fluorpyridin-3-yl)-1H-pyrazol-4-yl]-2-methylimidazo[1,2-a]pyridin | LC-MS (Methode 1): Rₜ = 0.77 min |
| | 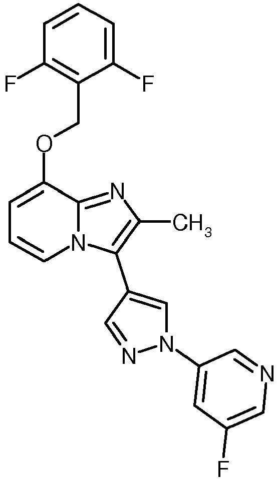 | MS (ESpos): m/z = 436 (M+H)⁺ |
| | | ¹H NMR (400 MHz, DMSO-*d*₆) δ = 2.42 (s, 3 H), 5.33 (s, 2 H), 6.84 - 6.91 (m, 2 H), 7.23 (t, 2 H), 7.55 - 7.64 (m, 1 H), 8.11 (d, 1 H), 8.23 (s, 1 H), 8.33 - 8.39 (m, 1 H), 8.60 (d, 1 H), 9.06 (s, 1 H), 9.18 (s, 1 H). |
| | (23% d. Th.) | |

### Beispiel 123

### 8-[(2,6-Difluorbenzyl)oxy]-3-[1-(4-fluorphenyl)-1H-pyrazol-4-yl]-2-methylimidazo[1,2-a]pyridin

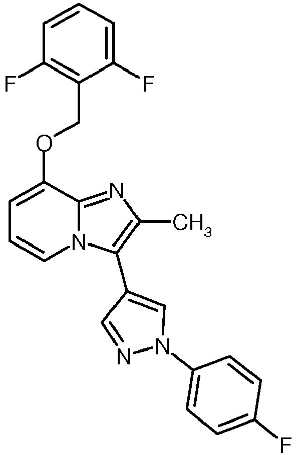

100 mg (0.28 mmol) 3-Brom-8-[(2,6-difluorbenzyl)oxy]-2-methylimidazo[1,2-a]pyridin aus Beispiel 28A im Lösungsmittelgemisch Toluol/Ethanol/Wasser (1 ml/2 ml/1 ml) wurden unter Argon mit 175 mg (6.81 mmol) [1-(4-Fluorphenyl)-1H-pyrazol-4-yl]boronsäure, 180 mg (0.85 mmol) Kaliumphosphat und 14.5 mg (0.03 mmol) Bis(tri-tert.-butyl-phosphin)palladium(0) versetzt und 15 min in einem auf 120°C vorgeheizten Ölbad gerührt. Das Reaktionsgemisch wurde eingeengt und das Rohprodukt wurde mittels präparativer HPLC gereinigt (RP18 Säule, Laufmittel: Acetonitril/Wasser-Gradient unter Zusatz von 0.1% TFA). Die Produktfraktion wurde mit Acetonitril verrührt und vom vorhandenen Feststoff abfiltriert. Es wurden 62 mg der Zielverbindung (50% d. Th.) erhalten.
LC-MS (Methode 1): Rₜ = 0.92 min
MS (ESpos): m/z = 435 (M+H)⁺
¹H NMR (400 MHz, DMSO-*d*₆) δ = 2.39 (s, 3 H), 5.32 (s, 2 H), 6.81 - 6.89 (m, 2 H), 7.23 (t, 2 H), 7.40 (t, 2 H), 7.55 - 7.64 (m, 1 H), 7.96 - 8.05 (m, 2 H), 8.08 - 8.13 (m, 2 H), 8.88 (s, 1 H).

### Beispiel 124

### 5-{8-[(2,6-Difluorbenzyl)oxy]-2-methylimidazo[1,2-a]pyridin-3-yl}-1H-1,2,4-triazol-3-amin

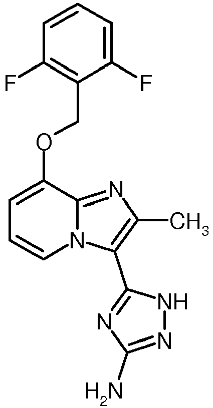

1.248 g (23.099 mmol) Natriummethylat in 20 ml Methanol wurden auf 0°C abgekühlt. Danach wurden 2.844 g (11.549 mmol) Aminoguanidin Hemisulfat zugegeben und es wurde 10 min bei RT gerührt. Anschliessend wurden 2.00 g (5.775 mmol) Ethyl-8-[(2,6-difluorbenzyl)oxy]-2-methylimidazo[1,2-a]pyridin-3-carboxylat aus Beispiel 2A in 20 ml Methanol suspendiert zugegeben und es wurde über Nacht zum Rückfluss erhitzt. Dann wurde eingeengt und der Rückstand wurde mittels präparativer HPLC gereinigt (Methanol:Wasser:Wasser (+1 % Trifluoressigsäure) - 55:40:5- isokratisch). Es wurden 60 mg der Zielverbindung erhalten (2.6 % d. Th.).
LC-MS (Methode 1): Rₜ = 0.64 min
MS (ESpos): m/z = 357.2 (M+H)⁺
¹H-NMR (400 MHz, DMSO-d₆): δ = 2.72 (s, 3 H), 5.46 (s, 2 H), 6.45 (s br, 2H), 7.23-7.29 (m, 2 H), 7.41 - 7.51 (br s, 1 H), 7.59-7.66 (m, 2H), 9.33 (d, 1 H), 12.63 (br s, 1H).

### Beispiel 125

### 3-{8-[(2,6-Difluorbenzyl)oxy]-2-methylimidazo[1,2-a]pyridin-3-yl}-1-(2,2,2-trifluorethyl)-1H-1,2,4-triazol-5-amin

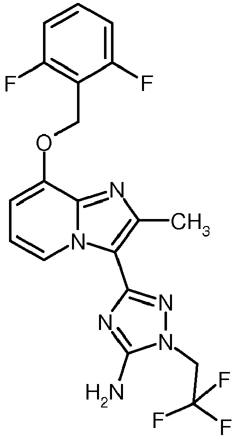

45 mg (0.127 mmol) 5-{8-[(2,6-Difluorbenzyl)oxy]-2-methylimidazo[1,2-a]pyridin-3-yl}-1H-1,2,4-triazol-3-amin aus Beispiel 124 und 49.5 mg (0.152 mmol) Cäsiumcarbonat in DMF (2 ml) wurden mit 25 µl (0.152 mmol) 2,2,2-Trifluorethyltrichlormethansulfonat versetzt. Es wurde 3 Tage bei RT gerührt und danach nochmals 20.6 mg (0.063 mmol) Cäsiumcarbonat und 10 µl (0.063 mmol) 2,2,2-Trifluorethyltrichlormethansulfonat zugegeben und über Nacht bei RT gerührt. Die Reaktionslösung wurde filtriert und mittels päparativer HPLC gereinigt (Eluent: Acetonitril/Wasser mit 0.05% Ameisensäure, Gradient). Es wurden 12 mg (21 % d. Th.) der Zielverbindung erhalten.
LC-MS (Methode 1): Rₜ = 0.76 min
MS (ESpos): m/z = 439.2 (M+H)⁺
¹H-NMR (400 MHz, DMSO-d₆): δ = 2.60 (s, 3 H), 4.98-5.05 (dd, 2H), 5.31 (s, 2 H), 6.88 (s br, 2H), 6.96 (d, 2H), 7.22-7.26 (m, 2 H), 7.55 - 7.63 (m, 1 H), 9.02 (t, 1 H).

### Beispiel 126

### 8-[(2,6-Difluorbenzyl)oxy]-2,6-dimethyl-3-[5-(trifluormethyl)-1H-1,2,4-triazol-3-yl]imidazo[1,2-a]pyridin

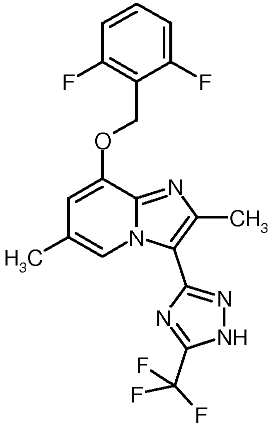

300 mg 8-[(2,6-Difluorbenzyl)oxy]-2,6-dimethylimidazo[1,2-a]pyridin-3-carboximidohydrazid des Rohproduktes aus Beispiel 43A in Dichlormethan (1 ml) wurden mit 1 ml (7.080 mmol) Trifluoressigsaeureanhydrid versetzt. Es wurde über Nacht bei RT gerührt. Die Reaktionslösung wurde mittels päparativer HPLC gereinigt (Eluent: Acetonitril/Wasser mit 0.05% Ameisensäure, Gradient). Es wurden 34 mg (43 % d. Th.) der Zielverbindung erhalten.
LC-MS (Methode 1): Rₜ = 1.00 min
MS (ESpos): m/z = 424.2 (M+H)⁺
¹H-NMR (400 MHz, DMSO-d₆): δ = 2.36 (s, 3 H), 5.32 (s, 2 H), 7.02 (s, 1H), 7.22-7.28 (m, 2 H), 7.57 - 7.64 (m, 1 H), 8.47 (br s, 1 H), 15.05 (br s, 1H), 1 Signal wahrscheinlich unter DMSO-Signal.

### Beispiel 127

### 1-(4-{8-[(2,6-Difluorbenzyl)oxy]-2,6-dimethylimidazo[1,2-a]pyridin-3-yl}-3-methyl-1H-pyrazol-1-yl)-2-methylpropan-2-amin Formiat

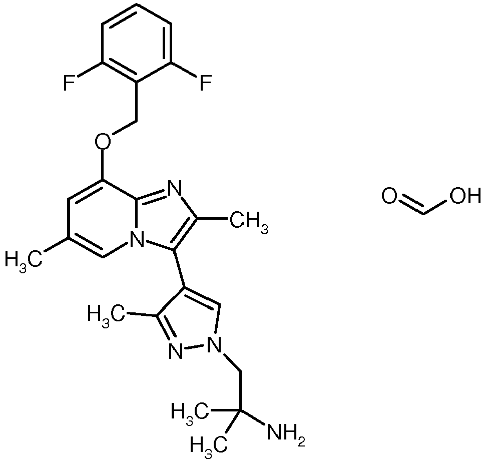

59 mg (0.13 mmol) 8-[(2,6-Difluorbenzyl)oxy]-2,6-dimethyl-3-[3-methyl-1-(2-methyl-2-nitropropyl)-1H-pyrazol-4-yl]imidazo[1,2-a] aus Beispiel 74A in 2 ml Ethanol wurden unter Argon mit ca. 203 mg Raney-Nickel (50%ige wässrige Aufschlämmung) versetzt und über Nacht bei RT und Normaldruck mit Wasserstoff gerührt. Das Reaktionsgemisch wurde über Kieselgur filtriert, gut mit Ethanol nachgewaschen und das Filtrat zur Trockene eingeengt. Der Rückstand wurde über präparative HPLC (RP18 Säule, Laufmittel: Acetonitril/Wasser-Gradient unter Zusatz von 0.05% Ameisensäure) getrennt und die Produktfraktionen wurden am Rotationsverdampfer eingeengt. Es wurden 45 mg der Zielverbindung (74% d. Th.) erhalten.
LC-MS (Methode 17): Rₜ = 1.47 min
MS (ESpos): m/z = 440 (M+H)⁺
¹H-NMR (400 MHz, DMSO-d₆): δ = 1.12 - 1.22 (m, 6 H), 2.01 (s, 3 H), 2.14 - 2.19 (m, 3 H), 2.25 (s, 3 H), 4.10 - 4.18 (m, 2 H), 5.28 (s, 2 H), 6.75 (s, 1 H), 7.20 - 7.30 (m, 2 H), 7.39 (s, 1 H), 7.55 - 7.65 (m, 1 H), 7.91 (s, 1 H), 8.25 - 8.32 (m, 2 H).

### Beispiel 128

### 8-[(2,6-Difluorbenzyl)oxy]-2,6-dimethyl-3-(1H-1,2,4-triazol-3-yl)imidazo[1,2-a]pyridin

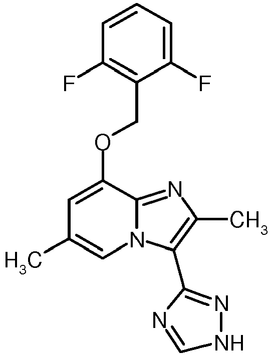

1.00 g (3.02 mmol) 8-[(2,6-Difluorbenzyl)oxy]-2,6-dimethylimidazo[1,2-a]pyridin-3-carboxamid aus Beispiel 40A in 6.30 ml (47.58 mmol) *N,N*-Dimethylformamiddimethylacetal wurde 2 Stunden auf 120°C erhitzt. Dann wurde abgekühlt und am Rotationsverdampfer eingeengt. Der Rückstand wurde mit 14.5 ml (252.6 mmol) Essigsäure und 0.135 ml (3.47 mmol) Hydrazinhydrat versetzt und anschließend wurde über Nacht bei 90°C gerührt. Nach Abkühlen wurde das Lösungsmittel Rotationsverdampfer entfernt. Der Rückstand wurde mit Essigsäureethylester und gesättigter, wässriger Natriumhydrogencarbonat-Lösung kräftig verrührt. Der dabei entstandene Feststoff wurde abfiltriert, mit Wasser und Diethylether gewaschen und anschließend im Hochvakuum getrocknet. Es wurden 1.04 g der Zielverbindung (97% d. Th.) erhalten.
LC-MS (Methode 1): Rₜ = 0.71 min
MS (ESpos): m/z = 356 (M+H)⁺
¹H-NMR (400 MHz, DMSO-d₆): δ = 2.36 (s, 3 H), 2.62 (s, 3 H), 5.30 (s, 2 H), 6.89 (s, 1 H), 7.20 - 7.29 (m, 2 H), 7.55 - 7.65 (m, 1 H), 8.73 (s, 1 H), 8.91 (s, 1 H).

### Beispiel 129

### 1-(3-{8-[(2,6-Difluorbenzyl)oxy]-2,6-dimethylimidazo[1,2-a]pyridin-3-yl}-1H-1,2,4-triazol-1-yl)-2-methylpropan-2-amin

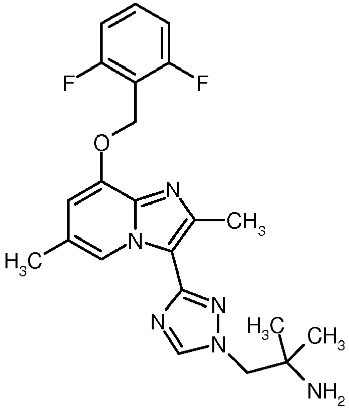

140 mg (0.31 mmol) 8-[(2,6-Difluorbenzyl)oxy]-2,6-dimethyl-3-[1-(2-methyl-2-nitropropyl)-1H-1,2,4-triazol-3-yl]imidazo[1,2-a]pyridin aus Beispiel 75A in 5 ml Ethanol wurden unter Argon mit ca. 500 mg Raney-Nickel (50%ige wässrige Aufschlämmung) versetzt und über Nacht bei RT und Normaldruck mit Wasserstoff gerührt. Das Reaktionsgemisch wurde über Kieselgur filtriert, gut mit Ethanol nachgewaschen und das Filtrat zur Trockene eingeengt. Der Rückstand wurde über präparative HPLC (RP18 Säule, Laufmittel: Acetonitril/Wasser-Gradient unter Zusatz von 0.05% Diethylamin) getrennt und die Produktfraktionen wurden am Rotationsverdampfer eingeengt. Es wurden 63 mg der Zielverbindung (48% d. Th.) erhalten.
LC-MS (Methode 1): Rₜ = 0.54 min
MS (ESpos): m/z = 427 (M+H)⁺
¹H-NMR (400 MHz, DMSO-d₆): δ = 1.07 (s, 6 H), 2.35 (s, 3 H), 2.61 (s, 3 H), 4.12 - 4.17 (m, 2 H), 5.30 (s, 2 H), 6.89 (s, 1 H), 7.21 - 7.29 (m, 2 H), 7.55 - 7.65 (m, 1 H), 8.66 (s, 1 H), 8.88 (s, 1 H).

### Beispiel 130

### 1-(5-{8-[(2,6-Difluorbenzyl)oxy]-2,6-dimethylimidazo[1,2-a]pyridin-3-yl}-1,3,4-oxadiazol-2-yl)-2-methylpropan-2-amin

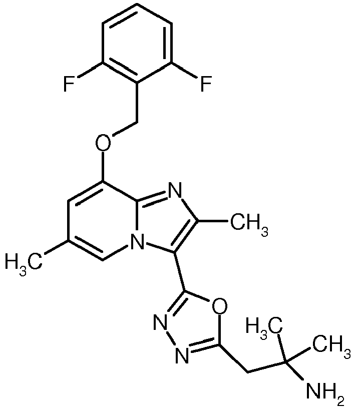

Zu 125 mg (0.24 mmol) *tert*.-Butyl-[1-(5-{8-[(2,6-difluorbenzyl)oxy]-2,6-dimethylimidazo[1,2-a]pyridin-3-yl}-1,3,4-oxadiazol-2-yl)-2-methylpropan-2-yl]carbamat aus Beispiel 66A wurden in 4 ml Dichlormethan 1.0 ml TFA getropft und das Reaktionsgemisch wurde 30 min bei RT gerührt. Es wurde am Rotationsverdampfer eingeengt und der Rückstand über präparative HPLC (RP18 Säule, Laufmittel: Acetonitril/Wasser-Gradient unter Zusatz von 0.05% Diethylamin) getrennt. Die Produktfraktionen wurden am Rotationsverdampfer eingeengt. Der Rückstand wurde mit Dichlormethan und gesättigter, wässriger Natriumhydrogencarbonat-Lösung verrührt. Die Phasen wurden getrennt, die wässrige Phase wurde zweimal mit Dichlormethan gewaschen und die vereinigten organischen Phasen wurden über Natriumsulfat getrocknet. Es wurde filtriert und das Filtrat am Rotationsverdampfer eingeengt. Die produkthaltigen Fraktionen wurden nochmals gereinigt [Säule: Kromasil 100 C18, 5 µm, 250 x 20 mm; Eluent: 56% Wasser, 30% Methanol + 14% 1%ige wässrige TFA; Fluß: 24 ml/min; 40°C; Detektion: 210 nm], eingeengt und lyophilisiert. Es wurden 46 mg der Zielverbindung (45% d. Th.) erhalten.
LC-MS (Methode 1): Rₜ = 0.73 min
MS (ESpos): m/z = 428 (M+H)⁺
¹H-NMR (400 MHz, DMSO-d₆): δ = 1.42 (s, 6 H), 2.43 (s, 3 H), 2.62 (s, 3 H), 5.35 (s, 2 H), 7.15 (s, 1 H), 7.21 - 7.30 (m, 2 H), 7.55 - 7.67 (m, 1 H), 8.10 (br. s., 2 H), 8.78 (s, 1 H).

### Beispiel 131

### 1-(3-{8-[(2,6-Difluorbenzyl)oxy]-2,6-dimethylimidazo[1,2-a]pyridin-3-yl}-1,2,4-oxadiazol-5-yl)-2-methylpropan-2-amin

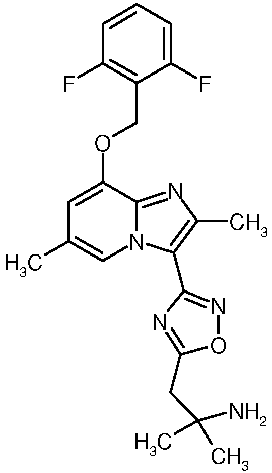

Zu 160 mg (0.30 mmol) tert-Butyl-[1-(3-{8-[(2,6-difluorbenzyl)oxy]-2,6-dimethylimidazo[1,2-a]pyridin-3-yl}-1,2,4-oxadiazol-5-yl)-2-methylpropan-2-yl]carbamat aus Beispiel 69A in 4 ml Dichlormethan wurden 1.0 ml TFA getropft und das Reaktionsgemisch wurde 1 h bei RT gerührt. Es wurde am Rotationsverdampfer eingeengt, über präparative HPLC (RP18 Säule, Laufmittel: Acetonitril/Wasser-Gradient unter Zusatz von 0.05% Diethylamin) getrennt und die Produktfraktionen wurden am Rotationsverdampfer eingeengt. Der Rückstand wurde zweimal mit 5 ml Toluol versetzt, bis zur Trockene eingeengt und anschließend mit 5 ml Acetonitril/Wasser-Gemisch versetzt. Die Acetonitril-Reste wurden am Rotationsverdampfer entfernt, der wässrige Rückstand im Trockeneisbad eingefroren und über Nacht lyophilisiert. Es wurden 31 mg der Zielverbindung (23% d. Th.; Reinheit 95%) erhalten.
LC-MS (Methode 1): Rₜ = 0.79 min
MS (ESpos): m/z = 428 (M+H)⁺
¹H-NMR (400 MHz, DMSO-d₆): δ = 1.25 (s, 6 H), 2.39 (s, 3 H), 2.64 (s, 3 H), 3.17 (s, 2 H), 5.33 (s, 2 H), 7.06 (s, 1 H), 7.21 - 7.29 (m, 2 H), 7.56 - 7.65 (m, 1 H), 8.64 (s, 1 H).

### Beispiel 132

### 1-(5-{8-[(2,6-difluorbenzyl)oxy]-2,6-dimethylimidazo[1,2-a]pyridin-3-yl}-1,2,4-oxadiazol-3-yl)-2-methylpropan-2-amin Formiat

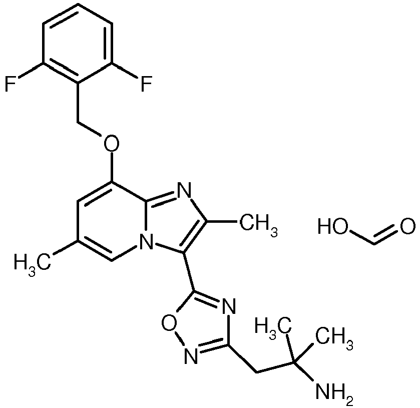

52 mg (0.09 mmol) Benzyl-[1-(5-{8-[(2,6-difluorbenzyl)oxy]-2,6-dimethylimidazo[1,2-a]pyridin-3-yl}-1,2,4-oxadiazol-3-yl)-2-methylpropan-2-yl]carbamat aus Beispiel 72A in 5 ml Ethanol wurden mit 5.2 mg 10%iger Palladium auf Kohle versetzt und das Reaktionsgemisch wurde unter Argon 40 min bei RT gerührt. Die Reaktionsmischung wurde abfiltriert und das Lösungsmittel wurde am Rotationsverdampfer entfernt. Das Rohprodukt wurde über präparative HPLC (RP18 Säule, Laufmittel: Acetonitril/Wasser-Gradient unter Zusatz von 0.05% Ameisensäure) getrennt und die Produktfraktionen wurden am Rotationsverdampfer eingeengt. Es wurden 21 mg der Zielverbindung (44% d. Th.; Reinheit 95%) erhalten.
LC-MS (Methode 1): Rₜ = 0.78 min
MS (ESpos): m/z = 428 (M+H)⁺
¹H-NMR (400 MHz, DMSO-d₆): δ = 1.22 (s, 6 H), 2.32 (s, 3 H), 2.93 (s, 2 H), 5.29 (s, 2 H), 6.94 - 6.99 (m, 1 H), 7.20 - 7.28 (m, 2 H), 7.55 - 7.64 (m, 1 H), 8.36 - 8.41 (m, 1 H), 10.18 - 10.23 (m, 1 H).

### Beispiel 133

### 1-(5-{8-[(2,6-Difluorbenzyl)oxy]-2,6-dimethylimidazo[1,2-a]pyridin-3-yl}-1,3,4-thiadiazol-2-yl)-2-methylpropan-2-amin Formiat

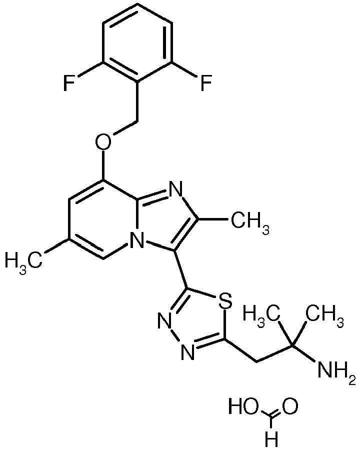

12 mg (0.02 mmol; Rohprodukt) *tert*.-Butyl-[1-(5-{8-[(2,6-difluorbenzyl)oxy]-2,6-dimethylimidazo[1,2-a]pyridin-3-yl}-1,3,4-thiadiazol-2-yl)-2-methylpropan-2-yl]carbamat aus Beispiel 65A in 3 ml Dichlormethan wurden mit 0.5 ml TFA versetzt und 0.5 h bei RT gerührt. Das Reaktionsgemisch wurde eingeengt und anschließend mittels präparativer HPLC gereinigt (RP18 Säule, Laufmittel: Acetonitril/Wasser-Gradient unter Zusatz von 0.05% Ameisensäure). Die Produktfraktionen wurden lyophilisiert. Es wurden 6 mg (62% d. Th.) der Titelverbindung erhalten.
LC-MS (Methode 1): Rₜ = 0.78 min
MS (ESpos): m/z = 444 (M+H)⁺
¹H NMR (400 MHz, DMSO-*d*₆) δ = 1.15 (s, 6 H), 2.41 (s, 3 H), 2.56 (s, 3 H), 3.20 (br. s, 2 H), 5.34 (s, 2 H), 7.08 (s, 1 H), 7.24 (t, 2 H), 7.55 - 7.65 (m, 1 H), 9.17 (s, 1 H).

### Beispiel 134

### 8-[(2,6-Difluorbenzyl)oxy]-2-methyl-3-(2H-1,2,3-triazol-4-yl)imidazo[1,2-a]pyridin

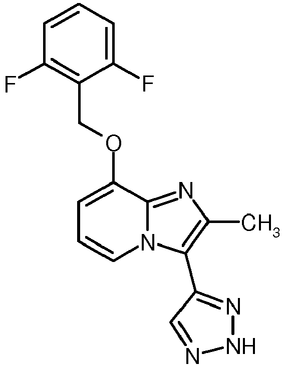

1.30 g (4.36 mmol) 8-[(2,6-Difluorbenzyl)oxy]-3-ethinyl-2-methylimidazo[1,2-a]pyridin aus Beispiel 77A, 0.57 ml (4.36 mmol) Azidotrimethylsilan, 33 ml Wasser/Ethanol (2/1), 345 mg (1.74 mmol) (2R)-2-[(1S)-1,2-Dihydroxyethyl]-4-hydroxy-5-oxo-2H-furan-3-olat und 152 mg (0.61 mmol) Kupfer(II)-tetraoxosulfat(VI)-Pentahydrat wurden über Nacht bei 50°C gerührt. Dann wurde mit 173 mg (0.87 mmol) (2R)-2-[(1S)-1,2-Dihydroxyethyl]-4-hydroxy-5-oxo-2*H*-furan-3-olat und 76 mg (0.31 mmol) Kupfer(II)-tetraoxosulfat(VI)-Pentahydrat versetzt und weiter über Nacht bei Rückfluss gerührt. Es wurde mit 173 mg (0.87 mmol) (2R)-2-[(1S)-1,2-Dihydroxyethyl]-4-hydroxy-5-oxo-2*H*-furan-3-olat, 76 mg (0.31 mmol) Kupfer(II)-tetraoxosulfat(VI)-Pentahydrat und 0.573 ml (4.36 mmol) Azidotrimethylsilan versetzt und weiter über Nacht bei 85°C gerührt. Es wurde abgekühlt, filtriert und mit Wasser gewaschen. Der Feststoff wurde mit Essigsäureethylester gewaschen. Das Filtrat wurde abfiltriert, mit Wasser und Diethylether gewaschen und im Hochvakuum getrocknet. Beide Feststofffraktionen wurden vereinigt und nochmals gereinigt [Säule: Sunfire C18, 5 µm, 250 x 20 mm; Eluent: 56% Wasser, 30% Acetonitril + 14% 1%ige wässrige TFA; Fluß: 25 ml/min; 25°C; Detektion: 210 nm]. Es wurden 293 mg der Zielverbindung (19% d. Th.) erhalten.
LC-MS (Methode 16): Rₜ = 0.62 min
MS (ESpos): m/z = 342 (M+H)⁺
¹H-NMR (400 MHz, DMSO-d₆): δ = 2.56 (br. s., 3 H), 5.45 (s, 2 H), 7.22 - 7.31 (m, 2 H), 7.32 - 7.44 (m, 1 H), 7.45 - 7.57 (m, 1 H), 7.57 - 7.66 (m, 1 H), 8.30 - 8.93 (m, 2 H).

### Beispiel 135

### 8-[(2,6-Difluorbenzyl)oxy]-2,6-dimethyl-3-(4-methylphenyl)imidazo[1,2-a]pyridin

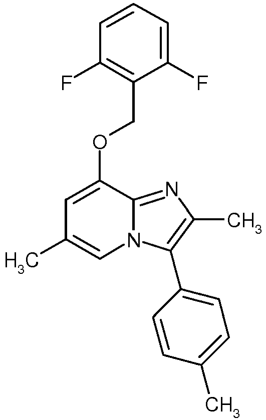

Eine Mischung von 100 mg 8-[(2,6-Difluorbenzyl)oxy]-2,6-dimethylimidazo[1,2-a]pyridin-3-carbonsäure (0.301 mmol, 1.0 eq.) aus Beispiel 16A, 56 µl 4-Bromtoluol (0.45 mmol, 1.5 eq.), 49.9 mg Kaliumcarbonat (0.361 mmol, 1.2 eq.), 11.5 mg Kupfer(I)iodid (0.060 mmol, 0.2 eq.) und 16.3 mg 1,10-Phenanthrolein in 2.0 ml N-Methylpyrrolidon wurde im Argonstrom entgast, anschließend mit 3.4 mg Palladium(II)acetat (0.015 mmol, 0.05 eq.) versetzt und in der Mikrowelle für 30 min auf 200°C erhitzt. Dann wurde über Kieselgur filtriert, mit Essigsäureethylester eluiert und eingeengt. Es wurde mit Wasser versetzt, zweimal mit Essigsäureethylester extrahiert, die vereinigten organischen Phasen wurden mit gesättigter wässriger Natriumchlorid-Lösung gewaschen, mit Magesiumsulfat getrocknet, filtriert und eingeengt. Der Rückstand wurde mittels Biotage Isolera (10 g Kieselgelkartusche, Cyclohexan/Essigsäureethylester-Gradient 10% ->100% Essigsäureethylester) gereinigt. Es wurden 50.7 mg (44% d. Th.) der Titelverbindung erhalten.
DC (Cyclohexan/Essigsäureethylester 1:1): R_{F} = 0.66
LC-MS (Methode 17): Rₜ = 2.02 min
MS (ESpos): m/z = 379 (M+H)⁺
¹H-NMR (400 MHz, DMSO-d₆): δ [ppm] = 2.24 (s, 3 H), 2.26 (s, 3 H), 2.40 (s, 3 H), 5.29 (s, 2 H), 6.76 (br. s, 1 H), 7.25 - 7.30 (m, 2 H), 7.38 (s, 4 H), 7.55 - 7.64 (m, 1 H), 7.66 (br. s, 1 H).

### Beispiel 136

### 5-{8-[(2,6-Difluorbenzyl)oxy]-2,6-dimethylimidazo[1,2-a]pyridin-3-yl}pyridin-2-carbonitril

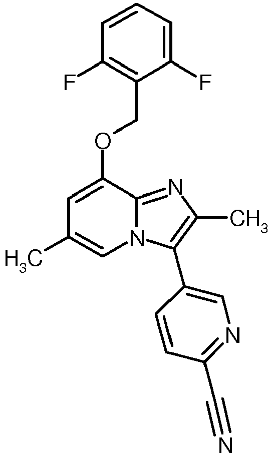

Eine Mischung von 100 mg 8-[(2,6-Difluorbenzyl)oxy]-2,6-dimethylimidazo[1,2-a]pyridin-3-carbonsäure (0.301 mmol, 1.0 eq.) aus Beispiel 16A, 82.6 mg 5-Brom-2-pyridincarbonitril (0.45 mmol, 1.5 eq.), 49.9 mg Kaliumcarbonat (0.361 mmol, 1.2 eq.), 5.7 mg Kupfer(I)iodid (0.030 mmol, 0.1 eq.) und 8.1 mg 1,10-Phenanthrolein (0.045 mmol, 0.015 eq.) in 1.0 ml *N-*Methylpyrrolidon wurde im Argonstrom entgast, anschließend mit 3.4 mg Palladium(II)acetat (0.015 mmol, 0.05 eq.) versetzt und in der Mikrowelle für 30 min auf 190°C erhitzt. Dann wurde über Kieselgur filtriert, mit Essigsäureethylester eluiert und eingeengt. Es wurde mit Wasser versetzt, zweimal mit Essigsäureethylester extrahiert, die vereinigten organischen Phasen wurden mit gesättigter wässriger Natriumchlorid-Lösung gewaschen, mit Magnesiumsulfat getrocknet, filtriert und eingeengt. Der Rückstand wurde mittels Biotage Isolera (10 g Kieselgelkartusche, Cyclohexan/Essigsäureethylester-Gradient 10% ->66% Essigsäureethylester) gereinigt, wobei 32.0 mg (Reinheit: 53%) der Titelverbindung erhalten wurden. Das verunreinigte Produkt wurde gemeinsam mit dem Rohprodukt einer weiteren Reaktion mittels präperativer HPLC (Methode 19) gereinigt. Das erhaltene Rohprodukt wurde aus einer Mischung von Wasser, Methanol und Acetonitril umkristallisiert. Es wurden 20.3 mg der Titelverbindung erhalten.
DC (Cyclohexan/Essigsäureethylester 2:1): R_{F} = 0.17
LC-MS (Methode 1): Rₜ = 0.78 min
MS (ESpos): m/z = 391 (M+H)⁺
¹H-NMR (400 MHz, DMSO-d₆): δ [ppm] = 2.28 (s, 3 H), 2.34 (s, 3 H), 5.30 (s, 2 H), 6.89 (s, 1 H), 7.21 - 7.29 (m, 2 H), 7.55 - 7.65 (m, 1 H), 7.91 (s, 1 H), 8.18 - 8.27 (m, 2 H), 8.93 (d, 1H).

### Beispiel 137

### 8-[(2,6-Difluorbenzyl)oxy]-2,6-dimethyl-3-(5-methyl-1,3-oxazol-2-yl)imidazo[1,2-a]pyridin

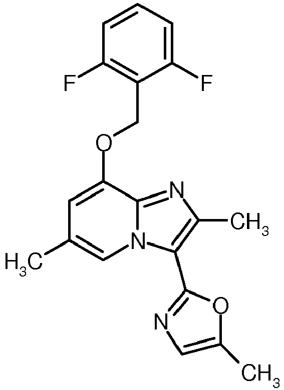

Eine Suspension von 100 mg 8-[(2,6-Difluorbenzyl)oxy]-2,6-dimethyl-*N*-(prop-2-in-1-yl)imidazo[1,2-a]pyridin-3-carboxamid (0.271 mmol, 1.0 eq.) aus Beispiel 58A in 5.0 ml Acetonitril wurde mit 4.1 mg Gold(III)chlorid (0.014 mmol, 0.05 eq.) versetzt und über Nacht bei 80°C gerührt. Anschließend wurde über Kieselgur filtriert und mit einer Mischung aus Acetonitril und Dichlormethan eluiert. Das Filtrat wurde eingeengt und mittels Biotage Isolera (10 g Kieselgelkartusche, Cyclohexan/Essigsäureethylester-Gradient) gereinigt. Es wurden 59 mg (59% d. Th.) der Titelverbindung erhalten.
LC-MS (Methode 1): Rₜ = 0.98 min
MS (ESpos): m/z = 370 (M+H)⁺
¹H-NMR (400 MHz, DMSO-d₆): δ [ppm]= 2.38 (s, 3 H), 2.42 (s, 3 H), 2.60 (s, 3 H), 5.31 (s, 2 H), 6.99 - 7.01 (m, 1 H), 7.09 - 7.11 (m, 1 H), 7.20 - 7.30 (m, 2 H), 7.53 - 7.65 (m, 1 H), 8.87 - 8.91 (m, 1 H).

### Beispiel 138

### Ethyl-5-{8-[(2,6-difluorbenzyl)oxy]-2,6-dimethylimidazo[1,2-a]pyridin-3-yl}-1,3-thiazol-2-carboxylat

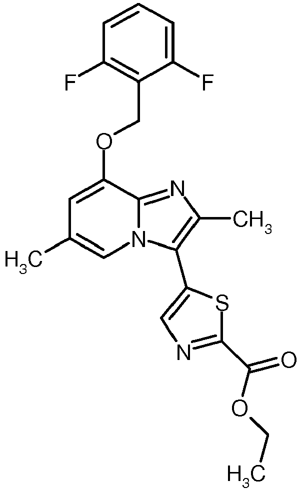

Eine Mischung von 43.0 mg 2-Brom-1-{8-[(2,6-difluorbenzyl)oxy]-2,6-dimethylimidazo[1,2-a]pyridin-3-yl}ethanon (0.105 mmol, 1.0 eq.) aus Beispiel 61A und 28.0 mg Thiooxamidsäureethylester (0.210 mmol, 2.0 eq.) in 5.0 ml Ethanol wurden 5 h auf Rückfluss erhitzt. Anschließend wurde eingeengt und mittels Biotage Isolera (25 g Kieselgelkartusche, Cyclohexan/Essigsäureethylester-Gradient, sowie Dichlormethan/Methanol-Gradient) gereinigt. Das isolierte Produktgemisch wurde mittels präparativer HPLC (Methode 21) gereingt, wobei 8.9 mg (19% d. Th) der Titelverbindung isoliert wurden.
LC-MS (Methode 1): Rₜ = 0.92 min
MS (ESpos): m/z = 444 (M+H)⁺
¹H-NMR (400 MHz, CDCl₃): δ [ppm]= 1.48 (t, 3 H), 2.35 (s, 3 H), 2.58 (s, 3 H), 4.53 (q, 2 H), 5.32 (s, 2 H), 6.60 (s, 1 H), 6.89 - 6.99 (m, 2 H), 7.29 - 7.40 (m, 1 H), 7.61 (s, 1 H), 8.37 (s, 1 H).

### Beispiel 139

### 5-{8-[(2,6-Difluorbenzyl)oxy]-2,6-dimethylimidazo[1,2-a]pyridin-3-yl}-3-ethoxythiophen-2-amin

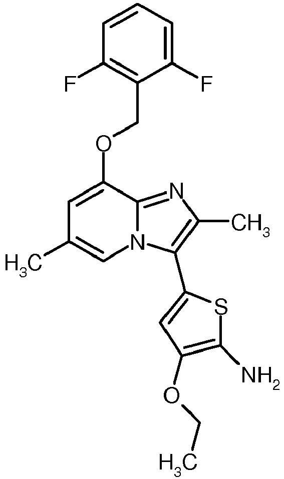

Die Titelverbindung entstand als Nebenprodukt in der Synthese von Ethyl-5-{8-[(2,6-difluorbenzyl)oxy]-2,6-dimethylimidazo[1,2-a]pyridin-3-yl}-1,3-thiazol-2-carboxylat.
LC-MS (Methode 1): Rₜ = 1.01 min
MS (ESpos): m/z = 430 (M+H)⁺
¹H-NMR (400 MHz, CDCl₃): δ [ppm]= 1.40 (t, 3 H), 2.38 (s, 3 H), 2.78 (s, 3 H), 4.38 (q, 2 H), 5.32 (s, 2 H), 6.53 (s, 1 H), 6.75 (s, 1 H), 6.89 - 6.98 (m, 2 H), 7.29 - 7.41 (m, 1 H), 9.18 (s, 1 H). (NH₂ wurde nicht beobachtet).

### Beispiel 140

### 8-[(2,6-Difluorbenzyl)oxy]-2,6-dimethyl-3-(pyridin-3-yl)imidazo[1,2-a]pyridin

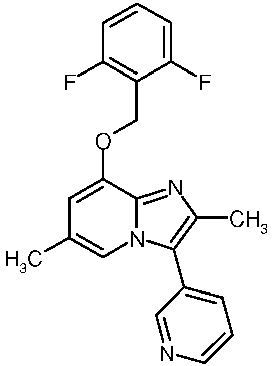

Eine Mischung von 69.5 mg 3-Brom-8-[(2,6-difluorbenzyl)oxy]-2,6-dimethylimidazo[1,2-a]pyridin (0.185 mmol, 1.0 eq.) aus Beispiel 30A, 34.2 mg Pyridin-3-boronsäure (0.278 mmol, 1.5 eq.) und (2'-Aminobiphenyl-2-yl)(chlor)palladium - dicyclohexyl[2',4',6'-tri(propan-2-yl)biphenyl-2-yl]phosphan (1:1) [CAS Nr: 1028206-56-5; Sigma Aldrich] (0.009 mmol, 0.05 eq.) 2.0 ml Acetonitril und 1.1 ml 0.5 M wässrige Kaliumphosphatlösung (0.56 ml, 3.0 eq.) wurden für 48 h bei 60°C gerührt. Dann wurde über eine Extrelut-Kartusche filtriert, mit Essigsäureethylester eluiert und das Filtrat wurde eingeengt. Das Rohprodukt wurde mittels präperativer HPLC (Methode 19) gereinigt, wobei 32.7 mg (68 % d. Th) der Titelverbindung erhalten wurden.
LC-MS (Methode 1): Rₜ = 0.75 min
MS (ESpos): m/z = 366 (M+H)⁺
¹H-NMR (400 MHz, DMSO-d₆): δ [ppm]= 2.27 (s, 3 H), 2.29 (s, 3 H), 5.30 (s, 2 H), 6.83 (br. s, 1 H), 7.22 - 7.29 (m, 2 H), 7.55 - 7.65 (m, 2 H), 7.74 (br. s, 1 H), 7.94 - 8.01 (m, 1 H), 8.63 - 8.67 (m, 1 H), 8.69 - 8.74 (m, 1 H)

### Beispiel 141

### Ethyl-5-{8-[(2,6-difluorbenzyl)oxy]-2,6-dimethylimidazo[1,2-a]pyridin-3-yl}-1,2-oxazol-3-carboxylat

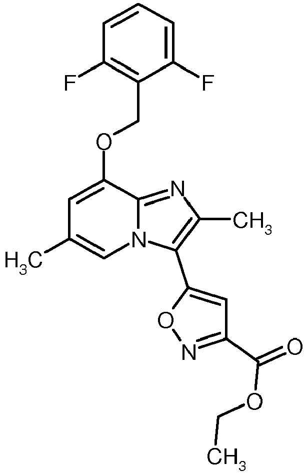

Eine Mischung von 815 mg Ethyl-4-{8-[(2,6-difluorbenzyl)oxy]-2,6-dimethylimidazo[1,2-a]pyridin-3-yl}-2,4-dioxobutanoat (80%ig, 1.89 mmol) aus Beispiel 62A und 461 mg Hydroxylaminhydrochlorid (6.63 mmol, 3.5 eq.) in 70 ml Ethanol wurden 7 Tage auf Rückfluss erhitzt. Anschließend wurde das Reaktionsgemisch eingeengt und aus Wasser/Acetonitril in der Siedehitze umkristallisiert. Es wurden 473 mg (51% d. Th., Reinheit: 87%) der Titelverbindung erhalten.
DC (Cyclohexan/Essigsäureethylester 1:1): R_{F} = 0.51
LC-MS (Methode 1): Rₜ = 1.12 min
MS (ESpos): m/z = 428 (M+H)⁺
¹H-NMR (400 MHz, DMSO-d₆): δ [ppm]= 1.32 - 1.41 (m, 3 H), 2.39 (s, 3 H), 4.37 - 4.49 (m, 2 H), 5.32 (s, 2 H), 7.05 (s, 1 H), 7.16 - 7.29 (m, 2 H), 7.34 (s, 1 H), 7.55 - 7.65 (m, 1 H), 8.24 (s, 1 H), (weiterer Peak unter Lösungsmittelsignal).

### Beispiel 142

### (5-{8-[(2,6-Difluorbenzyl)oxy]-2,6-dimethylimidazo[1,2-a]pyridin-3-yl}-1,2-oxazol-3-yl)methanol

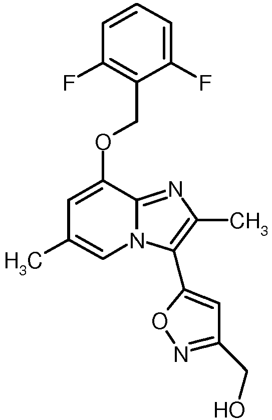

Eine Suspension von 470 mg Ethyl-5-{8-[(2,6-difluorbenzyl)oxy]-2,6-dimethylimidazo[1,2-a]pyridin-3-yl}-1,2-oxazol-3-carboxylat (0.990 mmol, 1.0 eq.) aus Beispiel 141 in 20 ml Ethanol wurde mit 37.4 mg Natriumborhydrid (0.990 mmol, 1.0 eq.) versetzt und für 2 h bei Raumtemperatur sowie 1 h bei Rückfluss gerührt. Anschließend wurde das Gemisch zur Hälfte eingeengt, mit Wasser versetzt und mehrfach mit Dichlormethan extrahiert. Die vereinigten organischen Phasen wurden mit Magnesiumsulfat getrocknet, filtriert und eingeengt. Der Rückstand wurde mittels Biotage Isolera (50 g Kieselgelkartusche, Dichlormethan/Mehtanol-Gradient) gereinigt, wobei 125 mg (32% d. Th) der Titelverbindung erhalten wurden.
DC (Dichlormethan/Methanol 100:5): R_{F} 0.33
LC-MS (Methode 1): Rₜ = 0.85 min
MS (ESpos): m/z = 386 (M+H)⁺
¹H-NMR (400 MHz, DMSO-d₆): δ [ppm]= 2.38 (br. s, 3 H), 2.49 (s, 3 H), 4.61 (d, 2 H), 5.31 (s, 2 H), 5.59 (t, 1 H), 6.88 (s, 1 H), 6.99 (br. s, 1 H), 7.20 - 7.30 (m, 2 H), 7.54 - 7.67 (m, 1 H), 8.18 - 8.21 (m, 1 H).

### Beispiel 143

### 1-(3-{8-[(2,6-Difluorbenzyl)oxy]-2,6-dimethylimidazo[1,2-a]pyridin-3-yl}-1H-pyrazol-5-yl)-2-methylpropan-2-amin

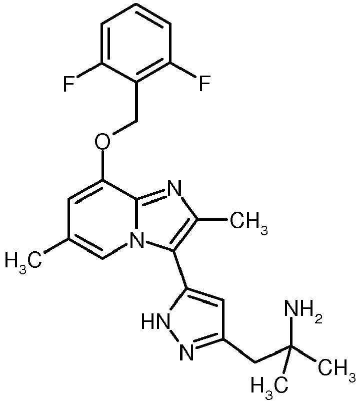

Eine Lösung von 120 mg (0.018 mmol der Mischung aus Beispiel 80A [1-(3-{8-[(2,6-Difluorbenzyl)oxy]-2,6-dimethylimidazo[1,2-a]pyridin-3-yl}-1H-pyrazol-5-yl)-2-methylpropan-2-yl]carbamidsäure-tert.-butylester in 2 ml Dichlormethan wurde mit 0.028 ml (0.365 mmol) Trifluoressigsäure versetzt. Die erhaltene Lösung wurde über Nacht bei Raumtemperatur gerührt. Nach dem Ende der Umsetzung wurde das Lösungsmittel im Vakuum abgezogen und der verbliebene Rückstand wurde durch präparative HPLC-Chromatographie (Methode 26) aufgereinigt, wodurch man 5.3 mg (60% d. Th., Reinheit 88%) der Titelverbindung (Beispiel 149) erhielt.
LC MS (Methode 25): Rₜ = 7.26 min; m/z= 426.19 (M+H)⁺
¹H-NMR (600 MHz, DMSO-d₆): δ [ppm] = 1.13 (s, 6 H), 2.30 (s, 3 H), 2.42 (s, 3H), 2.79 (s, 2H), 5.28 (s. 2 H), 6.42 (s, 1 H), 6.79 (s, 1 H), 7.14 -7.31 (m, 2 H), 7.53 - 7.66 (m, 1 H), 8.55 - 8.74 (m, 1 H).
¹³C-NMR (151 MHz, DMSO-d₆): δ [ppm] = 14.8, 18.3, 28.0, 38.7, 50.5, 58.1, 103.7, 105.9, 111.9, 115.2, 116.9, 121.0, 132.1, 136.4, 139.5, 140.2, 145.9, 160.6.

### Example 144

### 1-(5-{8-[(2,6-Difluorbenzyl)oxy]-2,6-dimethylimidazo[1,2-a]pyridin-3-yl}-1,2-oxazol-3-yl)-2-methylpropan-2-amin

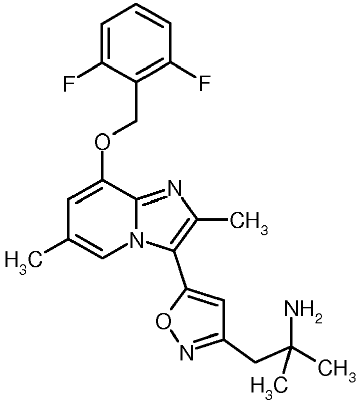

Eine Mischung von 200 mg (0.238 mmol, Reinheit 63%) (6-{8-[(2,6-Difluorbenzyl)oxy]-2,6-dimethylimidazo[1,2-a]pyridin-3-yl}-2-methyl-4,6-dioxohexan-2-yl)carbamidsäure-tert.-butylester (Beispiel 79A) und 165.3 mg (2.38 mmol) Hydroxylamin-Hydrochlorid in 10 ml Ethanol wurden in der Mikrowelle 30 min bei 120°C unter Rühren erhitzt. Die Reaktionsmischung wurde auf RT abgekühlt und im Vakuum eingedampft. Der Rückstand wurde mit Essigsäureethylester (15 ml) und Wasser (10 ml) versetzt, geschüttelt und die Phasen wurden anschließend getrennt. Die organische Phase wurde im Vakuum zur Trockne eingedampft. Der Rückstand wurde anschließend mittels präparativer HPLC gereinigt (Methode 26). Es wurden 47 mg (46% d. Th.) der Titelverbindung erhalten.
LC MS (Methode 25): Rₜ = 7.71 min; m/z = 427.09 (M+H)⁺
¹H-NMR (600 MHz, DMSO-d₆): d [ppm] = 1.12 (s, 6 H), 2.38 (s, 3 H), 2.49 (s, 3 H), 2.75 (s, 2 H), 5.32 (s, 2 H), 6.80 (s, 1 H), 6.98 (s, 1 H), 7.25 (t, 2 H), 7.60 (ddd, 1 H), 8.20 (s, 1 H)
¹³C-NMR (151 MHz, DMSO-d₆): δ [ppm] = 15.0, 18.2, 30.3, 40.4, 49.4, 58.3, 102.1, 107.8, 111.3, 111.6, 111.9, 116.4, 123.2, 132.2, 138.0, 143.3, 146.0, 159.6, 161.3, 161.5.

### B. Bewertung der pharmakologischen Wirksamkeit

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

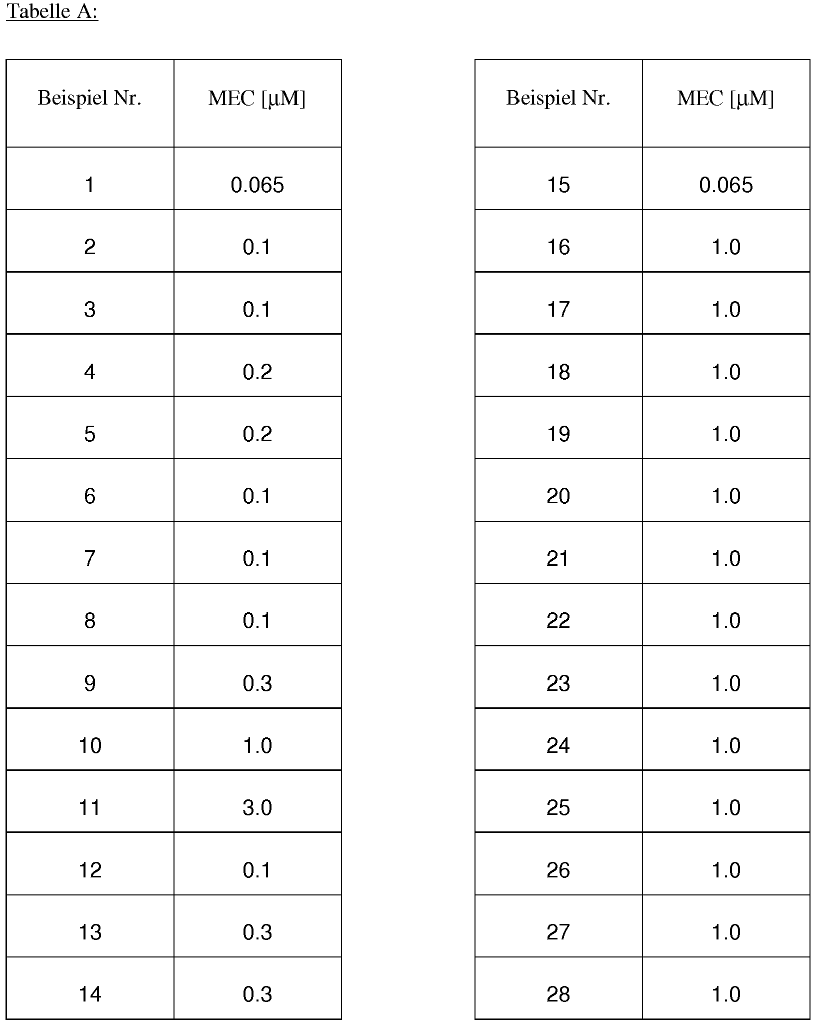

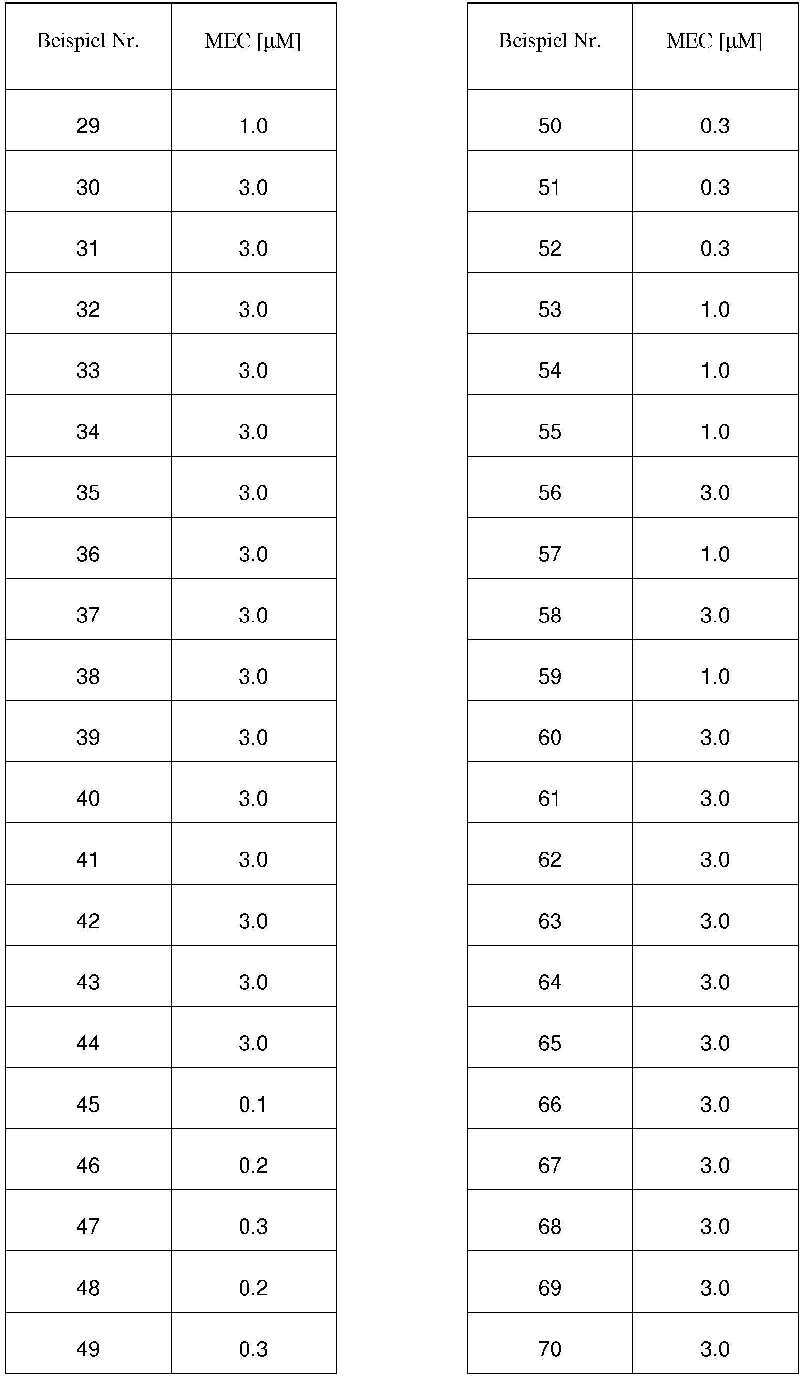

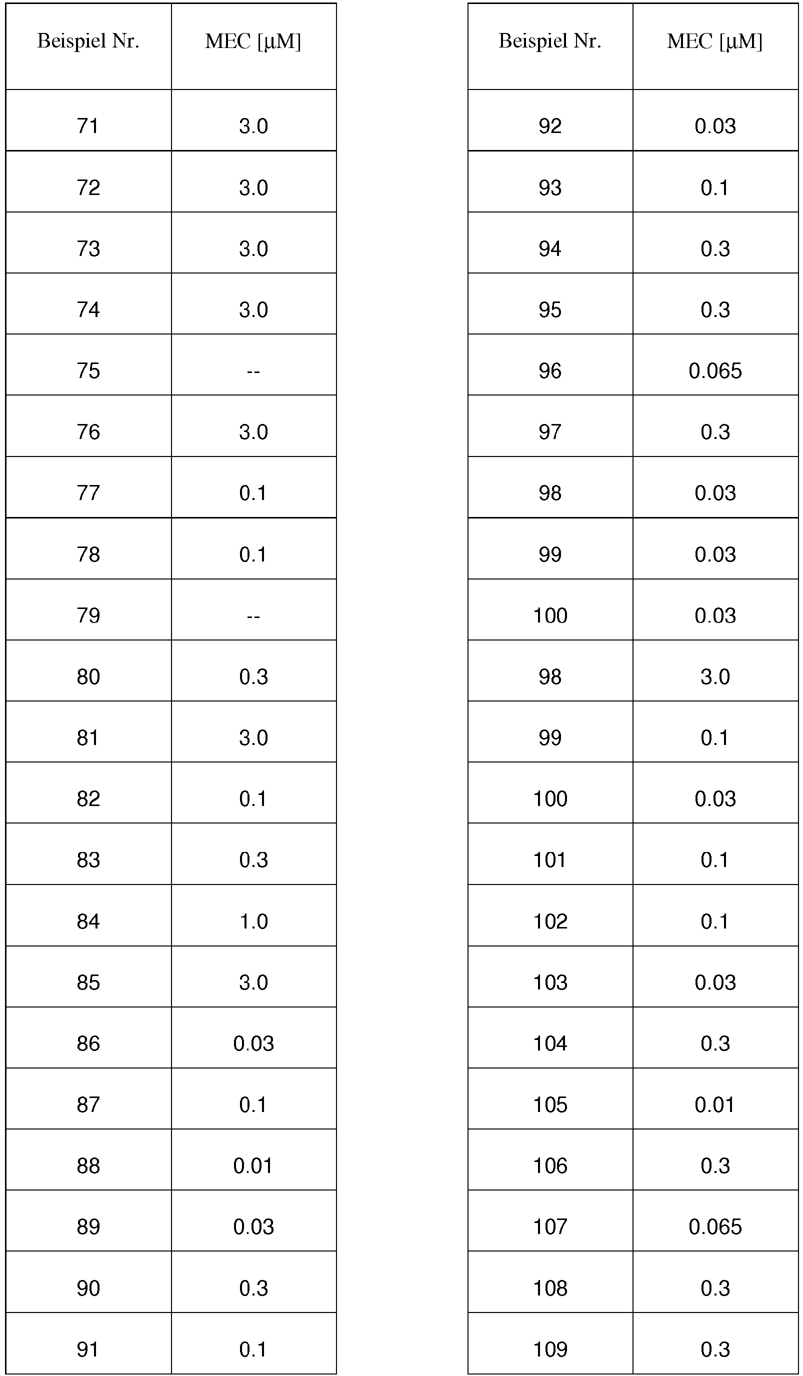

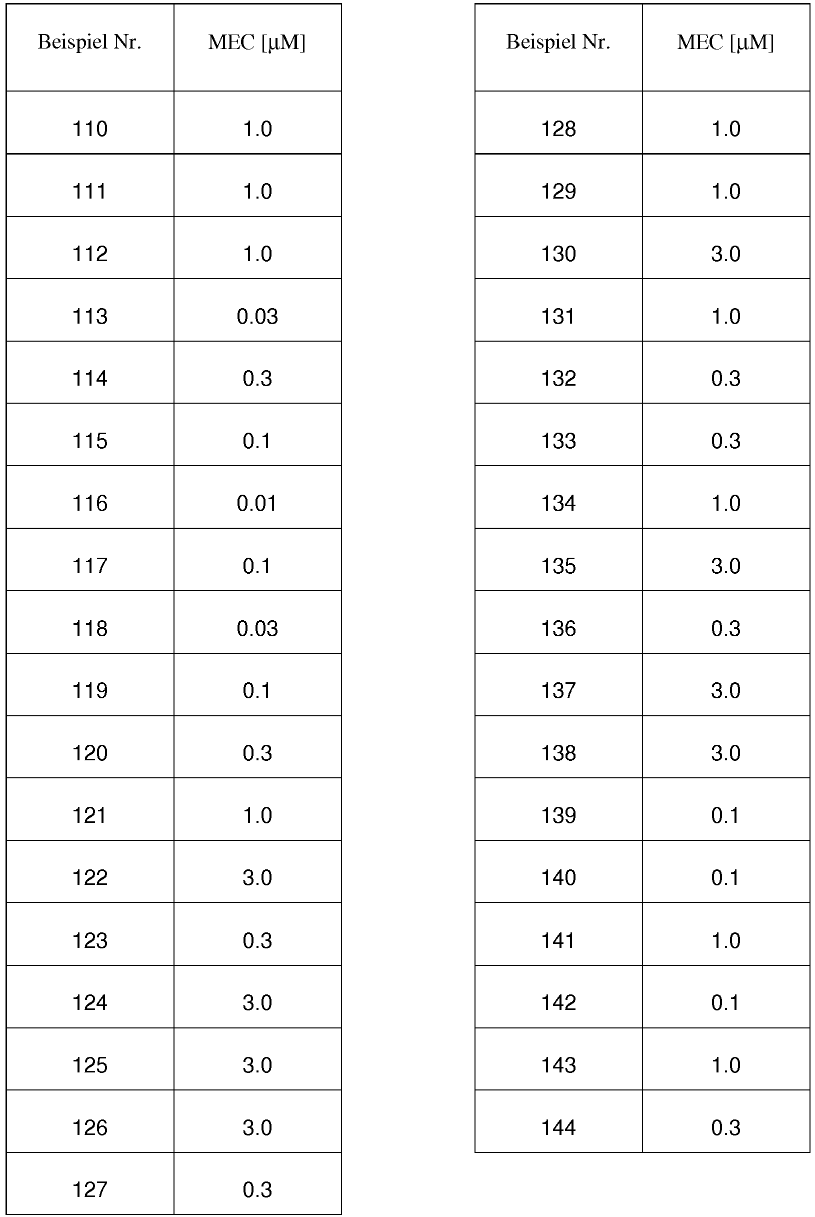

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1 %.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.
Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis exterua* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay.

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 Zell-Linie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I) 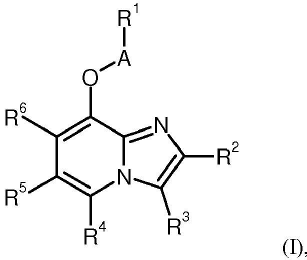 in welcher
A für CH₂, CD₂ oder CH(CH₃) steht,
R¹ für (C₃-C₇)-Cycloalkyl, Phenyl oder Pyridyl steht,
wobei (C₃-C₇)-Cycloalkyl mit 1 bis 4 Substituenten unabhängig voneinander ausgewählt aus der Gruppe Fluor, Trifluormethyl und (C₁-C₄)-Alkyl substituiert sein kann,
wobei Phenyl mit 1 bis 4 Substituenten unabhängig voneinander ausgewählt aus der Gruppe Halogen, Cyano, Monofluormethyl, Difluormethyl, Trifluormethyl, (C₁-C₄)-Alkyl, (C₁-C₄)-Alkoxy und Difluormethoxy substituiert ist,
und
wobei Pyridyl mit 1 oder 2 Substituenten unabhängig voneinander ausgewählt aus der Gruppe Halogen, Cyano und (C₁-C₄)-Alkyl substituiert ist,
R² für (C₁-C₄)-Alkyl, Cyclopropyl, Monofluormethyl, Difluormethyl oder Trifluormethyl steht,
R³ für Phenyl oder 5- bis 10-gliedriges Heteroaryl steht,
wobei Phenyl mit 1 bis 3 Substituenten unabhängig voneinander ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxycarbonyl, Hydroxycarbonyl, -(C=O)NR⁷R⁸, (C₁-C₄)-Alkylsulfonyl, (C₃-C₆)-Cycloalkylsulfonyl, (C₁-C₄)-Alkylthio, (C₁-C₄)-Alkoxy, Trifluormethoxy, Difluormethoxy, Phenoxy, Hydroxy und (C₃-C₇)-Cycloalkyl substituiert sein kann, worin (C₁-C₆)-Alkyl mit 1 oder 2 Substituenten ausgewählt aus der Gruppe Fluor, Trifluormethoxy, (C₁-C₄)-Alkylcarbonyl, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus (C₁-C₆)-Alkyl, (C₁-C₄)-Alkylcarbonyl, (C₃-C₆)-Cycloalkylsulfonyl, (C₁-C₄)-Alkylsulfonyl und Methoxy-(C₁-C₄)-alkyl substituiert sein kann,
und
worin (C₃-C₆)-Cycloalkyl mit Amino oder Hydroxy substituiert sein kann,
worin
R⁷ und R⁸ jeweils unabhängig voneinander für Wasserstoff, (C₁-C₄)-Alkyl oder (C₃-C₇)-Cycloalkyl stehen,
wobei 5- bis 10-gliedriges Heteroaryl mit 1 bis 3 Substituenten unabhängig voneinander ausgewählt aus der Gruppe Fluor, Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl und (C₃-C₇)-Cycloalkyl substituiert sein kann,
worin (C₁-C₆)-Alkyl mit 1 bis 3 Substituenten ausgewählt aus der Gruppe Halogen, Cyano, Hydroxy, Amino, Trifluormethyl, Difluormethyl, (C₁-C₄)-Alkylsulfonyl, (C₁-C₄)-Alkylcarbonyl, (C₁-C₄)-Alkoxycarbonyl, Hydroxycarbonyl, -(C=O)NR⁷R⁸, -O(C=O)NR⁷R⁸, (C₁-C₄)-Alkylthio, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 bis 3 Substituenten ausgewählt aus der Gruppe Halogen, (C₁-C₄)-Alkyl und (C₁-C₄)-Alkoxy substituiert sein kann,
worin Piperidinyl mit 1 bis 4 Substituenten Fluor substituiert sein kann,
worin Phenyl mit 1 bis 3 Substituenten ausgewählt aus der Gruppe Halogen, (C₁-C₄)-Alkyl und (C₁-C₄)-Alkoxy substituiert sein kann,
worin Azetidin mit Hydroxy substituiert sein kann,
und
worin Piperazinyl mit 1 bis 3 Substituenten unabhängig voneinander ausgewählt aus der Gruppe (C₁-C₄)-Alkyl, (C₃-C₇)-Cycloalkyl und Trifluormethyl substituiert sein kann,
worin (C₃-C₇)-Cycloalkyl mit 1 oder 2 Substituenten unabhängig ausgewählt aus der Gruppe Halogen, (C₁-C₆)-Alkyl, (C₁-C₄)-Alkoxycarbonyl und Hydroxycarbonyl substituiert sein kann,
worin Amino mit (C₁-C₄)-Alkyl substituiert sein kann,
worin Phenyl, Pyridyl, Pyrimidyl und 1,3-Thiazol-5-yl mit 1 oder 2 Substituenten ausgewählt aus der Gruppe Methyl, Ethyl und Fluor substituiert sein können,
worin
R⁷ und R⁸ jeweils unabhängig voneinander für Wasserstoff, (C₁-C₄)-Alkyl oder (C₃-C₇)-Cycloalkyl stehen,
und
mit der Maßgabe, dass wenn 5- bis 10-gliedriges Heteroaryl für Pyridyl steht, Pyridyl nicht mit Amino substituiert sein kann,
R⁴ für Wasserstoff steht,
R⁵ für Wasserstoff, Halogen, Cyano, (C₁-C₄)-Alkyl, (C₁-C₄)-Alkoxy, (C₃-C₅)-Cycloalkyl, Difluormethoxy, Difluormethyl, Trifluormethyl, 4- bis 7-gliedriges Heterocyclyl oder 5- oder 6-gliedriges Heteroaryl steht,
R⁶ für Wasserstoff oder Halogen steht,
sowie ihre N-Oxide, Salze, Solvate, Salze der N-Oxide und Solvate der N-Oxide und Salze.

2. Verbindung der Formel (I) nach Anspruch 1, in welcher
A für CH₂ oder CD₂ steht,
R¹ für Cyclohexyl, Phenyl oder Pyridyl steht,
wobei Phenyl mit 1 bis 4 Substituenten unabhängig voneinander ausgewählt aus der Gruppe Fluor, Brom, Chlor, Cyano und Methyl substituiert ist,
und
wobei Pyridyl mit 1 oder 2 Substituenten unabhängig voneinander ausgewählt aus der Gruppe Fluor, Cyano und Methyl substituiert ist,
R² für (C₁-C₄)-Alkyl, Cyclopropyl oder Trifluormethyl steht,
R³ für Phenyl steht,
wobei Phenyl mit 1 bis 3 Substituenten ausgewählt aus der Gruppe Halogen, Cyano, Trifluormethyl, Difluormethyl, (C₁-C₆)-Alkyl, (C₁-C₄)-Alkylcarbonyl, (C₁-C₄)-Alkoxycarbonyl, Hydroxycarbonyl, -(C=O)NR⁷R⁸, (C₁-C₄)-Alkylsulfonyl, (C₁-C₄)-Alkylthio, (C₁-C₄)-Alkoxy, Trifluormethoxy, Difluormethoxy, Phenoxy, Hydroxy und (C₃-C₇)-Cycloalkyl substituiert sein kann,
worin (C₁-C₆)-Alkyl mit 1 oder 2 Substituenten ausgewählt aus der Gruppe Trifluormethoxy, (C₁-C₄)-Alkylcarbonyl, -(C=O)NR⁷R⁸, (C₁-C₄)-Alkoxy, (C₃-C₆)-Cycloalkyl, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus (C₁₋C₆)-Alkyl, (C₁-C₄)-Alkylcarbonyl, (C₁-C₄)-Alkylsulfonyl und Methoxy-(C₁-C₄)-alkyl substituiert sein kann,
und
worin (C₃-C₆)-Cycloalkyl mit Amino oder Hydroxy substituiert sein kann,
und worin
R⁷ und R⁸ jeweils unabhängig voneinander für Wasserstoff, (C₁-C₄)-Alkyl oder (C₃-C₅)-Cycloalkyl stehen
oder für 5-gliedriges Heteroaryl steht,
wobei 5-gliedriges Heteroaryl mit 1 oder 2 Substituenten unabhängig voneinander ausgewählt aus der Gruppe Chlor, Cyano, (C₁-C₆)-Alkyl, (C₁-C₄)-Alkoxy, Amino, (C₁-C₄)-Alkoxycarbonyl, Hydroxycarbonyl, -(C=O)NR⁷R⁸, Phenyl, Pyridyl, Pyrimidyl, 1,3-Thiazol-5-yl oder (C₃-C₆)-Cycloalkyl substituiert ist,
worin (C₁-C₆)-Alkyl mit 1 bis 3 Substituenten ausgewählt aus der Gruppe Fluor, Cyano, Trifluormethyl, Difluormethyl, (C₁-C₄)-Alkylcarbonyl, (C₁-C₄)-Alkoxycarbonyl, Hydroxycarbonyl, -(C=O)NR⁷R⁸, -O(C=O)NR⁷R⁸, (C₁-C₄)-Alkylsulfonyl, (C₁-C₄)-Alkoxy, Trifluormethoxy, Difluormethoxy, Phenoxy, Phenyl, Pyridyl, Pyrimidyl, 5-gliedriges Heteroaryl, Tetrahydrothiophenyl-1,1-dioxid, Hydroxy, Amino, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 5-gliedriges Heteroaryl mit 1 oder 2 Substituenten Methyl oder Ethyl substituiert sein kann,
worin Piperidinyl mit 1 bis 4 Substituenten Fluor substituiert sein kann,
worin Phenyl mit 1 oder 2 Substituenten ausgewählt aus der Gruppe Fluor, Chlor und Methyl substituiert sein kann,
worin Azetidin mit Hydroxy substituiert sein kann,
worin
R⁷ und R⁸ jeweils unabhängig voneinander für Wasserstoff, (C₁-C₄)-Alkyl oder (C₃-C₇)-Cycloalkyl stehen,
und
worin Piperazinyl mit 1 oder 2 Substituenten Methyl oder Ethyl substituiert sein kann,
worin Phenyl, Pyridyl, Pyrimidyl und 1,3-Thiazol-5-yl mit 1 oder 2 Substituenten ausgewählt aus der Gruppe Methyl, Ethyl und Fluor substituiert sein können,
worin (C₃-C₆)-Cycloalkyl mit 1 oder 2 Substituenten unabhängig ausgewählt aus der Gruppe Fluor, Methyl, Ethyl, (C₁-C₄)-Alkoxycarbonyl und Hydroxycarbonyl substituiert sein kann,
und worin
R⁷ und R⁸ jeweils unabhängig voneinander für Wasserstoff, (C₁-C₄)-Alkyl oder (C₃-C₇)-Cycloalkyl stehen,
R⁴ für Wasserstoff steht,
R⁵ für Wasserstoff, Halogen, Cyano, (C₁-C₄)-Alkyl, Methoxy, Ethoxy, (C₃-C₅)-Cycloalkyl oder Difluormethyl steht,
R⁶ für Wasserstoff oder Fluor steht,
sowie ihre N-Oxide, Salze, Solvate, Salze der N-Oxide und Solvate der N-Oxide und Salze.

3. Verbindung der Formel (I) nach Anspruch 1 oder 2, in welcher
A für CH₂ steht,
R¹ für Cyclohexyl oder Phenyl steht,
wobei Phenyl mit 1 bis 3 Substituenten Fluor substituiert ist,
oder
für eine Pyridyl-Gruppe der Formel 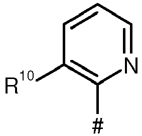
steht, wobei
# für die Anknüpfstelle an A steht,
und
R¹⁰ für Fluor steht,
R² für Methyl oder Ethyl steht,
R³ für Phenyl steht,
wobei Phenyl mit 1 oder 2 Substituenten ausgewählt aus der Gruppe Fluor, Brom, Chlor, Cyano, Trifluormethyl, Difluormethyl, Methyl, Ethyl, -(C=O)NR⁷R⁸, Amino, Hydroxycarbonyl, Methylsulfonyl, Ethylsulfonyl, Methoxy, Ethoxy, Trifluormethoxy, Difluormethoxy und Cyclobutyl substituiert sein kann,
worin Methyl und Ethyl mit 1 oder 2 Substituenten ausgewählt aus der Gruppe Trifluormethoxy, -(C=O)NR⁷R⁸, Methoxy, Ethoxy, Morpholinyl, Piperidinyl, Pyrrolidinyl, Piperazinyl, Hydroxy und Amino substituiert sein können,
worin Amino mit 1 oder 2 Substituenten unabhängig voneinander ausgewählt aus der Gruppe (C₁-C₄)-Alkyl, Methylcarbonyl, Ethylcarbonyl, Methylsulfonyl, Ethylsulfonyl und Methoxyethyl substituiert sein kann,
worin Cyclobutyl mit Amino oder Hydroxy substituiert sein kann,
worin Amino mit 1 oder 2 Substituenten unabhängig voneinander ausgewählt aus Methyl, Ethyl, Methylcarbonyl, Ethylcarbonyl, Methylsulfonyl oder Ethylsulfonyl substituiert sein kann,
und worin
R⁷ und R⁸ unabhängig voneinander für Wasserstoff, Methyl, Ethyl oder Cyclopropyl stehen,
oder
für eine Gruppe der Formel 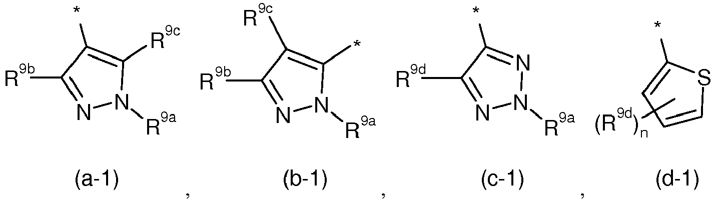 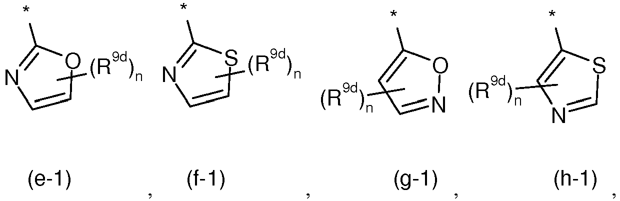 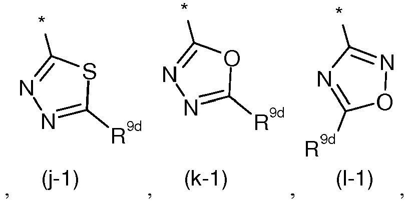 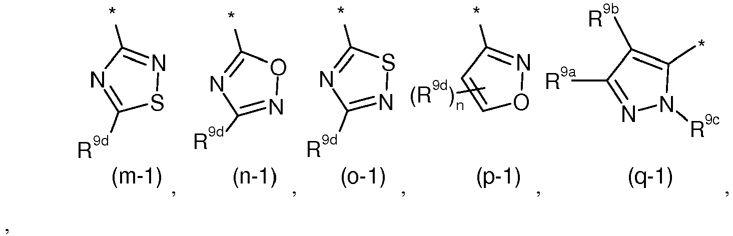 steht, wobei
* für die Anknüpfungsstelle an das Imidazopyridin steht,
n für eine Zahl 1 oder 2 steht,
R^{9a} für (C₁-C₆)-Alkyl, Phenyl, Pyridyl oder Cyclopropyl steht,
wobei (C₁-C₆)-Alkyl mit 1 oder 2 Substituenten ausgewählt aus der Gruppe Fluor, Cyano, Trifluormethyl, Difluormethyl, Methylcarbonyl, Methoxycarbonyl, Ethoxycarbonyl, Hydroxycarbonyl, -(C=O)NR⁷R⁸, O(C=O)NR⁷R⁸, Methylsulfonyl, Ethylsulfonyl, Methoxy, Phenyl, Pyridyl, 1H-Pyrazolyl, 1H-Tetrazolyl, 1,2-Oxazolyl, Hydroxy, Amino, (C₃-C₅)-Cycloalkyl, Morpholinyl, Piperidinyl, Pyrrolidinyl, Piperazinyl, Thiomorpholinyl-1,1-dioxid und Azetidin substituiert sein kann,
worin 1H-Pyrazolyl, 1H-Tetrazolyl und 1,2-Oxazolyl mit 1 oder 2 Substituenten Methyl oder Ethyl substituiert sein können,
worin Piperidinyl mit 1 bis 2 Substituenten Fluor substituiert sein kann,
worin Azetidin mit Hydroxy substituiert sein kann,
und
worin Piperazinyl mit Methyl substituiert sein kann,
wobei Cyclopropyl mit 1 oder 2 Substituenten unabhängig ausgewählt aus der Gruppe Methyl, Ethyl, Methoxycarbonyl, Ethoxycarbonyl und Hydroxycarbonyl substituiert sein kann,
wobei Phenyl und Pyridyl mit 1 oder 2 Substituenten Fluor substituiert sein können,
und worin
R⁷ und R⁸ unabhängig voneinander für Wasserstoff, Methyl, Ethyl oder Cyclopropyl stehen,
R^{9b} für Wasserstoff oder (C₁-C₄)-Alkyl steht,
R^{9c} für Wasserstoff oder (C₁-C₄)-Alkyl steht,
R^{9d} für Wasserstoff, (C₁-C₆)-Alkyl, Trifluormethyl, Methoxy, Ethoxy, Amino, Methoxycarbonyl, Ethoxycarbonyl, Hydroxycarbonyl, -(C=O)NR⁷R⁸, Phenyl, Pyridyl, Pyrimidyl, 1,3-Thiazolyl, Tetrahydrothiophenyl-1,1-dioxid oder Cyclopropyl steht,
wobei (C₁-C₆)-Alkyl mit 1 oder 2 Substituenten ausgewählt aus der Gruppe Trifluormethyl, Difluormethyl, (C₁-C₄)-Alkoxy, 2-Oxopyrrolidin-1-yl, Phenyl, Pyridyl, Pyrimidyl, 1H-1,2,4-Triazolyl, Hydroxy und Amino substituiert sein kann,
worin 1H-1,2,4-Triazolyl mit 1 oder 2 Substituenten Methyl oder Ethyl substituiert sein kann,
und
worin Amino mit (C₁-C₄)-Alkyl substituiert sein kann,
wobei Amino mit (C₁-C₄)-Alkyl substituiert sein kann, wobei Phenyl, Pyridyl, Pyrimidyl und 1,3-Thiazolyl jeweils mit 1 oder 2 Substituenten Methyl oder Ethyl substituiert sein können,
und wobei
R⁷ und R⁸ jeweils unabhängig voneinander für Wasserstoff, Methyl, Ethyl oder Cyclopropyl stehen,
R⁴ für Wasserstoff steht,
R⁵ für Wasserstoff, Chlor, Fluor, Methyl, Ethyl, Difluormethyl oder Cyclopropyl steht,
R⁶ für Wasserstoff steht,
sowie ihre N-Oxide, Salze, Solvate, Salze der N-Oxide und Solvate der N-Oxide und Salze.

4. Verbindung der Formel (I) nach Anspruch 1, 2 oder 3 in welcher
A für CH₂ steht,
R¹ für Cyclohexyl steht,
oder
für eine Phenyl-Gruppe der Formel 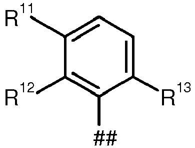
steht, wobei
## für die Anknüpfstelle an A steht,
und
R¹¹, R¹² und R¹³ unabhängig voneinander für Wasserstoff oder Fluor stehen,
mit der Maßgabe, dass mindestens zwei der Reste R¹¹, R¹², R¹³ von Wasserstoff verschieden sind,
oder
für eine Pyridyl-Gruppe der Formel 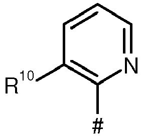 steht, wobei
# für die Anknüpfstelle an A steht,
und
R¹⁰ für Fluor steht,
R² für Methyl oder Ethyl steht,
R³ für Phenyl steht,
wobei Phenyl mit 1 oder 2 Substituenten ausgewählt aus der Gruppe Fluor, Chlor, Cyano, Amino, Trifluormethyl, Difluormethyl, Methyl, -(C=O)NR⁷R⁸, Methoxy, Piperidinyl und Cyclobutyl substituiert sein kann,
worin Methyl mit 1 oder 2 Substituenten ausgewählt aus der Gruppe-(C=O)NR⁷R⁸, Methoxy, Morpholinyl, Piperidinyl, Pyrrolidinyl, Piperazinyl, Hydroxy und Amino substituiert sein kann,
worin Amino mit 1 oder 2 Substituenten unabhängig voneinander ausgewählt aus Methyl, Ethyl und Methoxyethyl substituiert sein kann,
worin Amino mit 1 oder 2 Substituenten unabhängig voneinander ausgewählt aus Methyl, Ethyl, Methylcarbonyl, Ethylcarbonyl, Methylsulfonyl oder Ethylsulfonyl substituiert sein kann,
worin Cyclobutyl mit Amino substituiert ist,
und worin
R⁷ und R⁸ jeweils unabhängig voneinander für Wasserstoff, Methyl, Ethyl oder Cyclopropyl stehen,
oder für eine Gruppe der Formel 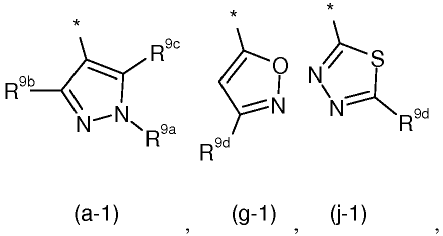 steht, wobei
* für die Anknüpfungsstelle an das Imidazopyridin steht,
R^{9a} für (C₁-C₆)-Alkyl, Phenyl, Pyridyl oder Cyclopropyl steht,
wobei (C₁-C₆)-Alkyl mit Fluor, Cyano, Trifluormethyl, Difluormethyl, Methylcarbonyl, Methoxycarbonyl, Ethoxycarbonyl, Hydroxycarbonyl,-(C=O)NR⁷R⁸, -O(C=O)NR⁷R⁸, Methylsulfonyl, Ethylsulfonyl, Methoxy, Ethoxy, Trifluormethoxy, Difluormethoxy, Phenyl, Pyridyl, 1H-Pyrazolyl, 1H-Tetrazolyl, 1,2-Oxazolyl, Hydroxy, Amino, Cyclopropyl, Cyclobutyl, Morpholinyl, Piperidinyl, Pyrrolidinyl, Piperazinyl, Thiomorpholinyl-1,1-dioxid oder Azetidin substituiert sein kann,
worin 1H-Pyrazolyl, 1H-Tetrazolyl und 1,2-Oxazolyl mit 1 oder 2 Substituenten Methyl oder Ethyl substituiert sein können,
worin Piperidinyl mit 1 bis 2 Substituenten Fluor substituiert sein kann,
worin Azetidin mit Hydroxy substituiert sein kann,
und
worin Piperazinyl mit Methyl substituiert sein kann,
wobei Cyclopropyl mit Methoxycarbonyl, Ethoxycarbonyl oder Hydroxycarbonyl substituiert sein kann,
wobei Phenyl und Pyridyl mit 1 oder 2 Substituenten Fluor substituiert sein können,
und worin
R⁷ und R⁸ unabhängig voneinander für Wasserstoff, Methyl, Ethyl oder Cyclopropyl stehen
R^{9b} für Wasserstoff oder Methyl steht,
R^{9c} für Wasserstoff oder Methyl steht,
R^{9d} für Wasserstoff, (C₁-C₆)-Alkyl, Trifluormethyl, Methoxy, Ethoxy, Amino, Methoxycarbonyl, Ethoxycarbonyl, Hydroxycarbonyl, -(C=O)NR⁷R⁸, Phenyl, Pyridyl, Pyrimidyl, 1,3-Thiazolyl, Tetrahydrothiophenyl-1,1-dioxid oder Cyclopropyl steht,
wobei (C₁-C₆)-Alkyl mit 1 oder 2 Substituenten ausgewählt aus der Gruppe Trifluormethyl, Difluormethyl, (C₁-C₄)-Alkoxy, 2-Oxopyrrolidin-1-yl, Phenyl, Pyridyl, Pyrimidyl, 1H-1,2,4-Triazolyl, Hydroxy und Amino substituiert sein kann,
worin 1H-1,2,4-Triazolyl mit 1 oder 2 Substituenten Methyl oder Ethyl substituiert sein kann,
und
worin Amino mit (C₁-C₄)-Alkyl substituiert sein kann,
wobei Amino mit (C₁-C₄)-Alkyl substituiert sein kann,
wobei Phenyl, Pyridyl, Pyrimidyl und 1,3-Thiazolyl jeweils mit 1 oder 2 Substituenten Methyl oder Ethyl substituiert sein können,
und worin
R⁷ und R⁸ jeweils unabhängig voneinander für Wasserstoff, Methyl, Ethyl oder Cyclopropyl stehen,
R⁴ für Wasserstoff steht,
R⁵ für Wasserstoff, Chlor, Fluor, Methyl, Ethyl, Difluormethyl oder Cyclopropyl steht,
R⁶ für Wasserstoff steht,
sowie ihre N-Oxide, Salze, Solvate, Salze der N-Oxide und Solvate der N-Oxide und Salze.

5. Verfahren zur Herstellung von Verbindungen der Formel (I), wie in den Ansprüchen 1 bis 4 definiert, **dadurch gekennzeichnet, dass** man
[A] eine Verbindung der Formel (II) 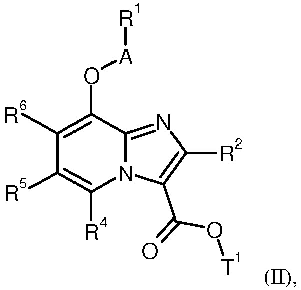 in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 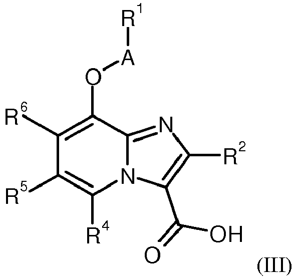 in welcher A, R¹, R², R⁴, R⁵ und R⁶ jeweils die oben angegebenen Bedeutungen haben, umsetzt,
[A] und diese in der Folge in Gegenwart einer geeigneten Säure zu einem Imidazo[1,2-a]-pyridin der Formel (IV) 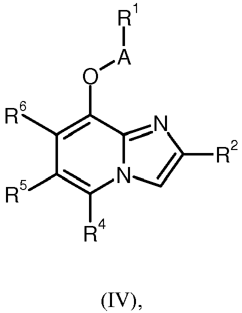 in welcher A, R¹, R², R⁴, R⁵ und R⁶ jeweils die oben angegebenen Bedeutungen haben, umsetzt, und dieses dann mit einem Halogen-Äquivalent in eine Verbindung der Formel (V) 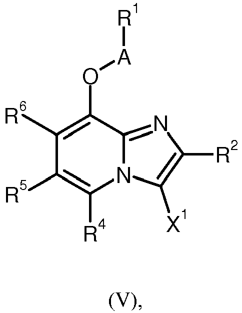 in welcher A, R¹ R², R⁴, R⁵ und R⁶ jeweils die oben angegebenen Bedeutungen haben und
X¹ für Chlor, Brom oder Iod steht,
überführt, und diese im Anschluß in einem inerten Lösungsmittel in Gegenwart eines geeigneten Übergangsmetallkatalysators mit einer Verbindung der Formel (VI), 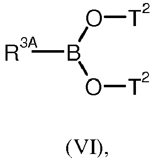 in welcher
R^{3A} die oben für R³ angegebenen Bedeutungen hat und
T² für Wasserstoff oder (C₁-C₄)-Alkyl steht, oder beide Reste T² zusammen eine -C(CH₃)₂-C(CH₃)₂-Brücke bilden,
zu einer Verbindung der Formel (I-A) 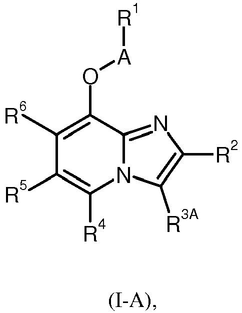 umsetzt und anschließend für den Fall, dass R^{3A} für 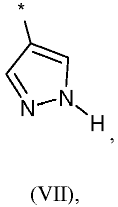 steht, diese Verbindungen in einem inerten Lösungsmittel in Gegenwart einer geeigneten Base mit einer Verbindung der Formel (VIII)
R¹⁴-X¹ (VIII),
in welcher
X¹ für eine geeignete Abgangsgruppe, insbesondere Chlor, Brom, Iod, Mesylat, Triflat oder Tosylat steht,
und
R¹⁴ für (C₁-C₆)-Alkyl steht,
wobei (C₁-C₆)-Alkyl mit 1 bis 3 Substituenten ausgewählt aus der Gruppe Fluor, Cyano, Trifluormethyl, Difluormethyl, (C₁-C₄)-Alkylcarbonyl, (C₁-C₄)-Alkoxycarbonyl, Hydroxycarbonyl, -(C=O)NR⁷R⁸, -O(C=O)NR⁷R⁸, (C₁-C₄)-Alkylsulfonyl, (C₁-C₄)-Alkoxy, Trifluormethoxy, Difluormethoxy, Phenyl, 1H-Pyrazolyl, 1H-1,2,4-Triazolyl, 1H-Tetrazolyl, 1,2-Oxazolyl, Tetrahydrothiophenyl-1,1-dioxid, Hydroxy, Amino, (C₃-C₇)-Cycloalkyl, Morpholinyl, Piperidinyl, Pyrrolidinyl, 2-Oxopyrrolidin-1-yl, Piperazinyl, Tetrahydrothiophenyl-1,1-dioxid, Thiomorpholinyl-1,1-dioxid und Azetidin substituiert sein kann,
worin 1H-Pyrazolyl, 1H-1,2,4-Triazolyl, 1H-Tetrazolyl und 1,2-Oxazolyl mit 1 oder 2 Substituenten Methyl oder Ethyl substituiert sein können,
worin Piperidinyl mit 1 oder 2 Substituenten Fluor substituiert sein kann,
worin Phenyl mit 1 oder 2 Substituenten Fluor substituiert sein kann,
worin Piperazinyl mit Methyl substituiert sein kann,
und worin
R⁷ und R⁸ jeweils unabhängig voneinander für Wasserstoff, Methyl oder Cyclopropyl stehen,
oder
R⁷ und R⁸ zusammen mit dem Kohlenstoffatom, an das sie gebunden sind, einen 3- bis 5-gliedrigen Carbocyclus bilden,
zu einer Verbindung der Formel (I-B) 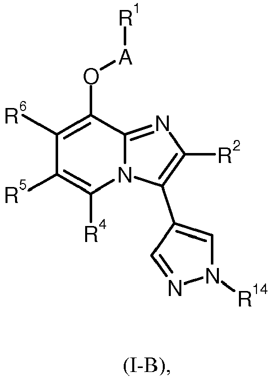 umsetzt,
in welcher A, R¹, R², R⁴, R⁵, R⁶ und R¹⁴ jeweils die oben angegebenen Bedeutungen haben und
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,
oder
[B] eine Verbindung der Formel (II) in Gegenwart von Hydrazinhydrat in eine Verbindung der Formel (IX) 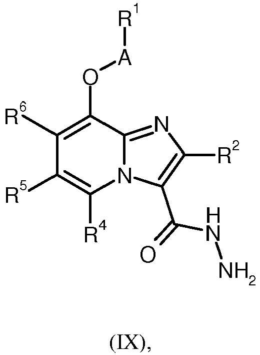 in welcher A, R¹ R², R⁴, R⁵ und R⁶ jeweils die oben angegebenen Bedeutungen haben, überführt, und diese in der Folge in einem inerten Lösungsmittel unter Amidkupplungsbedingungen mit einer Carbonsäure der Formel (X) 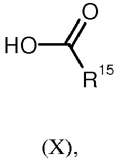 in welchem R¹⁵ für (C₁-C₆)-Alkyl steht,
wobei (C₁-C₆)-Alkyl mit 1 bis 3 Substituenten ausgewählt aus der Gruppe Trifluormethyl, Difluormethyl, Hydroxy und Amino substituiert sein kann,
zu einer Verbindung der Formel (XI) umsetzt, 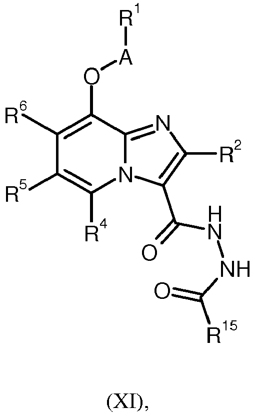 in welcher A, R¹, R², R⁴, R⁵, R⁶ und R¹⁵ jeweils die oben angegebenen Bedeutungen haben,
und diese Verbindung anschließend mit 2,4-Bis(4-methoxyphenyl)-1,3,2,4-dithiadiphosphetan-2,4-disulfid [Lawesson-Reagenz] in eine Verbindung der Formel (I-C) 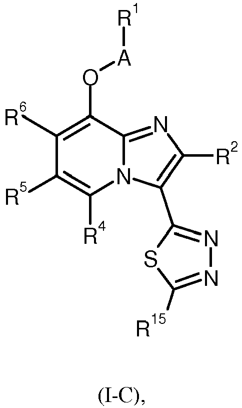 überführt,
in welcher A, R¹, R², R⁴, R⁵, R⁶ und R¹⁵ jeweils die oben angegebenen Bedeutungen haben,
anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

6. Verbindung der Formel (I), wie in einem der Ansprüche 1 bis 4 definiert, zur Verwendung in der Behandlung
und/oder Prophylaxe von Krankheiten.

7. Verwendung einer Verbindung der Formel (I), wie in einem der Ansprüche 1 bis 4 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

8. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 4 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

## Claims

1. Compound of the formula (I) 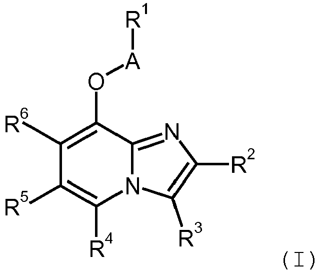 in which
A represents CH₂, CD₂ or CH(CH₃),
R¹ represents (C₃-C₇)-cycloalkyl, phenyl or pyridyl,
where (C₃-C₇)-cycloalkyl may be substituted by 1 to 4 substituents each independently selected from fluorine, trifluoromethyl and (C₁-C₄)-alkyl,
where phenyl may be substituted by 1 to 4 substituents independently selected from the group consisting of halogen, cyano, monofluoromethyl, difluoromethyl, trifluoromethyl, (C₁-C₄)-alkyl, (C₁-C₄)-alkoxy and difluoromethoxy
and
where pyridyl is substituted by 1 or 2 substituents each selected independently from the group of halogen, cyano and (C₁-C₄)-alkyl,
R² represents (C₁-C₄)-alkyl, cyclopropyl, monofluoromethyl, difluoromethyl or trifluoromethyl,
R³ represents phenyl or 5- to 10-membered heteroaryl,
where phenyl may be substituted by 1 to 3 substituents independently of one another selected from the group consisting of halogen, cyano, trifluoromethyl, difluoromethyl, (C₁-C₆)-alkyl, (C₁-C₄)-alkylcarbonyl, (C₁-C₄)-alkoxycarbonyl, hydroxycarbonyl, -(C=O)NR⁷R⁸, (C₁-C₄)-alkylsulphonyl, (C₃-C₆)-cycloalkylsulphonyl, (C₁-C₄) -alkylthio, (C₁-C₄)-alkoxy, trifluoromethoxy, difluoromethoxy, phenoxy, hydroxy and (C₃-C₇)-cycloalkyl,
in which (C₁-C₆)-alkyl may be substituted by 1 or 2 substituents selected from the group consisting of fluorine, trifluoromethoxy, (C₁-C₄)-alkylcarbonyl, -(C=O)NR⁷R⁸, (C₁-C₄)-alkoxy, (C₃-C₆)-cycloalkyl, morpholinyl, piperidinyl, pyrrolidinyl, piperazinyl, hydroxy and amino,
in which amino may be substituted by 1 or 2 substituents each independently selected from (C₁-C₆)-alkyl, (C₁-C₄)-alkylcarbonyl, (C₃-C₆)-cycloalkylsulphonyl, (C₁-C₄)-alkylsulphonyl and methoxy-(C₁-C₄)-alkyl,
and
in which (C₃-C₆)-cycloalkyl may be substituted by amino or hydroxyl,
in which
R⁷ and R⁸ each independently of one another represent hydrogen, (C₁-C₄)-alkyl or (C₃-C₇)-cycloalkyl,
where 5- to 10-membered heteroaryl may be substituted by 1 to 3 substituents independently of one another selected from the group consisting of fluorine, chlorine, cyano, (C₁-C₆)-alkyl, (C₁-C₄)-alkoxy, amino, (C₁-C₄)-alkoxycarbonyl, hydroxycarbonyl,-(C=O)NR⁷R⁸, phenyl, pyridyl, pyrimidyl, 1,3-thiazol-5-yl and (C₃-C₇)-cycloalkyl,
in which (C₁-C₆)-alkyl may be substituted by 1 to 3 substituents selected from the group consisting of halogen, cyano, hydroxy, amino, trifluoromethyl, difluoromethyl, (C₁-C₄)-alkylsulphonyl, (C₁-C₄)-alkylcarbonyl, (C₁-C₄)-alkoxycarbonyl, hydroxycarbonyl,-(C=O) NR⁷R⁸, -O(C=O)NR⁷R⁸, (C₁-C₄)-alkylthio, (C₁-C₄)-alkoxy, trifluoromethoxy, difluoromethoxy, phenoxy, phenyl, pyridyl, pyrimidyl, 5-membered heteroaryl, tetrahydrothiophenyl 1,1-dioxide, (C₃-C₇)-cycloalkyl, morpholinyl, piperidinyl, pyrrolidinyl, 2-oxopyrrolidin-1-yl, piperazinyl, tetrahydrothiophenyl 1,1-dioxide, thiomorpholinyl 1,1-dioxide and azetidine,
in which 5-membered heteroaryl may be substituted by 1 to 3 substituents selected from the group consisting of halogen, (C₁-C₄)-alkyl and (C₁-C₄)-alkoxy,
in which piperidinyl may be substituted by 1 to 4 fluorine substituents,
in which phenyl may be substituted by 1 to 3 substituents selected from the group consisting of halogen, (C₁-C₄)-alkyl and (C₁-C₄)-alkoxy,
in which azetidine may be substituted by hydroxyl,
and
in which piperazinyl may be substituted by 1 to 3 substituents each independently selected from the group of (C₁-C₄)-alkyl, (C₃-C₇)-cycloalkyl and trifluoromethyl,
in which (C₃-C₇)-alkyl may be substituted by 1 to 2 substituents independently of one another selected from the group consisting of halogen, (C₁-C₆)-alkyl, (C₁-C₄)-alkoxycarbonyl and hydroxycarbonyl,
in which amino may be substituted by (C₁-C₄)-alkyl,
in which phenyl, pyridyl, pyrimidyl and 1,3-thiazol-5-yl may be substituted by 1 or 2 substituents selected from the group consisting of methyl, ethyl and fluorine,
in which
R⁷ and R⁸ each independently of one another represent hydrogen, (C₁-C₄)-alkyl or (C₃-C₇)-cycloalkyl,
and
with the proviso that, if 5- to 10-membered heteroaryl represents pyridyl, pyridyl may not be substituted by amino,
R⁴ represents hydrogen,
R⁵ represents hydrogen, halogen, cyano, (C₁-C₄)-alkyl, (C₁-C₄)-alkoxy, (C₃-C₅)-cycloalkyl, difluoromethoxy, difluoromethyl, trifluoromethyl, 4- to 7-membered heterocyclyl or 5- or 6-membered heteroaryl,
R⁶ represents hydrogen or halogen,
and the *N*-oxides, salts, solvates, salts of the *N-*oxides and solvates of the *N*-oxides or salts thereof.

2. Compound of the formula (I) according to Claim 1 in which
A represents CH₂ or CD₂,
R¹ represents cyclohexyl, phenyl or pyridyl,
where phenyl may be substituted by 1 to 4 substituents independently of one another selected from the group consisting of fluorine, bromine, chlorine, cyano and methyl,
and
where pyridyl may be substituted by 1 to 2 substituents independently of one another selected from the group consisting of fluorine, cyano and methyl,
R² represents (C₁-C₄)-alkyl, cyclopropyl or trifluoromethyl,
R³ represents phenyl,
where phenyl may be substituted by 1 to 3 substituents selected from the group consisting of halogen, cyano, trifluoromethyl, difluoromethyl, (C₁-C₆)-alkyl, (C₁-C₄)-alkylcarbonyl, (C₁-C₄)-alkoxycarbonyl, hydroxycarbonyl, -(C=O)NR⁷R⁸, (C₁-C₄)-alkylsulphonyl, (C₁-C₄)-alkylthio, (C₁-C₄)-alkoxy, trifluoromethoxy, difluoromethoxy, phenoxy, hydroxy and (C₃-C₇)-cycloalkyl,
in which (C₁-C₆)-alkyl may be substituted by 1 or 2 substituents selected from the group consisting of trifluoromethoxy, (C₁-C₄) -alkylcarbonyl, - (C=O) NR⁷R⁸, (C₁-C₄)-alkoxy, (C₃-C₆)-cycloalkyl, morpholinyl, piperidinyl, pyrrolidinyl, piperazinyl, hydroxy and amino,
where amino may be substituted by 1 or 2 substituents independently of one another selected from (C₁-C₆)-alkyl, (C₁-C₄)-alkylcarbonyl, (C₁-C₄)-alkylsulphonyl and methoxy-(C₁-C₄)-alkyl,
and
in which (C₃-C₆)-cycloalkyl may be substituted by amino or hydroxyl,
and in which
R⁷ and R⁸ each independently of one another represent hydrogen, (C₁-C₄)-alkyl or (C₃-C₅)-cycloalkyl,
or
represents 5-membered heteroaryl,
where 5-membered heteroaryl is substituted by 1 or 2 substituents independently of one another selected from the group consisting of chlorine, cyano, (C₁-C₆)-alkyl, (C₁-C₄)-alkoxy, amino, (C₁-C₄)-alkoxycarbonyl, hydroxycarbonyl, -(C=O)NR⁷R⁸, phenyl, pyridyl, pyrimidyl, 1,3-thiazol-5-yl and (C₃-C₆)-cycloalkyl,
where (C₁-C₆)-alkyl may be substituted by 1 to 3 substituents selected from the group consisting of fluorine, cyano, trifluoromethyl, difluoromethyl, (C₁-C₄)-alkylcarbonyl, (C₁-C₄)-alkoxycarbonyl, hydroxycarbonyl, -(C=O)NR⁷R⁸,-O(C=O)NR⁷R⁸, (C₁-C₄)-alkylsulphonyl, (C₁-C₄)-alkoxy, trifluoromethoxy, difluoromethoxy, phenoxy, phenyl, pyridyl, pyrimidyl, 5-membered heteroaryl, tetrahydrothiophenyl 1,1-dioxide, hydroxy, amino, (C₃-C₇)-cycloalkyl, morpholinyl, piperidinyl, pyrrolidinyl, 2-oxopyrrolidin-1-yl, piperazinyl, tetrahydrothiophenyl 1,1-dioxide, thiomorpholinyl 1,1-dioxide and azetidine,
in which 5-membered heteroaryl may be substituted by 1 or 2 methyl or ethyl substituents,
in which piperidinyl may be substituted by 1 to 4 fluorine substituents,
in which phenyl may be substituted by 1 or 2 substituents selected from the group consisting of fluorine, chlorine and methyl,
in which azetidine may be substituted by hydroxyl,
in which
R⁷ and R⁸ each independently of one another represent hydrogen, (C₁-C₄)-alkyl or (C₃-C₇)-cycloalkyl,
and
in which piperazinyl may be substituted by 1 or 2 methyl or ethyl substituents,
in which phenyl, pyridyl, pyrimidyl and 1,3-thiazol-5-yl may be substituted by 1 or 2 substituents selected from the group consisting of methyl, ethyl and fluorine,
in which (C₃-C₆)-cycloalkyl may be substituted by 1 or 2 substituents independently of one another selected from the group consisting of fluorine, methyl, ethyl, (C₁-C₄)-alkoxycarbonyl and hydroxycarbonyl,
and in which
R⁷ and R⁸ each independently of one another represent hydrogen, (C₁-C₄)-alkyl or (C₃-C₇)-cycloalkyl,
R⁴ represents hydrogen,
R⁵ represents hydrogen, halogen, cyano, (C₁-C₄)-alkyl, methoxy, ethoxy, (C₃-C₅)-cycloalkyl or difluoromethyl,
R⁶ represents hydrogen or fluorine,
and the *N*-oxides, salts, solvates, salts of the *N-*oxides and solvates of the *N*-oxides or salts thereof.

3. Compound of the formula (I) according to Claim 1 or 2 in which
A represents CH₂,
R¹ represents cyclohexyl or phenyl,
where phenyl is substituted by 1 to 3 fluorine substituents,
or
represents a pyridyl group of the formula 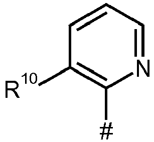 where
# is the attachment site to A,
and
R¹⁰ represents fluorine,
R² represents methyl or ethyl,
R³ represents phenyl,
where phenyl may be substituted by 1 or 2 substituents selected from the group consisting of fluorine, bromine, chlorine, cyano, trifluoromethyl, difluoromethyl, methyl, ethyl, -(C=O)NR⁷R⁸, amino, hydroxycarbonyl, methylsulphonyl, ethylsulphonyl, methoxy, ethoxy, trifluoromethoxy, difluoromethoxy and cyclobutyl,
in which methyl and ethyl may be substituted by 1 or 2 substituents selected from the group consisting of trifluoromethoxy, -(C=O)NR⁷R⁸, methoxy, ethoxy, morpholinyl, piperidinyl, pyrrolidinyl, piperazinyl, hydroxy and amino,
in which amino may be substituted by 1 or 2 substituents independently of one another selected from the group consisting of (C₁-C₄)-alkyl, methylcarbonyl, ethylcarbonyl, methylsulphonyl, ethylsulphonyl and methoxyethyl,
in which cyclobutyl may be substituted by amino or hydroxy,
in which amino may be substituted by 1 or 2 substituents independently of one another selected from methyl, ethyl, methylcarbonyl, ethylcarbonyl, methylsulphonyl or ethylsulphonyl, and in which
R⁷ and R⁸ independently of one another represent hydrogen, methyl, ethyl or cyclopropyl,
or
represent a group of the formula 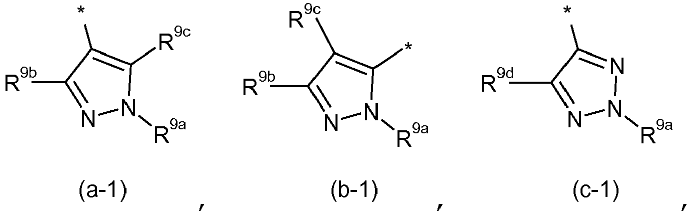 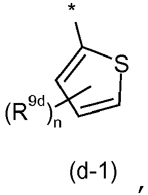 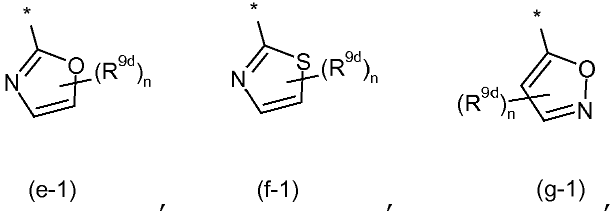 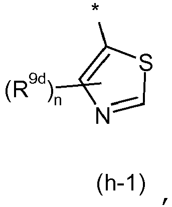 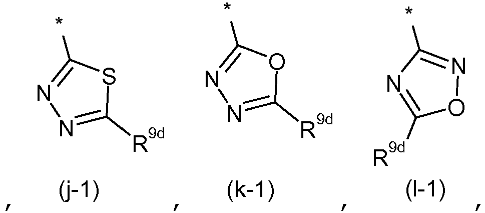 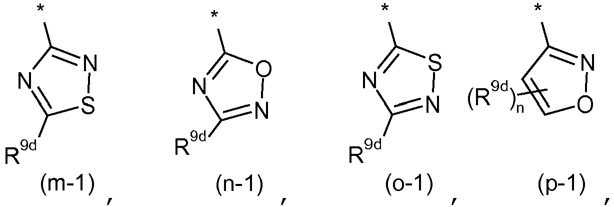 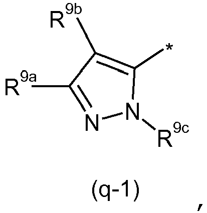 where
* represents the point of attachment to the imidazopyridine,
n represents a number 1 or 2,
R^{9a} represents (C₁-C₆)-alkyl, phenyl, pyridyl or cyclopropyl,
where (C₁-C₆)-alkyl may be substituted by 1 or 2 substituents selected from the group consisting of fluorine, cyano, trifluoromethyl, difluoromethyl, methylcarbonyl, methoxycarbonyl, ethoxycarbonyl, hydroxycarbonyl,-(C=O)NR⁷R⁸, -O(C=O)NR⁷R⁸, methylsulphonyl, ethylsulphonyl, methoxy, phenyl, pyridyl, 1H-pyrazolyl, 1H-tetrazolyl, 1, 2-oxazolyl, hydroxy, amino, (C₃-C₅)-cycloalkyl, morpholinyl, piperidinyl, pyrrolidinyl, piperazinyl, thiomorpholinyl 1,1-dioxide and azetidine,
in which 1H-pyrazolyl, 1H-tetrazolyl and 1,2-oxazolyl may be substituted by 1 or 2 methyl or ethyl substituents,
in which piperidinyl may be substituted by 1 to 2 fluorine substituents,
in which azetidine may be substituted by hydroxyl,
and
in which piperazinyl may be substituted by methyl,
where cyclopropyl may be substituted by 1 to 2 substituents independently selected from the group consisting of methyl, ethyl, methoxycarbonyl, ethoxycarbonyl and hydroxycarbonyl, where phenyl and pyridyl may be substituted by 1 or 2 fluorine substituents,
and in which
R⁷ and R⁸ independently of one another represent hydrogen, methyl, ethyl or cyclopropyl,
R^{9b} represents hydrogen or (C₁-C₄)-alkyl,
R^{9c} represents hydrogen or (C₁-C₄)-alkyl,
R^{9d} represents hydrogen, (C₁-C₆)-alkyl, trifluoromethyl, methoxy, ethoxy, amino, methoxycarbonyl, ethoxycarbonyl, hydroxycarbonyl, -(C=O)NR⁷R⁸, phenyl, pyridyl, pyrimidyl, 1,3-thiazolyl, tetrahydrothiophenyl 1,1-dioxide or cyclopropyl,
where (C₁-C₆)-alkyl may be substituted by 1 or 2 substituents selected from the group consisting of trifluoromethyl, difluoromethyl, (C₁-C₄)-alkoxy, 2-oxopyrrolidin-1-yl, phenyl, pyridyl, pyrimidyl, 1H-1,2,4-triazolyl, hydroxy and amino,
in which 1H-1,2,4-triazolyl may be substituted by 1 or 2 methyl or ethyl substituents,
and
in which amino may be substituted by (C₁-C₄)-alkyl,
where amino may be substituted by (C₁-C₄)-alkyl,
where phenyl, pyridyl, pyrimidyl and 1,3-thiazolyl may each be substituted by 1 or 2 methyl or ethyl substituents,
and where
R⁷ and R⁸ each independently of one
another represent hydrogen, methyl, ethyl or cyclopropyl,
R⁴ represents hydrogen,
R⁵ represents hydrogen, chlorine, fluorine, methyl, ethyl, difluoromethyl or cyclopropyl,
R⁶ represents hydrogen,
and the *N*-oxides, salts, solvates, salts of the *N-*oxides and solvates of the *N*-oxides or salts thereof.

4. Compound of the formula (I) according to Claim 1, 2 or 3 in which
A represents CH₂,
R¹ represents cyclohexyl,
or
represents a phenyl group of the formula 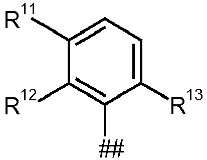 where
## represents the point of attachment to A, and
R¹¹, R¹² and R¹³ independently of one another represent hydrogen or fluorine,
with the proviso that at least two of the radicals R¹¹, R¹², R¹³ are different from hydrogen,
or
represents a pyridyl group of the formula 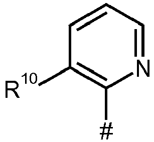 where
# represents the attachment site to A, and
R¹⁰ represents fluorine,
R² represents methyl or ethyl,
R³ represents phenyl,
where phenyl may be substituted by 1 or 2 substituents selected from the group consisting of fluorine, chlorine, cyano, amino, trifluoromethyl, difluoromethyl, methyl, -(C=O)NR⁷R⁸, methoxy, piperidinyl and cyclobutyl,
in which methyl may be substituted by 1 or 2 substituents selected from the group consisting of -(C=O)NR⁷R⁸, methoxy, morpholinyl, piperidinyl, pyrrolidinyl, piperazinyl, hydroxy and amino,
in which amino may be substituted by 1 or 2 substituents independently of one another selected from methyl, ethyl and methoxyethyl,
in which amino may be substituted by 1 or 2 substituents independently of one another selected from methyl, ethyl, methylcarbonyl, ethylcarbonyl, methylsulphonyl or ethylsulphonyl, in which cyclobutyl is substituted by amino,
and in which
R⁷ and R⁸ each independently of one another represent hydrogen, methyl, ethyl or cyclopropyl, or
represent a group of the formula 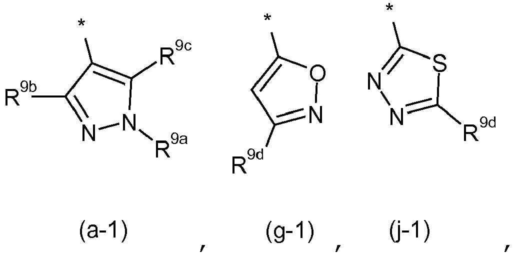 where
* represents the point of attachment to the imidazopyridine,
R^{9a} represents (C₁-C₆)-alkyl, phenyl, pyridyl or cyclopropyl,
where (C₁-C₆)-alkyl may be substituted by fluorine, cyano, trifluoromethyl, difluoromethyl, methylcarbonyl, methoxycarbonyl, ethoxycarbonyl, hydroxycarbonyl, -(C=O)NR⁷R⁸,-O(C=O)NR⁷R⁸, methylsulphonyl, ethylsulphonyl, methoxy, ethoxy, trifluoromethoxy, difluoromethoxy, phenyl, pyridyl, 1H-pyrazolyl, 1H-tetrazolyl, 1,2-oxazolyl, hydroxy, amino, cyclopropyl, cyclobutyl, morpholinyl, piperidinyl, pyrrolidinyl, piperazinyl, thiomorpholinyl 1,1-dioxide or azetidine,
in which 1H-pyrazolyl, 1H-tetrazolyl and 1,2-oxazolyl may be substituted by 1 or 2 methyl or ethyl substituents,
in which piperidinyl may be substituted by 1 to 2 fluorine substituents,
in which azetidine may be substituted by hydroxyl,
and
in which piperazinyl may be substituted by methyl,
where cyclopropyl may be substituted by methoxycarbonyl, ethoxycarbonyl or hydroxycarbonyl,
where phenyl and pyridyl may be substituted by 1 or 2 fluorine substituents,
and in which
R⁷ and R⁸ independently of one another represent hydrogen, methyl, ethyl or cyclopropyl,
R^{9b} represents hydrogen or methyl,
R^{9c} represents hydrogen or methyl,
R^{9d} represents hydrogen, (C₁-C₆)-alkyl, trifluoromethyl, methoxy, ethoxy, amino, methoxycarbonyl, ethoxycarbonyl, hydroxycarbonyl, -(C=O)NR⁷R⁸, phenyl, pyridyl, pyrimidyl, 1,3-thiazolyl, tetrahydrothiophenyl 1,1-dioxide or cyclopropyl,
where (C₁-C₆)-alkyl may be substituted by 1 or 2 substituents selected from the group consisting of trifluoromethyl, difluoromethyl, (C₁-C₄)-alkoxy, 2-oxopyrrolidin-1-yl, phenyl, pyridyl, pyrimidyl, 1H-1,2,4-triazolyl, hydroxy and amino,
in which 1H-1,2,4-triazolyl may be substituted by 1 or 2 methyl or ethyl substituents,
and
in which amino may be substituted by (C₁-C₄)-alkyl,
where amino may be substituted by (C₁-C₄)-alkyl,
where phenyl, pyridyl, pyrimidyl and 1,3-thiazolyl may each be substituted by 1 or 2 methyl or ethyl substituents,
and in which
R⁷ and R⁸ each independently of one another represent hydrogen, methyl, ethyl or cyclopropyl,
R⁴ represents hydrogen,
R⁵ represents hydrogen, chlorine, fluorine, methyl, ethyl, difluoromethyl or cyclopropyl,
R⁶ represents hydrogen,
and the *N*-oxides, salts, solvates, salts of the *N-*oxides and solvates of the *N*-oxides or salts thereof.

5. Process for preparing compounds of the formula (I) as defined in Claims 1 to 4, **characterized in that**
[A] a compound of the formula (II) 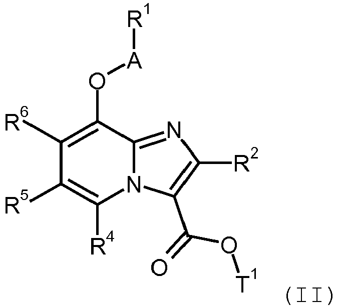
in which A, R¹, R², R⁴, R⁵ and R⁶ are each as defined above and
T¹ represents (C₁-C₄)-alkyl or benzyl,
is converted in an inert solvent in the presence of a suitable base or acid to a carboxylic acid of the formula (III) 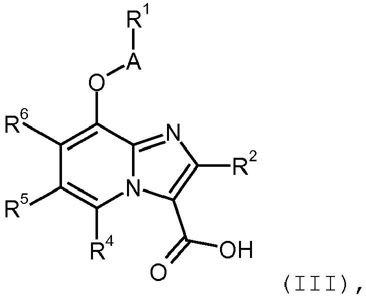 in which A, R¹, R², R⁴, R⁵ and R⁶ each have the meanings given above,
[A] and these are subsequently converted in the presence of a suitable acid into an imidazo[1,2-a]-pyridine of the formula (IV) 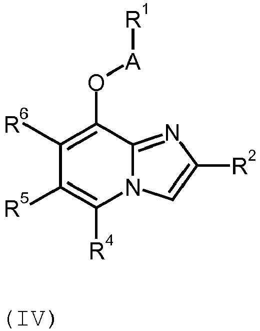 in which A, R¹, R², R⁴, R⁵ and R⁶ each have the meanings given above,
and this is then converted with a halogen equivalent into a compound of the formula (V) 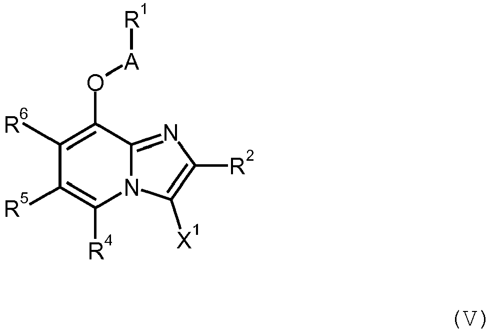 in which A, R¹, R², R⁴, R⁵ and R⁶ are each as defined above and
X¹ represents chlorine, bromine or iodine, and this is subsequently reacted in an inert solvent, in the presence of a suitable transition metal catalyst, with a compound of the formula (VI) 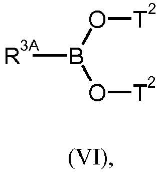 in which
R^{3A} has the meanings given above for R³ and
T² represents hydrogen or (C₁-C₄)-alkyl, or the two T² radicals together form a -C(CH₃)₂-C(CH₃)₂- bridge,
to give a compound of the formula (I-A) 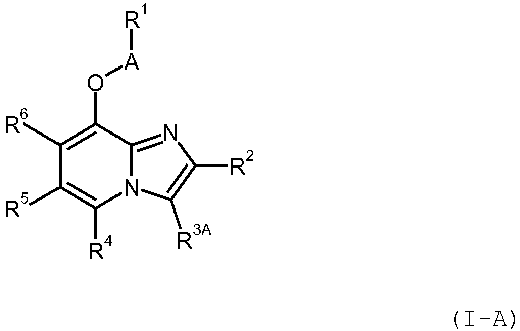 and these compounds are subsequently, if R^{3A} represents 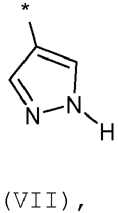 reacted in an inert solvent in the presence of a suitable base with a compound of the formula (VIII) 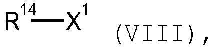 in which
X¹ represents a suitable leaving group, in particular chlorine, bromine, iodine, mesylate, triflate or tosylate,
and
R¹⁴ represents (C₁-C₆)-alkyl,
where (C₁-C₆)-alkyl may be substituted by 1 to 3 substituents selected from the group consisting of fluorine, cyano, trifluoromethyl, difluoromethyl, (C₁-C₄)-alkylcarbonyl, (C₁-C₄)-alkoxycarbonyl, hydroxycarbonyl, -(C=O)NR⁷R⁸, -O(C=O)NR⁷R⁸, (C₁-C₄) -alkylsulphonyl, (C₁-C₄) -alkoxy, trifluoromethoxy, difluoromethoxy, phenyl, 1H-pyrazolyl, 1H-1,2,4-triazolyl, 1H-tetrazolyl, 1,2-oxazolyl, tetrahydrothiophenyl 1,1-dioxide, hydroxy, amino, (C₃-C₇)-cycloalkyl, morpholinyl, piperidinyl, pyrrolidinyl, 2-oxopyrrolidin-1-yl, piperazinyl, tetrahydrothiophenyl 1,1-dioxide, thiomorpholinyl 1,1-dioxide and azetidine,
in which 1H-pyrazolyl, 1H-1,2,4-triazolyl, 1H-tetrazolyl and 1,2-oxazolyl may be substituted by 1 or 2 methyl or ethyl substituents,
in which piperidinyl may be substituted by 1 or 2 fluorine substituents,
in which phenyl may be substituted by 1 or 2 fluorine substituents,
in which piperazinyl may be substituted by methyl,
and in which
R⁷ and R⁸ each independently of one another represent hydrogen, methyl or cyclopropyl, or
R⁷ and R⁸ together with the carbon atom to which they are attached form a 3- to 5-membered carbocycle,
to give a compound of the formula (I-B) 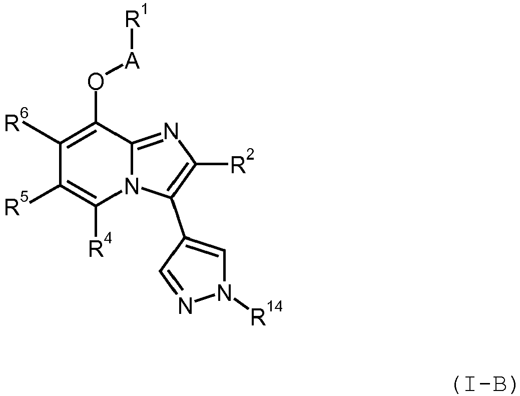 in which A, R¹, R², R⁴, R⁵, R⁶ and R¹⁴ each have the meanings given above and
any protecting groups present are subsequently removed, and the resulting compounds of the formula (I) are optionally converted with the appropriate (i) solvents and/or (ii) acids or bases into the solvates, salts and/or solvates of the salts thereof,
or
[B] a compound of the formula (II) is converted in the presence of hydrazine hydrate into a compound of the formula (IX) 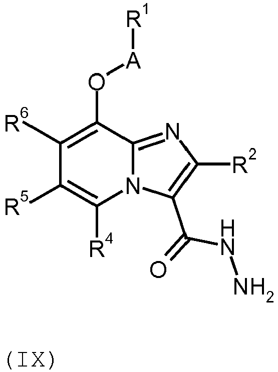 in which A, R¹, R², R⁴, R⁵ and R⁶ each have the meanings given above,
and these are subsequently reacted in an inert solvent under amide coupling conditions with a carboxylic acid of the formula (X) 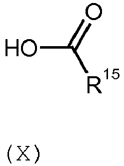 in which R¹⁵ represents (C₁-C₆)-alkyl,
where (C₁-C₆)-alkyl may be substituted by 1 to 3 substituents selected from the group consisting of trifluoromethyl, difluoromethyl, hydroxy and amino,
to give a compound of the formula (XI) 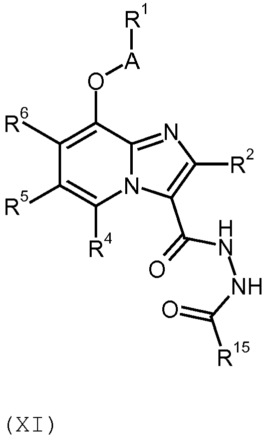 in which A, R¹, R², R⁴, R⁵, R⁶ and R¹⁵ each have the meanings given above,
and this compound is then converted with 2,4-bis(4-methoxyphenyl)-1,3,2,4-dithiadiphosphetane-2,4-disulphide [Lawesson's reagent] into a compound of the formula (I-C) 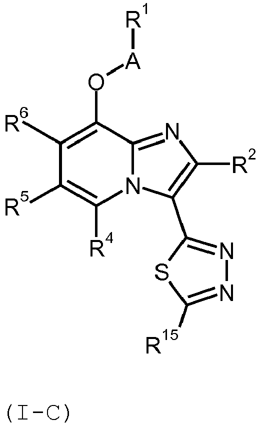 in which A, R¹, R², R⁴, R⁵, R⁶ and R¹⁵ each have the meanings given above,
then any protecting groups present are detached, and the resulting compounds of the formula (I) are optionally converted with the appropriate (i) solvents and/or (ii) acids or bases to the solvates, salts and/or solvates of the salts thereof.

6. Compound of the formula (I) as defined in any of Claims 1 to 4 for use in the treatment and/or prophylaxis of diseases.

7. Use of a compound of the formula (I) as defined in any of Claims 1 to 4 for production of a medicament for treatment and/or prophylaxis of heart failure, angina pectoris, hypertension, pulmonary hypertension, ischaemias, vascular disorders, renal insufficiency, thromboembolic disorders and arteriosclerosis.

8. Medicament comprising a compound of the formula (I) as defined in any of Claims 1 to 4 in combination with an inert, non-toxic, pharmaceutically suitable excipient.

9. Medicament comprising a compound of the formula (I) as defined in any of Claims 1 to 4 in combination with a further active ingredient selected from the group consisting of organic nitrates, NO donors, cGMP-PDE inhibitors, antithrombotic agents, hypotensive agents and lipid metabolism modifiers.

10. Medicament according to Claim 8 or 9 for use in the treatment and/or prophylaxis of heart failure, angina pectoris, hypertension, pulmonary hypertension, ischaemias, vascular disorders, kidney failure, thromboembolic disorders and arteriosclerosis.

## Revendications

1. Composé de formule (I) 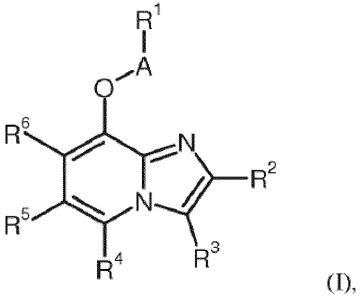 dans lequel
A représente CH₂, CD₂ ou CH(CH₃),
R¹ représente cycloalkyle en (C₃-C₇), phényle ou pyridyle,
le cycloalkyle en (C₃-C₇) pouvant être substitué avec 1 à 4 substituants choisis indépendamment les uns des autres dans le groupe constitué par fluor, trifluorométhyle et alkyle en (C₁-C₄),
le phényle étant substitué avec 1 à 4 substituants choisis indépendamment les uns des autres dans le groupe constitué par halogène, cyano, monofluorométhyle, difluorométhyle, trifluorométhyle, alkyle en (C₁-C₄), alcoxy en (C₁-C₄) et difluorométhoxy,
et
le pyridyle étant substitué avec 1 ou 2 substituants choisis indépendamment l'un de l'autre dans le groupe constitué par halogène, cyano et alkyle en (C₁-C₄),
R² représente alkyle en (C₁-C₄), cyclopropyle, monofluorométhyle, difluorométhyle ou trifluorométhyle,
R³ représente phényle ou hétéroaryle de 5 à 10 éléments,
le phényle pouvant être substitué avec 1 à 3 substituants choisis indépendamment les uns des autres dans le groupe constitué par halogène, cyano, trifluorométhyle, difluorométhyle, alkyle en (C₁-C₆), alkylcarbonyle en (C₁-C₄), alcoxycarbonyle en (C₁-C₄), hydroxycarbonyle, -(C=O)NR⁷R⁸, alkylsulfonyle en (C₁-C₄), cycloalkylsulfonyle en (C₃-C₆), alkylthio en (C₁-C₄), alcoxy en (C₁-C₄), trifluorométhoxy, difluorométhoxy, phénoxy, hydroxy et cycloalkyle en (C₃-C₇),
l'alkyle en (C₁-C₆) pouvant être substitué avec 1 ou 2 substituants choisis dans le groupe constitué par fluor, trifluorométhoxy, alkylcarbonyle en (C₁-C₄), -(C=O)NR⁷R⁸, alcoxy en (C₁-C₄), cycloalkyle en (C₃-C₆), morpholinyle, pipéridinyle, pyrrolidinyle, pipérazinyle, hydroxy et amino,
l'amino pouvant être substitué avec 1 ou 2 substituants choisis indépendamment l'un de l'autre parmi alkyle en (C₁-C₆), alkylcarbonyle en (C₁-C₄), cycloalkylsulfonyle en (C₃-C₆), alkylsulfonyle en (C₁-C₄) et méthoxy-alkyle en (C₁-C₄),
et
le cycloalkyle en (C₃-C₆) pouvant être substitué avec amino ou hydroxy,
R⁷ et R⁸ représentant chacun indépendamment l'un de l'autre hydrogène, alkyle en (C₁-C₄) ou cycloalkyle en (C₃-C₇),
l'hétéroaryle de 5 à 10 éléments pouvant être substitué avec 1 à 3 substituants choisis indépendamment les uns des autres dans le groupe constitué par fluor, chlore, cyano, alkyle en (C₁-C₆), alcoxy en (C₁-C₄), amino, alcoxycarbonyle en (C₁-C₄), hydroxycarbonyle, -(C=O)NR⁷R⁸, phényle, pyridyle, pyrimidyle, 1,3-thiazol-5-yle et cycloalkyle en (C₃-C₇),
l'alkyle en (C₁-C₆) pouvant être substitué avec 1 à 3 substituants choisis dans le groupe constitué par halogène, cyano, hydroxy, amino, trifluorométhyle, difluorométhyle, alkylsulfonyle en (C₁-C₄), alkylcarbonyle en (C₁-C₄), alcoxycarbonyle en (C₁-C₄), hydroxycarbonyle, -(C=O)NR⁷R⁸, -O(C=O)NR⁷R⁸, alkylthio en (C₁-C₄), alcoxy en (C₁-C₄), trifluorométhoxy, difluorométhoxy, phénoxy, phényle, pyridyle, pyrimidyle, hétéroaryle à 5 éléments, tétrahydrothiophényle-1,1-dioxyde, cycloalkyle en (C₃-C₇), morpholinyle, pipéridinyle, pyrrolidinyle, 2-oxopyrrolidin-1-yle, pipérazinyle, tétrahydrothiophényl-1,1-dioxyde, thiomorpholinyl-1,1-dioxyde et azétidine,
l'hétéroaryle à 5 éléments pouvant être substitué avec 1 à 3 substituants choisis dans le groupe constitué par halogène, alkyle en (C₁-C₄) et alcoxy en (C₁-C₄),
le pipéridinyle pouvant être substitué avec 1 à 4 substituants fluor,
le phényle pouvant être substitué avec 1 à 3 substituants choisis dans le groupe constitué par halogène, alkyle en (C₁-C₄) et alcoxy en (C₁-C₄),
l'azétidine pouvant être substitué avec hydroxy,
et
le pipérazinyle pouvant être substitué avec 1 à 3 substituants choisis indépendamment les uns des autres dans le groupe constitué par alkyle en (C₁-C₄), cycloalkyle en (C₃-C₇) et trifluorométhyle,
le cycloalkyle en (C₃-C₇) pouvant être substitué avec 1 ou 2 substituants choisis indépendamment l'un de l'autre dans le groupe constitué par halogène, alkyle en (C₁-C₆), alcoxycarbonyle en (C₁-C₄) et hydroxycarbonyle,
l'amino pouvant être substitué avec alkyle en (C₁-C₄), le phényle, le pyridyle, le pyrimidyle et le 1,3-thiazol-5-yle pouvant être substitués avec 1 ou 2 substituants choisis dans le groupe constitué par méthyle, éthyle et fluor,
R⁷ et R⁸ représentant chacun indépendamment l'un de l'autre hydrogène, alkyle en (C₁-C₄) ou cycloalkyle en (C₃-C₇),
à condition que lorsque l'hétéroaryle de 5 à 10 éléments représente pyridyle, le pyridyle ne puisse pas être substitué avec amino,
R⁴ représente hydrogène,
R⁵ représente hydrogène, halogène, cyano, alkyle en (C₁-C₄), alcoxy en (C₁-C₄), cycloalkyle en (C₃-C₅), difluorométhoxy, difluorométhyle, trifluorométhyle, hétérocyclyle de 4 à 7 éléments, ou hétéroaryle à 5 ou 6 éléments,
R⁶ représente hydrogène ou halogène,
ainsi que leurs N-oxydes, sels, solvates, sels des N-oxydes et solvates des N-oxydes et des sels.

2. Composé de formule (I) selon la revendication 1, dans lequel
A représente CH₂ ou CD₂,
R¹ représente cyclohexyle, phényle ou pyridyle,
le phényle étant substitué avec 1 à 4 substituants choisis indépendamment les uns des autres dans le groupe constitué par fluor, brome, chlore, cyano et méthyle,
et
le pyridyle étant substitué avec 1 ou 2 substituants choisis indépendamment l'un de l'autre dans le groupe constitué par fluor, cyano et méthyle,
R² représente alkyle en (C₁-C₄), cyclopropyle ou trifluorométhyle,
R³ représente phényle,
le phényle pouvant être substitué avec 1 à 3 substituants choisis dans le groupe constitué par halogène, cyano, trifluorométhyle, difluorométhyle, alkyle en (C₁-C₆), alkylcarbonyle en (C₁-C₄), alcoxycarbonyle en (C₁-C₄), hydroxycarbonyle,-(C=O)NR⁷R⁸, alkylsulfonyle en (C₁-C₄), alkylthio en (C₁-C₄), alcoxy en (C₁-C₄), trifluorométhoxy, difluorométhoxy, phénoxy, hydroxy et cycloalkyle en (C₃-C₇),
l'alkyle en (C₁-C₆) pouvant être substitué avec 1 ou 2 substituants choisis dans le groupe constitué par trifluorométhoxy, alkylcarbonyle en (C₁-C₄),-(C=O)NR⁷R⁸, alcoxy en (C₁-C₄), cycloalkyle en (C₃-C₆), morpholinyle, pipéridinyle, pyrrolidinyle, pipérazinyle, hydroxy et amino,
l'amino pouvant être substitué avec 1 ou 2 substituants choisis indépendamment l'un de l'autre parmi alkyle en (C₁-C₆), alkylcarbonyle en (C₁-C₄), alkylsulfonyle en (C₁-C₄) et méthoxy-alkyle en (C₁-C₄),
et
le cycloalkyle en (C₃-C₆) pouvant être substitué avec amino ou hydroxy,
R⁷ et R⁸ représentant chacun indépendamment l'un de l'autre hydrogène, alkyle en (C₁-C₄) ou cycloalkyle en (C₃-C₅),
ou
hétéroaryle à 5 éléments,
l'hétéroaryle à 5 éléments pouvant être substitué avec 1 ou 2 substituants choisis indépendamment l'un de l'autre dans le groupe constitué par chlore, cyano, alkyle en (C₁-C₆), alcoxy en (C₁-C₄), amino, alcoxycarbonyle en (C₁-C₄), hydroxycarbonyle,-(C=O)NR⁷R⁸, phényle, pyridyle, pyrimidyle, 1,3-thiazol-5-yle ou cycloalkyle en (C₃-C₆),
l'alkyle en (C₁-C₆) pouvant être substitué avec 1 à 3 substituants choisis dans le groupe constitué par fluor, cyano, trifluorométhyle, difluorométhyle, alkylcarbonyle en (C₁-C₄), alcoxycarbonyle en (C₁-C₄), hydroxycarbonyle, -(C=O)NR⁷R⁸, -O(C=O)NR⁷R⁸, alkylsulfonyle en (C₁-C₄), alcoxy en (C₁-C₄), trifluorométhoxy, difluorométhoxy, phénoxy, phényle, pyridyle, pyrimidyle, hétéroaryle à 5 éléments, tétrahydrothiophényle-1,1-dioxyde, hydroxy, amino, cycloalkyle en (C₃-C₇), morpholinyle, pipéridinyle, pyrrolidinyle, 2-oxopyrrolidin-1-yle, pipérazinyle, tétrahydrothiophényl-1,1-dioxyde, thiomorpholinyl-1,1-dioxyde et azétidine,
l'hétéroaryle à 5 éléments pouvant être substitué avec 1 ou 2 substituants méthyle ou éthyle,
le pipéridinyle pouvant être substitué avec 1 à 4 substituants fluor,
le phényle pouvant être substitué avec 1 ou 2 substituants choisis dans le groupe constitué par fluor, chlore et méthyle,
l'azétidine pouvant être substitué avec hydroxy,
R⁷ et R⁸ représentant chacun indépendamment l'un de l'autre hydrogène, alkyle en (C₁-C₄) ou cycloalkyle en (C₃-C₇),
et
le pipérazinyle pouvant être substitué avec 1 ou 2 substituants méthyle ou éthyle,
le phényle, le pyridyle, le pyrimidyle et le 1,3-thiazol-5-yle pouvant être substitués avec 1 ou 2 substituants choisis dans le groupe constitué par méthyle, éthyle et fluor,
le cycloalkyle en (C₃-C₆) pouvant être substitué avec 1 ou 2 substituants choisis indépendamment dans le groupe constitué par fluor, méthyle, éthyle, alcoxycarbonyle en (C₁-C₄) et hydroxycarbonyle,
et
R⁷ et R⁸ représentant chacun indépendamment l'un de l'autre hydrogène, alkyle en (C₁-C₄) ou cycloalkyle en (C₃-C₇),
R⁴ représente hydrogène,
R⁵ représente hydrogène, halogène, cyano, alkyle en (C₁-C₄), méthoxy, éthoxy, cycloalkyle en (C₃-C₅) ou difluorométhyle,
R⁶ représente hydrogène ou fluor,
ainsi que leurs N-oxydes, sels, solvates, sels des N-oxydes et solvates des N-oxydes et des sels.

3. Composé de formule (I) selon la revendication 1 ou 2, dans lequel
A représente CH₂,
R¹ représente cyclohexyle ou phényle,
le phényle étant substitué avec 1 à 3 substituants fluor,
ou
un groupe pyridyle de formule 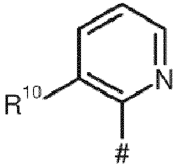 dans laquelle
# représente l'emplacement de liaison à A,
et
R¹⁰ représente fluor,
R² représente méthyle ou éthyle,
R³ représente phényle,
le phényle pouvant être substitué avec 1 ou 2 substituants choisis dans le groupe constitué par fluor, brome, chlore, cyano, trifluorométhyle, difluorométhyle, méthyle, éthyle, -(C=O)NR⁷R⁸, amino, hydroxycarbonyle, méthylsulfonyle, éthylsulfonyle, méthoxy, éthoxy, trifluorométhoxy, difluorométhoxy et cyclobutyle,
le méthyle et l'éthyle pouvant être substitués avec 1 ou 2 substituants choisis dans le groupe constitué par trifluorométhoxy, -(C=O)NR⁷R⁸, méthoxy, éthoxy, morpholinyle, pipéridinyle, pyrrolidinyle, pipérazinyle, hydroxy et amino,
l'amino pouvant être substitué avec 1 ou 2 substituants choisis indépendamment l'un de l'autre dans le groupe constitué par alkyle en (C₁-C₄), méthylcarbonyle, éthylcarbonyle, méthylsulfonyle, éthylsulfonyle et méthoxyéthyle,
le cyclobutyle pouvant être substitué avec amino ou hydroxy,
l'amino pouvant être substitué avec 1 ou 2 substituants choisis indépendamment l'un de l'autre parmi méthyle, éthyle, méthylcarbonyle, éthylcarbonyle, méthylsulfonyle ou éthylsulfonyle,
et
R⁷ et R⁸ représentant indépendamment l'un de l'autre hydrogène, méthyle, éthyle ou cyclopropyle,
ou
un groupe de formule 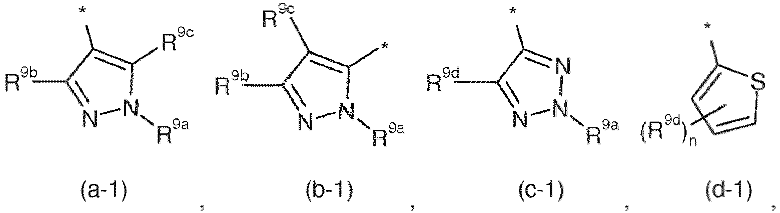 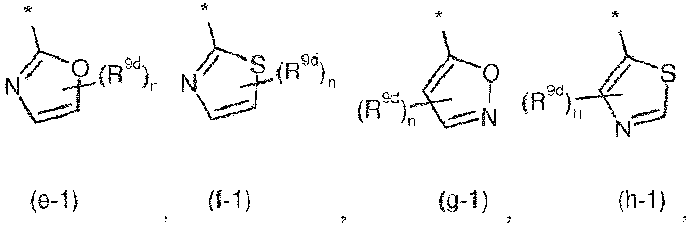 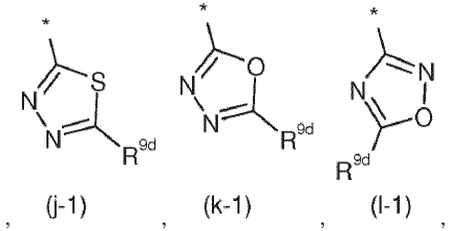 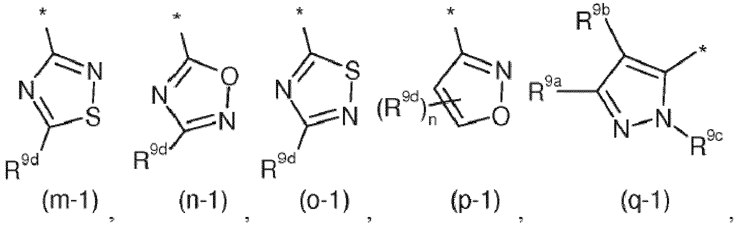 dans lesquelles
* représente l'emplacement de liaison à l'imidazopyridine,
n représente un nombre 1 ou 2,
R^{9a} représente alkyle en (C₁-C₆), phényle, pyridyle ou cyclopropyle,
l'alkyle en (C₁-C₆) pouvant être substitué avec 1 ou 2 substituants choisis dans le groupe constitué par fluor, cyano, trifluorométhyle, difluorométhyle, méthylcarbonyle, méthoxycarbonyle, éthoxycarbonyle, hydroxycarbonyle, -(C=O)NR⁷R⁸, -O(C=O)NR⁷R⁸, méthylsulfonyle, éthylsulfonyle, méthoxy, phényle, pyridyle, 1H-pyrazolyle, 1H-tétrazolyle, 1,2-oxazolyle, hydroxy, amino, cycloalkyle en (C₃-C₅), morpholinyle, pipéridinyle, pyrrolidinyle, pipérazinyle, thiomorpholinyl-1,1-dioxyde et azétidine,
le 1H-pyrazolyle, le 1H-tétrazolyle et le 1,2-oxazolyle pouvant être substitués avec 1 ou 2 substituants méthyle ou éthyle,
le pipéridinyle pouvant être substitué avec 1 à 2 substituants fluor,
l'azétidine pouvant être substituée avec hydroxy et
le pipérazinyle pouvant être substitué avec méthyle,
le cyclopropyle pouvant être substitué avec 1 ou 2 substituants choisis indépendamment dans le groupe constitué par méthyle, éthyle, méthoxycarbonyle, éthoxycarbonyle et hydroxycarbonyle,
le phényle et le pyridyle pouvant être substitués avec 1 ou 2 substituants fluor,
et
R⁷ et R⁸ représentant indépendamment l'un de l'autre hydrogène, méthyle, éthyle ou cyclopropyle,
R^{9b} représente hydrogène ou alkyle en (C₁-C₄),
R^{9c} représente hydrogène ou alkyle en (C₁-C₄),
R^{9d} représente hydrogène, alkyle en (C₁-C₆), trifluorométhyle, méthoxy, éthoxy, amino, méthoxycarbonyle, éthoxycarbonyle, hydroxycarbonyle, -(C=O)NR⁷R⁸, phényle, pyridyle, pyrimidyle, 1,3-thiazolyle, tétrahydrothiophényl-1,1-dioxyde ou cyclopropyle,
l'alkyle en (C₁-C₆) pouvant être substitué avec 1 ou 2 substituants choisis dans le groupe constitué par trifluorométhyle, difluorométhyle, alcoxy en (C₁-C₄), 2-oxopyrrolidin-1-yle, phényle, pyridyle, pyrimidyle, 1H-1,2,4-triazolyle, hydroxy et amino,
le 1H-1,2,4-triazolyle pouvant être substitué avec 1 ou 2 substituants méthyle ou éthyle,
et
l'amino pouvant être substitué avec alkyle en (C₁-C₄),
l'amino pouvant être substitué avec alkyle en (C₁-C₄), le phényle, le pyridyle, le pyrimidyle et le 1,3-thiazolyle pouvant chacun être substitués avec 1 ou 2 substituants méthyle ou éthyle,
et
R⁷ et R⁸ représentant chacun indépendamment l'un de l'autre hydrogène, méthyle, éthyle ou cyclopropyle,
R⁴ représente hydrogène,
R⁵ représente hydrogène, chlore, fluor, méthyle, éthyle, difluorométhyle ou cyclopropyle,
R⁶ représente hydrogène,
ainsi que leurs N-oxydes, sels, solvates, sels des N-oxydes et solvates des N-oxydes et des sels.

4. Composé de formule (I) selon la revendication 1, 2 ou 3, dans lequel
A représente CH₂,
R¹ représente cyclohexyle,
ou
un groupe phényle de formule 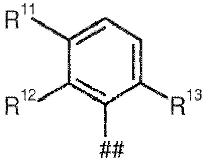 dans laquelle
## représente l'emplacement de liaison à A,
et
R¹¹, R¹² et R¹³ représentent indépendamment les uns des autres hydrogène ou fluor,
à condition qu'au moins deux des radicaux R¹¹, R¹², R¹³ soient différents de l'hydrogène,
ou
un groupe pyridyle de formule 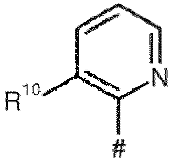 dans laquelle
# représente l'emplacement de liaison à A
et
R¹⁰ représente fluor,
R² représente méthyle ou éthyle,
R³ représente phényle,
le phényle pouvant être substitué avec 1 ou 2 substituants choisis dans le groupe constitué par fluor, chlore, cyano, amino, trifluorométhyle, difluorométhyle, méthyle, -(C=O)NR⁷R⁸, méthoxy, pipéridinyle et cyclobutyle,
le méthyle pouvant être substitué avec 1 ou 2 substituants choisis dans le groupe constitué par -(C=O)NR⁷R⁸, méthoxy, morpholinyle, pipéridinyle, pyrrolidinyle, pipérazinyle, hydroxy et amino,
l'amino pouvant être substitué avec 1 ou 2 substituants choisis indépendamment l'un de l'autre parmi méthyle, éthyle et méthoxyéthyle,
l'amino pouvant être substitué avec 1 ou 2 substituants choisis indépendamment l'un de l'autre parmi méthyle, éthyle, méthylcarbonyle, éthylcarbonyle, méthylsulfonyle ou éthylsulfonyle
le cyclobutyle étant substitué avec amino,
et
R⁷ et R⁸ représentant chacun indépendamment l'un de l'autre hydrogène, méthyle, éthyle ou cyclopropyle, ou
un groupe de formule 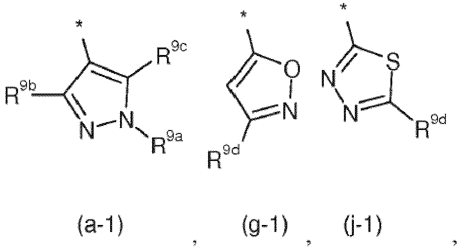 dans lesquelles
* représente l'emplacement de liaison à l'imidazopyridine,
R^{9a} représente alkyle en (C₁-C₆), phényle, pyridyle ou cyclopropyle,
l'alkyle en (C₁-C₆) pouvant être substitué avec fluor, cyano, trifluorométhyle, difluorométhyle, méthylcarbonyle, méthoxycarbonyle, éthoxycarbonyle, hydroxycarbonyle, -(C=O)NR⁷R⁸, -O(C=O)NR⁷R⁸, méthylsulfonyle, éthylsulfonyle, méthoxy, éthoxy, trifluorométhoxy, difluorométhoxy, phényle, pyridyle, 1H-pyrazolyle, 1H-tétrazolyle, 1,2-oxazolyle, hydroxy, amino, cyclopropyle, cyclobutyle, morpholinyle, pipéridinyle, pyrrolidinyle, pipérazinyle, thiomorpholinyl-1,1-dioxyde ou azétidine,
le 1H-pyrazolyle, le 1H-tétrazolyle et le 1,2-oxazolyle pouvant être substitués avec 1 ou 2 substituants méthyle ou éthyle,
le pipéridinyle pouvant être substitué avec 1 à 2 substituants fluor,
l'azétidine pouvant être substituée avec hydroxy et
le pipérazinyle pouvant être substitué avec méthyle,
le cyclopropyle pouvant être substitué avec méthoxycarbonyle, éthoxycarbonyle et hydroxycarbonyle,
le phényle et le pyridyle pouvant être substitués avec 1 ou 2 substituants fluor,
et
R⁷ et R⁸ représentant indépendamment l'un de l'autre hydrogène, méthyle, éthyle ou cyclopropyle,
R^{9b} représente hydrogène ou méthyle,
R^{9c} représente hydrogène ou méthyle,
R^{9d} représente hydrogène, alkyle en (C₁-C₆), trifluorométhyle, méthoxy, éthoxy, amino, méthoxycarbonyle, éthoxycarbonyle, hydroxycarbonyle, -(C=O)NR⁷R⁸, phényle, pyridyle, pyrimidyle, 1,3-thiazolyle, tétrahydrothiophényl-1,1-dioxyde ou cyclopropyle,
l'alkyle en (C₁-C₆) pouvant être substitué avec 1 ou 2 substituants choisis dans le groupe constitué par trifluorométhyle, difluorométhyle, alcoxy en (C₁-C₄), 2-oxopyrrolidin-1-yle, phényle, pyridyle, pyrimidyle, 1H-1,2,4-triazolyle, hydroxy et amino,
le 1H-1,2,4-triazolyle pouvant être substitué avec 1 ou 2 substituants méthyle ou éthyle,
et
l'amino pouvant être substitué avec alkyle en (C₁-C₄),
l'amino pouvant être substitué avec alkyle en (C₁-C₄), le phényle, le pyridyle, le pyrimidyle et le 1,3-thiazolyle pouvant chacun être substitués avec 1 ou 2 substituants méthyle ou éthyle,
et
R⁷ et R⁸ représentant chacun indépendamment l'un de l'autre hydrogène, méthyle, éthyle ou cyclopropyle,
R⁴ représente hydrogène,
R⁵ représente hydrogène, chlore, fluor, méthyle, éthyle, difluorométhyle ou cyclopropyle,
R⁶ représente hydrogène,
ainsi que leurs N-oxydes, sels, solvates, sels des N-oxydes et solvates des N-oxydes et des sels.

5. Procédé de fabrication de composés de formule (I), tels que définis dans les revendications 1 à 4, **caractérisé en ce que**
[A] un composé de formule (II) 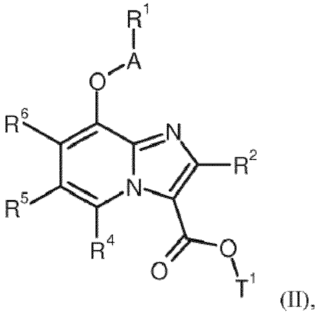
dans laquelle A, R¹, R², R⁴, R⁵ et R⁶ ont chacun les significations indiquées précédemment, et
T¹ représente alkyle en (C₁-C₄) ou benzyle,
est transformé dans un solvant inerte en présence d'une base ou d'un acide approprié en un acide carboxylique de formule (III) 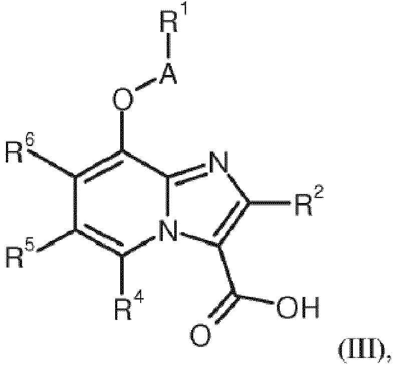 dans laquelle A, R¹, R², R⁴, R⁵ et R⁶ ont chacun les significations indiquées précédemment,
[A] puis celui-ci est mis en réaction en présence d'un acide approprié pour former une imidazo[1,2-a]-pyridine de formule (IV) 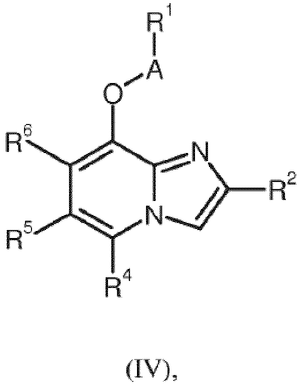 dans laquelle A, R¹, R², R⁴, R⁵ et R⁶ ont chacun les significations indiquées précédemment,
puis celui-ci est transformé avec un équivalent d'halogène en un composé de formule (V) 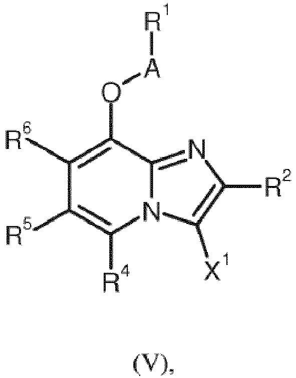
dans laquelle A, R¹, R², R⁴, R⁵ et R⁶ ont chacun les significations indiquées précédemment, et
X¹ représente chlore, brome ou iode,
puis celui-ci est mis en réaction dans un solvant inerte en présence d'un catalyseur de métal de transition approprié avec un composé de formule (VI) 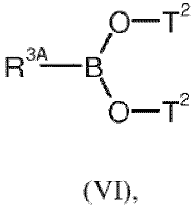 dans laquelle
R^{3A} a les significations indiquées précédemment pour R³, et
T² représente hydrogène ou alkyle en (C₁-C₄), ou les deux radicaux T² forment ensemble un pont -C(CH₃)₂-C(CH₃)₂,
pour former un composé de formule (I-A) 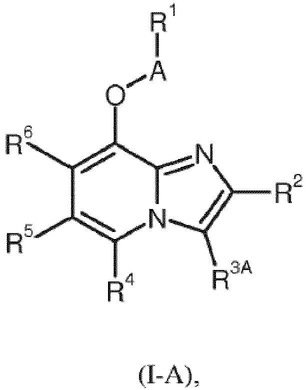 puis, lorsque R^{3A} représente 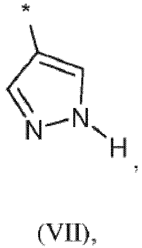 ces composés sont mis en réaction dans un solvant inerte en présence d'une base appropriée avec un composé de formule (VIII)
R¹⁴-X¹ (VIII),
dans laquelle
X¹ représente un groupe partant approprié, notamment chlore, brome, iode, mésylate, triflate ou tosylate,
et
R¹⁴ représente alkyle en (C₁-C₆),
l'alkyle en (C₁-C₆) pouvant être substitué avec 1 à 3 substituants choisis dans le groupe constitué par fluor, cyano, trifluorométhyle, difluorométhyle, alkylcarbonyle en (C₁-C₄), alcoxycarbonyle en (C₁-C₄), hydroxycarbonyle, -(C=O)NR⁷R⁸, -O(C=O)NR⁷R⁸, alkylsulfonyle en (C₁-C₄), alcoxy en (C₁-C₄), trifluorométhoxy, difluorométhoxy, phényle, 1H-pyrazolyle, 1H-1,2,4-triazolyle, 1H-tétrazolyle, 1,2-oxazolyle, tétrahydrothiophényl-1,1-dioxyde, hydroxy, amino, cycloalkyle en (C₃-C₇), morpholinyle, pipéridinyle, pyrrolidinyle, 2-oxopyrrolidin-1-yle, pipérazinyle, tétrahydrothiophényl-1,1-dioxyde, thiomorpholinyl-1,1-dioxyde et azétidine,
le 1H-pyrazolyle, le 1H-1,2,4-triazolyle, le 1H-tétrazolyle et le 1,2-oxazolyle pouvant être substitués avec 1 ou 2 substituants méthyle ou éthyle,
le pipéridinyle pouvant être substitué avec 1 ou 2 substituants fluor,
le phényle pouvant être substitué avec 1 ou 2 substituants fluor,
le pipérazinyle pouvant être substitué avec méthyle,
et
R⁷ et R⁸ représentant chacun indépendamment l'un de l'autre hydrogène, méthyle ou cyclopropyle,
ou
R⁷ et R⁸ formant ensemble avec l'atome de carbone auquel ils sont reliés un carbocycle de 3 à 5 éléments, pour former un composé de formule (I-B) 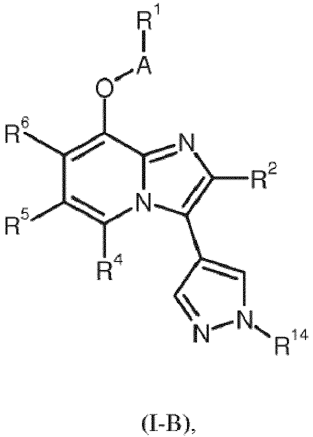 dans laquelle A, R¹, R², R⁴, R⁵, R⁶ et R¹⁴ ont chacun les significations indiquées précédemment, puis
les groupes protecteurs éventuellement présents sont clivés, et les composés de formule (I) résultants sont éventuellement transformés avec (i) les solvants et/ou (ii) les acides ou les bases appropriés en leurs solvates, sels et/ou solvates des sels,
ou
[B] un composé de formule (II) est transformé en présence d'hydrate d'hydrazine en un composé de formule (IX) 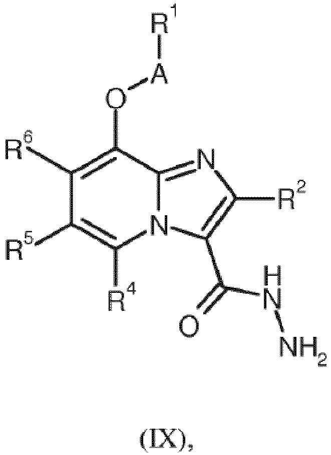 dans laquelle A, R¹, R², R⁴, R⁵ et R⁶ ont chacun les significations indiquées précédemment,
puis celui-ci est mis en réaction dans un solvant inerte dans des conditions de couplage d'amide avec un acide carboxylique de formule (X) 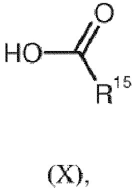 dans laquelle R¹⁵ représente alkyle en (C₁-C₆),
l'alkyle en (C₁-C₆) pouvant être substitué avec 1 à 3 substituants choisis dans le groupe constitué par trifluorométhyle, difluorométhyle, hydroxy et amino, pour former un composé de formule (XI) 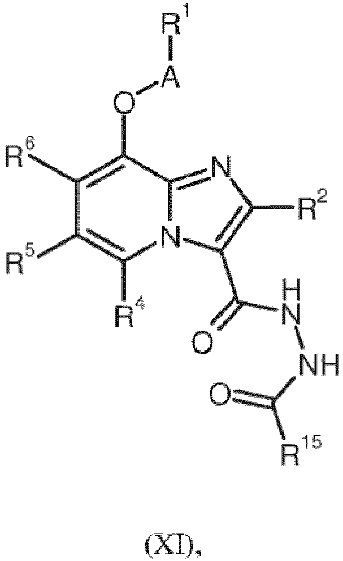 dans laquelle A, R¹, R², R⁴, R⁵, R⁶ et R¹⁵ ont chacun les significations indiquées précédemment, puis ce composé est mis en réaction avec du 2,4-disulfure de 2,4-bis(4-méthoxyphényl)-1,3,2,4-dithiadiphosphétane [réactif de Lawesson] pour former un composé de formule (I-C) 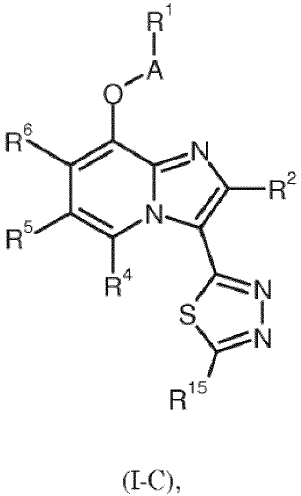 dans laquelle A, R¹, R², R⁴, R⁵, R⁶ et R¹⁵ ont chacun les significations indiquées précédemment,
puis les groupes protecteurs éventuellement présents sont clivés, et les composés de formule (I) résultants sont éventuellement transformés avec (i) les solvants et/ou (ii) les acides ou les bases appropriés en leurs solvates, sels et/ou solvates des sels.

6. Composé de formule (I), tel que défini dans l'une quelconque des revendications 1 à 4, destiné à une utilisation pour le traitement et/ou la prophylaxie de maladies.

7. Utilisation d'un composé de formule (I), tel que défini dans l'une quelconque des revendications 1 à 4, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques et l'artériosclérose.

8. Médicament contenant un composé de formule (I), tel que défini dans l'une quelconque des revendications 1 à 4, en combinaison avec un adjuvant inerte, non toxique, pharmaceutiquement approprié.

9. Médicament contenant un composé de formule (I), tel que défini dans l'une quelconque des revendications 1 à 4, en combinaison avec un autre agent actif choisi dans le groupe constitué par les nitrates organiques, les donneurs de NO, les inhibiteurs de cGMP-PDE, les agents à effet antithrombotique, les agents abaissant la tension artérielle, ainsi que les agents modifiant le métabolisme des lipides.

10. Médicament selon la revendication 8 ou 9, destiné à une utilisation pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques et l'artériosclérose.
